# FASCICULE DE BREVET EUROPEEN

(11) **EP 3 010 533 B1**
(45) Date de publication et mention de la délivrance du brevet: **26.09.2018**
(21) Numéro de dépôt: 14750245.4
(22) Date de dépôt: 20.06.2014
(51) Int. Cl.: A61K 38/48, C12N 9/64

(54) **FACTEUR X DEPOURVU DE DOMAINE GLA**
GLA-DOMÄNENLOSER FAKTOR X
GLA-DOMAINLESS FACTOR X

(30) Priorité: 21.06.2013 FR 1355896
(43) Date de publication de la demande: 27.04.2016
(73) Titulaire: Laboratoire Français du Fractionnement et des Biotechnologies, 91940 Les Ulis (FR); Centre National de la Recherche Scientifique - CNRS, 75794 Paris Cedex 16 (FR); Université Grenoble I Joseph Fourier, 38400 Saint Martin D'heres (FR)
(72) Inventeur: THOMAS, Aline, F-38240 Meylan (FR); DAGHER, Marie-Claire, F-38600 Fontaine (FR); MARLU, Raphaël, F-38700 La Tronche (FR); POLACK, Benoit, F-38950 Saint Martin Le Vinoux (FR); PLANTIER, Jean-Luc, F-59170 Croix (FR); FONTAYNE, Alexandre, F-59110 La Madeleine (FR); ABACHE, Toufik, F-59211 Santes (FR)
(74) Mandataire: Ipside
(86) Numéro de dépôt international: PCT/FR2014/051542
(87) Numéro de publication internationale: WO 2014/202916

(56) Documents cités:
- WO-A1-92/04378
- WO-A2-2009/042962
- FR-A1- 2 972 114
- US-A1- 2005 260 194

### SEQUENCE LISTING

<110> LFB et al
<120> FACTEUR X DEPOURVU DE DOMAINE GLA
<130> BFF130101
<160> 93
<170> PatentIn version 3.5
<210> 1
   <211> 306
   <212> PRT
   <213> Homo sapiens
<400> 1
   Ser Val Ala Gln Ala Thr Ser Ser Ser Gly Glu Ala Pro Asp Ser Ile
   1               5                   10                  15
   Thr Trp Lys Pro Tyr Asp Ala Ala Asp Leu Asp Pro Thr Glu Asn Pro
               20                  25                  30
   Phe Asp Leu Leu Asp Phe Asn Gln Thr Gln Pro Glu Arg Gly Asp Asn
           35                  40                  45
   Asn Leu Thr Arg Ile Val Gly Gly Gln Glu Cys Lys Asp Gly Glu Cys
       50                  55                  60
   Pro Trp Gln Ala Leu Leu Ile Asn Glu Glu Asn Glu Gly Phe Cys Gly
   65                  70                  75                  80
   Gly Thr Ile Leu Ser Glu Phe Tyr Ile Leu Thr Ala Ala His Cys Leu
                   85                  90                  95
   Tyr Gln Ala Lys Arg Phe Lys Val Arg Val Gly Asp Arg Asn Thr Glu
               100                 105                 110
   Gln Glu Glu Gly Gly Glu Ala Val His Glu Val Glu Val Val Ile Lys
           115                 120                 125
   His Asn Arg Phe Thr Lys Glu Thr Tyr Asp Phe Asp Ile Ala Val Leu
       130                 135                 140
   Arg Leu Lys Thr Pro Ile Thr Phe Arg Met Asn Val Ala Pro Ala Cys
   145                 150                 155                 160
   Leu Pro Glu Arg Asp Trp Ala Glu Ser Thr Leu Met Thr Gln Lys Thr
                   165                 170                 175
   Gly Ile Val Ser Gly Phe Gly Arg Thr His Glu Lys Gly Arg Gln Ser
               180                 185                 190
   Thr Arg Leu Lys Met Leu Glu Val Pro Tyr Val Asp Arg Asn Ser Cys
           195                 200                 205
   Lys Leu Ser Ser Ser Phe Ile Ile Thr Gln Asn Met Phe Cys Ala Gly
       210                 215                 220
   Tyr Asp Thr Lys Gln Glu Asp Ala Cys Gln Gly Asp Ser Gly Gly Pro
   225                 230                 235                 240
   His Val Thr Arg Phe Lys Asp Thr Tyr Phe Val Thr Gly Ile Val Ser
                   245                 250                 255
   Trp Gly Glu Gly Cys Ala Arg Lys Gly Lys Tyr Gly Ile Tyr Thr Lys
               260                 265                 270
   Val Thr Ala Phe Leu Lys Trp Ile Asp Arg Ser Met Lys Thr Arg Gly
           275                 280                 285
   Leu Pro Lys Ala Lys Ser His Ala Pro Glu Val Ile Thr Ser Ser Pro
       290                 295                 300
   Leu Lys
   305
<210> 2
   <211> 182
   <212> PRT
   <213> Homo sapiens
<400> 2
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Thr Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg
               180
<210> 3
   <211> 52
   <212> PRT
   <213> Homo sapiens
<400> 3
   Ser Val Ala Gln Ala Thr Ser Ser Ser Gly Glu Ala Pro Asp Ser Ile
   1               5                   10                  15
   Thr Trp Lys Pro Tyr Asp Ala Ala Asp Leu Asp Pro Thr Glu Asn Pro
               20                  25                  30
   Phe Asp Leu Leu Asp Phe Asn Gln Thr Gln Pro Glu Arg Gly Asp Asn
           35                  40                  45
   Asn Leu Thr Arg
       50
<210> 4
   <211> 488
   <212> PRT
   <213> Homo sapiens
<400> 4
<210> 5
   <211> 142
   <212> PRT
   <213> Homo sapiens
<400> 5
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg
       130                 135                 140
<210> 6
   <211> 254
   <212> PRT
   <213> Homo sapiens
<400> 6
<210> 7
   <211> 357
   <212> PRT
   <213> Homo sapiens
<400> 7
   Lys Tyr Lys Asp Gly Asp Gln Cys Glu Thr Ser Pro Cys Gln Asn Gln
   1 5 10 15
   Gly Lys Cys Lys Asp Gly Leu Gly Glu Tyr Thr Cys Thr Cys Leu Glu
   20 25 30
   Gly Phe Glu Gly Lys Asn Cys Glu Leu Phe Thr Arg Lys Leu Cys Ser
   35 40 45
   Leu Asp Asn Gly Asp Cys Asp Gln Phe Cys His Glu Glu Gln Asn Ser
       50                  55                  60
   Val Val Cys Ser Cys Ala Arg Gly Tyr Thr Leu Ala Asp Asn Gly Lys
   65                  70                  75                  80
   Ala Cys Ile Pro Thr Gly Pro Tyr Pro Cys Gly Lys Gln Thr Leu Glu
                   85                  90                  95
   Arg Arg Lys Arg Arg Lys Arg Ile Val Gly Gly Gln Glu Cys Lys Asp
               100                 105                 110
   Gly Glu Cys Pro Trp Gln Ala Leu Leu Ile Asn Glu Glu Asn Glu Gly
           115                 120                 125
   Phe Cys Gly Gly Thr Ile Leu Ser Glu Phe Tyr Ile Leu Thr Ala Ala
       130                 135                 140
   His Cys Leu Tyr Gln Ala Lys Arg Phe Lys Val Arg Val Gly Asp Arg
   145                 150                 155                 160
   Asn Thr Glu Gln Glu Glu Gly Gly Glu Ala Val His Glu Val Glu Val
                   165                 170                 175
   Val Ile Lys His Asn Arg Phe Thr Lys Glu Thr Tyr Asp Phe Asp Ile
               180                 185                 190
   Ala Val Leu Arg Leu Lys Thr Pro Ile Thr Phe Arg Met Asn Val Ala
           195                 200                 205
   Pro Ala Cys Leu Pro Glu Arg Asp Trp Ala Glu Ser Thr Leu Met Thr
       210                 215                 220
   Gln Lys Thr Gly Ile Val Ser Gly Phe Gly Arg Thr His Glu Lys Gly
   225                 230                 235                 240
   Arg Gln Ser Thr Arg Leu Lys Met Leu Glu Val Pro Tyr Val Asp Arg
                   245                 250                 255
   Asn Ser Cys Lys Leu Ser Ser Ser Phe Ile Ile Thr Gln Asn Met Phe
               260                 265                 270
   Cys Ala Gly Tyr Asp Thr Lys Gln Glu Asp Ala Cys Gln Gly Asp Ser
           275                 280                 285
   Gly Gly Pro His Val Thr Arg Phe Lys Asp Thr Tyr Phe Val Thr Gly
       290                 295                 300
   Ile Val Ser Trp Gly Glu Gly Cys Ala Arg Lys Gly Lys Tyr Gly Ile
   305                 310                 315                 320
   Tyr Thr Lys Val Thr Ala Phe Leu Lys Trp Ile Asp Arg Ser Met Lys
                   325                 330                 335
   Thr Arg Gly Leu Pro Lys Ala Lys Ser His Ala Pro Glu Val Ile Thr
               340                 345                 350
   Ser Ser Pro Leu Lys
           355
<210> 8
   <211> 1071
   <212> DNA
   <213> Homo sapiens
<400> 8
   aaatacaaag atggcgacca gtgtgagacc agtccttgcc agaaccaggg caaatgtaaa      60
   gacggcctcg gggaatacac ctgcacctgt ttagaaggat tcgaaggcaa aaactgtgaa     120
   ttattcacac ggaagctctg cagcctggac aacggggact gtgaccagtt ctgccacgag     180
   gaacagaact ctgtggtgtg ctcctgcgcc cgcgggtaca ccctggctga caacggcaag     240
   gcctgcattc ccacagggcc ctacccctgt gggaaacaga ccctggaacg caggaagagg     300
   aggaagagga tcgtgggagg ccaggaatgc aaggacgggg agtgtccctg gcaggccctg     360
   ctcatcaatg aggaaaacga gggtttctgt ggtggaacca ttctgagcga gttctacatc     420
   ctaacggcag cccactgtct ctaccaagcc aagagattca aggtgagggt aggggaccgg     480
   aacacggagc aggaggaggg cggtgaggcg gtgcacgagg tggaggtggt catcaagcac     540
   aaccggttca caaaggagac ctatgacttc gacatcgccg tgctccggct caaggaagac     600
   caggtggacc caagactgat tgacggaaaa tgagaattcg gatcccccga cctcgagtcc     660
   acgctgatga cgcagaagac ggggattgtg agcggcttcg ggcgcaccca cgagaagggc     720
   cggcagtcca ccaggctcaa gatgctggag gtgccctacg tggaccgcaa cagctgcaag     780
   ctgtccagca gcttcatcat cacccagaac atgttctgtg ccggctacga caccaagcag     840
   gaggatgcct gccaggggga cagcgggggc ccgcacgtca cccgcttcaa ggacacctac     900
   ttcgtgacag gcatcgtcag ctggggagag ggctgtgccc gtaaggggaa gtacgggatc     960
   tacaccaagg tcaccgcctt cctcaagtgg atcgacaggt ccatgaaaac caggggcttg    1020
   cccaaggcca agagccatgc cccggaggtc ataacgtcct ctccattaaa g            1071
<210> 9
   <211> 354
   <212> PRT
   <213> Homo sapiens
<400> 9
   Lys Tyr Lys Asp Gly Asp Gln Cys Glu Thr Ser Pro Cys Gln Asn Gln
   1               5                   10                  15
   Gly Lys Cys Lys Asp Gly Leu Gly Glu Tyr Thr Cys Thr Cys Leu Glu
               20                  25                  30
   Gly Phe Glu Gly Lys Asn Cys Glu Leu Phe Thr Arg Lys Leu Cys Ser
           35                  40                  45
   Leu Asp Asn Gly Asp Cys Asp Gln Phe Cys His Glu Glu Gln Asn Ser
       50                  55                  60
   Val Val Cys Ser Cys Ala Arg Gly Tyr Thr Leu Ala Asp Asn Gly Lys
   65                  70                  75                  80
   Ala Cys Ile Pro Thr Gly Pro Tyr Pro Cys Gly Lys Gln Thr Leu Glu
                   85                  90                  95
   Arg Arg Lys Arg Ile Val Gly Gly Gln Glu Cys Lys Asp Gly Glu Cys
               100                 105                 110
   Pro Trp Gln Ala Leu Leu Ile Asn Glu Glu Asn Glu Gly Phe Cys Gly
           115                 120                 125
   Gly Thr Ile Leu Ser Glu Phe Tyr Ile Leu Thr Ala Ala His Cys Leu
       130                 135                 140
   Tyr Gln Ala Lys Arg Phe Lys Val Arg Val Gly Asp Arg Asn Thr Glu
   145                 150                 155                 160
   Gln Glu Glu Gly Gly Glu Ala Val His Glu Val Glu Val Val Ile Lys
                   165                 170                 175
   His Asn Arg Phe Thr Lys Glu Thr Tyr Asp Phe Asp Ile Ala Val Leu
               180                 185                 190
   Arg Leu Lys Thr Pro Ile Thr Phe Arg Met Asn Val Ala Pro Ala Cys
           195                 200                 205
   Leu Pro Glu Arg Asp Trp Ala Glu Ser Thr Leu Met Thr Gln Lys Thr
       210                 215                 220
   Gly Ile Val Ser Gly Phe Gly Arg Thr His Glu Lys Gly Arg Gln Ser
   225                 230                 235                 240
   Thr Arg Leu Lys Met Leu Glu Val Pro Tyr Val Asp Arg Asn Ser Cys
                   245                 250                 255
   Lys Leu Ser Ser Ser Phe Ile Ile Thr Gln Asn Met Phe Cys Ala Gly
               260                 265                 270
   Tyr Asp Thr Lys Gln Glu Asp Ala Cys Gln Gly Asp Ser Gly Gly Pro
           275                 280                 285
   His Val Thr Arg Phe Lys Asp Thr Tyr Phe Val Thr Gly Ile Val Ser
       290                 295                 300
   Trp Gly Glu Gly Cys Ala Arg Lys Gly Lys Tyr Gly Ile Tyr Thr Lys
   305                 310                 315                 320
   Val Thr Ala Phe Leu Lys Trp Ile Asp Arg Ser Met Lys Thr Arg Gly
                   325                 330                 335
   Leu Pro Lys Ala Lys Ser His Ala Pro Glu Val Ile Thr Ser Ser Pro
               340                 345                 350
   Leu Lys
<210> 10
   <211> 1062
   <212> DNA
   <213> Homo sapiens
<400> 10
   aaatacaaag atggcgacca gtgtgagacc agtccttgcc agaaccaggg caaatgtaaa      60
   gacggcctcg gggaatacac ctgcacctgt ttagaaggat tcgaaggcaa aaactgtgaa     120
   ttattcacac ggaagctctg cagcctggac aacggggact gtgaccagtt ctgccacgag     180
   gaacagaact ctgtggtgtg ctcctgcgcc cgcgggtaca ccctggctga caacggcaag     240
   gcctgcattc ccacagggcc ctacccctgt gggaaacaga ccctggaacg caggaagagg     300
   atcgtgggag gccaggaatg caaggacggg gagtgtccct ggcaggccct gctcatcaat     360
   gaggaaaacg agggtttctg tggtggaacc attctgagcg agttctacat cctaacggca     420
   gcccactgtc tctaccaagc caagagattc aaggtgaggg taggggaccg gaacacggag     480
   caggaggagg gcggtgaggc ggtgcacgag gtggaggtgg tcatcaagca caaccggttc     540
   acaaaggaga cctatgactt cgacatcgcc gtgctccggc tcaagacccc catcaccttc     600
   cgcatgaacg tggcgcctgc ctgcctcccc gagcgtgact gggccgagtc cacgctgatg     660
   acgcagaaga cggggattgt gagcggcttc gggcgcaccc acgagaaggg ccggcagtcc     720
   accaggctca agatgctgga ggtgccctac gtggaccgca acagctgcaa gctgtccagc      780
   agcttcatca tcacccagaa catgttctgt gccggctacg acaccaagca ggaggatgcc      840
   tgccaggggg acagcggggg cccgcacgtc acccgcttca aggacaccta cttcgtgaca      900
   ggcatcgtca gctggggaga gggctgtgcc cgtaagggga agtacgggat ctacaccaag      960
   gtcaccgcct tcctcaagtg gatcgacagg tccatgaaaa ccaggggctt gcccaaggcc     1020
   aagagccatg ccccggaggt cataacgtcc tctccattaa ag                        1062
<210> 11
   <211> 100
   <212> PRT
   <213> Homo sapiens
<400> 11
   Lys Tyr Lys Asp Gly Asp Gln Cys Glu Thr Ser Pro Cys Gln Asn Gln
   1               5                   10                  15
   Gly Lys Cys Lys Asp Gly Leu Gly Glu Tyr Thr Cys Thr Cys Leu Glu
               20                  25                  30
   Gly Phe Glu Gly Lys Asn Cys Glu Leu Phe Thr Arg Lys Leu Cys Ser
           35                  40                  45
   Leu Asp Asn Gly Asp Cys Asp Gln Phe Cys His Glu Glu Gln Asn Ser
       50                  55                  60
   Val Val Cys Ser Cys Ala Arg Gly Tyr Thr Leu Ala Asp Asn Gly Lys
   65                  70                  75                  80
   Ala Cys Ile Pro Thr Gly Pro Tyr Pro Cys Gly Lys Gln Thr Leu Glu
                   85                  90                  95
   Arg Arg Lys Arg
               100
<210> 12
   <211> 357
   <212> PRT
   <213> Homo sapiens
<400> 12
   Lys Tyr Lys Asp Gly Asp Gln Cys Glu Thr Ser Pro Cys Gln Asn Gln
   1               5                   10                  15
   Gly Lys Cys Lys Asp Gly Leu Gly Glu Tyr Thr Cys Thr Cys Leu Glu
               20                  25                  30
   Gly Phe Glu Gly Lys Asn Cys Glu Leu Phe Thr Arg Lys Leu Cys Ser
           35                  40                  45
   Leu Asp Asn Gly Asp Cys Asp Gln Phe Cys His Glu Glu Gln Asn Ser
       50                  55                  60
   Val Val Cys Ser Cys Ala Arg Gly Tyr Thr Leu Ala Asp Asn Gly Lys
   65                  70                  75                  80
   Ala Cys Ile Pro Thr Gly Pro Tyr Pro Cys Gly Lys Gln Thr Leu Glu
                   85                  90                  95
   Arg Arg Lys Arg Arg Lys Arg Ile Val Gly Gly Gln Glu Cys Lys Asp
               100                 105                 110
   Gly Glu Cys Pro Trp Gln Ala Leu Leu Ile Asn Glu Glu Asn Glu Gly
           115                 120                 125
   Phe Cys Gly Gly Thr Ile Leu Ser Glu Phe Tyr Ile Leu Thr Ala Ala
       130                 135                 140
   His Cys Leu Tyr Gln Ala Lys Arg Phe Lys Val Arg Val Gly Asp Arg
   145                 150                 155                 160
   Asn Thr Glu Gln Glu Glu Gly Gly Glu Ala Val His Glu Val Glu Val
                   165                 170                 175
   Val Ile Lys His Asn Arg Phe Thr Tyr Glu Thr Tyr Asp Phe Asp Ile
               180                 185                 190
   Ala Val Leu Arg Leu Lys Thr Pro Ile Thr Phe Arg Met Asn Val Ala
           195                 200                 205
   Pro Ala Cys Leu Pro Glu Arg Asp Trp Ala Glu Ser Thr Leu Met Thr
       210                 215                 220
   Gln Lys Thr Gly Ile Val Ser Gly Phe Gly Arg Thr His Glu Lys Gly
   225                 230                 235                 240
   Arg Gln Ser Thr Arg Leu Lys Met Leu Glu Val Pro Tyr Val Asp Arg
                   245                 250                 255
   Asn Ser Cys Lys Leu Ser Ser Ser Phe Ile Ile Thr Gln Asn Met Phe
               260                 265                 270
   Cys Ala Gly Tyr Asp Thr Lys Gln Glu Asp Ala Cys Gln Gly Asp Ser
           275                 280                 285
   Gly Gly Pro His Val Thr Arg Phe Lys Asp Thr Tyr Phe Val Thr Gly
       290                 295                 300
   Ile Val Ser Trp Gly Glu Gly Cys Ala Arg Lys Gly Lys Tyr Gly Ile
   305                 310                 315                 320
   Tyr Thr Lys Val Thr Ala Phe Leu Lys Trp Ile Asp Arg Ser Met Lys
                   325                 330                 335
   Thr Arg Gly Leu Pro Lys Ala Lys Ser His Ala Pro Glu Val Ile Thr
               340                 345                 350
   Ser Ser Pro Leu Lys
           355
<210> 13
   <211> 357
   <212> PRT
   <213> Homo sapiens
<400> 13
   Lys Tyr Lys Asp Gly Asp Gln Cys Glu Thr Ser Pro Cys Gln Asn Gln
   1               5                   10                  15
   Gly Lys Cys Lys Asp Gly Leu Gly Glu Tyr Thr Cys Thr Cys Leu Glu
               20                  25                  30
   Gly Phe Glu Gly Lys Asn Cys Glu Leu Phe Thr Arg Lys Leu Cys Ser
           35                  40                  45
   Leu Asp Asn Gly Asp Cys Asp Gln Phe Cys His Glu Glu Gln Asn Ser
       50                  55                  60
   Val Val Cys Ser Cys Ala Arg Gly Tyr Thr Leu Ala Asp Asn Gly Lys
   65                  70                  75                  80
   Ala Cys Ile Pro Thr Gly Pro Tyr Pro Cys Gly Lys Gln Thr Leu Glu
                   85                  90                  95
   Arg Arg Lys Arg Arg Lys Arg Ile Val Gly Gly Gln Glu Cys Lys Asp
               100                 105                 110
   Gly Glu Cys Pro Trp Gln Ala Leu Leu Ile Asn Glu Glu Asn Glu Gly
           115                 120                 125
   Phe Cys Gly Gly Thr Ile Leu Ser Glu Phe Tyr Ile Leu Thr Ala Ala
       130                 135                 140
   His Cys Leu Tyr Gln Ala Lys Arg Phe Lys Val Arg Val Gly Asp Arg
   145 150 155 160
   Asn Thr Glu Gln Glu Glu Gly Gly Glu Ala Val His Glu Val Glu Val
   165 170 175
   Val Ile Lys His Asn Arg Phe Thr Lys Glu Thr Tyr Asp Phe Asp Ile
   180 185 190
   Ala Val Leu Arg Leu Lys Thr Pro Ile Thr Phe Arg Met Asn Val Ala
   195 200 205
   Pro Ala Cys Leu Pro Glu Arg Asp Trp Ala Glu Ser Thr Leu Met Thr
   210 215 220
   Gln Lys Thr Gly Ile Val Ser Gly Phe Gly Arg Thr His Glu Lys Gly
   225 230 235 240
   Gly Gln Ser Thr Arg Leu Lys Met Leu Glu Val Pro Tyr Val Asp Arg
   245 250 255
   Asn Ser Cys Lys Leu Ser Ser Ser Phe Ile Ile Thr Gln Asn Met Phe
   260 265 270
   Cys Ala Gly Tyr Asp Thr Lys Gln Glu Asp Ala Cys Gln Gly Asp Ser
   275 280 285
   Gly Gly Pro His Val Thr Arg Phe Lys Asp Thr Tyr Phe Val Thr Gly
   290 295 300
   Ile Val Ser Trp Gly Glu Gly Cys Ala Arg Lys Gly Lys Tyr Gly Ile
   305 310 315 320
   Tyr Thr Lys Val Thr Ala Phe Leu Lys Trp Ile Asp Arg Ser Met Lys
   325 330 335
   Thr Arg Gly Leu Pro Lys Ala Lys Ser His Ala Pro Glu Val Ile Thr
   340 345 350
   Ser Ser Pro Leu Lys
   355
<210> 14
   <211> 357
   <212> PRT
   <213> Homo sapiens
<400> 14
   Lys Tyr Lys Asp Gly Asp Gln Cys Glu Thr Ser Pro Cys Gln Asn Gln
   1               5                   10                  15
   Gly Lys Cys Lys Asp Gly Leu Gly Glu Tyr Thr Cys Thr Cys Leu Glu
               20                  25                  30
   Gly Phe Glu Gly Lys Asn Cys Glu Leu Phe Thr Arg Lys Leu Cys Ser
           35                  40                  45
   Leu Asp Asn Gly Asp Cys Asp Gln Phe Cys His Glu Glu Gln Asn Ser
       50                  55                  60
   Val Val Cys Ser Cys Ala Arg Gly Tyr Thr Leu Ala Asp Asn Gly Lys
   65                  70                  75                  80
   Ala Cys Ile Pro Thr Gly Pro Tyr Pro Cys Gly Lys Gln Thr Leu Glu
                   85                  90                  95
   Arg Arg Lys Arg Arg Lys Arg Ile Val Gly Gly Gln Glu Cys Lys Asp
               100                 105                 110
   Gly Glu Cys Pro Trp Gln Ala Leu Leu Ile Asn Glu Glu Asn Glu Gly
           115                 120                 125
   Phe Cys Gly Gly Thr Ile Leu Ser Glu Phe Tyr Ile Leu Thr Ala Ala
       130                 135                 140
   His Cys Leu Tyr Gln Ala Lys Arg Phe Lys Val Arg Val Gly Asp Arg
   145                 150                 155                 160
   Asn Thr Glu Gln Glu Glu Gly Gly Glu Ala Val His Glu Val Glu Val
                   165                 170                 175
   Val Ile Lys His Asn Arg Phe Thr Lys Glu Thr Tyr Asp Phe Asp Ile
               180                 185                 190
   Ala Val Leu Arg Leu Lys Thr Pro Ile Thr Phe Arg Met Asn Val Ala
           195                 200                 205
   Pro Ala Cys Leu Pro Glu Arg Asp Trp Ala Glu Ser Thr Leu Met Thr
       210                 215                 220
   Gln Lys Thr Gly Ile Val Ser Gly Phe Gly Arg Thr His Glu Lys Gly
   225                 230                 235                 240
   Ile Gln Ser Thr Arg Leu Lys Met Leu Glu Val Pro Tyr Val Asp Arg
                   245                 250                 255
   Asn Ser Cys Lys Leu Ser Ser Ser Phe Ile Ile Thr Gln Asn Met Phe
               260                 265                 270
   Cys Ala Gly Tyr Asp Thr Lys Gln Glu Asp Ala Cys Gln Gly Asp Ser
           275                 280                 285
   Gly Gly Pro His Val Thr Arg Phe Lys Asp Thr Tyr Phe Val Thr Gly
       290                 295                 300
   Ile Val Ser Trp Gly Glu Gly Cys Ala Arg Lys Gly Lys Tyr Gly Ile
   305                 310                 315                 320
   Tyr Thr Lys Val Thr Ala Phe Leu Lys Trp Ile Asp Arg Ser Met Lys
                   325                 330                 335
   Thr Arg Gly Leu Pro Lys Ala Lys Ser His Ala Pro Glu Val Ile Thr
               340                 345                 350
   Ser Ser Pro Leu Lys
           355
<210> 15
   <211> 357
   <212> PRT
   <213> Homo sapiens
<400> 15
   Lys Tyr Lys Asp Gly Asp Gln Cys Glu Thr Ser Pro Cys Gln Asn Gln
   1               5                   10                  15
   Gly Lys Cys Lys Asp Gly Leu Gly Glu Tyr Thr Cys Thr Cys Leu Glu
               20                  25                  30
   Gly Phe Glu Gly Lys Asn Cys Glu Leu Phe Thr Arg Lys Leu Cys Ser
           35                  40                  45
   Leu Asp Asn Gly Asp Cys Asp Gln Phe Cys His Glu Glu Gln Asn Ser
       50                  55                  60
   Val Val Cys Ser Cys Ala Arg Gly Tyr Thr Leu Ala Asp Asn Gly Lys
   65                  70                  75                  80
   Ala Cys Ile Pro Thr Gly Pro Tyr Pro Cys Gly Lys Gln Thr Leu Glu
                   85                  90                  95
   Arg Arg Lys Arg Arg Lys Arg Ile Val Gly Gly Gln Glu Cys Lys Asp
               100                 105                 110
   Gly Glu Cys Pro Trp Gln Ala Leu Leu Ile Asn Glu Glu Asn Glu Gly
           115                 120                 125
   Phe Cys Gly Gly Thr Ile Leu Ser Glu Phe Tyr Ile Leu Thr Ala Ala
       130                 135                 140
   His Cys Leu Tyr Gln Ala Lys Arg Phe Lys Val Arg Val Gly Asp Arg
   145                 150                 155                 160
   Asn Thr Glu Gln Glu Glu Gly Gly Glu Ala Val His Glu Val Glu Val
                   165                 170                 175
   Val Ile Lys His Asn Arg Phe Thr Lys Glu Thr Tyr Asp Phe Asp Ile
               180                 185                 190
   Ala Val Leu Arg Leu Lys Thr Pro Ile Thr Phe Arg Met Asn Val Ala
           195                 200                 205
   Pro Ala Cys Leu Pro Glu Arg Asp Trp Ala Glu Ser Thr Leu Met Thr
       210                 215                 220
   Gln Lys Thr Gly Ile Val Ser Gly Phe Gly Arg Thr His Glu Lys Gly
   225                 230                 235                 240
   Tyr Gln Ser Thr Arg Leu Lys Met Leu Glu Val Pro Tyr Val Asp Arg
                   245                 250                 255
   Asn Ser Cys Lys Leu Ser Ser Ser Phe Ile Ile Thr Gln Asn Met Phe
               260                 265                 270
   Cys Ala Gly Tyr Asp Thr Lys Gln Glu Asp Ala Cys Gln Gly Asp Ser
           275                 280                 285
   Gly Gly Pro His Val Thr Arg Phe Lys Asp Thr Tyr Phe Val Thr Gly
       290                 295                 300
   Ile Val Ser Trp Gly Glu Gly Cys Ala Arg Lys Gly Lys Tyr Gly Ile
   305                 310                 315                 320
   Tyr Thr Lys Val Thr Ala Phe Leu Lys Trp Ile Asp Arg Ser Met Lys
                   325                 330                 335
   Thr Arg Gly Leu Pro Lys Ala Lys Ser His Ala Pro Glu Val Ile Thr
               340                 345                 350
   Ser Ser Pro Leu Lys
   355
<210> 16
   <211> 357
   <212> PRT
   <213> Homo sapiens
<400> 16
   Lys Tyr Lys Asp Gly Asp Gln Cys Glu Thr Ser Pro Cys Gln Asn Gln
   1               5                   10                  15
   Gly Lys Cys Lys Asp Gly Leu Gly Glu Tyr Thr Cys Thr Cys Leu Glu
               20                  25                  30
   Gly Phe Glu Gly Lys Asn Cys Glu Leu Phe Thr Arg Lys Leu Cys Ser
           35                  40                  45
   Leu Asp Asn Gly Asp Cys Asp Gln Phe Cys His Glu Glu Gln Asn Ser
       50                  55                  60
   Val Val Cys Ser Cys Ala Arg Gly Tyr Thr Leu Ala Asp Asn Gly Lys
   65                  70                  75                      80
   Ala Cys Ile Pro Thr Gly Pro Tyr Pro Cys Gly Lys Gln Thr Leu Glu
                   85                  90                  95
   Arg Arg Lys Arg Arg Lys Arg Ile Val Gly Gly Gln Glu Cys Lys Asp
               100                 105                 110
   Gly Glu Cys Pro Trp Gln Ala Leu Leu Ile Asn Glu Glu Asn Glu Gly
           115                 120                 125
   Phe Cys Gly Gly Thr Ile Leu Ser Glu Phe Tyr Ile Leu Thr Ala Ala
       130                 135                 140
   His Cys Leu Tyr Gln Ala Lys Arg Phe Lys Val Arg Val Gly Asp Arg
   145                 150                 155                 160
   Asn Thr Glu Gln Glu Glu Gly Gly Glu Ala Val His Glu Val Glu Val
                   165                 170                 175
   Val Ile Lys His Asn Arg Phe Thr Lys Glu Thr Tyr Asp Phe Asp Ile
               180                 185                 190
   Ala Val Leu Arg Leu Lys Thr Pro Ile Thr Phe Arg Met Asn Val Ala
           195                 200                 205
   Pro Ala Cys Leu Pro Glu Arg Asp Trp Ala Glu Ser Thr Leu Met Thr
           210                 215                 220
   Gln Lys Thr Gly Ile Val Ser Gly Phe Gly Arg Thr His Glu Lys Gly
   225                 230                 235                 240
   Phe Gln Ser Thr Arg Leu Lys Met Leu Glu Val Pro Tyr Val Asp Arg
                   245                 250                 255
   Asn Ser Cys Lys Leu Ser Ser Ser Phe Ile Ile Thr Gln Asn Met Phe
               260                 265                 270
   Cys Ala Gly Tyr Asp Thr Lys Gln Glu Asp Ala Cys Gln Gly Asp Ser
           275                 280                 285
   Gly Gly Pro His Val Thr Arg Phe Lys Asp Thr Tyr Phe Val Thr Gly
       290                 295                 300
   Ile Val Ser Trp Gly Glu Gly Cys Ala Arg Lys Gly Lys Tyr Gly Ile
   305                 310                 315                 320
   Tyr Thr Lys Val Thr Ala Phe Leu Lys Trp Ile Asp Arg Ser Met Lys
                   325                 330                 335
   Thr Arg Gly Leu Pro Lys Ala Lys Ser His Ala Pro Glu Val Ile Thr
               340                 345                 350
   Ser Ser Pro Leu Lys
           355
<210> 17
   <211> 39
   <212> DNA
   <213> Artificial Sequence
<220>
   <223> primer
<400> 17
   ctggaacgca ggaagaggag gaagaggatc gtgggaggc 39
<210> 18
   <211> 39
   <212> DNA
   <213> Artificial Sequence
<220>
   <223> primer
<400> 18
   gcctcccacg atcctcttcc tcctcttcct gcgttccag 39
<210> 19
   <211> 18
   <212> PRT
   <213> Artificial Sequence
<220>
   <223> MB7
<400> 19
   Met Arg Trp Ser Trp Ile Phe Leu Leu Leu Leu Ser Ile Thr Ser Ala
   1               5                   10                  15
   Asn Ala
<210> 20
   <211> 13
   <212> PRT
   <213> Artificial Sequence
<220>
   <223> Etiquette HPC4 de GPAD1 et GPAD2
<400> 20
   Cys Glu Asp Gln Val Asp Pro Arg Leu Ile Asp Gly Lys
   1               5                   10
<210> 21
   <211> 356
   <212> PRT
   <213> Artificial Sequence
<220>
   <223> GPAD1
<400> 21
   Ser Asn Lys Tyr Lys Asp Gly Asp Gln Cys Glu Thr Ser Pro Cys Gln
   1               5                   10                  15
   Asn Gln Gly Lys Cys Lys Asp Gly Leu Gly Glu Tyr Thr Cys Thr Cys
               20                  25                  30
   Leu Glu Gly Phe Glu Gly Lys Asn Cys Glu Leu Phe Thr Arg Lys Leu
           35                  40                  45
   Cys Ser Leu Asp Asn Gly Asp Cys Asp Gln Phe Cys His Glu Glu Gln
       50                  55                  60
   Asn Ser Val Val Cys Ser Cys Ala Arg Gly Tyr Thr Leu Ala Asp Asn
   65                  70                  75                  80
   Gly Lys Ala Cys Ile Pro Thr Gly Pro Tyr Pro Cys Gly Lys Gln Thr
                   85                  90                  95
   Leu Glu Arg Arg Lys Arg Ile Val Gly Gly Gln Glu Cys Lys Asp Gly
               100                 105                 110
   Glu Cys Pro Trp Gln Ala Leu Leu Ile Asn Glu Glu Asn Glu Gly Phe
           115                 120                 125
   Cys Gly Gly Thr Ile Leu Ser Glu Phe Tyr Ile Leu Thr Ala Ala His
       130                 135                 140
   Cys Leu Tyr Gln Ala Lys Arg Phe Lys Val Arg Val Gly Asp Arg Asn
   145                 150                 155                 160
   Thr Glu Gln Glu Glu Gly Gly Glu Ala Val His Glu Val Glu Val Val
                   165                 170                 175
   Ile Lys His Asn Arg Phe Thr Lys Glu Thr Tyr Asp Phe Asp Ile Ala
               180                 185                 190
   Val Leu Arg Leu Lys Thr Pro Ile Thr Phe Arg Met Asn Val Ala Pro
           195                 200                 205
   Ala Cys Leu Pro Glu Arg Asp Trp Ala Glu Ser Thr Leu Met Thr Gln
       210                 215                 220
   Lys Thr Gly Ile Val Ser Gly Phe Gly Arg Thr His Glu Lys Gly Arg
   225                 230                 235                 240
   Gln Ser Thr Arg Leu Lys Met Leu Glu Val Pro Tyr Val Asp Arg Asn
                   245                 250                 255
   Ser Cys Lys Leu Ser Ser Ser Phe Ile Ile Thr Gln Asn Met Phe Cys
               260                 265                 270
   Ala Gly Tyr Asp Thr Lys Gln Glu Asp Ala Cys Gln Gly Asp Ser Gly
           275                 280                 285
   Gly Pro His Val Thr Arg Phe Lys Asp Thr Tyr Phe Val Thr Gly Ile
       290                 295                 300
   Val Ser Trp Gly Glu Gly Cys Ala Arg Lys Gly Lys Tyr Gly Ile Tyr
   305                 310                 315                 320
   Thr Lys Val Thr Ala Phe Leu Lys Trp Ile Asp Arg Ser Met Lys Thr
                   325                 330                 335
   Arg Gly Leu Pro Lys Ala Lys Ser His Ala Pro Glu Val Ile Thr Ser
   340 345 350
   Ser Pro Leu Lys
   355
<210> 22
   <211> 359
   <212> PRT
   <213> Artificial Sequence
<220>
   <223> GPAD2
<400> 22
   Ser Asn Lys Tyr Lys Asp Gly Asp Gln Cys Glu Thr Ser Pro Cys Gln
   1               5                   10                  15
   Asn Gln Gly Lys Cys Lys Asp Gly Leu Gly Glu Tyr Thr Cys Thr Cys
               20                  25                  30
   Leu Glu Gly Phe Glu Gly Lys Asn Cys Glu Leu Phe Thr Arg Lys Leu
           35                  40                  45
   Cys Ser Leu Asp Asn Gly Asp Cys Asp Gln Phe Cys His Glu Glu Gln
       50                  55                  60
   Asn Ser Val Val Cys Ser Cys Ala Arg Gly Tyr Thr Leu Ala Asp Asn
   65                  70                  75                  80
   Gly Lys Ala Cys Ile Pro Thr Gly Pro Tyr Pro Cys Gly Lys Gln Thr
                   85                  90                  95
   Leu Glu Arg Arg Lys Arg Arg Lys Arg Ile Val Gly Gly Gln Glu Cys
               100                 105                 110
   Lys Asp Gly Glu Cys Pro Trp Gln Ala Leu Leu Ile Asn Glu Glu Asn
           115                 120                 125
   Glu Gly Phe Cys Gly Gly Thr Ile Leu Ser Glu Phe Tyr Ile Leu Thr
       130                 135                 140
   Ala Ala His Cys Leu Tyr Gln Ala Lys Arg Phe Lys Val Arg Val Gly
   145                 150                 155                 160
   Asp Arg Asn Thr Glu Gln Glu Glu Gly Gly Glu Ala Val His Glu Val
                   165                 170                 175
   Glu Val Val Ile Lys His Asn Arg Phe Thr Lys Glu Thr Tyr Asp Phe
                   180                 185                 190
   Asp Ile Ala Val Leu Arg Leu Lys Thr Pro Ile Thr Phe Arg Met Asn
               195                 200                 205
   Val Ala Pro Ala Cys Leu Pro Glu Arg Asp Trp Ala Glu Ser Thr Leu
       210                 215                 220
   Met Thr Gln Lys Thr Gly Ile Val Ser Gly Phe Gly Arg Thr His Glu
   225                 230                 235                 240
   Lys Gly Arg Gln Ser Thr Arg Leu Lys Met Leu Glu Val Pro Tyr Val
                   245                 250                 255
   Asp Arg Asn Ser Cys Lys Leu Ser Ser Ser Phe Ile Ile Thr Gln Asn
               260                 265                 270
   Met Phe Cys Ala Gly Tyr Asp Thr Lys Gln Glu Asp Ala Cys Gln Gly
           275                 280                 285
   Asp Ser Gly Gly Pro His Val Thr Arg Phe Lys Asp Thr Tyr Phe Val
       290                 295                 300
   Thr Gly Ile Val Ser Trp Gly Glu Gly Cys Ala Arg Lys Gly Lys Tyr
   305                 310                 315                 320
   Gly Ile Tyr Thr Lys Val Thr Ala Phe Leu Lys Trp Ile Asp Arg Ser
                   325                 330                 335
   Met Lys Thr Arg Gly Leu Pro Lys Ala Lys Ser His Ala Pro Glu Val
               340                 345                 350
   Ile Thr Ser Ser Pro Leu Lys
           355
<210> 23
   <211> 355
   <212> PRT
   <213> Artificial Sequence
<220>
   <223> GPAD1 optimisé
<400> 23
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Arg Arg Lys Arg Ile Val Gly Gly Gln Glu Cys Lys Asp Gly Glu
               100                 105                 110
   Cys Pro Trp Gln Ala Leu Leu Ile Asn Glu Glu Asn Glu Gly Phe Cys
           115                 120                 125
   Gly Gly Thr Ile Leu Ser Glu Phe Tyr Ile Leu Thr Ala Ala His Cys
       130                 135                 140
   Leu Tyr Gln Ala Lys Arg Phe Lys Val Arg Val Gly Asp Arg Asn Thr
   145                 150                 155                 160
   Glu Gln Glu Glu Gly Gly Glu Ala Val His Glu Val Glu Val Val Ile
                   165                 170                 175
   Lys His Asn Arg Phe Thr Lys Glu Thr Tyr Asp Phe Asp Ile Ala Val
               180                 185                 190
   Leu Arg Leu Lys Thr Pro Ile Thr Phe Arg Met Asn Val Ala Pro Ala
           195                 200                 205
   Cys Leu Pro Glu Arg Asp Trp Ala Glu Ser Thr Leu Met Thr Gln Lys
       210                 215                 220
   Thr Gly Ile Val Ser Gly Phe Gly Arg Thr His Glu Lys Gly Arg Gln
   225                 230                 235                 240
   Ser Thr Arg Leu Lys Met Leu Glu Val Pro Tyr Val Asp Arg Asn Ser
                   245                 250                 255
   Cys Lys Leu Ser Ser Ser Phe Ile Ile Thr Gln Asn Met Phe Cys Ala
               260                 265                 270
   Gly Tyr Asp Thr Lys Gln Glu Asp Ala Cys Gln Gly Asp Ser Gly Gly
           275                 280                 285
   Pro His Val Thr Arg Phe Lys Asp Thr Tyr Phe Val Thr Gly Ile Val
       290                 295                 300
   Ser Trp Gly Glu Gly Cys Ala Arg Lys Gly Lys Tyr Gly Ile Tyr Thr
   305                 310                 315                 320
   Lys Val Thr Ala Phe Leu Lys Trp Ile Asp Arg Ser Met Lys Thr Arg
                   325                 330                 335
   Gly Leu Pro Lys Ala Lys Ser His Ala Pro Glu Val Ile Thr Ser Ser
               340                 345                 350
   Pro Leu Lys
           355
<210> 24
   <211> 358
   <212> PRT
   <213> Artificial Sequence
<220>
   <223> GPAD2 optimisé
<400> 24
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Arg Arg Lys Arg Arg Lys Arg Ile Val Gly Gly Gln Glu Cys Lys
               100                 105                 110
   Asp Gly Glu Cys Pro Trp Gln Ala Leu Leu Ile Asn Glu Glu Asn Glu
           115                 120                 125
   Gly Phe Cys Gly Gly Thr Ile Leu Ser Glu Phe Tyr Ile Leu Thr Ala
       130                 135                 140
   Ala His Cys Leu Tyr Gln Ala Lys Arg Phe Lys Val Arg Val Gly Asp
   145                 150                 155                 160
   Arg Asn Thr Glu Gln Glu Glu Gly Gly Glu Ala Val His Glu Val Glu
                   165                 170                 175
   Val Val Ile Lys His Asn Arg Phe Thr Lys Glu Thr Tyr Asp Phe Asp
               180                 185                 190
   Ile Ala Val Leu Arg Leu Lys Thr Pro Ile Thr Phe Arg Met Asn Val
           195                 200                 205
   Ala Pro Ala Cys Leu Pro Glu Arg Asp Trp Ala Glu Ser Thr Leu Met
       210                 215                 220
   Thr Gln Lys Thr Gly Ile Val Ser Gly Phe Gly Arg Thr His Glu Lys
   225                 230                 235                 240
   Gly Arg Gln Ser Thr Arg Leu Lys Met Leu Glu Val Pro Tyr Val Asp
                   245                 250                 255
   Arg Asn Ser Cys Lys Leu Ser Ser Ser Phe Ile Ile Thr Gln Asn Met
               260                 265                 270
   Phe Cys Ala Gly Tyr Asp Thr Lys Gln Glu Asp Ala Cys Gln Gly Asp
           275                 280                 285
   Ser Gly Gly Pro His Val Thr Arg Phe Lys Asp Thr Tyr Phe Val Thr
       290                 295                 300
   Gly Ile Val Ser Trp Gly Glu Gly Cys Ala Arg Lys Gly Lys Tyr Gly
   305                 310                 315                 320
   Ile Tyr Thr Lys Val Thr Ala Phe Leu Lys Trp Ile Asp Arg Ser Met
                   325                 330                 335
   Lys Thr Arg Gly Leu Pro Lys Ala Lys Ser His Ala Pro Glu Val Ile
               340                 345                 350
   Thr Ser Ser Pro Leu Lys
           355
<210> 25
   <211> 102
   <212> PRT
   <213> Artificial Sequence
<220>
   <223> SEQ ID NO:11 avec Ser-Asn- en N terminal
<400> 25
   Ser Asn Lys Tyr Lys Asp Gly Asp Gln Cys Glu Thr Ser Pro Cys Gln
   1               5                   10                  15
   Asn Gln Gly Lys Cys Lys Asp Gly Leu Gly Glu Tyr Thr Cys Thr Cys
               20                  25                  30
   Leu Glu Gly Phe Glu Gly Lys Asn Cys Glu Leu Phe Thr Arg Lys Leu
           35                  40                  45
   Cys Ser Leu Asp Asn Gly Asp Cys Asp Gln Phe Cys His Glu Glu Gln
       50                  55                  60
   Asn Ser Val Val Cys Ser Cys Ala Arg Gly Tyr Thr Leu Ala Asp Asn
   65                  70                  75                  80
   Gly Lys Ala Cys Ile Pro Thr Gly Pro Tyr Pro Cys Gly Lys Gln Thr
                   85                  90                  95
   Leu Glu Arg Arg Lys Arg
               100
<210> 26
   <211> 101
   <212> PRT
   <213> Artificial Sequence
<220>
   <223> SEQ ID NO:11 avec Asn- en N terminal
<400> 26
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Arg Arg Lys Arg
               100
<210> 27
   <211> 359
   <212> PRT
   <213> Artificial Sequence
<220>
   <223> Exemple de GPAD3
<400> 27
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Gly Ser Ser Gly Arg Arg Lys Arg Ile Val Gly Gly Gln Glu Cys
               100                 105                 110
   Lys Asp Gly Glu Cys Pro Trp Gln Ala Leu Leu Ile Asn Glu Glu Asn
           115                 120                 125
   Glu Gly Phe Cys Gly Gly Thr Ile Leu Ser Glu Phe Tyr Ile Leu Thr
       130                 135                 140
   Ala Ala His Cys Leu Tyr Gln Ala Lys Arg Phe Lys Val Arg Val Gly
   145                 150                 155                 160
   Asp Arg Asn Thr Glu Gln Glu Glu Gly Gly Glu Ala Val His Glu Val
   165 170 175
   Glu Val Val Ile Lys His Asn Arg Phe Thr Lys Glu Thr Tyr Asp Phe
   180 185 190
   Asp Ile Ala Val Leu Arg Leu Lys Thr Pro Ile Thr Phe Arg Met Asn
   195 200 205
   Val Ala Pro Ala Cys Leu Pro Glu Arg Asp Trp Ala Glu Ser Thr Leu
   210 215 220
   Met Thr Gln Lys Thr Gly Ile Val Ser Gly Phe Gly Arg Thr His Glu
   225 230 235 240
   Lys Gly Arg Gln Ser Thr Arg Leu Lys Met Leu Glu Val Pro Tyr Val
   245 250 255
   Asp Arg Asn Ser Cys Lys Leu Ser Ser Ser Phe Ile Ile Thr Gln Asn
   260 265 270
   Met Phe Cys Ala Gly Tyr Asp Thr Lys Gln Glu Asp Ala Cys Gln Gly
   275 280 285
   Asp Ser Gly Gly Pro His Val Thr Arg Phe Lys Asp Thr Tyr Phe Val
   290 295 300
   Thr Gly Ile Val Ser Trp Gly Glu Gly Cys Ala Arg Lys Gly Lys Tyr
   305 310 315 320
   Gly Ile Tyr Thr Lys Val Thr Ala Phe Leu Lys Trp Ile Asp Arg Ser
   325 330 335
   Met Lys Thr Arg Gly Leu Pro Lys Ala Lys Ser His Ala Pro Glu Val
   340 345 350
   Ile Thr Ser Ser Pro Leu Lys
   355
<210> 28
   <211> 360
   <212> PRT
   <213> Artificial Sequence
<220>
   <223> GPAD3-LC
<400> 28
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Arg Gly Ser Ser Gly Arg Arg Lys Arg Ile Val Gly Gly Gln Glu
               100                 105                 110
   Cys Lys Asp Gly Glu Cys Pro Trp Gln Ala Leu Leu Ile Asn Glu Glu
           115                 120                 125
   Asn Glu Gly Phe Cys Gly Gly Thr Ile Leu Ser Glu Phe Tyr Ile Leu
       130                 135                 140
   Thr Ala Ala His Cys Leu Tyr Gln Ala Lys Arg Phe Lys Val Arg Val
   145                 150                 155                 160
   Gly Asp Arg Asn Thr Glu Gln Glu Glu Gly Gly Glu Ala Val His Glu
                   165                 170                 175
   Val Glu Val Val Ile Lys His Asn Arg Phe Thr Lys Glu Thr Tyr Asp
               180                 185                 190
   Phe Asp Ile Ala Val Leu Arg Leu Lys Thr Pro Ile Thr Phe Arg Met
           195                 200                 205
   Asn Val Ala Pro Ala Cys Leu Pro Glu Arg Asp Trp Ala Glu Ser Thr
       210                 215                 220
   Leu Met Thr Gln Lys Thr Gly Ile Val Ser Gly Phe Gly Arg Thr His
   225                 230                 235                 240
   Glu Lys Gly Arg Gln Ser Thr Arg Leu Lys Met Leu Glu Val Pro Tyr
                   245                 250                 255
   Val Asp Arg Asn Ser Cys Lys Leu Ser Ser Ser Phe Ile Ile Thr Gln
                   260                 265                 270
   Asn Met Phe Cys Ala Gly Tyr Asp Thr Lys Gln Glu Asp Ala Cys Gln
           275                 280                 285
   Gly Asp Ser Gly Gly Pro His Val Thr Arg Phe Lys Asp Thr Tyr Phe
       290                 295                 300
   Val Thr Gly Ile Val Ser Trp Gly Glu Gly Cys Ala Arg Lys Gly Lys
   305                 310                 315                 320
   Tyr Gly Ile Tyr Thr Lys Val Thr Ala Phe Leu Lys Trp Ile Asp Arg
                   325                 330                 335
   Ser Met Lys Thr Arg Gly Leu Pro Lys Ala Lys Ser His Ala Pro Glu
               340                 345                 350
   Val Ile Thr Ser Ser Pro Leu Lys
           355                 360
<210> 29
   <211> 366
   <212> PRT
   <213> Artificial Sequence
<220>
   <223> GPAD3-LL
<400> 29
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Arg Gly Gly Gly Gly Ser Gly Gly Gly Gly Ser Arg Arg Lys Arg
               100                 105                 110
   Ile Val Gly Gly Gln Glu Cys Lys Asp Gly Glu Cys Pro Trp Gln Ala
   115 120 125
   Leu Leu Ile Asn Glu Glu Asn Glu Gly Phe Cys Gly Gly Thr Ile Leu
   130 135 140
   Ser Glu Phe Tyr Ile Leu Thr Ala Ala His Cys Leu Tyr Gln Ala Lys
   145 150 155 160
   Arg Phe Lys Val Arg Val Gly Asp Arg Asn Thr Glu Gln Glu Glu Gly
   165 170 175
   Gly Glu Ala Val His Glu Val Glu Val Val Ile Lys His Asn Arg Phe
   180 185 190
   Thr Lys Glu Thr Tyr Asp Phe Asp Ile Ala Val Leu Arg Leu Lys Thr
   195 200 205
   Pro Ile Thr Phe Arg Met Asn Val Ala Pro Ala Cys Leu Pro Glu Arg
   210 215 220
   Asp Trp Ala Glu Ser Thr Leu Met Thr Gln Lys Thr Gly Ile Val Ser
   225 230 235 240
   Gly Phe Gly Arg Thr His Glu Lys Gly Arg Gln Ser Thr Arg Leu Lys
   245 250 255
   Met Leu Glu Val Pro Tyr Val Asp Arg Asn Ser Cys Lys Leu Ser Ser
   260 265 270
   Ser Phe Ile Ile Thr Gln Asn Met Phe Cys Ala Gly Tyr Asp Thr Lys
   275 280 285
   Gln Glu Asp Ala Cys Gln Gly Asp Ser Gly Gly Pro His Val Thr Arg
   290 295 300
   Phe Lys Asp Thr Tyr Phe Val Thr Gly Ile Val Ser Trp Gly Glu Gly
   305 310 315 320
   Cys Ala Arg Lys Gly Lys Tyr Gly Ile Tyr Thr Lys Val Thr Ala Phe
   325 330 335
   Leu Lys Trp Ile Asp Arg Ser Met Lys Thr Arg Gly Leu Pro Lys Ala
   340 345 350
   Lys Ser His Ala Pro Glu Val Ile Thr Ser Ser Pro Leu Lys
   355 360 365
<210> 30
   <211> 369
   <212> PRT
   <213> Artificial Sequence
<220>
   <223> GPAD3-2F
<400> 30
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Arg Arg Lys Arg Gly Gly Gly Gly Ser Gly Gly Gly Gly Ser Arg
               100                 105                 110
   Arg Lys Arg Ile Val Gly Gly Gln Glu Cys Lys Asp Gly Glu Cys Pro
           115                 120                 125
   Trp Gln Ala Leu Leu Ile Asn Glu Glu Asn Glu Gly Phe Cys Gly Gly
       130                 135                 140
   Thr Ile Leu Ser Glu Phe Tyr Ile Leu Thr Ala Ala His Cys Leu Tyr
   145                 150                 155                 160
   Gln Ala Lys Arg Phe Lys Val Arg Val Gly Asp Arg Asn Thr Glu Gln
                   165                 170                 175
   Glu Glu Gly Gly Glu Ala Val His Glu Val Glu Val Val Ile Lys His
               180                 185                 190
   Asn Arg Phe Thr Lys Glu Thr Tyr Asp Phe Asp Ile Ala Val Leu Arg
           195                 200                 205
   Leu Lys Thr Pro Ile Thr Phe Arg Met Asn Val Ala Pro Ala Cys Leu
       210                 215                 220
   Pro Glu Arg Asp Trp Ala Glu Ser Thr Leu Met Thr Gln Lys Thr Gly
   225                 230                 235                 240
   Ile Val Ser Gly Phe Gly Arg Thr His Glu Lys Gly Arg Gln Ser Thr
                   245                 250                 255
   Arg Leu Lys Met Leu Glu Val Pro Tyr Val Asp Arg Asn Ser Cys Lys
               260                 265                 270
   Leu Ser Ser Ser Phe Ile Ile Thr Gln Asn Met Phe Cys Ala Gly Tyr
           275                 280                 285
   Asp Thr Lys Gln Glu Asp Ala Cys Gln Gly Asp Ser Gly Gly Pro His
       290                 295                 300
   Val Thr Arg Phe Lys Asp Thr Tyr Phe Val Thr Gly Ile Val Ser Trp
   305                 310                 315                 320
   Gly Glu Gly Cys Ala Arg Lys Gly Lys Tyr Gly Ile Tyr Thr Lys Val
                   325                 330                 335
   Thr Ala Phe Leu Lys Trp Ile Asp Arg Ser Met Lys Thr Arg Gly Leu
               340                 345                 350
   Pro Lys Ala Lys Ser His Ala Pro Glu Val Ile Thr Ser Ser Pro Leu
           355                 360                 365
   Lys
<210> 31
   <211> 266
   <212> PRT
   <213> Artificial Sequence
<220>
   <223> Chaîne lourde de FXa fusionné à SEQ ID NO:27
<400> 31
   Ile Val Gly Gly Gln Glu Cys Lys Asp Gly Glu Cys Pro Trp Gln Ala
   1               5                   10                  15
   Leu Leu Ile Asn Glu Glu Asn Glu Gly Phe Cys Gly Gly Thr Ile Leu
               20                  25                  30
   Ser Glu Phe Tyr Ile Leu Thr Ala Ala His Cys Leu Tyr Gln Ala Lys
               35                  40                  45
   Arg Phe Lys Val Arg Val Gly Asp Arg Asn Thr Glu Gln Glu Glu Gly
       50                  55                  60
   Gly Glu Ala Val His Glu Val Glu Val Val Ile Lys His Asn Arg Phe
   65                  70                  75                  80
   Thr Lys Glu Thr Tyr Asp Phe Asp Ile Ala Val Leu Arg Leu Lys Thr
                   85                  90                  95
   Pro Ile Thr Phe Arg Met Asn Val Ala Pro Ala Cys Leu Pro Glu Arg
               100                 105                 110
   Asp Trp Ala Glu Ser Thr Leu Met Thr Gln Lys Thr Gly Ile Val Ser
           115                 120                 125
   Gly Phe Gly Arg Thr His Glu Lys Gly Arg Gln Ser Thr Arg Leu Lys
       130                 135                 140
   Met Leu Glu Val Pro Tyr Val Asp Arg Asn Ser Cys Lys Leu Ser Ser
   145                 150                 155                 160
   Ser Phe Ile Ile Thr Gln Asn Met Phe Cys Ala Gly Tyr Asp Thr Lys
                   165                 170                 175
   Gln Glu Asp Ala Cys Gln Gly Asp Ser Gly Gly Pro His Val Thr Arg
               180                 185                 190
   Phe Lys Asp Thr Tyr Phe Val Thr Gly Ile Val Ser Trp Gly Glu Gly
           195                 200                 205
   Cys Ala Arg Lys Gly Lys Tyr Gly Ile Tyr Thr Lys Val Thr Ala Phe
       210                 215                 220
   Leu Lys Trp Ile Asp Arg Ser Met Lys Thr Arg Gly Leu Pro Lys Ala
   225                 230                 235                 240
   Lys Ser His Ala Pro Glu Val Ile Thr Ser Ser Pro Leu Lys Glu Asp
                   245                 250                 255
   Gln Val Asp Pro Arg Leu Ile Asp Gly Lys
               260                 265
<210> 32
   <211> 586
   <212> PRT
   <213> Artificial Sequence
<220>
   <223> GPAD2-FX-Fc
<400> 32
   Ser Asn Lys Tyr Lys Asp Gly Asp Gln Cys Glu Thr Ser Pro Cys Gln
   1               5                   10                  15
   Asn Gln Gly Lys Cys Lys Asp Gly Leu Gly Glu Tyr Thr Cys Thr Cys
               20                  25                  30
   Leu Glu Gly Phe Glu Gly Lys Asn Cys Glu Leu Phe Thr Arg Lys Leu
           35                  40                  45
   Cys Ser Leu Asp Asn Gly Asp Cys Asp Gln Phe Cys His Glu Glu Gln
       50                  55                  60
   Asn Ser Val Val Cys Ser Cys Ala Arg Gly Tyr Thr Leu Ala Asp Asn
   65                  70                  75                  80
   Gly Lys Ala Cys Ile Pro Thr Gly Pro Tyr Pro Cys Gly Lys Gln Thr
                   85                  90                  95
   Leu Glu Arg Arg Lys Arg Arg Lys Arg Ile Val Gly Gly Gln Glu Cys
               100                 105                 110
   Lys Asp Gly Glu Cys Pro Trp Gln Ala Leu Leu Ile Asn Glu Glu Asn
           115                 120                 125
   Glu Gly Phe Cys Gly Gly Thr Ile Leu Ser Glu Phe Tyr Ile Leu Thr
       130                 135                 140
   Ala Ala His Cys Leu Tyr Gln Ala Lys Arg Phe Lys Val Arg Val Gly
   145                 150                 155                 160
   Asp Arg Asn Thr Glu Gln Glu Glu Gly Gly Glu Ala Val His Glu Val
                   165                 170                 175
   Glu Val Val Ile Lys His Asn Arg Phe Thr Lys Glu Thr Tyr Asp Phe
               180                 185                 190
   Asp Ile Ala Val Leu Arg Leu Lys Thr Pro Ile Thr Phe Arg Met Asn
           195                 200                 205
   Val Ala Pro Ala Cys Leu Pro Glu Arg Asp Trp Ala Glu Ser Thr Leu
       210                 215                 220
   Met Thr Gln Lys Thr Gly Ile Val Ser Gly Phe Gly Arg Thr His Glu
   225 230 235 240
   Lys Gly Arg Gln Ser Thr Arg Leu Lys Met Leu Glu Val Pro Tyr Val
   245 250 255
   Asp Arg Asn Ser Cys Lys Leu Ser Ser Ser Phe Ile Ile Thr Gln Asn
   260 265 270
   Met Phe Cys Ala Gly Tyr Asp Thr Lys Gln Glu Asp Ala Cys Gln Gly
   275 280 285
   Asp Ser Gly Gly Pro His Val Thr Arg Phe Lys Asp Thr Tyr Phe Val
   290 295 300
   Thr Gly Ile Val Ser Trp Gly Glu Gly Cys Ala Arg Lys Gly Lys Tyr
   305 310 315 320
   Gly Ile Tyr Thr Lys Val Thr Ala Phe Leu Lys Trp Ile Asp Arg Ser
   325 330 335
   Met Lys Thr Arg Gly Leu Pro Lys Ala Lys Ser His Ala Pro Glu Val
   340 345 350
   Ile Thr Ser Ser Pro Leu Lys Asp Lys Thr His Thr Cys Pro Pro Cys
   355 360 365
   Pro Ala Pro Glu Leu Leu Gly Gly Pro Ser Val Phe Leu Phe Pro Pro
   370 375 380
   Lys Pro Lys Asp Thr Leu Met Ile Ser Arg Thr Pro Glu Val Thr Cys
   385 390 395 400
   Val Val Val Asp Val Ser His Glu Asp Pro Glu Val Lys Phe Asn Trp
   405 410 415
   Tyr Val Asp Gly Val Glu Val His Asn Ala Lys Thr Lys Pro Arg Glu
   420 425 430
   Glu Gln Tyr Asn Ser Thr Tyr Arg Val Val Ser Val Leu Thr Val Leu
   435 440 445
   His Gln Asp Trp Leu Asn Gly Lys Glu Tyr Lys Cys Lys Val Ser Asn
   450 455 460
   Lys Ala Leu Pro Ala Pro Ile Glu Lys Thr Ile Ser Lys Ala Lys Gly
   465 470 475 480
   Gln Pro Arg Glu Pro Gln Val Tyr Thr Leu Pro Pro Ser Arg Asp Glu
                   485                 490                 495
   Leu Thr Lys Asn Gln Val Ser Leu Thr Cys Leu Val Lys Gly Phe Tyr
               500                 505                 510
   Pro Ser Asp Ile Ala Val Glu Trp Glu Ser Asn Gly Gln Pro Glu Asn
           515                 520                 525
   Asn Tyr Lys Thr Thr Pro Pro Val Leu Asp Ser Asp Gly Ser Phe Phe
       530                 535                 540
   Leu Tyr Ser Lys Leu Thr Val Asp Lys Ser Arg Trp Gln Gln Gly Asn
   545                 550                 555                 560
   Val Phe Ser Cys Ser Val Met His Glu Ala Leu His Asn His Tyr Thr
                   565                 570                 575
   Gln Lys Ser Leu Ser Leu Ser Pro Gly Lys
               580                 585
<210> 33
   <211> 591
   <212> PRT
   <213> Artificial Sequence
<220>
   <223> GPAD2-Fcl
<400> 33
   Ser Asn Lys Tyr Lys Asp Gly Asp Gln Cys Glu Thr Ser Pro Cys Gln
   1               5                   10                  15
   Asn Gln Gly Lys Cys Lys Asp Gly Leu Gly Glu Tyr Thr Cys Thr Cys
               20                  25                  30
   Leu Glu Gly Phe Glu Gly Lys Asn Cys Glu Leu Phe Thr Arg Lys Leu
           35                  40                  45
   Cys Ser Leu Asp Asn Gly Asp Cys Asp Gln Phe Cys His Glu Glu Gln
       50                  55                  60
   Asn Ser Val Val Cys Ser Cys Ala Arg Gly Tyr Thr Leu Ala Asp Asn
   65                  70                  75                  80
   Gly Lys Ala Cys Ile Pro Thr Gly Pro Tyr Pro Cys Gly Lys Gln Thr
                   85                  90                  95
   Leu Glu Arg Arg Lys Arg Arg Lys Arg Ile Val Gly Gly Gln Glu Cys
               100                 105                 110
   Lys Asp Gly Glu Cys Pro Trp Gln Ala Leu Leu Ile Asn Glu Glu Asn
           115                 120                 125
   Glu Gly Phe Cys Gly Gly Thr Ile Leu Ser Glu Phe Tyr Ile Leu Thr
       130                 135                 140
   Ala Ala His Cys Leu Tyr Gln Ala Lys Arg Phe Lys Val Arg Val Gly
   145                 150                 155                 160
   Asp Arg Asn Thr Glu Gln Glu Glu Gly Gly Glu Ala Val His Glu Val
                   165                 170                 175
   Glu Val Val Ile Lys His Asn Arg Phe Thr Lys Glu Thr Tyr Asp Phe
               180                 185                 190
   Asp Ile Ala Val Leu Arg Leu Lys Thr Pro Ile Thr Phe Arg Met Asn
           195                 200                 205
   Val Ala Pro Ala Cys Leu Pro Glu Arg Asp Trp Ala Glu Ser Thr Leu
       210                 215                 220
   Met Thr Gln Lys Thr Gly Ile Val Ser Gly Phe Gly Arg Thr His Glu
   225                 230                 235                 240
   Lys Gly Arg Gln Ser Thr Arg Leu Lys Met Leu Glu Val Pro Tyr Val
                   245                 250                 255
   Asp Arg Asn Ser Cys Lys Leu Ser Ser Ser Phe Ile Ile Thr Gln Asn
               260                 265                 270
   Met Phe Cys Ala Gly Tyr Asp Thr Lys Gln Glu Asp Ala Cys Gln Gly
           275                 280                 285
   Asp Ser Gly Gly Pro His Val Thr Arg Phe Lys Asp Thr Tyr Phe Val
       290                 295                 300
   Thr Gly Ile Val Ser Trp Gly Glu Gly Cys Ala Arg Lys Gly Lys Tyr
   305                 310                 315                 320
   Gly Ile Tyr Thr Lys Val Thr Ala Phe Leu Lys Trp Ile Asp Arg Ser
                   325                 330                 335
   Met Lys Thr Arg Gly Leu Pro Lys Ala Lys Ser His Ala Pro Glu Val
               340                 345                 350
   Ile Thr Ser Ser Pro Leu Lys Glu Pro Lys Ser Cys Asp Lys Thr His
               355                 360                 365
   Thr Cys Pro Pro Cys Pro Ala Pro Glu Leu Leu Gly Gly Pro Ser Val
       370                 375                 380
   Phe Leu Phe Pro Pro Lys Pro Lys Asp Thr Leu Met Ile Ser Arg Thr
   385                 390                 395                 400
   Pro Glu Val Thr Cys Val Val Val Asp Val Ser His Glu Asp Pro Glu
                   405                 410                 415
   Val Lys Phe Asn Trp Tyr Val Asp Gly Val Glu Val His Asn Ala Lys
               420                 425                 430
   Thr Lys Pro Arg Glu Glu Gln Tyr Asn Ser Thr Tyr Arg Val Val Ser
           435                 440                 445
   Val Leu Thr Val Leu His Gln Asp Trp Leu Asn Gly Lys Glu Tyr Lys
       450                 455                 460
   Cys Lys Val Ser Asn Lys Ala Leu Pro Ala Pro Ile Glu Lys Thr Ile
   465                 470                 475                 480
   Ser Lys Ala Lys Gly Gln Pro Arg Glu Pro Gln Val Tyr Thr Leu Pro
                   485                 490                 495
   Pro Ser Arg Asp Glu Leu Thr Lys Asn Gln Val Ser Leu Thr Cys Leu
               500                 505                 510
   Val Lys Gly Phe Tyr Pro Ser Asp Ile Ala Val Glu Trp Glu Ser Asn
           515                 520                 525
   Gly Gln Pro Glu Asn Asn Tyr Lys Thr Thr Pro Pro Val Leu Asp Ser
       530                 535                 540
   Asp Gly Ser Phe Phe Leu Tyr Ser Lys Leu Thr Val Asp Lys Ser Arg
   545                 550                 555                 560
   Trp Gln Gln Gly Asn Val Phe Ser Cys Ser Val Met His Glu Ala Leu
                   565                 570                 575
   His Asn His Tyr Thr Gln Lys Ser Leu Ser Leu Ser Pro Gly Lys
               580                 585                 590
<210> 34
   <211> 590
   <212> PRT
   <213> Artificial Sequence
<220>
   <223> GPAD2-FcLss
<400> 34
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Arg Arg Lys Arg Arg Lys Arg Ile Val Gly Gly Gln Glu Cys Lys
               100                 105                 110
   Asp Gly Glu Cys Pro Trp Gln Ala Leu Leu Ile Asn Glu Glu Asn Glu
           115                 120                 125
   Gly Phe Cys Gly Gly Thr Ile Leu Ser Glu Phe Tyr Ile Leu Thr Ala
       130                 135                 140
   Ala His Cys Leu Tyr Gln Ala Lys Arg Phe Lys Val Arg Val Gly Asp
   145                 150                 155                 160
   Arg Asn Thr Glu Gln Glu Glu Gly Gly Glu Ala Val His Glu Val Glu
                   165                 170                 175
   Val Val Ile Lys His Asn Arg Phe Thr Lys Glu Thr Tyr Asp Phe Asp
               180                 185                 190
   Ile Ala Val Leu Arg Leu Lys Thr Pro Ile Thr Phe Arg Met Asn Val
           195                 200                 205
   Ala Pro Ala Cys Leu Pro Glu Arg Asp Trp Ala Glu Ser Thr Leu Met
       210                 215                 220
   Thr Gln Lys Thr Gly Ile Val Ser Gly Phe Gly Arg Thr His Glu Lys
   225 230 235 240
   Gly Arg Gln Ser Thr Arg Leu Lys Met Leu Glu Val Pro Tyr Val Asp
   245 250 255
   Arg Asn Ser Cys Lys Leu Ser Ser Ser Phe Ile Ile Thr Gln Asn Met
   260 265 270
   Phe Cys Ala Gly Tyr Asp Thr Lys Gln Glu Asp Ala Cys Gln Gly Asp
   275 280 285
   Ser Gly Gly Pro His Val Thr Arg Phe Lys Asp Thr Tyr Phe Val Thr
   290 295 300
   Gly Ile Val Ser Trp Gly Glu Gly Cys Ala Arg Lys Gly Lys Tyr Gly
   305 310 315 320
   Ile Tyr Thr Lys Val Thr Ala Phe Leu Lys Trp Ile Asp Arg Ser Met
   325 330 335
   Lys Thr Arg Gly Leu Pro Lys Ala Lys Ser His Ala Pro Glu Val Ile
   340 345 350
   Thr Ser Ser Pro Leu Lys Glu Pro Lys Ser Cys Asp Lys Thr His Thr
   355 360 365
   Cys Pro Pro Cys Pro Ala Pro Glu Leu Leu Gly Gly Pro Ser Val Phe
   370 375 380
   Leu Phe Pro Pro Lys Pro Lys Asp Thr Leu Met Ile Ser Arg Thr Pro
   385 390 395 400
   Glu Val Thr Cys Val Val Val Asp Val Ser His Glu Asp Pro Glu Val
   405 410 415
   Lys Phe Asn Trp Tyr Val Asp Gly Val Glu Val His Asn Ala Lys Thr
   420 425 430
   Lys Pro Arg Glu Glu Gln Tyr Asn Ser Thr Tyr Arg Val Val Ser Val
   435 440 445
   Leu Thr Val Leu His Gln Asp Trp Leu Asn Gly Lys Glu Tyr Lys Cys
   450 455 460
   Lys Val Ser Asn Lys Ala Leu Pro Ala Pro Ile Glu Lys Thr Ile Ser
   465                 470                 475                 480
   Lys Ala Lys Gly Gln Pro Arg Glu Pro Gln Val Tyr Thr Leu Pro Pro
                   485                 490                 495
   Ser Arg Asp Glu Leu Thr Lys Asn Gln Val Ser Leu Thr Cys Leu Val
               500                 505                 510
   Lys Gly Phe Tyr Pro Ser Asp Ile Ala Val Glu Trp Glu Ser Asn Gly
           515                 520                 525
   Gln Pro Glu Asn Asn Tyr Lys Thr Thr Pro Pro Val Leu Asp Ser Asp
       530                 535                 540
   Gly Ser Phe Phe Leu Tyr Ser Lys Leu Thr Val Asp Lys Ser Arg Trp
   545                 550                 555                 560
   Gln Gln Gly Asn Val Phe Ser Cys Ser Val Met His Glu Ala Leu His
                   565                 570                 575
   Asn His Tyr Thr Gln Lys Ser Leu Ser Leu Ser Pro Gly Lys
               580                 585                 590
<210> 35
   <211> 831
   <212> PRT
   <213> Artificial Sequence
<220>
   <223> GPAD2-scFcL
<400> 35
   Ser Asn Lys Tyr Lys Asp Gly Asp Gln Cys Glu Thr Ser Pro Cys Gln
   1               5                   10                  15
   Asn Gln Gly Lys Cys Lys Asp Gly Leu Gly Glu Tyr Thr Cys Thr Cys
               20                  25                  30
   Leu Glu Gly Phe Glu Gly Lys Asn Cys Glu Leu Phe Thr Arg Lys Leu
           35                  40                  45
   Cys Ser Leu Asp Asn Gly Asp Cys Asp Gln Phe Cys His Glu Glu Gln
       50                  55                  60
   Asn Ser Val Val Cys Ser Cys Ala Arg Gly Tyr Thr Leu Ala Asp Asn
   65                  70                  75                  80
   Gly Lys Ala Cys Ile Pro Thr Gly Pro Tyr Pro Cys Gly Lys Gln Thr
                   85                  90                  95
   Leu Glu Arg Arg Lys Arg Arg Lys Arg Ile Val Gly Gly Gln Glu Cys
               100                 105                 110
   Lys Asp Gly Glu Cys Pro Trp Gln Ala Leu Leu Ile Asn Glu Glu Asn
           115                 120                 125
   Glu Gly Phe Cys Gly Gly Thr Ile Leu Ser Glu Phe Tyr Ile Leu Thr
       130                 135                 140
   Ala Ala His Cys Leu Tyr Gln Ala Lys Arg Phe Lys Val Arg Val Gly
   145                 150                 155                 160
   Asp Arg Asn Thr Glu Gln Glu Glu Gly Gly Glu Ala Val His Glu Val
                   165                 170                 175
   Glu Val Val Ile Lys His Asn Arg Phe Thr Lys Glu Thr Tyr Asp Phe
               180                 185                 190
   Asp Ile Ala Val Leu Arg Leu Lys Thr Pro Ile Thr Phe Arg Met Asn
           195                 200                 205
   Val Ala Pro Ala Cys Leu Pro Glu Arg Asp Trp Ala Glu Ser Thr Leu
       210                 215                 220
   Met Thr Gln Lys Thr Gly Ile Val Ser Gly Phe Gly Arg Thr His Glu
   225                 230                 235                 240
   Lys Gly Arg Gln Ser Thr Arg Leu Lys Met Leu Glu Val Pro Tyr Val
                   245                 250                 255
   Asp Arg Asn Ser Cys Lys Leu Ser Ser Ser Phe Ile Ile Thr Gln Asn
               260                 265                 270
   Met Phe Cys Ala Gly Tyr Asp Thr Lys Gln Glu Asp Ala Cys Gln Gly
           275                 280                 285
   Asp Ser Gly Gly Pro His Val Thr Arg Phe Lys Asp Thr Tyr Phe Val
       290                 295                 300
   Thr Gly Ile Val Ser Trp Gly Glu Gly Cys Ala Arg Lys Gly Lys Tyr
   305                 310                 315                 320
   Gly Ile Tyr Thr Lys Val Thr Ala Phe Leu Lys Trp Ile Asp Arg Ser
                   325                 330                 335
   Met Lys Thr Arg Gly Leu Pro Lys Ala Lys Ser His Ala Pro Glu Val
               340                 345                 350
   Ile Thr Ser Ser Pro Leu Lys Asp Lys Thr His Thr Cys Pro Pro Cys
           355                 360                 365
   Pro Ala Pro Glu Leu Leu Gly Gly Pro Ser Val Phe Leu Phe Pro Pro
       370                 375                 380
   Lys Pro Lys Asp Thr Leu Met Ile Ser Arg Thr Pro Glu Val Thr Cys
   385                 390                 395                 400
   Val Val Val Asp Val Ser His Glu Asp Pro Glu Val Lys Phe Asn Trp
                   405                 410                 415
   Tyr Val Asp Gly Val Glu Val His Asn Ala Lys Thr Lys Pro Arg Glu
               420                 425                 430
   Glu Gln Tyr Asn Ser Thr Tyr Arg Val Val Ser Val Leu Thr Val Leu
           435                 440                 445
   His Gln Asp Trp Leu Asn Gly Lys Glu Tyr Lys Cys Lys Val Ser Asn
       450                 455                 460
   Lys Ala Leu Pro Ala Pro Ile Glu Lys Thr Ile Ser Lys Ala Lys Gly
   465                 470                 475                 480
   Gln Pro Arg Glu Pro Gln Val Tyr Thr Leu Pro Pro Ser Arg Asp Glu
                   485                 490                 495
   Leu Thr Lys Asn Gln Val Ser Leu Thr Cys Leu Val Lys Gly Phe Tyr
               500                 505                 510
   Pro Ser Asp Ile Ala Val Glu Trp Glu Ser Asn Gly Gln Pro Glu Asn
           515                 520                 525
   Asn Tyr Lys Thr Thr Pro Pro Val Leu Asp Ser Asp Gly Ser Phe Phe
       530                 535                 540
   Leu Tyr Ser Lys Leu Thr Val Asp Lys Ser Arg Trp Gln Gln Gly Asn
   545                 550                 555                 560
   Val Phe Ser Cys Ser Val Met His Glu Ala Leu His Asn His Tyr Thr
                   565                 570                 575
   Gln Lys Ser Leu Ser Leu Ser Pro Gly Gly Gly Gly Gly Ser Gly Gly
               580                 585                 590
   Gly Gly Ser Gly Gly Gly Gly Ser Glu Pro Lys Ser Ser Asp Lys Thr
           595                 600                 605
   His Thr Cys Pro Pro Cys Pro Ala Pro Glu Leu Leu Gly Gly Pro Ser
       610                 615                 620
   Val Phe Leu Phe Pro Pro Lys Pro Lys Asp Thr Leu Met Ile Ser Arg
   625                 630                 635                 640
   Thr Pro Glu Val Thr Cys Val Val Val Asp Val Ser His Glu Asp Pro
                   645                 650                 655
   Glu Val Lys Phe Asn Trp Tyr Val Asp Gly Val Glu Val His Asn Ala
               660                 665                 670
   Lys Thr Lys Pro Arg Glu Glu Gln Tyr Asn Ser Thr Tyr Arg Val Val
           675                 680                 685
   Ser Val Leu Thr Val Leu His Gln Asp Trp Leu Asn Gly Lys Glu Tyr
       690                 695                 700
   Lys Cys Lys Val Ser Asn Lys Ala Leu Pro Ala Pro Ile Glu Lys Thr
   705                 710                 715                 720
   Ile Ser Lys Ala Lys Gly Gln Pro Arg Glu Pro Gln Val Tyr Thr Leu
                   725                 730                 735
   Pro Pro Ser Arg Asp Glu Leu Thr Lys Asn Gln Val Ser Leu Thr Cys
               740                 745                 750
   Leu Val Lys Gly Phe Tyr Pro Ser Asp Ile Ala Val Glu Trp Glu Ser
           755                 760                 765
   Asn Gly Gln Pro Glu Asn Asn Tyr Lys Thr Thr Pro Pro Val Leu Asp
       770                 775                 780
   Ser Asp Gly Ser Phe Phe Leu Tyr Ser Lys Leu Thr Val Asp Lys Ser
   785                 790                 795                 800
   Arg Trp Gln Gln Gly Asn Val Phe Ser Cys Ser Val Met His Glu Ala
                   805                 810                 815
   Leu His Asn His Tyr Thr Gln Lys Ser Leu Ser Leu Ser Pro Gly
               820                 825                 830
<210> 36
   <211> 821
   <212> PRT
   <213> Artificial Sequence
<220>
   <223> GPAD2-scFcS
<400> 36
   Ser Asn Lys Tyr Lys Asp Gly Asp Gln Cys Glu Thr Ser Pro Cys Gln
   1               5                   10                  15
   Asn Gln Gly Lys Cys Lys Asp Gly Leu Gly Glu Tyr Thr Cys Thr Cys
               20                  25                  30
   Leu Glu Gly Phe Glu Gly Lys Asn Cys Glu Leu Phe Thr Arg Lys Leu
           35                  40                  45
   Cys Ser Leu Asp Asn Gly Asp Cys Asp Gln Phe Cys His Glu Glu Gln
       50                  55                  60
   Asn Ser Val Val Cys Ser Cys Ala Arg Gly Tyr Thr Leu Ala Asp Asn
   65                  70                  75                  80
   Gly Lys Ala Cys Ile Pro Thr Gly Pro Tyr Pro Cys Gly Lys Gln Thr
                   85                  90                  95
   Leu Glu Arg Arg Lys Arg Arg Lys Arg Ile Val Gly Gly Gln Glu Cys
               100                 105                 110
   Lys Asp Gly Glu Cys Pro Trp Gln Ala Leu Leu Ile Asn Glu Glu Asn
           115                 120                 125
   Glu Gly Phe Cys Gly Gly Thr Ile Leu Ser Glu Phe Tyr Ile Leu Thr
       130                 135                 140
   Ala Ala His Cys Leu Tyr Gln Ala Lys Arg Phe Lys Val Arg Val Gly
   145                 150                 155                 160
   Asp Arg Asn Thr Glu Gln Glu Glu Gly Gly Glu Ala Val His Glu Val
                   165                 170                 175
   Glu Val Val Ile Lys His Asn Arg Phe Thr Lys Glu Thr Tyr Asp Phe
               180                 185                 190
   Asp Ile Ala Val Leu Arg Leu Lys Thr Pro Ile Thr Phe Arg Met Asn
           195                 200                 205
   Val Ala Pro Ala Cys Leu Pro Glu Arg Asp Trp Ala Glu Ser Thr Leu
       210                 215                 220
   Met Thr Gln Lys Thr Gly Ile Val Ser Gly Phe Gly Arg Thr His Glu
   225                 230                 235                 240
   Lys Gly Arg Gln Ser Thr Arg Leu Lys Met Leu Glu Val Pro Tyr Val
                   245                 250                 255
   Asp Arg Asn Ser Cys Lys Leu Ser Ser Ser Phe Ile Ile Thr Gln Asn
               260                 265                 270
   Met Phe Cys Ala Gly Tyr Asp Thr Lys Gln Glu Asp Ala Cys Gln Gly
           275                 280                 285
   Asp Ser Gly Gly Pro His Val Thr Arg Phe Lys Asp Thr Tyr Phe Val
       290                 295                 300
   Thr Gly Ile Val Ser Trp Gly Glu Gly Cys Ala Arg Lys Gly Lys Tyr
   305                 310                 315                 320
   Gly Ile Tyr Thr Lys Val Thr Ala Phe Leu Lys Trp Ile Asp Arg Ser
                   325                 330                 335
   Met Lys Thr Arg Gly Leu Pro Lys Ala Lys Ser His Ala Pro Glu Val
               340                 345                 350
   Ile Thr Ser Ser Pro Leu Lys Asp Lys Thr His Thr Cys Pro Pro Cys
           355                 360                 365
   Pro Ala Pro Glu Leu Leu Gly Gly Pro Ser Val Phe Leu Phe Pro Pro
       370                 375                 380
   Lys Pro Lys Asp Thr Leu Met Ile Ser Arg Thr Pro Glu Val Thr Cys
   385                 390                 395                 400
   Val Val Val Asp Val Ser His Glu Asp Pro Glu Val Lys Phe Asn Trp
                   405                 410                 415
   Tyr Val Asp Gly Val Glu Val His Asn Ala Lys Thr Lys Pro Arg Glu
               420                 425                 430
   Glu Gln Tyr Asn Ser Thr Tyr Arg Val Val Ser Val Leu Thr Val Leu
           435                 440                 445
   His Gln Asp Trp Leu Asn Gly Lys Glu Tyr Lys Cys Lys Val Ser Asn
       450                 455                 460
   Lys Ala Leu Pro Ala Pro Ile Glu Lys Thr Ile Ser Lys Ala Lys Gly
   465                 470                 475                 480
   Gln Pro Arg Glu Pro Gln Val Tyr Thr Leu Pro Pro Ser Arg Asp Glu
   485 490 495
   Leu Thr Lys Asn Gln Val Ser Leu Thr Cys Leu Val Lys Gly Phe Tyr
   500 505 510
   Pro Ser Asp Ile Ala Val Glu Trp Glu Ser Asn Gly Gln Pro Glu Asn
   515 520 525
   Asn Tyr Lys Thr Thr Pro Pro Val Leu Asp Ser Asp Gly Ser Phe Phe
   530 535 540
   Leu Tyr Ser Lys Leu Thr Val Asp Lys Ser Arg Trp Gln Gln Gly Asn
   545 550 555 560
   Val Phe Ser Cys Ser Val Met His Glu Ala Leu His Asn His Tyr Thr
   565 570 575
   Gln Lys Ser Leu Ser Leu Ser Pro Gly Gly Gly Gly Gly Ser Glu Pro
   580 585 590
   Lys Ser Ser Asp Lys Thr His Thr Cys Pro Pro Cys Pro Ala Pro Glu
   595 600 605
   Leu Leu Gly Gly Pro Ser Val Phe Leu Phe Pro Pro Lys Pro Lys Asp
   610 615 620
   Thr Leu Met Ile Ser Arg Thr Pro Glu Val Thr Cys Val Val Val Asp
   625 630 635 640
   Val Ser His Glu Asp Pro Glu Val Lys Phe Asn Trp Tyr Val Asp Gly
   645 650 655
   Val Glu Val His Asn Ala Lys Thr Lys Pro Arg Glu Glu Gln Tyr Asn
   660 665 670
   Ser Thr Tyr Arg Val Val Ser Val Leu Thr Val Leu His Gln Asp Trp
   675 680 685
   Leu Asn Gly Lys Glu Tyr Lys Cys Lys Val Ser Asn Lys Ala Leu Pro
   690 695 700
   Ala Pro Ile Glu Lys Thr Ile Ser Lys Ala Lys Gly Gln Pro Arg Glu
   705 710 715 720
   Pro Gln Val Tyr Thr Leu Pro Pro Ser Arg Asp Glu Leu Thr Lys Asn
   725 730 735
   Gln Val Ser Leu Thr Cys Leu Val Lys Gly Phe Tyr Pro Ser Asp Ile
   740 745 750
   Ala Val Glu Trp Glu Ser Asn Gly Gln Pro Glu Asn Asn Tyr Lys Thr
   755 760 765
   Thr Pro Pro Val Leu Asp Ser Asp Gly Ser Phe Phe Leu Tyr Ser Lys
   770 775 780
   Leu Thr Val Asp Lys Ser Arg Trp Gln Gln Gly Asn Val Phe Ser Cys
   785 790 795 800
   Ser Val Met His Glu Ala Leu His Asn His Tyr Thr Gln Lys Ser Leu
   805 810 815
   Ser Leu Ser Pro Gly
   820
<210> 37
   <211> 835
   <212> PRT
   <213> Artificial Sequence
<220>
   <223> scFcL-GPAD2
<400> 37
   Asp Lys Thr His Thr Cys Pro Pro Cys Pro Ala Pro Glu Leu Leu Gly
   1 5 10 15
   Gly Pro Ser Val Phe Leu Phe Pro Pro Lys Pro Lys Asp Thr Leu Met
   20 25 30
   Ile Ser Arg Thr Pro Glu Val Thr Cys Val Val Val Asp Val Ser His
   35 40 45
   Glu Asp Pro Glu Val Lys Phe Asn Trp Tyr Val Asp Gly Val Glu Val
   50 55 60
   His Asn Ala Lys Thr Lys Pro Arg Glu Glu Gln Tyr Asn Ser Thr Tyr
   65 70 75 80
   Arg Val Val Ser Val Leu Thr Val Leu His Gln Asp Trp Leu Asn Gly
   85 90 95
   Lys Glu Tyr Lys Cys Lys Val Ser Asn Lys Ala Leu Pro Ala Pro Ile
   100 105 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Gly Gly Gly Gly Ser Gly Gly Gly Gly Ser Gly Gly Gly Gly
   225                 230                 235                 240
   Ser Glu Pro Lys Ser Ser Asp Lys Thr His Thr Cys Pro Pro Cys Pro
                   245                 250                 255
   Ala Pro Glu Leu Leu Gly Gly Pro Ser Val Phe Leu Phe Pro Pro Lys
               260                 265                 270
   Pro Lys Asp Thr Leu Met Ile Ser Arg Thr Pro Glu Val Thr Cys Val
           275                 280                 285
   Val Val Asp Val Ser His Glu Asp Pro Glu Val Lys Phe Asn Trp Tyr
       290                 295                 300
   Val Asp Gly Val Glu Val His Asn Ala Lys Thr Lys Pro Arg Glu Glu
   305                 310                 315                 320
   Gln Tyr Asn Ser Thr Tyr Arg Val Val Ser Val Leu Thr Val Leu His
                   325                 330                 335
   Gln Asp Trp Leu Asn Gly Lys Glu Tyr Lys Cys Lys Val Ser Asn Lys
               340                 345                 350
   Ala Leu Pro Ala Pro Ile Glu Lys Thr Ile Ser Lys Ala Lys Gly Gln
           355                 360                 365
   Pro Arg Glu Pro Gln Val Tyr Thr Leu Pro Pro Ser Arg Asp Glu Leu
       370                 375                 380
   Thr Lys Asn Gln Val Ser Leu Thr Cys Leu Val Lys Gly Phe Tyr Pro
   385                 390                 395                 400
   Ser Asp Ile Ala Val Glu Trp Glu Ser Asn Gly Gln Pro Glu Asn Asn
                   405                 410                 415
   Tyr Lys Thr Thr Pro Pro Val Leu Asp Ser Asp Gly Ser Phe Phe Leu
               420                 425                 430
   Tyr Ser Lys Leu Thr Val Asp Lys Ser Arg Trp Gln Gln Gly Asn Val
           435                 440                 445
   Phe Ser Cys Ser Val Met His Glu Ala Leu His Asn His Tyr Thr Gln
       450                 455                 460
   Lys Ser Leu Ser Leu Ser Pro Gly Gly Gly Gly Gly Ser Asn Lys Tyr
   465                 470                 475                 480
   Lys Asp Gly Asp Gln Cys Glu Thr Ser Pro Cys Gln Asn Gln Gly Lys
                   485                 490                 495
   Cys Lys Asp Gly Leu Gly Glu Tyr Thr Cys Thr Cys Leu Glu Gly Phe
               500                 505                 510
   Glu Gly Lys Asn Cys Glu Leu Phe Thr Arg Lys Leu Cys Ser Leu Asp
           515                 520                 525
   Asn Gly Asp Cys Asp Gln Phe Cys His Glu Glu Gln Asn Ser Val Val
       530                 535                 540
   Cys Ser Cys Ala Arg Gly Tyr Thr Leu Ala Asp Asn Gly Lys Ala Cys
   545                 550                 555                 560
   Ile Pro Thr Gly Pro Tyr Pro Cys Gly Lys Gln Thr Leu Glu Arg Arg
                   565                 570                 575
   Lys Arg Arg Lys Arg Ile Val Gly Gly Gln Glu Cys Lys Asp Gly Glu
               580                 585                 590
   Cys Pro Trp Gln Ala Leu Leu Ile Asn Glu Glu Asn Glu Gly Phe Cys
           595                 600                 605
   Gly Gly Thr Ile Leu Ser Glu Phe Tyr Ile Leu Thr Ala Ala His Cys
   610 615 620
   Leu Tyr Gln Ala Lys Arg Phe Lys Val Arg Val Gly Asp Arg Asn Thr
   625 630 635 640
   Glu Gln Glu Glu Gly Gly Glu Ala Val His Glu Val Glu Val Val Ile
   645 650 655
   Lys His Asn Arg Phe Thr Lys Glu Thr Tyr Asp Phe Asp Ile Ala Val
   660 665 670
   Leu Arg Leu Lys Thr Pro Ile Thr Phe Arg Met Asn Val Ala Pro Ala
   675 680 685
   Cys Leu Pro Glu Arg Asp Trp Ala Glu Ser Thr Leu Met Thr Gln Lys
   690 695 700
   Thr Gly Ile Val Ser Gly Phe Gly Arg Thr His Glu Lys Gly Arg Gln
   705 710 715 720
   Ser Thr Arg Leu Lys Met Leu Glu Val Pro Tyr Val Asp Arg Asn Ser
   725 730 735
   Cys Lys Leu Ser Ser Ser Phe Ile Ile Thr Gln Asn Met Phe Cys Ala
   740 745 750
   Gly Tyr Asp Thr Lys Gln Glu Asp Ala Cys Gln Gly Asp Ser Gly Gly
   755 760 765
   Pro His Val Thr Arg Phe Lys Asp Thr Tyr Phe Val Thr Gly Ile Val
   770 775 780
   Ser Trp Gly Glu Gly Cys Ala Arg Lys Gly Lys Tyr Gly Ile Tyr Thr
   785 790 795 800
   Lys Val Thr Ala Phe Leu Lys Trp Ile Asp Arg Ser Met Lys Thr Arg
   805 810 815
   Gly Leu Pro Lys Ala Lys Ser His Ala Pro Glu Val Ile Thr Ser Ser
   820 825 830
   Pro Leu Lys
   835
<210> 38
   <211> 832
   <212> PRT
   <213> Artificial Sequence
<220>
   <223> scFcL-GPAD1
<400> 38
   Asp Lys Thr His Thr Cys Pro Pro Cys Pro Ala Pro Glu Leu Leu Gly
   1 5 10 15
   Gly Pro Ser Val Phe Leu Phe Pro Pro Lys Pro Lys Asp Thr Leu Met
   20 25 30
   Ile Ser Arg Thr Pro Glu Val Thr Cys Val Val Val Asp Val Ser His
   35 40 45
   Glu Asp Pro Glu Val Lys Phe Asn Trp Tyr Val Asp Gly Val Glu Val
   50 55 60
   His Asn Ala Lys Thr Lys Pro Arg Glu Glu Gln Tyr Asn Ser Thr Tyr
   65 70 75 80
   Arg Val Val Ser Val Leu Thr Val Leu His Gln Asp Trp Leu Asn Gly
   85 90 95
   Lys Glu Tyr Lys Cys Lys Val Ser Asn Lys Ala Leu Pro Ala Pro Ile
   100 105 110
   Glu Lys Thr Ile Ser Lys Ala Lys Gly Gln Pro Arg Glu Pro Gln Val
   115 120 125
   Tyr Thr Leu Pro Pro Ser Arg Asp Glu Leu Thr Lys Asn Gln Val Ser
   130 135 140
   Leu Thr Cys Leu Val Lys Gly Phe Tyr Pro Ser Asp Ile Ala Val Glu
   145 150 155 160
   Trp Glu Ser Asn Gly Gln Pro Glu Asn Asn Tyr Lys Thr Thr Pro Pro
   165 170 175
   Val Leu Asp Ser Asp Gly Ser Phe Phe Leu Tyr Ser Lys Leu Thr Val
   180 185 190
   Asp Lys Ser Arg Trp Gln Gln Gly Asn Val Phe Ser Cys Ser Val Met
   195 200 205
   His Glu Ala Leu His Asn His Tyr Thr Gln Lys Ser Leu Ser Leu Ser
   210 215 220
   Pro Gly Gly Gly Gly Gly Ser Gly Gly Gly Gly Ser Gly Gly Gly Gly
   225 230 235 240
   Ser Glu Pro Lys Ser Ser Asp Lys Thr His Thr Cys Pro Pro Cys Pro
   245 250 255
   Ala Pro Glu Leu Leu Gly Gly Pro Ser Val Phe Leu Phe Pro Pro Lys
   260 265 270
   Pro Lys Asp Thr Leu Met Ile Ser Arg Thr Pro Glu Val Thr Cys Val
   275 280 285
   Val Val Asp Val Ser His Glu Asp Pro Glu Val Lys Phe Asn Trp Tyr
   290 295 300
   Val Asp Gly Val Glu Val His Asn Ala Lys Thr Lys Pro Arg Glu Glu
   305 310 315 320
   Gln Tyr Asn Ser Thr Tyr Arg Val Val Ser Val Leu Thr Val Leu His
   325 330 335
   Gln Asp Trp Leu Asn Gly Lys Glu Tyr Lys Cys Lys Val Ser Asn Lys
   340 345 350
   Ala Leu Pro Ala Pro Ile Glu Lys Thr Ile Ser Lys Ala Lys Gly Gln
   355 360 365
   Pro Arg Glu Pro Gln Val Tyr Thr Leu Pro Pro Ser Arg Asp Glu Leu
   370 375 380
   Thr Lys Asn Gln Val Ser Leu Thr Cys Leu Val Lys Gly Phe Tyr Pro
   385 390 395 400
   Ser Asp Ile Ala Val Glu Trp Glu Ser Asn Gly Gln Pro Glu Asn Asn
   405 410 415
   Tyr Lys Thr Thr Pro Pro Val Leu Asp Ser Asp Gly Ser Phe Phe Leu
   420 425 430
   Tyr Ser Lys Leu Thr Val Asp Lys Ser Arg Trp Gln Gln Gly Asn Val
   435 440 445
   Phe Ser Cys Ser Val Met His Glu Ala Leu His Asn His Tyr Thr Gln
   450 455 460
   Lys Ser Leu Ser Leu Ser Pro Gly Gly Gly Gly Gly Ser Asn Lys Tyr
   465 470 475 480
   Lys Asp Gly Asp Gln Cys Glu Thr Ser Pro Cys Gln Asn Gln Gly Lys
                   485                 490                 495
   Cys Lys Asp Gly Leu Gly Glu Tyr Thr Cys Thr Cys Leu Glu Gly Phe
               500                 505                 510
   Glu Gly Lys Asn Cys Glu Leu Phe Thr Arg Lys Leu Cys Ser Leu Asp
           515                 520                 525
   Asn Gly Asp Cys Asp Gln Phe Cys His Glu Glu Gln Asn Ser Val Val
       530                 535                 540
   Cys Ser Cys Ala Arg Gly Tyr Thr Leu Ala Asp Asn Gly Lys Ala Cys
   545                 550                 555                 560
   Ile Pro Thr Gly Pro Tyr Pro Cys Gly Lys Gln Thr Leu Glu Arg Arg
                   565                 570                 575
   Lys Arg Ile Val Gly Gly Gln Glu Cys Lys Asp Gly Glu Cys Pro Trp
               580                 585                 590
   Gln Ala Leu Leu Ile Asn Glu Glu Asn Glu Gly Phe Cys Gly Gly Thr
           595                 600                 605
   Ile Leu Ser Glu Phe Tyr Ile Leu Thr Ala Ala His Cys Leu Tyr Gln
       610                 615                 620
   Ala Lys Arg Phe Lys Val Arg Val Gly Asp Arg Asn Thr Glu Gln Glu
   625                 630                 635                 640
   Glu Gly Gly Glu Ala Val His Glu Val Glu Val Val Ile Lys His Asn
                   645                 650                 655
   Arg Phe Thr Lys Glu Thr Tyr Asp Phe Asp Ile Ala Val Leu Arg Leu
               660                 665                 670
   Lys Thr Pro Ile Thr Phe Arg Met Asn Val Ala Pro Ala Cys Leu Pro
           675                 680                 685
   Glu Arg Asp Trp Ala Glu Ser Thr Leu Met Thr Gln Lys Thr Gly Ile
       690                 695                 700
   Val Ser Gly Phe Gly Arg Thr His Glu Lys Gly Arg Gln Ser Thr Arg
   705                 710                 715                 720
   Leu Lys Met Leu Glu Val Pro Tyr Val Asp Arg Asn Ser Cys Lys Leu
                   725                 730                 735
   Ser Ser Ser Phe Ile Ile Thr Gln Asn Met Phe Cys Ala Gly Tyr Asp
   740 745 750
   Thr Lys Gln Glu Asp Ala Cys Gln Gly Asp Ser Gly Gly Pro His Val
   755 760 765
   Thr Arg Phe Lys Asp Thr Tyr Phe Val Thr Gly Ile Val Ser Trp Gly
   770 775 780
   Glu Gly Cys Ala Arg Lys Gly Lys Tyr Gly Ile Tyr Thr Lys Val Thr
   785 790 795 800
   Ala Phe Leu Lys Trp Ile Asp Arg Ser Met Lys Thr Arg Gly Leu Pro
   805 810 815
   Lys Ala Lys Ser His Ala Pro Glu Val Ile Thr Ser Ser Pro Leu Lys
   820 825 830
<210> 39
   <211> 226
   <212> PRT
   <213> Artificial Sequence
<220>
   <223> Fragment Fc, éventuellement suivi d'une lysine
<400> 39
   Asp Lys Thr His Thr Cys Pro Pro Cys Pro Ala Pro Glu Leu Leu Gly
   1 5 10 15
   Gly Pro Ser Val Phe Leu Phe Pro Pro Lys Pro Lys Asp Thr Leu Met
   20 25 30
   Ile Ser Arg Thr Pro Glu Val Thr Cys Val Val Val Asp Val Ser His
   35 40 45
   Glu Asp Pro Glu Val Lys Phe Asn Trp Tyr Val Asp Gly Val Glu Val
   50 55 60
   His Asn Ala Lys Thr Lys Pro Arg Glu Glu Gln Tyr Asn Ser Thr Tyr
   65 70 75 80
   Arg Val Val Ser Val Leu Thr Val Leu His Gln Asp Trp Leu Asn Gly
   85 90 95
   Lys Glu Tyr Lys Cys Lys Val Ser Asn Lys Ala Leu Pro Ala Pro Ile
   100 105 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly
   225
<210> 40
   <211> 1068
   <212> DNA
   <213> Artificial Sequence
<220>
   <223> Séquence nucléique codant GPAD1 (SEQ ID NO:21)
<400> 40
   agcaataaat acaaagatgg cgaccagtgt gagaccagtc cttgccagaa ccagggcaaa 60
   tgtaaagacg gcctcgggga atacacctgc acctgtttag aaggattcga aggcaaaaac 120
   tgtgaattat tcacacggaa gctctgcagc ctggacaacg gggactgtga ccagttctgc 180
   cacgaggaac agaactctgt ggtgtgctcc tgcgcccgcg ggtacaccct ggctgacaac 240
   ggcaaggcct gcattcccac agggccctac ccctgtggga aacagaccct ggaacgcagg 300
   aagaggatcg tgggaggcca ggaatgcaag gacggggagt gtccctggca ggccctgctc 360
   atcaatgagg aaaacgaggg tttctgtggt ggaaccattc tgagcgagtt ctacatccta 420
   acggcagccc actgtctcta ccaagccaag agattcaagg tgagggtagg ggaccggaac 480
   acggagcagg aggagggcgg tgaggcggtg cacgaggtgg aggtggtcat caagcacaac 540
   cggttcacaa aggagaccta tgacttcgac atcgccgtgc tccggctcaa gacccccatc 600
   accttccgca tgaacgtggc gcctgcctgc ctccccgagc gtgactgggc cgagtccacg 660
   ctgatgacgc agaagacggg gattgtgagc ggcttcgggc gcacccacga gaagggccgg 720
   cagtccacca ggctcaagat gctggaggtg ccctacgtgg accgcaacag ctgcaagctg 780
   tccagcagct tcatcatcac ccagaacatg ttctgtgccg gctacgacac caagcaggag 840
   gatgcctgcc agggggacag cgggggcccg cacgtcaccc gcttcaagga cacctacttc 900
   gtgacaggca tcgtcagctg gggagagggc tgtgcccgta aggggaagta cgggatctac 960
   accaaggtca ccgccttcct caagtggatc gacaggtcca tgaaaaccag gggcttgccc 1020
   aaggccaaga gccatgcccc ggaggtcata acgtcctctc cattaaag 1068
<210> 41
   <211> 1077
   <212> DNA
   <213> Artificial Sequence
<220>
   <223> Séquence nucléique codant GPAD2 (SEQ ID NO:22)
<400> 41
   agcaataaat acaaagatgg cgaccagtgt gagaccagtc cttgccagaa ccagggcaaa      60
   tgtaaagacg gcctcgggga atacacctgc acctgtttag aaggattcga aggcaaaaac     120
   tgtgaattat tcacacggaa gctctgcagc ctggacaacg gggactgtga ccagttctgc     180
   cacgaggaac agaactctgt ggtgtgctcc tgcgcccgcg ggtacaccct ggctgacaac     240
   ggcaaggcct gcattcccac agggccctac ccctgtggga aacagaccct ggaacgcagg     300
   aagaggagga agaggatcgt gggaggccag gaatgcaagg acggggagtg tccctggcag     360
   gccctgctca tcaatgagga aaacgagggt ttctgtggtg gaaccattct gagcgagttc     420
   tacatcctaa cggcagccca ctgtctctac caagccaaga gattcaaggt gagggtaggg     480
   gaccggaaca cggagcagga ggagggcggt gaggcggtgc acgaggtgga ggtggtcatc     540
   aagcacaacc ggttcacaaa ggagacctat gacttcgaca tcgccgtgct ccggctcaag     600
   acccccatca ccttccgcat gaacgtggcg cctgcctgcc tccccgagcg tgactgggcc     660
   gagtccacgc tgatgacgca gaagacgggg attgtgagcg gcttcgggcg cacccacgag     720
   aagggccggc agtccaccag gctcaagatg ctggaggtgc cctacgtgga ccgcaacagc     780
   tgcaagctgt ccagcagctt catcatcacc cagaacatgt tctgtgccgg ctacgacacc     840
   aagcaggagg atgcctgcca gggggacagc gggggcccgc acgtcacccg cttcaaggac     900
   acctacttcg tgacaggcat cgtcagctgg ggagagggct gtgcccgtaa ggggaagtac     960
   gggatctaca ccaaggtcac cgccttcctc aagtggatcg acaggtccat gaaaaccagg    1020
   ggcttgccca aggccaagag ccatgccccg gaggtcataa cgtcctctcc attaaag       1077
<210> 42
   <211> 1065
   <212> DNA
   <213> Artificial Sequence
<220>
   <223> Séquence nucléique codant GPAD1optimisé (SEQ ID NO:23)
<400> 42
   aacaagtata aagacggaga ccagtgtgag actagccctt gccagaacca ggggaagtgt      60
   aaagatggac tgggcgagta cacctgcaca tgtctggagg gattcgaagg caagaattgc     120
   gaactgttta ctagaaaact gtgtagcctg gataacggcg actgcgatca gttctgtcac     180
   gaggaacaga attcagtggt ctgcagctgt gccaggggat acaccctggc cgacaacggc     240
   aaggcttgca tccctaccgg cccctatcct tgtgggaaac agacactgga gcggagaaag     300
   aggatcgtgg gcgggcagga gtgcaaggat ggagaatgtc catggcaggc cctgctgatt     360
   aacgaggaaa atgagggctt ctgcggaggc actatcctgt ccgaatttta cattctgacc     420
   gccgctcatt gtctgtatca ggccaagcgg ttcaaggtgc gggtcggcga cagaaacaca     480
   gagcaggagg aaggggagaa agctgtgcac gaggtcgaag tggtcatcaa gcataatcgc     540
   ttcactaaag agacctacga ctttgatatc gcagtgctga gactgaagac accaattact     600
   ttcaggatga atgtcgcacc agcatgcctg ccagagcgag attgggctga atccaccctg     660
   atgacacaga aaactggcat tgtgtctggg tttggacgga cacacgagaa ggggaggcag     720
   agcactcgcc tgaaaatgct ggaagtgccc tacgtcgaca ggaactcctg taagctgagc     780
   tcctctttca tcattacaca gaatatgttt tgcgcagggt atgacactaa gcaggaggat     840
   gcctgtcagg gagactctgg aggacctcac gtgacccgct tcaaagatac ttattttgtg     900
   accggaatcg tcagttgggg agagggatgc gctcgaaagg ggaaatacgg aatctatacc     960
   aaggtgacag cattcctgaa atggattgac cgaagtatga agacccgggg cctgcctaag    1020
   gctaaatcac atgcaccaga agtgatcaca agttcacccc tgaag                    1065
<210> 43
   <211> 1074
   <212> DNA
   <213> Artificial Sequence
<220>
   <223> Séquence nucléique codant GPAD2 optimisé (SEQ ID NO:24)
<400> 43
   aacaagtata aagacggaga ccagtgtgag actagccctt gccagaacca ggggaagtgt      60
   aaagatggac tgggcgagta cacctgcaca tgtctggagg gattcgaagg caagaattgc     120
   gaactgttta ctagaaaact gtgtagcctg gataacggcg actgcgatca gttctgtcac     180
   gaggaacaga attcagtggt ctgcagctgt gccaggggat acaccctggc cgacaacggc     240
   aaggcttgca tccctaccgg cccctatcct tgtgggaaac agacactgga gcggagaaag     300
   aggcgcaaac ggatcgtggg cgggcaggag tgcaaggatg gagaatgtcc atggcaggcc      360
   ctgctgatta acgaggaaaa tgagggcttc tgcggaggca ctatcctgtc cgaattttac      420
   attctgaccg ccgctcattg tctgtatcag gccaagcggt tcaaggtgcg ggtcggcgac      480
   agaaacacag agcaggagga agggggagaa gctgtgcacg aggtcgaagt ggtcatcaag      540
   cataatcgct tcactaaaga gacctacgac tttgatatcg cagtgctgag actgaagaca      600
   ccaattactt tcaggatgaa tgtcgcacca gcatgcctgc cagagcgaga ttgggctgaa      660
   tccaccctga tgacacagaa aactggcatt gtgtctgggt ttggacggac acacgagaag      720
   gggaggcaga gcactcgcct gaaaatgctg gaagtgccct acgtcgacag gaactcctgt      780
   aagctgagct cctctttcat cattacacag aatatgtttt gcgcagggta tgacactaag      840
   caggaggatg cctgtcaggg agactctgga ggacctcacg tgacccgctt caaagatact      900
   tattttgtga ccggaatcgt cagttgggga gagggatgcg ctcgaaaggg gaaatacgga      960
   atctatacca aggtgacagc attcctgaaa tggattgacc gaagtatgaa gacccggggc     1020
   ctgcctaagg ctaaatcaca tgcaccagaa gtgatcacaa gttcacccct gaag           1074
<210> 44
   <211> 762
   <212> DNA
   <213> Artificial Sequence
<220>
   <223> Séquence nucléique codant SEQ ID NO:31
<400> 44
   atcgtgggcg ggcaggagtg caaggatgga gaatgtccat ggcaggccct gctgattaac        60
   gaggaaaatg agggcttctg cggaggcact atcctgtccg aattttacat tctgaccgcc       120
   gctcattgtc tgtatcaggc caagcggttc aaggtgcggg tcggcgacag aaacacagag       180
   caggaggaag ggggagaagc tgtgcacgag gtcgaagtgg tcatcaagca taatcgcttc        240
   actaaagaga cctacgactt tgatatcgca gtgctgagac tgaagacacc aattactttc       300
   aggatgaatg tcgcaccagc atgcctgcca gagcgagatt gggctgaatc caccctgatg       360
   acacagaaaa ctggcattgt gtctgggttt ggacggacac acgagaaggg gaggcagagc       420
   actcgcctga aaatgctgga agtgccctac gtcgacagga actcctgtaa gctgagctcc       480
   tctttcatca ttacacagaa tatgttttgc gcagggtatg acactaagca ggaggatgcc       540
   tgtcagggag actctggagg acctcacgtg acccgcttca aagatactta ttttgtgacc       600
   ggaatcgtca gttggggaga gggatgcgct cgaaagggga aatacggaat ctataccaag       660
   gtgacagcat tcctgaaatg gattgaccga agtatgaaga cccggggcct gcctaaggct       720
   aaatcacatg caccagaagt gatcacaagt tcacccctga ag                          762
<210> 45
   <211> 1758
   <212> DNA
   <213> Artificial Sequence
<220>
   <223> Séquence nucléique codant GPAD2-FX-Fc (SEQ ID NO:32)
<400> 45
   agcaataaat acaaagatgg cgaccagtgt gagaccagtc cttgccagaa ccagggcaaa 60
   tgtaaagacg gcctcgggga atacacctgc acctgtttag aaggattcga aggcaaaaac 120
   tgtgaattat tcacacggaa gctctgcagc ctggacaacg gggactgtga ccagttctgc 180
   cacgaggaac agaactctgt ggtgtgctcc tgcgcccgcg ggtacaccct ggctgacaac 240
   ggcaaggcct gcattcccac agggccctac ccctgtggga aacagaccct ggaacgcagg 300
   aagaggagga agaggatcgt gggaggccag gaatgcaagg acggggagtg tccctggcag 360
   gccctgctca tcaatgagga aaacgagggt ttctgtggtg gaaccattct gagcgagttc 420
   tacatcctaa cggcagccca ctgtctctac caagccaaga gattcaaggt gagggtaggg 480
   gaccggaaca cggagcagga ggagggcggt gaggcggtgc acgaggtgga ggtggtcatc 540
   aagcacaacc ggttcacaaa ggagacctat gacttcgaca tcgccgtgct ccggctcaag 600
   acccccatca ccttccgcat gaacgtggcg cctgcctgcc tccccgagcg tgactgggcc 660
   gagtccacgc tgatgacgca gaagacgggg attgtgagcg gcttcgggcg cacccacgag 720
   aagggccggc agtccaccag gctcaagatg ctggaggtgc cctacgtgga ccgcaacagc 780
   tgcaagctgt ccagcagctt catcatcacc cagaacatgt tctgtgccgg ctacgacacc 840
   aagcaggagg atgcctgcca gggggacagc gggggcccgc acgtcacccg cttcaaggac 900
   acctacttcg tgacaggcat cgtcagctgg ggagagggct gtgcccgtaa ggggaagtac 960
   gggatctaca ccaaggtcac cgccttcctc aagtggatcg acaggtccat gaaaaccagg 1020
   ggcttgccca aggccaagag ccatgccccg gaggtcataa cgtcctctcc attaaaggac 1080
   aagacacaca catgccctcc ttgtccagcc cctgagctgc tgggcggccc ctccgtgttc 1140
   ctgttccccc ccaagcctaa ggataccctg atgatcagca gaacccccga ggtgacctgc 1200
   gtggtggtgg acgtgtccca cgaggatccc gaggtgaagt tcaactggta cgtggacggc 1260
   gtggaggtgc acaacgctaa gaccaagccc agagaggagc agtacaacag cacatacaga 1320
   gtggtgtctg tgctgaccgt gctgcaccag gactggctga acgggaagga gtacaagtgc 1380
   aaggtgtcca acaaggccct gcctgcccct atcgagaaga ccatctctaa ggctaagggg 1440
   cagccccggg agccacaggt gtacaccctg ccacccagcc gcgacgagct gaccaagaac 1500
   caggtgtccc tgacatgcct ggtgaaggga ttctacccca gcgacatcgc cgtggagtgg 1560
   gagagcaacg gccagcccga gaacaactac aagacaaccc ctcccgtgct ggacagcgat 1620
   ggatccttct tcctgtactc caagctgacc gtggacaaga gcaggtggca gcagggaaac 1680
   gtgttctctt gttccgtgat gcacgaggct ctgcacaacc actacaccca gaagtccctg 1740
   agcctgtctc caggcaag 1758
<210> 46
   <211> 1773
   <212> DNA
   <213> Artificial Sequence
<220>
   <223> Séquence nucléique codant GPAD2-Fcl (SEQ ID NO:33)
<400> 46
   agcaataagt ataaagacgg ggatcagtgc gagacctccc catgtcagaa ccagggcaag      60
   tgcaaagacg ggctgggaga gtacacatgc acttgtctgg aggggttcga aggaaagaat     120
   tgcgaactgt ttacaagaaa gctgtgcagc ctggataacg gcgactgcga tcagttctgt     180
   cacgaggaac agaatagtgt ggtctgctca tgtgccaggg ggtacactct ggctgacaac     240
   ggaaaggcat gcatccctac tggaccttat ccatgtggca aacagaccct ggagcggaga     300
   aagaggcgca aacgcatcgt gggcgggcag gagtgcaagg atggagaatg tccatggcag     360
   gctctgctga ttaacgagga aaatgagggc ttctgcggag gcacaatcct gagcgagttc     420
   tacattctga ctgccgctca ttgtctgtat caggctaagc ggttcaaggt gcgggtcggc     480
   gacagaaaca ccgagcagga ggaaggggga gaagcagtgc acgaggtcga agtggtcatc     540
   aagcataatc gcttcacaaa agagacttac gactttgata tcgccgtgct gagactgaag     600
   acccccatta cattcaggat gaatgtggca ccagcatgcc tgcctgagcg agattgggct     660
   gaatcaactc tgatgaccca gaaaacagga attgtgagcg gctttgggcg aactcacgag     720
   aagggcaggc agagcacccg cctgaaaatg ctggaagtgc cctacgtcga ccggaacagc     780
   tgtaagctga gctcctcttt catcattacc cagaatatgt tttgcgccgg ctatgacaca     840
   aagcaggagg atgcttgtca gggggactcc ggaggacctc atgtgaccag attcaaagat     900
   acatattttg tgactggcat cgtctcttgg ggagaaggct gcgccaggaa gggcaaatac     960
   gggatctata ctaaggtgac cgccttcctg aaatggattg atcgatccat gaagactcgg    1020
   ggcctgccaa aggcaaaatc tcacgccccc gaagtgatca ccagttcacc tctgaaggaa    1080
   cctaagtctt gcgacaaaac ccatacatgc ccaccttgtc cagcacctga actgctggga    1140
   ggaccatccg tgttcctgtt tccacccaag cccaaagata cactgatgat tagtcggacc    1200
   cctgaggtga catgcgtggt cgtggatgtc tcacacgagg acccagaagt gaagtttaac    1260
   tggtacgtgg acggcgtgga agtccataat gccaagacca aacctcgcga ggaacagtac    1320
   aacagtacat atcgagtcgt gtcagtgctg actgtcctgc accaggattg gctgaacgga    1380
   aaggagtata agtgcaaagt gagcaataag gctctgccag cacccatcga gaaaacaatt    1440
   tccaaggcaa aaggccagcc aagggaaccc caggtgtaca ctctgcctcc aagccgcgat     1500
   gagctgacaa agaaccaggt gtccctgact tgtctggtca aagggttcta tccctccgac     1560
   atcgccgtgg agtgggaatc taatggacag cctgagaaca attacaagac cacaccccct     1620
   gtgctggact cagatgggag cttctttctg tattctaagc tgactgtgga caaaagtaga     1680
   tggcagcagg gaaacgtgtt ttcttgcagt gtcatgcacg aggccctgca caatcattac     1740
   acccagaagt cactgagcct gtccccagga aag                                  1773
<210> 47
   <211> 1770
   <212> DNA
   <213> Artificial Sequence
<220>
   <223> Séquence nucléique codant GPAD2-FcLss (SEQ ID NO:34)
<400> 47
   aataagtata aagacgggga tcagtgcgag acctctcctt gtcagaacca gggcaagtgc 60
   aaagacgggc tgggagagta cacatgcact tgtctggagg ggttcgaagg aaagaattgc 120
   gaactgttta caagaaaact gtgtagcctg gataacgggg actgcgatca gttctgtcac 180
   gaggaacaga attccgtggt ctgctcttgt gcaagggggt acaccctggc tgacaacgga 240
   aaggcatgca tccctactgg accttatcca tgtggcaaac agaccctgga gcggagaaag 300
   aggcgcaaac gcatcgtggg cgggcaggag tgcaaggatg gagaatgtcc atggcaggct 360
   ctgctgatta acgaggaaaa tgagggcttc tgcggaggca caatcctgag cgaattttac 420
   attctgactg ccgctcattg tctgtatcag gctaagcggt tcaaggtgcg ggtcggcgac 480
   agaaacaccg agcaggagga agggggagaa gcagtgcacg aggtcgaagt ggtcatcaag 540
   cataatcgct tcacaaaaga gacttacgac tttgatatcg ccgtgctgag actgaagacc 600
   cccattacat tcaggatgaa tgtggcacca gcatgcctgc ctgagcgaga ttgggctgaa 660
   tctactctga tgacccagaa aacaggaatt gtgagtggct ttgggcgaac tcacgagaag 720
   ggcaggcagt ctacccgcct gaaaatgctg gaagtgccct acgtcgaccg gaacagctgt 780
   aagctgagct cctctttcat cattacccag aatatgtttt gcgccggcta tgacacaaag 840
   caggaggatg cttgtcaggg ggacagcgga ggacctcatg tgaccagatt caaagataca 900
   tattttgtga ctggcatcgt ctcctgggga gaaggctgcg caaggaaggg caaatacggg 960
   atctatacta aggtgaccgc cttcctgaaa tggattgatc gatcaatgaa gactcggggc 1020
   ctgccaaagg caaaaagcca cgcccccgaa gtgatcacca gttcacctct gaaggaacca 1080
   aagagctgcg acaaaaccca tacatgccca ccttgtccag cacctgaact gctgggagga 1140
   ccatccgtgt tcctgtttcc acccaagccc aaagatacac tgatgatttc ccggacccct 1200
   gaggtgacat gtgtggtcgt ggatgtctct cacgaggacc cagaagtgaa gtttaactgg 1260
   tacgtggacg gcgtggaagt ccataatgcc aagaccaaac cccgcgagga acagtacaac 1320
   tccacatatc gagtcgtgtc tgtgctgact gtcctgcacc aggattggct gaacggaaaa 1380
   gagtacaagt gcaaagtgag taataaggct ctgccagcac ccatcgagaa aacaatttcc 1440
   aaggcaaaag gccagccaag ggaaccccag gtgtacactc tgcctccaag tcgcgatgag 1500
   ctgacaaaga accaggtgtc actgacttgt ctggtcaaag ggttctatcc ctcagacatc 1560
   gccgtggagt gggaaagcaa tggacagcct gagaacaatt acaagaccac accccctgtg 1620
   ctggactctg atgggagttt ctttctgtat agcaagctga ctgtggacaa atccagatgg 1680
   cagcagggaa acgtgttttc ttgcagtgtc atgcacgagg ccctgcacaa tcattacacc 1740
   cagaagtcac tgagcctgtc cccaggaaag 1770
<210> 48
   <211> 2493
   <212> DNA
   <213> Artificial Sequence
<220>
   <223> Séquence nucléique codant GPAD2-scFcL (SEQ ID NO:35)
<400> 48
   agcaataagt ataaagacgg ggatcagtgc gagacctccc catgtcagaa ccagggcaag      60
   tgcaaagacg ggctgggaga gtacacatgc acttgtctgg aggggttcga aggaaagaat     120
   tgcgaactgt ttacaagaaa gctgtgcagc ctggataacg gcgactgcga tcagttctgt     180
   cacgaggaac agaatagtgt ggtctgctca tgtgccaggg ggtacactct ggctgacaac     240
   ggaaaggcat gcatccctac tggaccttat ccatgtggca aacagaccct ggagcggaga     300
   aagaggcgca aacgcatcgt gggcgggcag gagtgcaagg atggagaatg tccatggcag     360
   gctctgctga ttaacgagga aaatgagggc ttctgcggag gcacaatcct gagcgagttc     420
   tacattctga ctgccgctca ttgtctgtat caggctaagc ggttcaaggt gcgggtcggc     480
   gacagaaaca ccgagcagga ggaaggggga gaagcagtgc acgaggtcga agtggtcatc     540
   aagcataatc gcttcacaaa agagacttac gactttgata tcgccgtgct gagactgaag     600
   acccccatta cattcaggat gaatgtggca ccagcatgcc tgcctgagcg agattgggct     660
   gaatcaactc tgatgaccca gaaaacagga attgtgagcg gctttgggcg aactcacgag     720
   aagggcaggc agagcacccg cctgaaaatg ctggaagtgc cctacgtcga ccggaacagc     780
   tgtaagctga gctcctcttt catcattacc cagaatatgt tttgcgccgg ctatgacaca     840
   aagcaggagg atgcttgtca gggggactcc ggaggacctc atgtgaccag attcaaagat     900
   acatattttg tgactggcat cgtctcttgg ggagaaggct gcgccaggaa gggcaaatac     960
   gggatctata ctaaggtgac cgccttcctg aaatggattg atcgatccat gaagactcgg    1020
   ggcctgccaa aggcaaaatc tcacgccccc gaagtgatca ccagttcacc tctgaaggac      1080
   aaaacccata catgcccacc ttgtccagca cctgaactgc tgggaggacc atccgtgttc      1140
   ctgtttccac ccaagcccaa agatacactg atgattagtc ggacccctga ggtgacatgc      1200
   gtggtcgtgg atgtctcaca cgaggaccca gaagtgaagt ttaactggta cgtggacggc      1260
   gtggaagtcc ataatgccaa gaccaaacct cgcgaggaac agtacaacag tacatatcga      1320
   gtcgtgtcag tgctgactgt cctgcaccag gattggctga acggaaagga gtataagtgc      1380
   aaagtgagca ataaggctct gccagcaccc atcgagaaaa caatttccaa ggcaaaaggc      1440
   cagccaaggg aaccccaggt gtacactctg cctccaagcc gcgatgagct gacaaagaac      1500
   caggtgtccc tgacttgtct ggtcaaaggg ttctatccct ccgacatcgc cgtggagtgg      1560
   gaatctaatg gacagcctga gaacaattac aagaccacac cccctgtgct ggactcagat      1620
   gggagcttct ttctgtattc taagctgact gtggacaaaa gtagatggca gcagggaaac      1680
   gtgttttctt gcagtgtcat gcacgaggcc ctgcacaatc attacaccca gaagtcactg      1740
   agcctgtccc caggaggagg aggaggaagc ggaggaggag gctccggggg aggcgggtct      1800
   gagcccaaga gctccgataa aactcatacc tgcccaccct gtcctgctcc agaactgctg      1860
   ggaggcccta gcgtgttcct gtttcctcca aagccaaaag acacactgat gatttctagg      1920
   actcccgagg tgacctgcgt ggtggtcgat gtcagtcacg aggaccctga agtgaagttc      1980
   aactggtacg tggatggagt cgaggtgcac aacgccaaga ccaaaccccg ggaggaacag      2040
   tacaacagca cctatagagt ggtctccgtg ctgacagtcc tgcaccagga ctggctgaac      2100
   gggaaggaat acaagtgcaa agtgtccaat aaggccctgc ccgctcctat cgaaaaaacc      2160
   atttctaagg ctaaaggcca gccccgggag ccacaggtgt acacactgcc cccttctcgg      2220
   gatgaactga ccaagaacca ggtgagtctg acatgtctgg tcaaaggctt ctatccaagt      2280
   gacatcgcag tggagtggga atcaaatggg cagcccgaga acaattacaa gactacccca      2340
   cccgtgctgg actccgatgg ctctttcttt ctgtattcaa agctgaccgt ggacaaaagc      2400
   agatggcagc aggggaacgt gttcagctgc agtgtcatgc acgaagcact gcacaatcat      2460
   tacactcaga aatcactgtc actgtcacct gga                                   2493
<210> 49
   <211> 2463
   <212> DNA
   <213> Artificial Sequence
<220>
   <223> Séquence nucléique codant GPAD2-scFcS (SEQ ID NO:36)
<400> 49
   agcaataagt ataaagacgg ggatcagtgc gagacctccc catgtcagaa ccagggcaag 60
   tgcaaagacg ggctgggaga gtacacatgc acttgtctgg aggggttcga aggaaagaat 120
   tgcgaactgt ttacaagaaa gctgtgcagc ctggataacg gcgactgcga tcagttctgt 180
   cacgaggaac agaatagtgt ggtctgctca tgtgccaggg ggtacactct ggctgacaac 240
   ggaaaggcat gcatccctac tggaccttat ccatgtggca aacagaccct ggagcggaga 300
   aagaggcgca aacgcatcgt gggcgggcag gagtgcaagg atggagaatg tccatggcag 360
   gctctgctga ttaacgagga aaatgagggc ttctgcggag gcacaatcct gagcgagttc 420
   tacattctga ctgccgctca ttgtctgtat caggctaagc ggttcaaggt gcgggtcggc 480
   gacagaaaca ccgagcagga ggaaggggga gaagcagtgc acgaggtcga agtggtcatc 540
   aagcataatc gcttcacaaa agagacttac gactttgata tcgccgtgct gagactgaag 600
   acccccatta cattcaggat gaatgtggca ccagcatgcc tgcctgagcg agattgggct 660
   gaatcaactc tgatgaccca gaaaacagga attgtgagcg gctttgggcg aactcacgag 720
   aagggcaggc agagcacccg cctgaaaatg ctggaagtgc cctacgtcga ccggaacagc 780
   tgtaagctga gctcctcttt catcattacc cagaatatgt tttgcgccgg ctatgacaca 840
   aagcaggagg atgcttgtca gggggactcc ggaggacctc atgtgaccag attcaaagat 900
   acatattttg tgactggcat cgtctcttgg ggagaaggct gcgccaggaa gggcaaatac 960
   gggatctata ctaaggtgac cgccttcctg aaatggattg atcgatccat gaagactcgg 1020
   ggcctgccaa aggcaaaatc tcacgccccc gaagtgatca ccagttcacc tctgaaggac 1080
   aaaacccata catgcccacc ttgtccagca cctgaactgc tgggaggacc atccgtgttc 1140
   ctgtttccac ccaagcccaa agatacactg atgattagtc ggacccctga ggtgacatgc 1200
   gtggtcgtgg atgtctcaca cgaggaccca gaagtgaagt ttaactggta cgtggacggc 1260
   gtggaagtcc ataatgccaa gaccaaacct cgcgaggaac agtacaacag tacatatcga 1320
   gtcgtgtcag tgctgactgt cctgcaccag gattggctga acggaaagga gtataagtgc 1380
   aaagtgagca ataaggctct gccagcaccc atcgagaaaa caatttccaa ggcaaaaggc 1440
   cagccaaggg aaccccaggt gtacactctg cctccaagcc gcgatgagct gacaaagaac 1500
   caggtgtccc tgacttgtct ggtcaaaggg ttctatccct ccgacatcgc cgtggagtgg 1560
   gaatctaatg gacagcctga gaacaattac aagaccacac cccctgtgct ggactcagat 1620
   gggagcttct ttctgtattc taagctgact gtggacaaaa gtagatggca gcagggaaac 1680
   gtgttttctt gcagtgtcat gcacgaggcc ctgcacaatc attacaccca gaagtcactg 1740
   agcctgtccc caggaggagg aggaggaagc gagcccaaga gctccgataa aactcatacc 1800
   tgcccaccct gtcctgctcc agaactgctg ggaggcccta gcgtgttcct gtttcctcca 1860
   aagccaaaag acacactgat gatttctagg actcccgagg tgacctgcgt ggtggtcgat 1920
   gtcagtcacg aggaccctga agtgaagttc aactggtacg tggatggagt cgaggtgcac 1980
   aacgccaaga ccaaaccccg ggaggaacag tacaacagca cctatagagt ggtctccgtg 2040
   ctgacagtcc tgcaccagga ctggctgaac gggaaggaat acaagtgcaa agtgtccaat 2100
   aaggccctgc ccgctcctat cgaaaaaacc atttctaagg ctaaaggcca gccccgggag 2160
   ccacaggtgt acacactgcc cccttctcgg gatgaactga ccaagaacca ggtgagtctg 2220
   acatgtctgg tcaaaggctt ctatccaagt gacatcgcag tggagtggga atcaaatggg 2280
   cagcccgaga acaattacaa gactacccca cccgtgctgg actccgatgg ctctttcttt 2340
   ctgtattcaa agctgaccgt ggacaaaagc agatggcagc aggggaacgt gttcagctgc 2400
   agtgtcatgc acgaagcact gcacaatcat tacactcaga aatcactgtc actgtcacct 2460
   gga 2463
<210> 50
   <211> 2505
   <212> DNA
   <213> Artificial Sequence
<220>
   <223> Séquence nucléique codant scFcL-GPAD2 (SEQ ID NO:37)
<400> 50
   gataagacac acacttgccc accctgccct gcccctgagc tgctgggcgg gccaagcgtg      60
   ttcctgtttc cccctaagcc aaaagataca ctgatgatca gtagaactcc cgaagtgacc     120
   tgcgtggtcg tggacgtctc acacgaggac cccgaagtga agttcaactg gtacgtggac     180
   ggcgtggagg tccataatgc caagaccaaa ccccgcgagg aacagtacaa ctccacctat     240
   cgagtcgtgt ctgtgctgac agtcctgcac caggattggc tgaacggcaa ggagtataag     300
   tgcaaagtgt ctaataaggc tctgccagca cccatcgaga aaaccattag taaggcaaaa     360
   gggcagccta gggaaccaca ggtgtacaca ctgccaccca gtcgcgatga gctgactaag     420
   aaccaggtgt cactgacctg tctggtcaaa ggattctatc cttcagacat cgccgtggag     480
   tgggaaagca atggccagcc agagaacaat tacaagacca cacctccagt gctggactct     540
   gatggaagtt tctttctgta tagcaagctg actgtggaca aatccagatg gcagcagggc     600
   aacgtgtttt cttgcagtgt catgcacgag gccctgcaca atcattacac ccagaagtca     660
   ctgagcctgt cccctggagg aggaggaggc agtggaggag gagggtcagg aggcggggga     720
   agcgagccaa agagctccga taaaacacat acttgccccc cttgtcctgc tccagaactg     780
   ctgggaggac cttccgtgtt cctgtttcca cccaagccta aagacacact gatgatttcc     840
   aggacaccag aagtgacttg tgtcgtggtc gacgtgtctc atgaggaccc cgaggtgaag     900
   tttaactggt acgtggatgg agtcgaagtg cacaacgcca agaccaaacc ccgggaggaa     960
   cagtacaata gtacttatag agtggtctca gtgctgaccg tcctgcacca ggactggctg    1020
   aatggcaagg aatataagtg caaagtgagc aataaggccc tgcccgctcc tatcgaaaaa 1080
   actatttcca aggctaaagg ccagccccga gagcctcagg tgtacaccct gcctccaagc 1140
   cgggatgaac tgacaaagaa ccaggtgtcc ctgacttgtc tggtcaaagg gttctatccc 1200
   tccgacatcg cagtggagtg ggaatctaat ggacagcctg agaacaatta caagactacc 1260
   ccccctgtgc tggactcaga tgggagcttc tttctgtatt ctaagctgac cgtggataaa 1320
   agtcgctggc agcagggaaa tgtgttttct tgtagtgtca tgcacgaagc cctgcataac 1380
   cactatactc aaaagtcact gagcctgagc ccaggaggag gaggaggctc caacaagtat 1440
   aaagacgggg atcagtgcga gacatctcct tgtcagaatc agggaaagtg taaagacggc 1500
   ctgggggagt acacctgcac atgtctggag ggcttcgaag ggaagaactg cgaactgttt 1560
   acaagaaaac tgtgtagcct ggataacggc gactgcgatc agttctgtca tgaggaacag 1620
   aattccgtgg tctgctcttg tgccaggggc tacaccctgg ctgacaatgg gaaggcatgc 1680
   atccctaccg ggccatatcc ctgtggaaaa cagacactgg agcggagaaa gaggcgcaaa 1740
   cggatcgtgg ggggacagga gtgcaaggat ggcgaatgtc catggcaggc tctgctgatt 1800
   aacgaggaaa atgaggggtt ctgcggcggg actatcctgt ccgaatttta cattctgacc 1860
   gccgctcact gtctgtatca ggctaagcgg ttcaaggtgc gggtcggcga cagaaacacc 1920
   gagcaggagg aaggaggcga agcagtgcac gaggtcgaag tggtcatcaa gcataatcgc 1980
   ttcactaaag agacctacga ctttgatatc gccgtgctga gactgaagac acccattact 2040
   ttcaggatga acgtggcacc agcatgcctg ccagagcgag attgggctga atctaccctg 2100
   atgacacaga aaactggcat tgtgagtgga tttggccgaa cacatgagaa ggggaggcag 2160
   tctactcgcc tgaaaatgct ggaagtgccc tacgtcgacc ggaactcctg taagctgtct 2220
   agttcattca tcatcacaca gaacatgttt tgcgccggat atgacactaa gcaggaagat 2280
   gcttgtcagg gcgacagcgg aggacctcac gtgaccagat tcaaagatac ctattttgtg 2340
   acaggcatcg tctcctgggg ggagggatgc gcaaggaagg gaaaatacgg catctatacc 2400
   aaggtgacag ccttcctgaa atggattgac cgatcaatga agacacgggg cctgcccaag 2460
   gcaaaaagcc atgcccctga agtgatcact agctccccac tgaaa 2505
<210> 51
   <211> 2496
   <212> DNA
   <213> Artificial Sequence
<220>
   <223> Séquence nucléique codant scFcL-GPAD1 (SEQ ID NO:38)
<400> 51
   gataagacac acacttgccc accctgccct gcccctgagc tgctgggcgg gccaagcgtg        60
   ttcctgtttc cccctaagcc aaaagataca ctgatgatca gtagaactcc cgaagtgacc       120
   tgcgtggtcg tggacgtctc acacgaggac cccgaagtga agttcaactg gtacgtggac 180
   ggcgtggagg tccataatgc caagaccaaa ccccgcgagg aacagtacaa ctccacctat 240
   cgagtcgtgt ctgtgctgac agtcctgcac caggattggc tgaacggcaa ggagtataag 300
   tgcaaagtgt ctaataaggc tctgccagca cccatcgaga aaaccattag taaggcaaaa 360
   gggcagccta gggaaccaca ggtgtacaca ctgccaccca gtcgcgatga gctgactaag 420
   aaccaggtgt cactgacctg tctggtcaaa ggattctatc cttcagacat cgccgtggag 480
   tgggaaagca atggccagcc agagaacaat tacaagacca cacctccagt gctggactct 540
   gatggaagtt tctttctgta tagcaagctg actgtggaca aatccagatg gcagcagggc 600
   aacgtgtttt cttgcagtgt catgcacgag gccctgcaca atcattacac ccagaagtca 660
   ctgagcctgt cccctggagg aggaggaggc agtggaggag gagggtcagg aggcggggga 720
   agcgagccaa agagctccga taaaacacat acttgccccc cttgtcctgc tccagaactg 780
   ctgggaggac cttccgtgtt cctgtttcca cccaagccta aagacacact gatgatttcc 840
   aggacaccag aagtgacttg tgtcgtggtc gacgtgtctc atgaggaccc cgaggtgaag 900
   tttaactggt acgtggatgg agtcgaagtg cacaacgcca agaccaaacc ccgggaggaa 960
   cagtacaata gtacttatag agtggtctca gtgctgaccg tcctgcacca ggactggctg 1020
   aatggcaagg aatataagtg caaagtgagc aataaggccc tgcccgctcc tatcgaaaaa 1080
   actatttcca aggctaaagg ccagccccga gagcctcagg tgtacaccct gcctccaagc 1140
   cgggatgaac tgacaaagaa ccaggtgtcc ctgacttgtc tggtcaaagg gttctatccc 1200
   tccgacatcg cagtggagtg ggaatctaat ggacagcctg agaacaatta caagactacc 1260
   ccccctgtgc tggactcaga tgggagcttc tttctgtatt ctaagctgac cgtggataaa 1320
   agtcgctggc agcagggaaa tgtgttttct tgtagtgtca tgcacgaagc cctgcataac 1380
   cactatactc aaaagtcact gagcctgagc ccaggaggag gaggaggctc caacaagtat 1440
   aaagacgggg atcagtgcga gacatctcct tgtcagaatc agggaaagtg taaagacggc 1500
   ctgggggagt acacctgcac atgtctggag ggcttcgaag ggaagaactg cgaactgttt 1560
   acaagaaaac tgtgtagcct ggataacggc gactgcgatc agttctgtca tgaggaacag 1620
   aattccgtgg tctgctcttg tgccaggggc tacaccctgg ctgacaatgg gaaggcatgc 1680
   atccctaccg ggccatatcc ctgtggaaaa cagacactgg agcggagaaa gaggatcgtg 1740
   gggggacagg agtgcaagga tggcgaatgt ccatggcagg ctctgctgat taacgaggaa 1800
   aatgaggggt tctgcggcgg gactatcctg tccgaatttt acattctgac cgccgctcac 1860
   tgtctgtatc aggctaagcg gttcaaggtg cgggtcggcg acagaaacac cgagcaggag 1920
   gaaggaggcg aagcagtgca cgaggtcgaa gtggtcatca agcataatcg cttcactaaa 1980
   gagacctacg actttgatat cgccgtgctg agactgaaga cacccattac tttcaggatg     2040
   aacgtggcac cagcatgcct gccagagcga gattgggctg aatctaccct gatgacacag     2100
   aaaactggca ttgtgagtgg atttggccga acacatgaga aggggaggca gtctactcgc     2160
   ctgaaaatgc tggaagtgcc ctacgtcgac cggaactcct gtaagctgtc tagttcattc     2220
   atcatcacac agaacatgtt ttgcgccgga tatgacacta agcaggaaga tgcttgtcag     2280
   ggcgacagcg gaggacctca cgtgaccaga ttcaaagata cctattttgt gacaggcatc     2340
   gtctcctggg gggagggatg cgcaaggaag ggaaaatacg gcatctatac caaggtgaca     2400
   gccttcctga aatggattga ccgatcaatg aagacacggg gcctgcccaa ggcaaaaagc     2460
   catgcccctg aagtgatcac tagctcccca ctgaaa                               2496
<210> 52
   <211> 364
   <212> PRT
   <213> Artificial Sequence
<220>
   <223> Exemple de GPAD3
<400> 52
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Arg Gly Gly Gly Gly Ser Arg Arg Lys Arg Arg Lys Arg Ile Val
               100                 105                 110
   Gly Gly Gln Glu Cys Lys Asp Gly Glu Cys Pro Trp Gln Ala Leu Leu
           115                 120                 125
   Ile Asn Glu Glu Asn Glu Gly Phe Cys Gly Gly Thr Ile Leu Ser Glu
       130                 135                 140
   Phe Tyr Ile Leu Thr Ala Ala His Cys Leu Tyr Gln Ala Lys Arg Phe
   145                 150                 155                 160
   Lys Val Arg Val Gly Asp Arg Asn Thr Glu Gln Glu Glu Gly Gly Glu
                   165                 170                 175
   Ala Val His Glu Val Glu Val Val Ile Lys His Asn Arg Phe Thr Lys
               180                 185                 190
   Glu Thr Tyr Asp Phe Asp Ile Ala Val Leu Arg Leu Lys Thr Pro Ile
           195                 200                 205
   Thr Phe Arg Met Asn Val Ala Pro Ala Cys Leu Pro Glu Arg Asp Trp
       210                 215                 220
   Ala Glu Ser Thr Leu Met Thr Gln Lys Thr Gly Ile Val Ser Gly Phe
   225                 230                 235                 240
   Gly Arg Thr His Glu Lys Gly Arg Gln Ser Thr Arg Leu Lys Met Leu
                   245                 250                 255
   Glu Val Pro Tyr Val Asp Arg Asn Ser Cys Lys Leu Ser Ser Ser Phe
               260                 265                 270
   Ile Ile Thr Gln Asn Met Phe Cys Ala Gly Tyr Asp Thr Lys Gln Glu
           275                 280                 285
   Asp Ala Cys Gln Gly Asp Ser Gly Gly Pro His Val Thr Arg Phe Lys
       290                 295                 300
   Asp Thr Tyr Phe Val Thr Gly Ile Val Ser Trp Gly Glu Gly Cys Ala
   305                 310                 315                 320
   Arg Lys Gly Lys Tyr Gly Ile Tyr Thr Lys Val Thr Ala Phe Leu Lys
                   325                 330                 335
   Trp Ile Asp Arg Ser Met Lys Thr Arg Gly Leu Pro Lys Ala Lys Ser
               340                 345                 350
   His Ala Pro Glu Val Ile Thr Ser Ser Pro Leu Lys
           355                 360
<210> 53
   <211> 360
   <212> PRT
   <213> Artificial Sequence
<220>
   <223> Exemple de GPAD3
<400> 53
   Asn Lys Tyr Lys Asp Gly Asp Gln Cys Glu Thr Ser Pro Cys Gln Asn
   1               5                   10                  15
   Gln Gly Lys Cys Lys Asp Gly Leu Gly Glu Tyr Thr Cys Thr Cys Leu
               20                  25                  30
   Glu Gly Phe Glu Gly Lys Asn Cys Glu Leu Phe Thr Arg Lys Leu Cys
           35                  40                  45
   Ser Leu Asp Asn Gly Asp Cys Asp Gln Phe Cys His Glu Glu Gln Asn
       50                  55                  60
   Ser Val Val Cys Ser Cys Ala Arg Gly Tyr Thr Leu Ala Asp Asn Gly
   65                  70                  75                  80
   Lys Ala Cys Ile Pro Thr Gly Pro Tyr Pro Cys Gly Lys Gln Thr Leu
                   85                  90                  95
   Glu Arg Gly Ser Ser Gly Arg Arg Lys Arg Ile Val Gly Gly Gln Glu
               100                 105                 110
   Cys Lys Asp Gly Glu Cys Pro Trp Gln Ala Leu Leu Ile Asn Glu Glu
           115                 120                 125
   Asn Glu Gly Phe Cys Gly Gly Thr Ile Leu Ser Glu Phe Tyr Ile Leu
       130                 135                 140
   Thr Ala Ala His Cys Leu Tyr Gln Ala Lys Arg Phe Lys Val Arg Val
   145                 150                 155                 160
   Gly Asp Arg Asn Thr Glu Gln Glu Glu Gly Gly Glu Ala Val His Glu
                   165                 170                 175
   Val Glu Val Val Ile Lys His Asn Arg Phe Thr Lys Glu Thr Tyr Asp
               180                 185                 190
   Phe Asp Ile Ala Val Leu Arg Leu Lys Thr Pro Ile Thr Phe Arg Met
           195                 200                 205
   Asn Val Ala Pro Ala Cys Leu Pro Glu Arg Asp Trp Ala Glu Ser Thr
       210                 215                 220
   Leu Met Thr Gln Lys Thr Gly Ile Val Ser Gly Phe Gly Arg Thr His
   225                 230                 235                 240
   Glu Lys Gly Arg Gln Ser Thr Arg Leu Lys Met Leu Glu Val Pro Tyr
                   245                 250                 255
   Val Asp Arg Asn Ser Cys Lys Leu Ser Ser Ser Phe Ile Ile Thr Gln
               260                 265                 270
   Asn Met Phe Cys Ala Gly Tyr Asp Thr Lys Gln Glu Asp Ala Cys Gln
           275                 280                 285
   Gly Asp Ser Gly Gly Pro His Val Thr Arg Phe Lys Asp Thr Tyr Phe
       290                 295                 300
   Val Thr Gly Ile Val Ser Trp Gly Glu Gly Cys Ala Arg Lys Gly Lys
   305                 310                 315                 320
   Tyr Gly Ile Tyr Thr Lys Val Thr Ala Phe Leu Lys Trp Ile Asp Arg
                   325                 330                 335
   Ser Met Lys Thr Arg Gly Leu Pro Lys Ala Lys Ser His Ala Pro Glu
               340                 345                 350
   Val Ile Thr Ser Ser Pro Leu Lys
           355                 360
<210> 54
   <211> 231
   <212> PRT
   <213> Artificial Sequence
<220>
   <223> SEQ ID NO:39 comprenant la région charnière entière en N-terminal
<400> 54
   Glu Pro Lys Ser Cys Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly
   225                 230
<210> 55
   <211> 10
   <212> PRT
   <213> Artificial Sequence
<220>
   <223> Région charnière partielle N-terminal
<400> 55
   Asp Lys Thr His Thr Cys Pro Pro Cys Pro
   1               5                   10
<210> 56
   <211> 15
   <212> PRT
   <213> Artificial Sequence
<220>
   <223> Région charnière entière N-terminal
<400> 56
   Glu Pro Lys Ser Cys Asp Lys Thr His Thr Cys Pro Pro Cys Pro
   1 5 10 15
<210> 57
   <211> 1080
   <212> DNA
   <213> Artificial Sequence
<220>
   <223> Séquence nucléique codant pour GPAD3-LC (SEQ ID NO:28)
<400> 57
   aacaagtaca aggacgggga ccagtgtgag acctcccctt gccagaacca gggcaagtgt      60
   aaagatggac tgggcgagta cacctgcaca tgtctggagg gattcgaagg caagaattgc     120
   gaactgttta ctagaaaact gtgttctctg gataacgggg actgcgatca gttctgtcac     180
   gaggaacaga attccgtggt ctgctcttgt gccagggggt acaccctggc cgacaacgga     240
   aaggcttgca tccctaccgg accctatcct tgtggcaaac agacactgga gcgagggagc     300
   tccggacgaa gaaagcgaat cgtgggagga caggagtgca aagatgggga atgtccatgg     360
   caggccctgc tgattaacga ggaaaatgag ggattctgcg gaggcactat cctgagcgaa     420
   ttttacattc tgaccgccgc tcattgtctg tatcaggcca agaggtttaa agtgcgcgtc     480
   ggcgaccgaa acacagagca ggaggaaggg ggagaagctg tgcacgaggt cgaagtggtc     540
   atcaagcata atcgcttcac taaagagacc tacgactttg atatcgcagt gctgagactg     600
   aagacaccaa ttactttcag gatgaatgtc gcaccagcat gcctgccaga gcgagattgg     660
   gctgaaagta ccctgatgac acagaagact ggaattgtgt cagggtttgg acggacacac     720
   gagaagggcc ggcagtctac tagactgaaa atgctggaag tgccctacgt cgacaggaac     780
   agttgtaagc tgtctagttc attcatcatc acacagaaca tgttttgcgc aggctatgac     840
   actaagcagg aggatgcctg tcagggggac agcggaggac ctcacgtgac ccgcttcaaa     900
   gatacttatt ttgtgaccgg aatcgtcagc tggggagagg gatgcgctcg aaagggcaaa     960
   tacgggatct ataccaaggt gacagcattc ctgaaatgga ttgacaggag catgaagacc    1020
   cgcggcctgc ctaaggctaa atcccatgca ccagaagtga tcacaagctc ccccctgaag    1080
<210> 58
   <211> 1098
   <212> DNA
   <213> Artificial Sequence
<220>
   <223> Séquence nucléique codant pour GPAD3-LL (SEQ ID NO:29)
<400> 58
   aacaagtata aagacggaga ccagtgcgag acatcccctt gccagaacca gggcaagtgt       60
   aaagatgggc tgggagagta cacctgcaca tgtctggagg ggttcgaagg aaagaattgc      120
   gaactgttta ctagaaaact gtgttctctg gataacgggg actgcgatca gttctgtcac      180
   gaggaacaga attcagtggt ctgcagctgt gccagggggt acaccctggc cgacaacgga      240
   aaggcttgca tccccaccgg accctatcct tgtggcaaac agacactgga gagaggagga      300
   ggaggatccg gaggaggagg gtctcggaga aagaggatcg tgggaggcca ggagtgcaaa      360
   gatggggaat gtccttggca ggccctgctg attaacgagg aaaatgaggg attctgcggg      420
   ggaactatcc tgagcgaatt ttacattctg accgccgctc actgtctgta tcaggccaag      480
   aggtttaaag tgcgcgtcgg cgaccgaaac acagagcagg aggaaggcgg ggaagctgtg      540
   cacgaggtcg aagtggtcat caagcataat cgcttcacta aagagaccta cgactttgat      600
   atcgcagtgc tgcgactgaa gacaccaatt actttccgga tgaatgtcgc accagcatgc      660
   ctgccagagc gagattgggc tgaaagtacc ctgatgacac agaagactgg aattgtgtca      720
   ggctttgggc ggacacatga gaagggccgg cagtctacta gactgaaaat gctggaagtg      780
   ccttacgtcg acaggaacag ttgtaagctg agctcctctt tcatcattac acagaatatg      840
   ttttgcgcag gctatgacac taagcaggag gatgcatgtc agggggacag cggaggacca      900
   cacgtgaccc gcttcaaaga tacttatttt gtgaccggaa tcgtcagctg gggagaggga      960
   tgcgctcgaa agggcaaata cgggatctat accaaggtga cagcattcct gaaatggatt     1020
   gacaggagca tgaagacccg cggcctgcct aaggctaaat cccatgcacc agaagtgatc     1080
   acaagttcac ccctgaag                                                   1098
<210> 59
   <211> 1107
   <212> DNA
   <213> Artificial Sequence
<220>
   <223> Séquence nucléique codant pour GPAD3-2F (SEQ ID NO:30)
<400> 59
   aacaagtaca aggacgggga tcagtgcgag acctcccctt gccagaacca gggcaagtgt      60
   aaagatgggc tgggagagta cacctgcaca tgtctggagg ggttcgaagg aaagaattgc     120
   gaactgttta ctcgcaaact gtgttctctg gataacgggg actgcgatca gttctgtcac     180
   gaggaacaga attcagtggt ctgcagctgt gccagaggct acaccctggc cgacaacgga     240
   aaggcttgca tccccaccgg accctatcct tgtggcaagc agacactgga gcgaagaaaa     300
   cgaggaggag gaggatccgg aggaggaggg tctaggcgca agcgaatcgt gggaggccag     360
   gagtgcaaag atggggaatg tccttggcag gccctgctga ttaacgagga aaatgaggga     420
   ttctgcgggg gaactatcct gagcgaattt tacattctga ccgccgctca ctgtctgtat     480
   caggccaagc ggttcaaggt gcgggtcggc gacagaaaca cagagcagga ggaaggcggg     540
   gaagctgtgc acgaggtcga agtggtcatc aagcataata gattcactaa agagacctac     600
   gactttgata tcgcagtgct gagactgaag acaccaatta ctttcaggat gaatgtcgca     660
   ccagcctgcc tgcccgagag agattgggct gaaagtaccc tgatgacaca gaaaactgga     720
   attgtgtcag gctttgggag gacacatgag aagggcaggc agtctactcg cctgaaaatg     780
   ctggaagtgc cttacgtcga caggaacagt tgtaagctga gctcctcttt catcattaca     840
   cagaatatgt tttgcgcagg ctatgacact aagcaggagg atgcatgtca gggggacagc     900
   ggaggaccac acgtgacccg cttcaaagat acttattttg tgaccggaat cgtcagctgg     960
   ggagagggat gcgctcgaaa gggcaaatac gggatctata ccaaggtgac agcattcctg    1020
   aaatggattg accgaagcat gaagacccgg ggcctgccta aggctaaatc ccatgcacca    1080
   gaagtgatca caagttcacc cctgaag                                        1107
<210> 60
   <211> 374
   <212> PRT
   <213> Artificial Sequence
<220>
   <223> GPAD1 avec peptide signal MB7
<400> 60
   Met Arg Trp Ser Trp Ile Phe Leu Leu Leu Leu Ser Ile Thr Ser Ala
   1               5                   10                  15
   Asn Ala Ser Asn Lys Tyr Lys Asp Gly Asp Gln Cys Glu Thr Ser Pro
               20                  25                  30
   Cys Gln Asn Gln Gly Lys Cys Lys Asp Gly Leu Gly Glu Tyr Thr Cys
           35                  40                  45
   Thr Cys Leu Glu Gly Phe Glu Gly Lys Asn Cys Glu Leu Phe Thr Arg
       50                  55                  60
   Lys Leu Cys Ser Leu Asp Asn Gly Asp Cys Asp Gln Phe Cys His Glu
   65                  70                  75                  80
   Glu Gln Asn Ser Val Val Cys Ser Cys Ala Arg Gly Tyr Thr Leu Ala
                   85                  90                  95
   Asp Asn Gly Lys Ala Cys Ile Pro Thr Gly Pro Tyr Pro Cys Gly Lys
               100                 105                 110
   Gln Thr Leu Glu Arg Arg Lys Arg Ile Val Gly Gly Gln Glu Cys Lys
           115                 120                 125
   Asp Gly Glu Cys Pro Trp Gln Ala Leu Leu Ile Asn Glu Glu Asn Glu
   130 135 140
   Gly Phe Cys Gly Gly Thr Ile Leu Ser Glu Phe Tyr Ile Leu Thr Ala
   145 150 155 160
   Ala His Cys Leu Tyr Gln Ala Lys Arg Phe Lys Val Arg Val Gly Asp
   165 170 175
   Arg Asn Thr Glu Gln Glu Glu Gly Gly Glu Ala Val His Glu Val Glu
   180 185 190
   Val Val Ile Lys His Asn Arg Phe Thr Lys Glu Thr Tyr Asp Phe Asp
   195 200 205
   Ile Ala Val Leu Arg Leu Lys Thr Pro Ile Thr Phe Arg Met Asn Val
   210 215 220
   Ala Pro Ala Cys Leu Pro Glu Arg Asp Trp Ala Glu Ser Thr Leu Met
   225 230 235 240
   Thr Gln Lys Thr Gly Ile Val Ser Gly Phe Gly Arg Thr His Glu Lys
   245 250 255
   Gly Arg Gln Ser Thr Arg Leu Lys Met Leu Glu Val Pro Tyr Val Asp
   260 265 270
   Arg Asn Ser Cys Lys Leu Ser Ser Ser Phe Ile Ile Thr Gln Asn Met
   275 280 285
   Phe Cys Ala Gly Tyr Asp Thr Lys Gln Glu Asp Ala Cys Gln Gly Asp
   290 295 300
   Ser Gly Gly Pro His Val Thr Arg Phe Lys Asp Thr Tyr Phe Val Thr
   305 310 315 320
   Gly Ile Val Ser Trp Gly Glu Gly Cys Ala Arg Lys Gly Lys Tyr Gly
   325 330 335
   Ile Tyr Thr Lys Val Thr Ala Phe Leu Lys Trp Ile Asp Arg Ser Met
   340 345 350
   Lys Thr Arg Gly Leu Pro Lys Ala Lys Ser His Ala Pro Glu Val Ile
   355 360 365
   Thr Ser Ser Pro Leu Lys
   370
<210> 61
   <211> 377
   <212> PRT
   <213> Artificial Sequence
<220>
   <223> GPAD2 avec peptide signal MB7
<400> 61
   Met Arg Trp Ser Trp Ile Phe Leu Leu Leu Leu Ser Ile Thr Ser Ala
   1               5                   10                  15
   Asn Ala Ser Asn Lys Tyr Lys Asp Gly Asp Gln Cys Glu Thr Ser Pro
               20                  25                  30
   Cys Gln Asn Gln Gly Lys Cys Lys Asp Gly Leu Gly Glu Tyr Thr Cys
           35                  40                  45
   Thr Cys Leu Glu Gly Phe Glu Gly Lys Asn Cys Glu Leu Phe Thr Arg
       50                  55                  60
   Lys Leu Cys Ser Leu Asp Asn Gly Asp Cys Asp Gln Phe Cys His Glu
   65                  70                  75                  80
   Glu Gln Asn Ser Val Val Cys Ser Cys Ala Arg Gly Tyr Thr Leu Ala
                   85                  90                  95
   Asp Asn Gly Lys Ala Cys Ile Pro Thr Gly Pro Tyr Pro Cys Gly Lys
               100                 105                 110
   Gln Thr Leu Glu Arg Arg Lys Arg Arg Lys Arg Ile Val Gly Gly Gln
           115                 120                 125
   Glu Cys Lys Asp Gly Glu Cys Pro Trp Gln Ala Leu Leu Ile Asn Glu
       130                 135                 140
   Glu Asn Glu Gly Phe Cys Gly Gly Thr Ile Leu Ser Glu Phe Tyr Ile
   145                 150                 155                 160
   Leu Thr Ala Ala His Cys Leu Tyr Gln Ala Lys Arg Phe Lys Val Arg
                   165                 170                 175
   Val Gly Asp Arg Asn Thr Glu Gln Glu Glu Gly Gly Glu Ala Val His
               180                 185                 190
   Glu Val Glu Val Val Ile Lys His Asn Arg Phe Thr Lys Glu Thr Tyr
           195                 200                 205
   Asp Phe Asp Ile Ala Val Leu Arg Leu Lys Thr Pro Ile Thr Phe Arg
       210                 215                 220
   Met Asn Val Ala Pro Ala Cys Leu Pro Glu Arg Asp Trp Ala Glu Ser
   225                 230                 235                 240
   Thr Leu Met Thr Gln Lys Thr Gly Ile Val Ser Gly Phe Gly Arg Thr
                   245                 250                 255
   His Glu Lys Gly Arg Gln Ser Thr Arg Leu Lys Met Leu Glu Val Pro
               260                 265                 270
   Tyr Val Asp Arg Asn Ser Cys Lys Leu Ser Ser Ser Phe Ile Ile Thr
           275                 280                 285
   Gln Asn Met Phe Cys Ala Gly Tyr Asp Thr Lys Gln Glu Asp Ala Cys
       290                 295                 300
   Gln Gly Asp Ser Gly Gly Pro His Val Thr Arg Phe Lys Asp Thr Tyr
   305                 310                 315                 320
   Phe Val Thr Gly Ile Val Ser Trp Gly Glu Gly Cys Ala Arg Lys Gly
                   325                 330                 335
   Lys Tyr Gly Ile Tyr Thr Lys Val Thr Ala Phe Leu Lys Trp Ile Asp
               340                 345                 350
   Arg Ser Met Lys Thr Arg Gly Leu Pro Lys Ala Lys Ser His Ala Pro
           355                 360                 365
   Glu Val Ile Thr Ser Ser Pro Leu Lys
       370                 375
<210> 62
   <211> 373
   <212> PRT
   <213> Artificial Sequence
<220>
   <223> GPAD1 optimisé avec peptide signal MB7
<400> 62
   Met Arg Trp Ser Trp Ile Phe Leu Leu Leu Leu Ser Ile Thr Ser Ala
   1               5                   10                  15
   Asn Ala Asn Lys Tyr Lys Asp Gly Asp Gln Cys Glu Thr Ser Pro Cys
               20                  25                  30
   Gln Asn Gln Gly Lys Cys Lys Asp Gly Leu Gly Glu Tyr Thr Cys Thr
   35 40 45
   Cys Leu Glu Gly Phe Glu Gly Lys Asn Cys Glu Leu Phe Thr Arg Lys
   50 55 60
   Leu Cys Ser Leu Asp Asn Gly Asp Cys Asp Gln Phe Cys His Glu Glu
   65 70 75 80
   Gln Asn Ser Val Val Cys Ser Cys Ala Arg Gly Tyr Thr Leu Ala Asp
   85 90 95
   Asn Gly Lys Ala Cys Ile Pro Thr Gly Pro Tyr Pro Cys Gly Lys Gln
   100 105 110
   Thr Leu Glu Arg Arg Lys Arg Ile Val Gly Gly Gln Glu Cys Lys Asp
   115 120 125
   Gly Glu Cys Pro Trp Gln Ala Leu Leu Ile Asn Glu Glu Asn Glu Gly
   130 135 140
   Phe Cys Gly Gly Thr Ile Leu Ser Glu Phe Tyr Ile Leu Thr Ala Ala
   145 150 155 160
   His Cys Leu Tyr Gln Ala Lys Arg Phe Lys Val Arg Val Gly Asp Arg
   165 170 175
   Asn Thr Glu Gln Glu Glu Gly Gly Glu Ala Val His Glu Val Glu Val
   180 185 190
   Val Ile Lys His Asn Arg Phe Thr Lys Glu Thr Tyr Asp Phe Asp Ile
   195 200 205
   Ala Val Leu Arg Leu Lys Thr Pro Ile Thr Phe Arg Met Asn Val Ala
   210 215 220
   Pro Ala Cys Leu Pro Glu Arg Asp Trp Ala Glu Ser Thr Leu Met Thr
   225 230 235 240
   Gln Lys Thr Gly Ile Val Ser Gly Phe Gly Arg Thr His Glu Lys Gly
   245 250 255
   Arg Gln Ser Thr Arg Leu Lys Met Leu Glu Val Pro Tyr Val Asp Arg
   260 265 270
   Asn Ser Cys Lys Leu Ser Ser Ser Phe Ile Ile Thr Gln Asn Met Phe
   275 280 285
   Cys Ala Gly Tyr Asp Thr Lys Gln Glu Asp Ala Cys Gln Gly Asp Ser
       290                 295                 300
   Gly Gly Pro His Val Thr Arg Phe Lys Asp Thr Tyr Phe Val Thr Gly
   305                 310                 315                 320
   Ile Val Ser Trp Gly Glu Gly Cys Ala Arg Lys Gly Lys Tyr Gly Ile
                   325                 330                 335
   Tyr Thr Lys Val Thr Ala Phe Leu Lys Trp Ile Asp Arg Ser Met Lys
               340                 345                 350
   Thr Arg Gly Leu Pro Lys Ala Lys Ser His Ala Pro Glu Val Ile Thr
           355                 360                 365
   Ser Ser Pro Leu Lys
       370
<210> 63
   <211> 376
   <212> PRT
   <213> Artificial Sequence
<220>
   <223> GPAD2 optimisé avec peptide signal MB7
<400> 63
   Met Arg Trp Ser Trp Ile Phe Leu Leu Leu Leu Ser Ile Thr Ser Ala
   1               5                   10                  15
   Asn Ala Asn Lys Tyr Lys Asp Gly Asp Gln Cys Glu Thr Ser Pro Cys
               20                  25                  30
   Gln Asn Gln Gly Lys Cys Lys Asp Gly Leu Gly Glu Tyr Thr Cys Thr
           35                  40                  45
   Cys Leu Glu Gly Phe Glu Gly Lys Asn Cys Glu Leu Phe Thr Arg Lys
       50                  55                  60
   Leu Cys Ser Leu Asp Asn Gly Asp Cys Asp Gln Phe Cys His Glu Glu
   65                  70                  75                  80
   Gln Asn Ser Val Val Cys Ser Cys Ala Arg Gly Tyr Thr Leu Ala Asp
                   85                  90                  95
   Asn Gly Lys Ala Cys Ile Pro Thr Gly Pro Tyr Pro Cys Gly Lys Gln
               100                 105                 110
   Thr Leu Glu Arg Arg Lys Arg Arg Lys Arg Ile Val Gly Gly Gln Glu
           115                 120                 125
   Cys Lys Asp Gly Glu Cys Pro Trp Gln Ala Leu Leu Ile Asn Glu Glu
       130                 135                 140
   Asn Glu Gly Phe Cys Gly Gly Thr Ile Leu Ser Glu Phe Tyr Ile Leu
   145                 150                 155                 160
   Thr Ala Ala His Cys Leu Tyr Gln Ala Lys Arg Phe Lys Val Arg Val
                   165                 170                 175
   Gly Asp Arg Asn Thr Glu Gln Glu Glu Gly Gly Glu Ala Val His Glu
               180                 185                 190
   Val Glu Val Val Ile Lys His Asn Arg Phe Thr Lys Glu Thr Tyr Asp
           195                 200                 205
   Phe Asp Ile Ala Val Leu Arg Leu Lys Thr Pro Ile Thr Phe Arg Met
       210                 215                 220
   Asn Val Ala Pro Ala Cys Leu Pro Glu Arg Asp Trp Ala Glu Ser Thr
   225                 230                 235                 240
   Leu Met Thr Gln Lys Thr Gly Ile Val Ser Gly Phe Gly Arg Thr His
                   245                 250                 255
   Glu Lys Gly Arg Gln Ser Thr Arg Leu Lys Met Leu Glu Val Pro Tyr
               260                 265                 270
   Val Asp Arg Asn Ser Cys Lys Leu Ser Ser Ser Phe Ile Ile Thr Gln
           275                 280                 285
   Asn Met Phe Cys Ala Gly Tyr Asp Thr Lys Gln Glu Asp Ala Cys Gln
       290                 295                 300
   Gly Asp Ser Gly Gly Pro His Val Thr Arg Phe Lys Asp Thr Tyr Phe
   305                 310                 315                 320
   Val Thr Gly Ile Val Ser Trp Gly Glu Gly Cys Ala Arg Lys Gly Lys
                   325                 330                 335
   Tyr Gly Ile Tyr Thr Lys Val Thr Ala Phe Leu Lys Trp Ile Asp Arg
               340                 345                 350
   Ser Met Lys Thr Arg Gly Leu Pro Lys Ala Lys Ser His Ala Pro Glu
           355                 360                 365
   Val Ile Thr Ser Ser Pro Leu Lys
   370 375
<210> 64
   <211> 377
   <212> PRT
   <213> Artificial Sequence
<220>
   <223> SEQ ID NO:27 avec peptide signal MB7
<400> 64
   Met Arg Trp Ser Trp Ile Phe Leu Leu Leu Leu Ser Ile Thr Ser Ala
   1 5 10 15
   Asn Ala Asn Lys Tyr Lys Asp Gly Asp Gln Cys Glu Thr Ser Pro Cys
   20 25 30
   Gln Asn Gln Gly Lys Cys Lys Asp Gly Leu Gly Glu Tyr Thr Cys Thr
   35 40 45
   Cys Leu Glu Gly Phe Glu Gly Lys Asn Cys Glu Leu Phe Thr Arg Lys
   50 55 60
   Leu Cys Ser Leu Asp Asn Gly Asp Cys Asp Gln Phe Cys His Glu Glu
   65 70 75 80
   Gln Asn Ser Val Val Cys Ser Cys Ala Arg Gly Tyr Thr Leu Ala Asp
   85 90 95
   Asn Gly Lys Ala Cys Ile Pro Thr Gly Pro Tyr Pro Cys Gly Lys Gln
   100 105 110
   Thr Leu Glu Gly Ser Ser Gly Arg Arg Lys Arg Ile Val Gly Gly Gln
   115 120 125
   Glu Cys Lys Asp Gly Glu Cys Pro Trp Gln Ala Leu Leu Ile Asn Glu
   130 135 140
   Glu Asn Glu Gly Phe Cys Gly Gly Thr Ile Leu Ser Glu Phe Tyr Ile
   145 150 155 160
   Leu Thr Ala Ala His Cys Leu Tyr Gln Ala Lys Arg Phe Lys Val Arg
   165 170 175
   Val Gly Asp Arg Asn Thr Glu Gln Glu Glu Gly Gly Glu Ala Val His
   180 185 190
   Glu Val Glu Val Val Ile Lys His Asn Arg Phe Thr Lys Glu Thr Tyr
       195                 200                 205
   Asp Phe Asp Ile Ala Val Leu Arg Leu Lys Thr Pro Ile Thr Phe Arg
       210                 215                 220
   Met Asn Val Ala Pro Ala Cys Leu Pro Glu Arg Asp Trp Ala Glu Ser
   225                 230                 235                 240
   Thr Leu Met Thr Gln Lys Thr Gly Ile Val Ser Gly Phe Gly Arg Thr
                   245                 250                 255
   His Glu Lys Gly Arg Gln Ser Thr Arg Leu Lys Met Leu Glu Val Pro
               260                 265                 270
   Tyr Val Asp Arg Asn Ser Cys Lys Leu Ser Ser Ser Phe Ile Ile Thr
           275                 280                 285
   Gln Asn Met Phe Cys Ala Gly Tyr Asp Thr Lys Gln Glu Asp Ala Cys
       290                 295                 300
   Gln Gly Asp Ser Gly Gly Pro His Val Thr Arg Phe Lys Asp Thr Tyr
   305                 310                 315                 320
   Phe Val Thr Gly Ile Val Ser Trp Gly Glu Gly Cys Ala Arg Lys Gly
                   325                 330                 335
   Lys Tyr Gly Ile Tyr Thr Lys Val Thr Ala Phe Leu Lys Trp Ile Asp
               340                 345                 350
   Arg Ser Met Lys Thr Arg Gly Leu Pro Lys Ala Lys Ser His Ala Pro
           355                 360                 365
   Glu Val Ile Thr Ser Ser Pro Leu Lys
       370                 375
<210> 65
   <211> 378
   <212> PRT
   <213> Artificial Sequence
<220>
   <223> GPAD3-LC avec peptide signal MB7
<400> 65
   Met Arg Trp Ser Trp Ile Phe Leu Leu Leu Leu Ser Ile Thr Ser Ala
   1               5                   10                  15
   Asn Ala Asn Lys Tyr Lys Asp Gly Asp Gln Cys Glu Thr Ser Pro Cys
               20                  25                  30
   Gln Asn Gln Gly Lys Cys Lys Asp Gly Leu Gly Glu Tyr Thr Cys Thr
           35                  40                  45
   Cys Leu Glu Gly Phe Glu Gly Lys Asn Cys Glu Leu Phe Thr Arg Lys
       50                  55                  60
   Leu Cys Ser Leu Asp Asn Gly Asp Cys Asp Gln Phe Cys His Glu Glu
   65                  70                  75                  80
   Gln Asn Ser Val Val Cys Ser Cys Ala Arg Gly Tyr Thr Leu Ala Asp
                   85                  90                  95
   Asn Gly Lys Ala Cys Ile Pro Thr Gly Pro Tyr Pro Cys Gly Lys Gln
               100                 105                 110
   Thr Leu Glu Arg Gly Ser Ser Gly Arg Arg Lys Arg Ile Val Gly Gly
           115                 120                 125
   Gln Glu Cys Lys Asp Gly Glu Cys Pro Trp Gln Ala Leu Leu Ile Asn
       130                 135                 140
   Glu Glu Asn Glu Gly Phe Cys Gly Gly Thr Ile Leu Ser Glu Phe Tyr
   145                 150                 155                 160
   Ile Leu Thr Ala Ala His Cys Leu Tyr Gln Ala Lys Arg Phe Lys Val
                   165                 170                 175
   Arg Val Gly Asp Arg Asn Thr Glu Gln Glu Glu Gly Gly Glu Ala Val
               180                 185                 190
   His Glu Val Glu Val Val Ile Lys His Asn Arg Phe Thr Lys Glu Thr
           195                 200                 205
   Tyr Asp Phe Asp Ile Ala Val Leu Arg Leu Lys Thr Pro Ile Thr Phe
       210                 215                 220
   Arg Met Asn Val Ala Pro Ala Cys Leu Pro Glu Arg Asp Trp Ala Glu
   225                 230                 235                 240
   Ser Thr Leu Met Thr Gln Lys Thr Gly Ile Val Ser Gly Phe Gly Arg
                   245                 250                 255
   Thr His Glu Lys Gly Arg Gln Ser Thr Arg Leu Lys Met Leu Glu Val
               260                 265                 270
   Pro Tyr Val Asp Arg Asn Ser Cys Lys Leu Ser Ser Ser Phe Ile Ile
           275                 280                 285
   Thr Gln Asn Met Phe Cys Ala Gly Tyr Asp Thr Lys Gln Glu Asp Ala
       290                 295                 300
   Cys Gln Gly Asp Ser Gly Gly Pro His Val Thr Arg Phe Lys Asp Thr
   305                 310                 315                 320
   Tyr Phe Val Thr Gly Ile Val Ser Trp Gly Glu Gly Cys Ala Arg Lys
                   325                 330                 335
   Gly Lys Tyr Gly Ile Tyr Thr Lys Val Thr Ala Phe Leu Lys Trp Ile
               340                 345                 350
   Asp Arg Ser Met Lys Thr Arg Gly Leu Pro Lys Ala Lys Ser His Ala
           355                 360                 365
   Pro Glu Val Ile Thr Ser Ser Pro Leu Lys
       370                 375
<210> 66
   <211> 384
   <212> PRT
   <213> Artificial Sequence
<220>
   <223> GPAD3-LL avec peptide signal MB7
<400> 66
   Met Arg Trp Ser Trp Ile Phe Leu Leu Leu Leu Ser Ile Thr Ser Ala
   1               5                   10                  15
   Asn Ala Asn Lys Tyr Lys Asp Gly Asp Gln Cys Glu Thr Ser Pro Cys
               20                  25                  30
   Gln Asn Gln Gly Lys Cys Lys Asp Gly Leu Gly Glu Tyr Thr Cys Thr
           35                  40                  45
   Cys Leu Glu Gly Phe Glu Gly Lys Asn Cys Glu Leu Phe Thr Arg Lys
       50                  55                  60
   Leu Cys Ser Leu Asp Asn Gly Asp Cys Asp Gln Phe Cys His Glu Glu
   65                  70                  75                  80
   Gln Asn Ser Val Val Cys Ser Cys Ala Arg Gly Tyr Thr Leu Ala Asp
                   85                  90                  95
   Asn Gly Lys Ala Cys Ile Pro Thr Gly Pro Tyr Pro Cys Gly Lys Gln
   100 105 110
   Thr Leu Glu Arg Gly Gly Gly Gly Ser Gly Gly Gly Gly Ser Arg Arg
   115 120 125
   Lys Arg Ile Val Gly Gly Gln Glu Cys Lys Asp Gly Glu Cys Pro Trp
   130 135 140
   Gln Ala Leu Leu Ile Asn Glu Glu Asn Glu Gly Phe Cys Gly Gly Thr
   145 150 155 160
   Ile Leu Ser Glu Phe Tyr Ile Leu Thr Ala Ala His Cys Leu Tyr Gln
   165 170 175
   Ala Lys Arg Phe Lys Val Arg Val Gly Asp Arg Asn Thr Glu Gln Glu
   180 185 190
   Glu Gly Gly Glu Ala Val His Glu Val Glu Val Val Ile Lys His Asn
   195 200 205
   Arg Phe Thr Lys Glu Thr Tyr Asp Phe Asp Ile Ala Val Leu Arg Leu
   210 215 220
   Lys Thr Pro Ile Thr Phe Arg Met Asn Val Ala Pro Ala Cys Leu Pro
   225 230 235 240
   Glu Arg Asp Trp Ala Glu Ser Thr Leu Met Thr Gln Lys Thr Gly Ile
   245 250 255
   Val Ser Gly Phe Gly Arg Thr His Glu Lys Gly Arg Gln Ser Thr Arg
   260 265 270
   Leu Lys Met Leu Glu Val Pro Tyr Val Asp Arg Asn Ser Cys Lys Leu
   275 280 285
   Ser Ser Ser Phe Ile Ile Thr Gln Asn Met Phe Cys Ala Gly Tyr Asp
   290 295 300
   Thr Lys Gln Glu Asp Ala Cys Gln Gly Asp Ser Gly Gly Pro His Val
   305 310 315 320
   Thr Arg Phe Lys Asp Thr Tyr Phe Val Thr Gly Ile Val Ser Trp Gly
   325 330 335
   Glu Gly Cys Ala Arg Lys Gly Lys Tyr Gly Ile Tyr Thr Lys Val Thr
   340 345 350
   Ala Phe Leu Lys Trp Ile Asp Arg Ser Met Lys Thr Arg Gly Leu Pro
   355 360 365
   Lys Ala Lys Ser His Ala Pro Glu Val Ile Thr Ser Ser Pro Leu Lys
   370 375 380
<210> 67
   <211> 387
   <212> PRT
   <213> Artificial Sequence
<220>
   <223> GPAD3-2F avec peptide signal MB7
<400> 67
   Met Arg Trp Ser Trp Ile Phe Leu Leu Leu Leu Ser Ile Thr Ser Ala
   1               5                   10                  15
   Asn Ala Asn Lys Tyr Lys Asp Gly Asp Gln Cys Glu Thr Ser Pro Cys
               20                  25                  30
   Gln Asn Gln Gly Lys Cys Lys Asp Gly Leu Gly Glu Tyr Thr Cys Thr
           35                  40                  45
   Cys Leu Glu Gly Phe Glu Gly Lys Asn Cys Glu Leu Phe Thr Arg Lys
       50                  55                  60
   Leu Cys Ser Leu Asp Asn Gly Asp Cys Asp Gln Phe Cys His Glu Glu
   65                  70                  75                  80
   Gln Asn Ser Val Val Cys Ser Cys Ala Arg Gly Tyr Thr Leu Ala Asp
                   85                  90                  95
   Asn Gly Lys Ala Cys Ile Pro Thr Gly Pro Tyr Pro Cys Gly Lys Gln
               100                 105                 110
   Thr Leu Glu Arg Arg Lys Arg Gly Gly Gly Gly Ser Gly Gly Gly Gly
           115                 120                 125
   Ser Arg Arg Lys Arg Ile Val Gly Gly Gln Glu Cys Lys Asp Gly Glu
       130                 135                 140
   Cys Pro Trp Gln Ala Leu Leu Ile Asn Glu Glu Asn Glu Gly Phe Cys
   145                 150                 155                 160
   Gly Gly Thr Ile Leu Ser Glu Phe Tyr Ile Leu Thr Ala Ala His Cys
                   165                 170                 175
   Leu Tyr Gln Ala Lys Arg Phe Lys Val Arg Val Gly Asp Arg Asn Thr
                   180                 185                 190
   Glu Gln Glu Glu Gly Gly Glu Ala Val His Glu Val Glu Val Val Ile
           195                 200                 205
   Lys His Asn Arg Phe Thr Lys Glu Thr Tyr Asp Phe Asp Ile Ala Val
       210                 215                 220
   Leu Arg Leu Lys Thr Pro Ile Thr Phe Arg Met Asn Val Ala Pro Ala
   225                 230                 235                 240
   Cys Leu Pro Glu Arg Asp Trp Ala Glu Ser Thr Leu Met Thr Gln Lys
                   245                 250                 255
   Thr Gly Ile Val Ser Gly Phe Gly Arg Thr His Glu Lys Gly Arg Gln
               260                 265                 270
   Ser Thr Arg Leu Lys Met Leu Glu Val Pro Tyr Val Asp Arg Asn Ser
           275                 280                 285
   Cys Lys Leu Ser Ser Ser Phe Ile Ile Thr Gln Asn Met Phe Cys Ala
       290                 295                 300
   Gly Tyr Asp Thr Lys Gln Glu Asp Ala Cys Gln Gly Asp Ser Gly Gly
   305                 310                 315                 320
   Pro His Val Thr Arg Phe Lys Asp Thr Tyr Phe Val Thr Gly Ile Val
                   325                 330                 335
   Ser Trp Gly Glu Gly Cys Ala Arg Lys Gly Lys Tyr Gly Ile Tyr Thr
               340                 345                 350
   Lys Val Thr Ala Phe Leu Lys Trp Ile Asp Arg Ser Met Lys Thr Arg
           355                 360                 365
   Gly Leu Pro Lys Ala Lys Ser His Ala Pro Glu Val Ile Thr Ser Ser
       370                 375                 380
   Pro Leu Lys
   385
<210> 68
   <211> 604
   <212> PRT
   <213> Artificial Sequence
<220>
   <223> GPAD2-FX-Fc avec peptide signal MB7
<400> 68
   Met Arg Trp Ser Trp Ile Phe Leu Leu Leu Leu Ser Ile Thr Ser Ala
   1               5                   10                  15
   Asn Ala Ser Asn Lys Tyr Lys Asp Gly Asp Gln Cys Glu Thr Ser Pro
               20                  25                  30
   Cys Gln Asn Gln Gly Lys Cys Lys Asp Gly Leu Gly Glu Tyr Thr Cys
           35                  40                  45
   Thr Cys Leu Glu Gly Phe Glu Gly Lys Asn Cys Glu Leu Phe Thr Arg
       50                  55                  60
   Lys Leu Cys Ser Leu Asp Asn Gly Asp Cys Asp Gln Phe Cys His Glu
   65                  70                  75                  80
   Glu Gln Asn Ser Val Val Cys Ser Cys Ala Arg Gly Tyr Thr Leu Ala
                   85                  90                  95
   Asp Asn Gly Lys Ala Cys Ile Pro Thr Gly Pro Tyr Pro Cys Gly Lys
               100                 105                 110
   Gln Thr Leu Glu Arg Arg Lys Arg Arg Lys Arg Ile Val Gly Gly Gln
           115                 120                 125
   Glu Cys Lys Asp Gly Glu Cys Pro Trp Gln Ala Leu Leu Ile Asn Glu
       130                 135                 140
   Glu Asn Glu Gly Phe Cys Gly Gly Thr Ile Leu Ser Glu Phe Tyr Ile
   145                 150                 155                 160
   Leu Thr Ala Ala His Cys Leu Tyr Gln Ala Lys Arg Phe Lys Val Arg
                   165                 170                 175
   Val Gly Asp Arg Asn Thr Glu Gln Glu Glu Gly Gly Glu Ala Val His
               180                 185                 190
   Glu Val Glu Val Val Ile Lys His Asn Arg Phe Thr Lys Glu Thr Tyr
           195                 200                 205
   Asp Phe Asp Ile Ala Val Leu Arg Leu Lys Thr Pro Ile Thr Phe Arg
       210                 215                 220
   Met Asn Val Ala Pro Ala Cys Leu Pro Glu Arg Asp Trp Ala Glu Ser
   225                 230                 235                 240
   Thr Leu Met Thr Gln Lys Thr Gly Ile Val Ser Gly Phe Gly Arg Thr
   245 250 255
   His Glu Lys Gly Arg Gln Ser Thr Arg Leu Lys Met Leu Glu Val Pro
   260 265 270
   Tyr Val Asp Arg Asn Ser Cys Lys Leu Ser Ser Ser Phe Ile Ile Thr
   275 280 285
   Gln Asn Met Phe Cys Ala Gly Tyr Asp Thr Lys Gln Glu Asp Ala Cys
   290 295 300
   Gln Gly Asp Ser Gly Gly Pro His Val Thr Arg Phe Lys Asp Thr Tyr
   305 310 315 320
   Phe Val Thr Gly Ile Val Ser Trp Gly Glu Gly Cys Ala Arg Lys Gly
   325 330 335
   Lys Tyr Gly Ile Tyr Thr Lys Val Thr Ala Phe Leu Lys Trp Ile Asp
   340 345 350
   Arg Ser Met Lys Thr Arg Gly Leu Pro Lys Ala Lys Ser His Ala Pro
   355 360 365
   Glu Val Ile Thr Ser Ser Pro Leu Lys Asp Lys Thr His Thr Cys Pro
   370 375 380
   Pro Cys Pro Ala Pro Glu Leu Leu Gly Gly Pro Ser Val Phe Leu Phe
   385 390 395 400
   Pro Pro Lys Pro Lys Asp Thr Leu Met Ile Ser Arg Thr Pro Glu Val
   405 410 415
   Thr Cys Val Val Val Asp Val Ser His Glu Asp Pro Glu Val Lys Phe
   420 425 430
   Asn Trp Tyr Val Asp Gly Val Glu Val His Asn Ala Lys Thr Lys Pro
   435 440 445
   Arg Glu Glu Gln Tyr Asn Ser Thr Tyr Arg Val Val Ser Val Leu Thr
   450 455 460
   Val Leu His Gln Asp Trp Leu Asn Gly Lys Glu Tyr Lys Cys Lys Val
   465 470 475 480
   Ser Asn Lys Ala Leu Pro Ala Pro Ile Glu Lys Thr Ile Ser Lys Ala
   485 490 495
   Lys Gly Gln Pro Arg Glu Pro Gln Val Tyr Thr Leu Pro Pro Ser Arg
               500                 505                 510
   Asp Glu Leu Thr Lys Asn Gln Val Ser Leu Thr Cys Leu Val Lys Gly
           515                 520                 525
   Phe Tyr Pro Ser Asp Ile Ala Val Glu Trp Glu Ser Asn Gly Gln Pro
       530                 535                 540
   Glu Asn Asn Tyr Lys Thr Thr Pro Pro Val Leu Asp Ser Asp Gly Ser
   545                 550                 555                 560
   Phe Phe Leu Tyr Ser Lys Leu Thr Val Asp Lys Ser Arg Trp Gln Gln
                   565                 570                 575
   Gly Asn Val Phe Ser Cys Ser Val Met His Glu Ala Leu His Asn His
               580                 585                 590
   Tyr Thr Gln Lys Ser Leu Ser Leu Ser Pro Gly Lys
           595                 600
<210> 69
   <211> 609
   <212> PRT
   <213> Artificial Sequence
<220>
   <223> GPAD2-Fcl avec peptide signal MB7
<400> 69
   Met Arg Trp Ser Trp Ile Phe Leu Leu Leu Leu Ser Ile Thr Ser Ala
   1               5                   10                  15
   Asn Ala Ser Asn Lys Tyr Lys Asp Gly Asp Gln Cys Glu Thr Ser Pro
               20                  25                  30
   Cys Gln Asn Gln Gly Lys Cys Lys Asp Gly Leu Gly Glu Tyr Thr Cys
           35                  40                  45
   Thr Cys Leu Glu Gly Phe Glu Gly Lys Asn Cys Glu Leu Phe Thr Arg
       50                  55                  60
   Lys Leu Cys Ser Leu Asp Asn Gly Asp Cys Asp Gln Phe Cys His Glu
   65                  70                  75                  80
   Glu Gln Asn Ser Val Val Cys Ser Cys Ala Arg Gly Tyr Thr Leu Ala
                   85                  90                  95
   Asp Asn Gly Lys Ala Cys Ile Pro Thr Gly Pro Tyr Pro Cys Gly Lys
               100                 105                 110
   Gln Thr Leu Glu Arg Arg Lys Arg Arg Lys Arg Ile Val Gly Gly Gln
           115                 120                 125
   Glu Cys Lys Asp Gly Glu Cys Pro Trp Gln Ala Leu Leu Ile Asn Glu
       130                 135                 140
   Glu Asn Glu Gly Phe Cys Gly Gly Thr Ile Leu Ser Glu Phe Tyr Ile
   145                 150                 155                 160
   Leu Thr Ala Ala His Cys Leu Tyr Gln Ala Lys Arg Phe Lys Val Arg
                   165                 170                 175
   Val Gly Asp Arg Asn Thr Glu Gln Glu Glu Gly Gly Glu Ala Val His
               180                 185                 190
   Glu Val Glu Val Val Ile Lys His Asn Arg Phe Thr Lys Glu Thr Tyr
           195                 200                 205
   Asp Phe Asp Ile Ala Val Leu Arg Leu Lys Thr Pro Ile Thr Phe Arg
       210                 215                 220
   Met Asn Val Ala Pro Ala Cys Leu Pro Glu Arg Asp Trp Ala Glu Ser
   225                 230                 235                 240
   Thr Leu Met Thr Gln Lys Thr Gly Ile Val Ser Gly Phe Gly Arg Thr
                   245                 250                 255
   His Glu Lys Gly Arg Gln Ser Thr Arg Leu Lys Met Leu Glu Val Pro
               260                 265                 270
   Tyr Val Asp Arg Asn Ser Cys Lys Leu Ser Ser Ser Phe Ile Ile Thr
           275                 280                 285
   Gln Asn Met Phe Cys Ala Gly Tyr Asp Thr Lys Gln Glu Asp Ala Cys
       290                 295                 300
   Gln Gly Asp Ser Gly Gly Pro His Val Thr Arg Phe Lys Asp Thr Tyr
   305                 310                 315                 320
   Phe Val Thr Gly Ile Val Ser Trp Gly Glu Gly Cys Ala Arg Lys Gly
                   325                 330                 335
   Lys Tyr Gly Ile Tyr Thr Lys Val Thr Ala Phe Leu Lys Trp Ile Asp
               340                 345                 350
   Arg Ser Met Lys Thr Arg Gly Leu Pro Lys Ala Lys Ser His Ala Pro
           355                 360                 365
   Glu Val Ile Thr Ser Ser Pro Leu Lys Glu Pro Lys Ser Cys Asp Lys
       370                 375                 380
   Thr His Thr Cys Pro Pro Cys Pro Ala Pro Glu Leu Leu Gly Gly Pro
   385                 390                 395                 400
   Ser Val Phe Leu Phe Pro Pro Lys Pro Lys Asp Thr Leu Met Ile Ser
                   405                 410                 415
   Arg Thr Pro Glu Val Thr Cys Val Val Val Asp Val Ser His Glu Asp
               420                 425                 430
   Pro Glu Val Lys Phe Asn Trp Tyr Val Asp Gly Val Glu Val His Asn
           435                 440                 445
   Ala Lys Thr Lys Pro Arg Glu Glu Gln Tyr Asn Ser Thr Tyr Arg Val
       450                 455                 460
   Val Ser Val Leu Thr Val Leu His Gln Asp Trp Leu Asn Gly Lys Glu
   465                 470                 475                 480
   Tyr Lys Cys Lys Val Ser Asn Lys Ala Leu Pro Ala Pro Ile Glu Lys
                   485                 490                 495
   Thr Ile Ser Lys Ala Lys Gly Gln Pro Arg Glu Pro Gln Val Tyr Thr
               500                 505                 510
   Leu Pro Pro Ser Arg Asp Glu Leu Thr Lys Asn Gln Val Ser Leu Thr
           515                 520                 525
   Cys Leu Val Lys Gly Phe Tyr Pro Ser Asp Ile Ala Val Glu Trp Glu
       530                 535                 540
   Ser Asn Gly Gln Pro Glu Asn Asn Tyr Lys Thr Thr Pro Pro Val Leu
   545                 550                 555                 560
   Asp Ser Asp Gly Ser Phe Phe Leu Tyr Ser Lys Leu Thr Val Asp Lys
                   565                 570                 575
   Ser Arg Trp Gln Gln Gly Asn Val Phe Ser Cys Ser Val Met His Glu
               580                 585                 590
   Ala Leu His Asn His Tyr Thr Gln Lys Ser Leu Ser Leu Ser Pro Gly
           595                 600                 605
   Lys
<210> 70
   <211> 608
   <212> PRT
   <213> Artificial Sequence
<220>
   <223> GPAD2-FcLss avec peptide signal MB7
<400> 70
   Met Arg Trp Ser Trp Ile Phe Leu Leu Leu Leu Ser Ile Thr Ser Ala
   1               5                   10                  15
   Asn Ala Asn Lys Tyr Lys Asp Gly Asp Gln Cys Glu Thr Ser Pro Cys
               20                  25                  30
   Gln Asn Gln Gly Lys Cys Lys Asp Gly Leu Gly Glu Tyr Thr Cys Thr
           35                  40                  45
   Cys Leu Glu Gly Phe Glu Gly Lys Asn Cys Glu Leu Phe Thr Arg Lys
       50                  55                  60
   Leu Cys Ser Leu Asp Asn Gly Asp Cys Asp Gln Phe Cys His Glu Glu
   65                  70                  75                  80
   Gln Asn Ser Val Val Cys Ser Cys Ala Arg Gly Tyr Thr Leu Ala Asp
                   85                  90                  95
   Asn Gly Lys Ala Cys Ile Pro Thr Gly Pro Tyr Pro Cys Gly Lys Gln
               100                 105                 110
   Thr Leu Glu Arg Arg Lys Arg Arg Lys Arg Ile Val Gly Gly Gln Glu
           115                 120                 125
   Cys Lys Asp Gly Glu Cys Pro Trp Gln Ala Leu Leu Ile Asn Glu Glu
       130                 135                 140
   Asn Glu Gly Phe Cys Gly Gly Thr Ile Leu Ser Glu Phe Tyr Ile Leu
   145                 150                 155                 160
   Thr Ala Ala His Cys Leu Tyr Gln Ala Lys Arg Phe Lys Val Arg Val
                   165                 170                 175
   Gly Asp Arg Asn Thr Glu Gln Glu Glu Gly Gly Glu Ala Val His Glu
               180                 185                 190
   Val Glu Val Val Ile Lys His Asn Arg Phe Thr Lys Glu Thr Tyr Asp
   195 200 205
   Phe Asp Ile Ala Val Leu Arg Leu Lys Thr Pro Ile Thr Phe Arg Met
   210 215 220
   Asn Val Ala Pro Ala Cys Leu Pro Glu Arg Asp Trp Ala Glu Ser Thr
   225 230 235 240
   Leu Met Thr Gln Lys Thr Gly Ile Val Ser Gly Phe Gly Arg Thr His
   245 250 255
   Glu Lys Gly Arg Gln Ser Thr Arg Leu Lys Met Leu Glu Val Pro Tyr
   260 265 270
   Val Asp Arg Asn Ser Cys Lys Leu Ser Ser Ser Phe Ile Ile Thr Gln
   275 280 285
   Asn Met Phe Cys Ala Gly Tyr Asp Thr Lys Gln Glu Asp Ala Cys Gln
   290 295 300
   Gly Asp Ser Gly Gly Pro His Val Thr Arg Phe Lys Asp Thr Tyr Phe
   305 310 315 320
   Val Thr Gly Ile Val Ser Trp Gly Glu Gly Cys Ala Arg Lys Gly Lys
   325 330 335
   Tyr Gly Ile Tyr Thr Lys Val Thr Ala Phe Leu Lys Trp Ile Asp Arg
   340 345 350
   Ser Met Lys Thr Arg Gly Leu Pro Lys Ala Lys Ser His Ala Pro Glu
   355 360 365
   Val Ile Thr Ser Ser Pro Leu Lys Glu Pro Lys Ser Cys Asp Lys Thr
   370 375 380
   His Thr Cys Pro Pro Cys Pro Ala Pro Glu Leu Leu Gly Gly Pro Ser
   385 390 395 400
   Val Phe Leu Phe Pro Pro Lys Pro Lys Asp Thr Leu Met Ile Ser Arg
   405 410 415
   Thr Pro Glu Val Thr Cys Val Val Val Asp Val Ser His Glu Asp Pro
   420 425 430
   Glu Val Lys Phe Asn Trp Tyr Val Asp Gly Val Glu Val His Asn Ala
   435 440 445
   Lys Thr Lys Pro Arg Glu Glu Gln Tyr Asn Ser Thr Tyr Arg Val Val
       450                 455                 460
   Ser Val Leu Thr Val Leu His Gln Asp Trp Leu Asn Gly Lys Glu Tyr
   465                 470                 475                 480
   Lys Cys Lys Val Ser Asn Lys Ala Leu Pro Ala Pro Ile Glu Lys Thr
                   485                 490                 495
   Ile Ser Lys Ala Lys Gly Gln Pro Arg Glu Pro Gln Val Tyr Thr Leu
               500                 505                 510
   Pro Pro Ser Arg Asp Glu Leu Thr Lys Asn Gln Val Ser Leu Thr Cys
           515                 520                 525
   Leu Val Lys Gly Phe Tyr Pro Ser Asp Ile Ala Val Glu Trp Glu Ser
       530                 535                 540
   Asn Gly Gln Pro Glu Asn Asn Tyr Lys Thr Thr Pro Pro Val Leu Asp
   545                 550                 555                 560
   Ser Asp Gly Ser Phe Phe Leu Tyr Ser Lys Leu Thr Val Asp Lys Ser
                   565                 570                 575
   Arg Trp Gln Gln Gly Asn Val Phe Ser Cys Ser Val Met His Glu Ala
               580                 585                 590
   Leu His Asn His Tyr Thr Gln Lys Ser Leu Ser Leu Ser Pro Gly Lys
           595                 600                 605
<210> 71
   <211> 849
   <212> PRT
   <213> Artificial Sequence
<220>
   <223> GPAD2-scFcL avec peptide signal MB7
<400> 71
   Met Arg Trp Ser Trp Ile Phe Leu Leu Leu Leu Ser Ile Thr Ser Ala
   1               5                   10                  15
   Asn Ala Ser Asn Lys Tyr Lys Asp Gly Asp Gln Cys Glu Thr Ser Pro
               20                  25                  30
   Cys Gln Asn Gln Gly Lys Cys Lys Asp Gly Leu Gly Glu Tyr Thr Cys
           35                  40                  45
   Thr Cys Leu Glu Gly Phe Glu Gly Lys Asn Cys Glu Leu Phe Thr Arg
       50                  55                  60
   Lys Leu Cys Ser Leu Asp Asn Gly Asp Cys Asp Gln Phe Cys His Glu
   65                  70                  75                  80
   Glu Gln Asn Ser Val Val Cys Ser Cys Ala Arg Gly Tyr Thr Leu Ala
                   85                  90                  95
   Asp Asn Gly Lys Ala Cys Ile Pro Thr Gly Pro Tyr Pro Cys Gly Lys
               100                 105                 110
   Gln Thr Leu Glu Arg Arg Lys Arg Arg Lys Arg Ile Val Gly Gly Gln
           115                 120                 125
   Glu Cys Lys Asp Gly Glu Cys Pro Trp Gln Ala Leu Leu Ile Asn Glu
       130                 135                 140
   Glu Asn Glu Gly Phe Cys Gly Gly Thr Ile Leu Ser Glu Phe Tyr Ile
   145                 150                 155                 160
   Leu Thr Ala Ala His Cys Leu Tyr Gln Ala Lys Arg Phe Lys Val Arg
                   165                 170                 175
   Val Gly Asp Arg Asn Thr Glu Gln Glu Glu Gly Gly Glu Ala Val His
               180                 185                 190
   Glu Val Glu Val Val Ile Lys His Asn Arg Phe Thr Lys Glu Thr Tyr
           195                 200                 205
   Asp Phe Asp Ile Ala Val Leu Arg Leu Lys Thr Pro Ile Thr Phe Arg
       210                 215                 220
   Met Asn Val Ala Pro Ala Cys Leu Pro Glu Arg Asp Trp Ala Glu Ser
   225                 230                 235                 240
   Thr Leu Met Thr Gln Lys Thr Gly Ile Val Ser Gly Phe Gly Arg Thr
                   245                 250                 255
   His Glu Lys Gly Arg Gln Ser Thr Arg Leu Lys Met Leu Glu Val Pro
               260                 265                 270
   Tyr Val Asp Arg Asn Ser Cys Lys Leu Ser Ser Ser Phe Ile Ile Thr
           275                 280                 285
   Gln Asn Met Phe Cys Ala Gly Tyr Asp Thr Lys Gln Glu Asp Ala Cys
       290                 295                 300
   Gln Gly Asp Ser Gly Gly Pro His Val Thr Arg Phe Lys Asp Thr Tyr
   305                 310                 315                 320
   Phe Val Thr Gly Ile Val Ser Trp Gly Glu Gly Cys Ala Arg Lys Gly
                   325                 330                 335
   Lys Tyr Gly Ile Tyr Thr Lys Val Thr Ala Phe Leu Lys Trp Ile Asp
               340                 345                 350
   Arg Ser Met Lys Thr Arg Gly Leu Pro Lys Ala Lys Ser His Ala Pro
           355                 360                 365
   Glu Val Ile Thr Ser Ser Pro Leu Lys Asp Lys Thr His Thr Cys Pro
       370                 375                 380
   Pro Cys Pro Ala Pro Glu Leu Leu Gly Gly Pro Ser Val Phe Leu Phe
   385                 390                 395                 400
   Pro Pro Lys Pro Lys Asp Thr Leu Met Ile Ser Arg Thr Pro Glu Val
                   405                 410                 415
   Thr Cys Val Val Val Asp Val Ser His Glu Asp Pro Glu Val Lys Phe
               420                 425                 430
   Asn Trp Tyr Val Asp Gly Val Glu Val His Asn Ala Lys Thr Lys Pro
           435                 440                 445
   Arg Glu Glu Gln Tyr Asn Ser Thr Tyr Arg Val Val Ser Val Leu Thr
       450                 455                 460
   Val Leu His Gln Asp Trp Leu Asn Gly Lys Glu Tyr Lys Cys Lys Val
   465                 470                 475                 480
   Ser Asn Lys Ala Leu Pro Ala Pro Ile Glu Lys Thr Ile Ser Lys Ala
                   485                 490                 495
   Lys Gly Gln Pro Arg Glu Pro Gln Val Tyr Thr Leu Pro Pro Ser Arg
               500                 505                 510
   Asp Glu Leu Thr Lys Asn Gln Val Ser Leu Thr Cys Leu Val Lys Gly
           515                 520                 525
   Phe Tyr Pro Ser Asp Ile Ala Val Glu Trp Glu Ser Asn Gly Gln Pro
       530                 535                 540
   Glu Asn Asn Tyr Lys Thr Thr Pro Pro Val Leu Asp Ser Asp Gly Ser
   545                 550                 555                 560
   Phe Phe Leu Tyr Ser Lys Leu Thr Val Asp Lys Ser Arg Trp Gln Gln
   565 570 575
   Gly Asn Val Phe Ser Cys Ser Val Met His Glu Ala Leu His Asn His
   580 585 590
   Tyr Thr Gln Lys Ser Leu Ser Leu Ser Pro Gly Gly Gly Gly Gly Ser
   595 600 605
   Gly Gly Gly Gly Ser Gly Gly Gly Gly Ser Glu Pro Lys Ser Ser Asp
   610 615 620
   Lys Thr His Thr Cys Pro Pro Cys Pro Ala Pro Glu Leu Leu Gly Gly
   625 630 635 640
   Pro Ser Val Phe Leu Phe Pro Pro Lys Pro Lys Asp Thr Leu Met Ile
   645 650 655
   Ser Arg Thr Pro Glu Val Thr Cys Val Val Val Asp Val Ser His Glu
   660 665 670
   Asp Pro Glu Val Lys Phe Asn Trp Tyr Val Asp Gly Val Glu Val His
   675 680 685
   Asn Ala Lys Thr Lys Pro Arg Glu Glu Gln Tyr Asn Ser Thr Tyr Arg
   690 695 700
   Val Val Ser Val Leu Thr Val Leu His Gln Asp Trp Leu Asn Gly Lys
   705 710 715 720
   Glu Tyr Lys Cys Lys Val Ser Asn Lys Ala Leu Pro Ala Pro Ile Glu
   725 730 735
   Lys Thr Ile Ser Lys Ala Lys Gly Gln Pro Arg Glu Pro Gln Val Tyr
   740 745 750
   Thr Leu Pro Pro Ser Arg Asp Glu Leu Thr Lys Asn Gln Val Ser Leu
   755 760 765
   Thr Cys Leu Val Lys Gly Phe Tyr Pro Ser Asp Ile Ala Val Glu Trp
   770 775 780
   Glu Ser Asn Gly Gln Pro Glu Asn Asn Tyr Lys Thr Thr Pro Pro Val
   785 790 795 800
   Leu Asp Ser Asp Gly Ser Phe Phe Leu Tyr Ser Lys Leu Thr Val Asp
   805 810 815
   Lys Ser Arg Trp Gln Gln Gly Asn Val Phe Ser Cys Ser Val Met His
   820 825 830
   Glu Ala Leu His Asn His Tyr Thr Gln Lys Ser Leu Ser Leu Ser Pro
   835 840 845
   Gly
<210> 72
   <211> 839
   <212> PRT
   <213> Artificial Sequence
<220>
   <223> GPAD2-scFcS avec peptide signal MB7
<400> 72
   Met Arg Trp Ser Trp Ile Phe Leu Leu Leu Leu Ser Ile Thr Ser Ala
   1               5                   10                  15
   Asn Ala Ser Asn Lys Tyr Lys Asp Gly Asp Gln Cys Glu Thr Ser Pro
               20                  25                  30
   Cys Gln Asn Gln Gly Lys Cys Lys Asp Gly Leu Gly Glu Tyr Thr Cys
           35                  40                  45
   Thr Cys Leu Glu Gly Phe Glu Gly Lys Asn Cys Glu Leu Phe Thr Arg
       50                  55                  60
   Lys Leu Cys Ser Leu Asp Asn Gly Asp Cys Asp Gln Phe Cys His Glu
   65                  70                  75                  80
   Glu Gln Asn Ser Val Val Cys Ser Cys Ala Arg Gly Tyr Thr Leu Ala
                   85                  90                  95
   Asp Asn Gly Lys Ala Cys Ile Pro Thr Gly Pro Tyr Pro Cys Gly Lys
               100                 105                 110
   Gln Thr Leu Glu Arg Arg Lys Arg Arg Lys Arg Ile Val Gly Gly Gln
           115                 120                 125
   Glu Cys Lys Asp Gly Glu Cys Pro Trp Gln Ala Leu Leu Ile Asn Glu
       130                 135                 140
   Glu Asn Glu Gly Phe Cys Gly Gly Thr Ile Leu Ser Glu Phe Tyr Ile
   145                 150                 155                 160
   Leu Thr Ala Ala His Cys Leu Tyr Gln Ala Lys Arg Phe Lys Val Arg
                   165                 170                 175
   Val Gly Asp Arg Asn Thr Glu Gln Glu Glu Gly Gly Glu Ala Val His
               180                 185                 190
   Glu Val Glu Val Val Ile Lys His Asn Arg Phe Thr Lys Glu Thr Tyr
           195                 200                 205
   Asp Phe Asp Ile Ala Val Leu Arg Leu Lys Thr Pro Ile Thr Phe Arg
       210                 215                 220
   Met Asn Val Ala Pro Ala Cys Leu Pro Glu Arg Asp Trp Ala Glu Ser
   225                 230                 235                 240
   Thr Leu Met Thr Gln Lys Thr Gly Ile Val Ser Gly Phe Gly Arg Thr
                   245                 250                 255
   His Glu Lys Gly Arg Gln Ser Thr Arg Leu Lys Met Leu Glu Val Pro
               260                 265                 270
   Tyr Val Asp Arg Asn Ser Cys Lys Leu Ser Ser Ser Phe Ile Ile Thr
           275                 280                 285
   Gln Asn Met Phe Cys Ala Gly Tyr Asp Thr Lys Gln Glu Asp Ala Cys
       290                 295                 300
   Gln Gly Asp Ser Gly Gly Pro His Val Thr Arg Phe Lys Asp Thr Tyr
   305                 310                 315                 320
   Phe Val Thr Gly Ile Val Ser Trp Gly Glu Gly Cys Ala Arg Lys Gly
                   325                 330                 335
   Lys Tyr Gly Ile Tyr Thr Lys Val Thr Ala Phe Leu Lys Trp Ile Asp
               340                 345                 350
   Arg Ser Met Lys Thr Arg Gly Leu Pro Lys Ala Lys Ser His Ala Pro
           355                 360                 365
   Glu Val Ile Thr Ser Ser Pro Leu Lys Asp Lys Thr His Thr Cys Pro
       370                 375                 380
   Pro Cys Pro Ala Pro Glu Leu Leu Gly Gly Pro Ser Val Phe Leu Phe
   385                 390                 395                 400
   Pro Pro Lys Pro Lys Asp Thr Leu Met Ile Ser Arg Thr Pro Glu Val
   405 410 415
   Thr Cys Val Val Val Asp Val Ser His Glu Asp Pro Glu Val Lys Phe
   420 425 430
   Asn Trp Tyr Val Asp Gly Val Glu Val His Asn Ala Lys Thr Lys Pro
   435 440 445
   Arg Glu Glu Gln Tyr Asn Ser Thr Tyr Arg Val Val Ser Val Leu Thr
   450 455 460
   Val Leu His Gln Asp Trp Leu Asn Gly Lys Glu Tyr Lys Cys Lys Val
   465 470 475 480
   Ser Asn Lys Ala Leu Pro Ala Pro Ile Glu Lys Thr Ile Ser Lys Ala
   485 490 495
   Lys Gly Gln Pro Arg Glu Pro Gln Val Tyr Thr Leu Pro Pro Ser Arg
   500 505 510
   Asp Glu Leu Thr Lys Asn Gln Val Ser Leu Thr Cys Leu Val Lys Gly
   515 520 525
   Phe Tyr Pro Ser Asp Ile Ala Val Glu Trp Glu Ser Asn Gly Gln Pro
   530 535 540
   Glu Asn Asn Tyr Lys Thr Thr Pro Pro Val Leu Asp Ser Asp Gly Ser
   545 550 555 560
   Phe Phe Leu Tyr Ser Lys Leu Thr Val Asp Lys Ser Arg Trp Gln Gln
   565 570 575
   Gly Asn Val Phe Ser Cys Ser Val Met His Glu Ala Leu His Asn His
   580 585 590
   Tyr Thr Gln Lys Ser Leu Ser Leu Ser Pro Gly Gly Gly Gly Gly Ser
   595 600 605
   Glu Pro Lys Ser Ser Asp Lys Thr His Thr Cys Pro Pro Cys Pro Ala
   610 615 620
   Pro Glu Leu Leu Gly Gly Pro Ser Val Phe Leu Phe Pro Pro Lys Pro
   625 630 635 640
   Lys Asp Thr Leu Met Ile Ser Arg Thr Pro Glu Val Thr Cys Val Val
   645 650 655
   Val Asp Val Ser His Glu Asp Pro Glu Val Lys Phe Asn Trp Tyr Val
   660 665 670
   Asp Gly Val Glu Val His Asn Ala Lys Thr Lys Pro Arg Glu Glu Gln
   675 680 685
   Tyr Asn Ser Thr Tyr Arg Val Val Ser Val Leu Thr Val Leu His Gln
   690 695 700
   Asp Trp Leu Asn Gly Lys Glu Tyr Lys Cys Lys Val Ser Asn Lys Ala
   705 710 715 720
   Leu Pro Ala Pro Ile Glu Lys Thr Ile Ser Lys Ala Lys Gly Gln Pro
   725 730 735
   Arg Glu Pro Gln Val Tyr Thr Leu Pro Pro Ser Arg Asp Glu Leu Thr
   740 745 750
   Lys Asn Gln Val Ser Leu Thr Cys Leu Val Lys Gly Phe Tyr Pro Ser
   755 760 765
   Asp Ile Ala Val Glu Trp Glu Ser Asn Gly Gln Pro Glu Asn Asn Tyr
   770 775 780
   Lys Thr Thr Pro Pro Val Leu Asp Ser Asp Gly Ser Phe Phe Leu Tyr
   785 790 795 800
   Ser Lys Leu Thr Val Asp Lys Ser Arg Trp Gln Gln Gly Asn Val Phe
   805 810 815
   Ser Cys Ser Val Met His Glu Ala Leu His Asn His Tyr Thr Gln Lys
   820 825 830
   Ser Leu Ser Leu Ser Pro Gly
   835
<210> 73
   <211> 853
   <212> PRT
   <213> Artificial Sequence
<220>
   <223> scFcL-GPAD2 avec peptide signal MB7
<400> 73
   Met Arg Trp Ser Trp Ile Phe Leu Leu Leu Leu Ser Ile Thr Ser Ala
   1               5                   10                  15
   Asn Ala Asp Lys Thr His Thr Cys Pro Pro Cys Pro Ala Pro Glu Leu
               20                  25                  30
   Leu Gly Gly Pro Ser Val Phe Leu Phe Pro Pro Lys Pro Lys Asp Thr
           35                  40                  45
   Leu Met Ile Ser Arg Thr Pro Glu Val Thr Cys Val Val Val Asp Val
       50                  55                  60
   Ser His Glu Asp Pro Glu Val Lys Phe Asn Trp Tyr Val Asp Gly Val
   65                  70                  75                  80
   Glu Val His Asn Ala Lys Thr Lys Pro Arg Glu Glu Gln Tyr Asn Ser
                   85                  90                  95
   Thr Tyr Arg Val Val Ser Val Leu Thr Val Leu His Gln Asp Trp Leu
               100                 105                 110
   Asn Gly Lys Glu Tyr Lys Cys Lys Val Ser Asn Lys Ala Leu Pro Ala
           115                 120                 125
   Pro Ile Glu Lys Thr Ile Ser Lys Ala Lys Gly Gln Pro Arg Glu Pro
       130                 135                 140
   Gln Val Tyr Thr Leu Pro Pro Ser Arg Asp Glu Leu Thr Lys Asn Gln
   145                 150                 155                 160
   Val Ser Leu Thr Cys Leu Val Lys Gly Phe Tyr Pro Ser Asp Ile Ala
                   165                 170                 175
   Val Glu Trp Glu Ser Asn Gly Gln Pro Glu Asn Asn Tyr Lys Thr Thr
               180                 185                 190
   Pro Pro Val Leu Asp Ser Asp Gly Ser Phe Phe Leu Tyr Ser Lys Leu
           195                 200                 205
   Thr Val Asp Lys Ser Arg Trp Gln Gln Gly Asn Val Phe Ser Cys Ser
       210                 215                 220
   Val Met His Glu Ala Leu His Asn His Tyr Thr Gln Lys Ser Leu Ser
   225                 230                 235                 240
   Leu Ser Pro Gly Gly Gly Gly Gly Ser Gly Gly Gly Gly Ser Gly Gly
                   245                 250                 255
   Gly Gly Ser Glu Pro Lys Ser Ser Asp Lys Thr His Thr Cys Pro Pro
               260                 265                 270
   Cys Pro Ala Pro Glu Leu Leu Gly Gly Pro Ser Val Phe Leu Phe Pro
           275                 280                 285
   Pro Lys Pro Lys Asp Thr Leu Met Ile Ser Arg Thr Pro Glu Val Thr
       290                 295                 300
   Cys Val Val Val Asp Val Ser His Glu Asp Pro Glu Val Lys Phe Asn
   305                 310                 315                 320
   Trp Tyr Val Asp Gly Val Glu Val His Asn Ala Lys Thr Lys Pro Arg
                   325                 330                 335
   Glu Glu Gln Tyr Asn Ser Thr Tyr Arg Val Val Ser Val Leu Thr Val
               340                 345                 350
   Leu His Gln Asp Trp Leu Asn Gly Lys Glu Tyr Lys Cys Lys Val Ser
           355                 360                 365
   Asn Lys Ala Leu Pro Ala Pro Ile Glu Lys Thr Ile Ser Lys Ala Lys
       370                 375                 380
   Gly Gln Pro Arg Glu Pro Gln Val Tyr Thr Leu Pro Pro Ser Arg Asp
   385                 390                 395                 400
   Glu Leu Thr Lys Asn Gln Val Ser Leu Thr Cys Leu Val Lys Gly Phe
                   405                 410                 415
   Tyr Pro Ser Asp Ile Ala Val Glu Trp Glu Ser Asn Gly Gln Pro Glu
               420                 425                 430
   Asn Asn Tyr Lys Thr Thr Pro Pro Val Leu Asp Ser Asp Gly Ser Phe
           435                 440                 445
   Phe Leu Tyr Ser Lys Leu Thr Val Asp Lys Ser Arg Trp Gln Gln Gly
       450                 455                 460
   Asn Val Phe Ser Cys Ser Val Met His Glu Ala Leu His Asn His Tyr
   465                 470                 475                 480
   Thr Gln Lys Ser Leu Ser Leu Ser Pro Gly Gly Gly Gly Gly Ser Asn
                   485                 490                 495
   Lys Tyr Lys Asp Gly Asp Gln Cys Glu Thr Ser Pro Cys Gln Asn Gln
               500                 505                 510
   Gly Lys Cys Lys Asp Gly Leu Gly Glu Tyr Thr Cys Thr Cys Leu Glu
           515                 520                 525
   Gly Phe Glu Gly Lys Asn Cys Glu Leu Phe Thr Arg Lys Leu Cys Ser
       530                 535                 540
   Leu Asp Asn Gly Asp Cys Asp Gln Phe Cys His Glu Glu Gln Asn Ser
   545                 550                 555                 560
   Val Val Cys Ser Cys Ala Arg Gly Tyr Thr Leu Ala Asp Asn Gly Lys
                   565                 570                 575
   Ala Cys Ile Pro Thr Gly Pro Tyr Pro Cys Gly Lys Gln Thr Leu Glu
               580                 585                 590
   Arg Arg Lys Arg Arg Lys Arg Ile Val Gly Gly Gln Glu Cys Lys Asp
           595                 600                 605
   Gly Glu Cys Pro Trp Gln Ala Leu Leu Ile Asn Glu Glu Asn Glu Gly
       610                 615                 620
   Phe Cys Gly Gly Thr Ile Leu Ser Glu Phe Tyr Ile Leu Thr Ala Ala
   625                 630                 635                 640
   His Cys Leu Tyr Gln Ala Lys Arg Phe Lys Val Arg Val Gly Asp Arg
                   645                 650                 655
   Asn Thr Glu Gln Glu Glu Gly Gly Glu Ala Val His Glu Val Glu Val
               660                 665                 670
   Val Ile Lys His Asn Arg Phe Thr Lys Glu Thr Tyr Asp Phe Asp Ile
           675                 680                 685
   Ala Val Leu Arg Leu Lys Thr Pro Ile Thr Phe Arg Met Asn Val Ala
       690                 695                 700
   Pro Ala Cys Leu Pro Glu Arg Asp Trp Ala Glu Ser Thr Leu Met Thr
   705                 710                 715                 720
   Gln Lys Thr Gly Ile Val Ser Gly Phe Gly Arg Thr His Glu Lys Gly
                   725                 730                 735
   Arg Gln Ser Thr Arg Leu Lys Met Leu Glu Val Pro Tyr Val Asp Arg
               740                 745                 750
   Asn Ser Cys Lys Leu Ser Ser Ser Phe Ile Ile Thr Gln Asn Met Phe
           755                 760                 765
   Cys Ala Gly Tyr Asp Thr Lys Gln Glu Asp Ala Cys Gln Gly Asp Ser
       770                 775                 780
   Gly Gly Pro His Val Thr Arg Phe Lys Asp Thr Tyr Phe Val Thr Gly
   785                 790                 795                 800
   Ile Val Ser Trp Gly Glu Gly Cys Ala Arg Lys Gly Lys Tyr Gly Ile
                   805                 810                 815
   Tyr Thr Lys Val Thr Ala Phe Leu Lys Trp Ile Asp Arg Ser Met Lys
               820                 825                 830
   Thr Arg Gly Leu Pro Lys Ala Lys Ser His Ala Pro Glu Val Ile Thr
           835                 840                 845
   Ser Ser Pro Leu Lys
       850
<210> 74
   <211> 850
   <212> PRT
   <213> Artificial Sequence
<220>
   <223> scFcL-GPAD1 avec peptide signal MB7
<400> 74
   Met Arg Trp Ser Trp Ile Phe Leu Leu Leu Leu Ser Ile Thr Ser Ala
   1               5                   10                  15
   Asn Ala Asp Lys Thr His Thr Cys Pro Pro Cys Pro Ala Pro Glu Leu
               20                  25                  30
   Leu Gly Gly Pro Ser Val Phe Leu Phe Pro Pro Lys Pro Lys Asp Thr
           35                  40                  45
   Leu Met Ile Ser Arg Thr Pro Glu Val Thr Cys Val Val Val Asp Val
       50                  55                  60
   Ser His Glu Asp Pro Glu Val Lys Phe Asn Trp Tyr Val Asp Gly Val
   65                  70                  75                  80
   Glu Val His Asn Ala Lys Thr Lys Pro Arg Glu Glu Gln Tyr Asn Ser
                   85                  90                  95
   Thr Tyr Arg Val Val Ser Val Leu Thr Val Leu His Gln Asp Trp Leu
               100                 105                 110
   Asn Gly Lys Glu Tyr Lys Cys Lys Val Ser Asn Lys Ala Leu Pro Ala
           115                 120                 125
   Pro Ile Glu Lys Thr Ile Ser Lys Ala Lys Gly Gln Pro Arg Glu Pro
       130                 135                 140
   Gln Val Tyr Thr Leu Pro Pro Ser Arg Asp Glu Leu Thr Lys Asn Gln
   145                 150                 155                 160
   Val Ser Leu Thr Cys Leu Val Lys Gly Phe Tyr Pro Ser Asp Ile Ala
                   165                 170                 175
   Val Glu Trp Glu Ser Asn Gly Gln Pro Glu Asn Asn Tyr Lys Thr Thr
               180                 185                 190
   Pro Pro Val Leu Asp Ser Asp Gly Ser Phe Phe Leu Tyr Ser Lys Leu
           195                 200                 205
   Thr Val Asp Lys Ser Arg Trp Gln Gln Gly Asn Val Phe Ser Cys Ser
       210                 215                 220
   Val Met His Glu Ala Leu His Asn His Tyr Thr Gln Lys Ser Leu Ser
   225                 230                 235                 240
   Leu Ser Pro Gly Gly Gly Gly Gly Ser Gly Gly Gly Gly Ser Gly Gly
                   245                 250                 255
   Gly Gly Ser Glu Pro Lys Ser Ser Asp Lys Thr His Thr Cys Pro Pro
               260                 265                 270
   Cys Pro Ala Pro Glu Leu Leu Gly Gly Pro Ser Val Phe Leu Phe Pro
           275                 280                 285
   Pro Lys Pro Lys Asp Thr Leu Met Ile Ser Arg Thr Pro Glu Val Thr
       290                 295                 300
   Cys Val Val Val Asp Val Ser His Glu Asp Pro Glu Val Lys Phe Asn
   305                 310                 315                 320
   Trp Tyr Val Asp Gly Val Glu Val His Asn Ala Lys Thr Lys Pro Arg
                   325                 330                 335
   Glu Glu Gln Tyr Asn Ser Thr Tyr Arg Val Val Ser Val Leu Thr Val
               340                 345                 350
   Leu His Gln Asp Trp Leu Asn Gly Lys Glu Tyr Lys Cys Lys Val Ser
           355                 360                 365
   Asn Lys Ala Leu Pro Ala Pro Ile Glu Lys Thr Ile Ser Lys Ala Lys
       370                 375                 380
   Gly Gln Pro Arg Glu Pro Gln Val Tyr Thr Leu Pro Pro Ser Arg Asp
   385 390 395 400
   Glu Leu Thr Lys Asn Gln Val Ser Leu Thr Cys Leu Val Lys Gly Phe
   405 410 415
   Tyr Pro Ser Asp Ile Ala Val Glu Trp Glu Ser Asn Gly Gln Pro Glu
   420 425 430
   Asn Asn Tyr Lys Thr Thr Pro Pro Val Leu Asp Ser Asp Gly Ser Phe
   435 440 445
   Phe Leu Tyr Ser Lys Leu Thr Val Asp Lys Ser Arg Trp Gln Gln Gly
   450 455 460
   Asn Val Phe Ser Cys Ser Val Met His Glu Ala Leu His Asn His Tyr
   465 470 475 480
   Thr Gln Lys Ser Leu Ser Leu Ser Pro Gly Gly Gly Gly Gly Ser Asn
   485 490 495
   Lys Tyr Lys Asp Gly Asp Gln Cys Glu Thr Ser Pro Cys Gln Asn Gln
   500 505 510
   Gly Lys Cys Lys Asp Gly Leu Gly Glu Tyr Thr Cys Thr Cys Leu Glu
   515 520 525
   Gly Phe Glu Gly Lys Asn Cys Glu Leu Phe Thr Arg Lys Leu Cys Ser
   530 535 540
   Leu Asp Asn Gly Asp Cys Asp Gln Phe Cys His Glu Glu Gln Asn Ser
   545 550 555 560
   Val Val Cys Ser Cys Ala Arg Gly Tyr Thr Leu Ala Asp Asn Gly Lys
   565 570 575
   Ala Cys Ile Pro Thr Gly Pro Tyr Pro Cys Gly Lys Gln Thr Leu Glu
   580 585 590
   Arg Arg Lys Arg Ile Val Gly Gly Gln Glu Cys Lys Asp Gly Glu Cys
   595 600 605
   Pro Trp Gln Ala Leu Leu Ile Asn Glu Glu Asn Glu Gly Phe Cys Gly
   610 615 620
   Gly Thr Ile Leu Ser Glu Phe Tyr Ile Leu Thr Ala Ala His Cys Leu
   625 630 635 640
   Tyr Gln Ala Lys Arg Phe Lys Val Arg Val Gly Asp Arg Asn Thr Glu
   645 650 655
   Gln Glu Glu Gly Gly Glu Ala Val His Glu Val Glu Val Val Ile Lys
   660 665 670
   His Asn Arg Phe Thr Lys Glu Thr Tyr Asp Phe Asp Ile Ala Val Leu
   675 680 685
   Arg Leu Lys Thr Pro Ile Thr Phe Arg Met Asn Val Ala Pro Ala Cys
   690 695 700
   Leu Pro Glu Arg Asp Trp Ala Glu Ser Thr Leu Met Thr Gln Lys Thr
   705 710 715 720
   Gly Ile Val Ser Gly Phe Gly Arg Thr His Glu Lys Gly Arg Gln Ser
   725 730 735
   Thr Arg Leu Lys Met Leu Glu Val Pro Tyr Val Asp Arg Asn Ser Cys
   740 745 750
   Lys Leu Ser Ser Ser Phe Ile Ile Thr Gln Asn Met Phe Cys Ala Gly
   755 760 765
   Tyr Asp Thr Lys Gln Glu Asp Ala Cys Gln Gly Asp Ser Gly Gly Pro
   770 775 780
   His Val Thr Arg Phe Lys Asp Thr Tyr Phe Val Thr Gly Ile Val Ser
   785 790 795 800
   Trp Gly Glu Gly Cys Ala Arg Lys Gly Lys Tyr Gly Ile Tyr Thr Lys
   805 810 815
   Val Thr Ala Phe Leu Lys Trp Ile Asp Arg Ser Met Lys Thr Arg Gly
   820 825 830
   Leu Pro Lys Ala Lys Ser His Ala Pro Glu Val Ile Thr Ser Ser Pro
   835 840 845
   Leu Lys
   850
<210> 75
   <211> 382
   <212> PRT
   <213> Artificial Sequence
<220>
   <223> GPAD 3 de SEQ ID NO:52 avec peptide signal MB7
<400> 75
   Met Arg Trp Ser Trp Ile Phe Leu Leu Leu Leu Ser Ile Thr Ser Ala
   1               5                   10                  15
   Asn Ala Asn Lys Tyr Lys Asp Gly Asp Gln Cys Glu Thr Ser Pro Cys
               20                  25                  30
   Gln Asn Gln Gly Lys Cys Lys Asp Gly Leu Gly Glu Tyr Thr Cys Thr
           35                  40                  45
   Cys Leu Glu Gly Phe Glu Gly Lys Asn Cys Glu Leu Phe Thr Arg Lys
       50                  55                  60
   Leu Cys Ser Leu Asp Asn Gly Asp Cys Asp Gln Phe Cys His Glu Glu
   65                  70                  75                  80
   Gln Asn Ser Val Val Cys Ser Cys Ala Arg Gly Tyr Thr Leu Ala Asp
                   85                  90                  95
   Asn Gly Lys Ala Cys Ile Pro Thr Gly Pro Tyr Pro Cys Gly Lys Gln
               100                 105                 110
   Thr Leu Glu Arg Gly Gly Gly Gly Ser Arg Arg Lys Arg Arg Lys Arg
           115                 120                 125
   Ile Val Gly Gly Gln Glu Cys Lys Asp Gly Glu Cys Pro Trp Gln Ala
       130                 135                 140
   Leu Leu Ile Asn Glu Glu Asn Glu Gly Phe Cys Gly Gly Thr Ile Leu
   145                 150                 155                 160
   Ser Glu Phe Tyr Ile Leu Thr Ala Ala His Cys Leu Tyr Gln Ala Lys
                   165                 170                 175
   Arg Phe Lys Val Arg Val Gly Asp Arg Asn Thr Glu Gln Glu Glu Gly
               180                 185                 190
   Gly Glu Ala Val His Glu Val Glu Val Val Ile Lys His Asn Arg Phe
           195                 200                 205
   Thr Lys Glu Thr Tyr Asp Phe Asp Ile Ala Val Leu Arg Leu Lys Thr
       210                 215                 220
   Pro Ile Thr Phe Arg Met Asn Val Ala Pro Ala Cys Leu Pro Glu Arg
   225                 230                 235                 240
   Asp Trp Ala Glu Ser Thr Leu Met Thr Gln Lys Thr Gly Ile Val Ser
                   245                 250                 255
   Gly Phe Gly Arg Thr His Glu Lys Gly Arg Gln Ser Thr Arg Leu Lys
               260                 265                 270
   Met Leu Glu Val Pro Tyr Val Asp Arg Asn Ser Cys Lys Leu Ser Ser
           275                 280                 285
   Ser Phe Ile Ile Thr Gln Asn Met Phe Cys Ala Gly Tyr Asp Thr Lys
       290                 295                 300
   Gln Glu Asp Ala Cys Gln Gly Asp Ser Gly Gly Pro His Val Thr Arg
   305                 310                 315                 320
   Phe Lys Asp Thr Tyr Phe Val Thr Gly Ile Val Ser Trp Gly Glu Gly
                   325                 330                 335
   Cys Ala Arg Lys Gly Lys Tyr Gly Ile Tyr Thr Lys Val Thr Ala Phe
               340                 345                 350
   Leu Lys Trp Ile Asp Arg Ser Met Lys Thr Arg Gly Leu Pro Lys Ala
           355                 360                 365
   Lys Ser His Ala Pro Glu Val Ile Thr Ser Ser Pro Leu Lys
       370                 375                 380
<210> 76
   <211> 378
   <212> PRT
   <213> Artificial Sequence
<220>
   <223> GPAD3 de SEQ ID NO:53 avec peptide signal MB7
<400> 76
   Met Arg Trp Ser Trp Ile Phe Leu Leu Leu Leu Ser Ile Thr Ser Ala
   1               5                   10                  15
   Asn Ala Asn Lys Tyr Lys Asp Gly Asp Gln Cys Glu Thr Ser Pro Cys
               20                  25                  30
   Gln Asn Gln Gly Lys Cys Lys Asp Gly Leu Gly Glu Tyr Thr Cys Thr
           35                  40                  45
   Cys Leu Glu Gly Phe Glu Gly Lys Asn Cys Glu Leu Phe Thr Arg Lys
       50                  55                  60
   Leu Cys Ser Leu Asp Asn Gly Asp Cys Asp Gln Phe Cys His Glu Glu
   65                  70                  75                  80
   Gln Asn Ser Val Val Cys Ser Cys Ala Arg Gly Tyr Thr Leu Ala Asp
                   85                  90                  95
   Asn Gly Lys Ala Cys Ile Pro Thr Gly Pro Tyr Pro Cys Gly Lys Gln
               100                 105                 110
   Thr Leu Glu Arg Gly Ser Ser Gly Arg Arg Lys Arg Ile Val Gly Gly
           115                 120                 125
   Gln Glu Cys Lys Asp Gly Glu Cys Pro Trp Gln Ala Leu Leu Ile Asn
       130                 135                 140
   Glu Glu Asn Glu Gly Phe Cys Gly Gly Thr Ile Leu Ser Glu Phe Tyr
   145                 150                 155                 160
   Ile Leu Thr Ala Ala His Cys Leu Tyr Gln Ala Lys Arg Phe Lys Val
                   165                 170                 175
   Arg Val Gly Asp Arg Asn Thr Glu Gln Glu Glu Gly Gly Glu Ala Val
               180                 185                 190
   His Glu Val Glu Val Val Ile Lys His Asn Arg Phe Thr Lys Glu Thr
           195                 200                 205
   Tyr Asp Phe Asp Ile Ala Val Leu Arg Leu Lys Thr Pro Ile Thr Phe
       210                 215                 220
   Arg Met Asn Val Ala Pro Ala Cys Leu Pro Glu Arg Asp Trp Ala Glu
   225                 230                 235                 240
   Ser Thr Leu Met Thr Gln Lys Thr Gly Ile Val Ser Gly Phe Gly Arg
                   245                 250                 255
   Thr His Glu Lys Gly Arg Gln Ser Thr Arg Leu Lys Met Leu Glu Val
               260                 265                 270
   Pro Tyr Val Asp Arg Asn Ser Cys Lys Leu Ser Ser Ser Phe Ile Ile
           275                 280                 285
   Thr Gln Asn Met Phe Cys Ala Gly Tyr Asp Thr Lys Gln Glu Asp Ala
       290                 295                 300
   Cys Gln Gly Asp Ser Gly Gly Pro His Val Thr Arg Phe Lys Asp Thr
   305                 310                 315                 320
   Tyr Phe Val Thr Gly Ile Val Ser Trp Gly Glu Gly Cys Ala Arg Lys
                   325                 330                 335
   Gly Lys Tyr Gly Ile Tyr Thr Lys Val Thr Ala Phe Leu Lys Trp Ile
               340                 345                 350
   Asp Arg Ser Met Lys Thr Arg Gly Leu Pro Lys Ala Lys Ser His Ala
           355                 360                 365
   Pro Glu Val Ile Thr Ser Ser Pro Leu Lys
       370                 375
<210> 77
   <211> 1122
   <212> DNA
   <213> Artificial Sequence
<220>
   <223> Séquence nucléique codant pour la protéine SEQ ID NO:60
<400> 77
   atgagatgga gttggatttt tctgctgctg ctgagtatta cctctgctaa cgcaagcaat 60
   aaatacaaag atggcgacca gtgtgagacc agtccttgcc agaaccaggg caaatgtaaa 120
   gacggcctcg gggaatacac ctgcacctgt ttagaaggat tcgaaggcaa aaactgtgaa 180
   ttattcacac ggaagctctg cagcctggac aacggggact gtgaccagtt ctgccacgag 240
   gaacagaact ctgtggtgtg ctcctgcgcc cgcgggtaca ccctggctga caacggcaag 300
   gcctgcattc ccacagggcc ctacccctgt gggaaacaga ccctggaacg caggaagagg 360
   atcgtgggag gccaggaatg caaggacggg gagtgtccct ggcaggccct gctcatcaat 420
   gaggaaaacg agggtttctg tggtggaacc attctgagcg agttctacat cctaacggca 480
   gcccactgtc tctaccaagc caagagattc aaggtgaggg taggggaccg gaacacggag 540
   caggaggagg gcggtgaggc ggtgcacgag gtggaggtgg tcatcaagca caaccggttc 600
   acaaaggaga cctatgactt cgacatcgcc gtgctccggc tcaagacccc catcaccttc 660
   cgcatgaacg tggcgcctgc ctgcctcccc gagcgtgact gggccgagtc cacgctgatg 720
   acgcagaaga cggggattgt gagcggcttc gggcgcaccc acgagaaggg ccggcagtcc 780
   accaggctca agatgctgga ggtgccctac gtggaccgca acagctgcaa gctgtccagc 840
   agcttcatca tcacccagaa catgttctgt gccggctacg acaccaagca ggaggatgcc 900
   tgccaggggg acagcggggg cccgcacgtc acccgcttca aggacaccta cttcgtgaca 960
   ggcatcgtca gctggggaga gggctgtgcc cgtaagggga agtacgggat ctacaccaag 1020
   gtcaccgcct tcctcaagtg gatcgacagg tccatgaaaa ccaggggctt gcccaaggcc 1080
   aagagccatg ccccggaggt cataacgtcc tctccattaa ag 1122
<210> 78
   <211> 1131
   <212> DNA
   <213> Artificial Sequence
<220>
   <223> Séquence nucléique codant pour la protéine SEQ ID NO:61
<400> 78
   atgagatgga gttggatttt tctgctgctg ctgagtatta cctctgctaa cgcaagcaat      60
   aaatacaaag atggcgacca gtgtgagacc agtccttgcc agaaccaggg caaatgtaaa     120
   gacggcctcg gggaatacac ctgcacctgt ttagaaggat tcgaaggcaa aaactgtgaa     180
   ttattcacac ggaagctctg cagcctggac aacggggact gtgaccagtt ctgccacgag     240
   gaacagaact ctgtggtgtg ctcctgcgcc cgcgggtaca ccctggctga caacggcaag     300
   gcctgcattc ccacagggcc ctacccctgt gggaaacaga ccctggaacg caggaagagg     360
   aggaagagga tcgtgggagg ccaggaatgc aaggacgggg agtgtccctg gcaggccctg     420
   ctcatcaatg aggaaaacga gggtttctgt ggtggaacca ttctgagcga gttctacatc     480
   ctaacggcag cccactgtct ctaccaagcc aagagattca aggtgagggt aggggaccgg     540
   aacacggagc aggaggaggg cggtgaggcg gtgcacgagg tggaggtggt catcaagcac     600
   aaccggttca caaaggagac ctatgacttc gacatcgccg tgctccggct caagaccccc     660
   atcaccttcc gcatgaacgt ggcgcctgcc tgcctccccg agcgtgactg ggccgagtcc     720
   acgctgatga cgcagaagac ggggattgtg agcggcttcg ggcgcaccca cgagaagggc     780
   cggcagtcca ccaggctcaa gatgctggag gtgccctacg tggaccgcaa cagctgcaag     840
   ctgtccagca gcttcatcat cacccagaac atgttctgtg ccggctacga caccaagcag     900
   gaggatgcct gccaggggga cagcgggggc ccgcacgtca cccgcttcaa ggacacctac     960
   ttcgtgacag gcatcgtcag ctggggagag ggctgtgccc gtaaggggaa gtacgggatc    1020
   tacaccaagg tcaccgcctt cctcaagtgg atcgacaggt ccatgaaaac caggggcttg    1080
   cccaaggcca agagccatgc cccggaggtc ataacgtcct ctccattaaa g             1131
<210> 79
   <211> 1119
   <212> DNA
   <213> Artificial Sequence
<220>
   <223> Séquence nucléique codant pour la protéine SEQ ID NO:62
<400> 79
   atgagatgga gttggatttt tctgctgctg ctgagtatta cctctgctaa cgcaaacaag      60
   tataaagacg gagaccagtg tgagactagc ccttgccaga accaggggaa gtgtaaagat      120
   ggactgggcg agtacacctg cacatgtctg gagggattcg aaggcaagaa ttgcgaactg      180
   tttactagaa aactgtgtag cctggataac ggcgactgcg atcagttctg tcacgaggaa      240
   cagaattcag tggtctgcag ctgtgccagg ggatacaccc tggccgacaa cggcaaggct      300
   tgcatcccta ccggccccta tccttgtggg aaacagacac tggagcggag aaagaggatc      360
   gtgggcgggc aggagtgcaa ggatggagaa tgtccatggc aggccctgct gattaacgag      420
   gaaaatgagg gcttctgcgg aggcactatc ctgtccgaat tttacattct gaccgccgct      480
   cattgtctgt atcaggccaa gcggttcaag gtgcgggtcg gcgacagaaa cacagagcag      540
   gaggaagggg gagaagctgt gcacgaggtc gaagtggtca tcaagcataa tcgcttcact      600
   aaagagacct acgactttga tatcgcagtg ctgagactga agacaccaat tactttcagg      660
   atgaatgtcg caccagcatg cctgccagag cgagattggg ctgaatccac cctgatgaca      720
   cagaaaactg gcattgtgtc tgggtttgga cggacacacg agaaggggag gcagagcact      780
   cgcctgaaaa tgctggaagt gccctacgtc gacaggaact cctgtaagct gagctcctct      840
   ttcatcatta cacagaatat gttttgcgca gggtatgaca ctaagcagga ggatgcctgt      900
   cagggagact ctggaggacc tcacgtgacc cgcttcaaag atacttattt tgtgaccgga      960
   atcgtcagtt ggggagaggg atgcgctcga aaggggaaat acggaatcta taccaaggtg     1020
   acagcattcc tgaaatggat tgaccgaagt atgaagaccc ggggcctgcc taaggctaaa     1080
   tcacatgcac cagaagtgat cacaagttca cccctgaag                            1119
<210> 80
   <211> 1128
   <212> DNA
   <213> Artificial Sequence
<220>
   <223> Séquence nucléique codant pour la protéine SEQ ID NO:63
<400> 80
   atgagatgga gttggatttt tctgctgctg ctgagtatta cctctgctaa cgcaaacaag      60
   tataaagacg gagaccagtg tgagactagc ccttgccaga accaggggaa gtgtaaagat     120
   ggactgggcg agtacacctg cacatgtctg gagggattcg aaggcaagaa ttgcgaactg     180
   tttactagaa aactgtgtag cctggataac ggcgactgcg atcagttctg tcacgaggaa     240
   cagaattcag tggtctgcag ctgtgccagg ggatacaccc tggccgacaa cggcaaggct     300
   tgcatcccta ccggccccta tccttgtggg aaacagacac tggagcggag aaagaggcgc     360
   aaacggatcg tgggcgggca ggagtgcaag gatggagaat gtccatggca ggccctgctg     420
   attaacgagg aaaatgaggg cttctgcgga ggcactatcc tgtccgaatt ttacattctg     480
   accgccgctc attgtctgta tcaggccaag cggttcaagg tgcgggtcgg cgacagaaac     540
   acagagcagg aggaaggggg agaagctgtg cacgaggtcg aagtggtcat caagcataat     600
   cgcttcacta aagagaccta cgactttgat atcgcagtgc tgagactgaa gacaccaatt     660
   actttcagga tgaatgtcgc accagcatgc ctgccagagc gagattgggc tgaatccacc     720
   ctgatgacac agaaaactgg cattgtgtct gggtttggac ggacacacga gaaggggagg     780
   cagagcactc gcctgaaaat gctggaagtg ccctacgtcg acaggaactc ctgtaagctg     840
   agctcctctt tcatcattac acagaatatg ttttgcgcag ggtatgacac taagcaggag     900
   gatgcctgtc agggagactc tggaggacct cacgtgaccc gcttcaaaga tacttatttt     960
   gtgaccggaa tcgtcagttg ggagagggga tgcgctcgaa aggggaaata cggaatctat    1020
   accaaggtga cagcattcct gaaatggatt gaccgaagta tgaagacccg gggcctgcct    1080
   aaggctaaat cacatgcacc agaagtgatc acaagttcac ccctgaag                 1128
<210> 81
   <211> 1134
   <212> DNA
   <213> Artificial Sequence
<220>
   <223> Séquence nucléique codant pour la protéine SEQ ID NO:65
<400> 81
   atgcgatgga gttggatctt tctgctgctg ctgagtatta ccagtgcaaa tgctaacaag      60
   tacaaggacg gggaccagtg tgagacctcc ccttgccaga accagggcaa gtgtaaagat     120
   ggactgggcg agtacacctg cacatgtctg gagggattcg aaggcaagaa ttgcgaactg     180
   tttactagaa aactgtgttc tctggataac ggggactgcg atcagttctg tcacgaggaa     240
   cagaattccg tggtctgctc ttgtgccagg gggtacaccc tggccgacaa cggaaaggct     300
   tgcatcccta ccggacccta tccttgtggc aaacagacac tggagcgagg gagctccgga     360
   cgaagaaagc gaatcgtggg aggacaggag tgcaaagatg gggaatgtcc atggcaggcc      420
   ctgctgatta acgaggaaaa tgagggattc tgcggaggca ctatcctgag cgaattttac     480
   attctgaccg ccgctcattg tctgtatcag gccaagaggt ttaaagtgcg cgtcggcgac     540
   cgaaacacag agcaggagga agggggagaa gctgtgcacg aggtcgaagt ggtcatcaag     600
   cataatcgct tcactaaaga gacctacgac tttgatatcg cagtgctgag actgaagaca     660
   ccaattactt tcaggatgaa tgtcgcacca gcatgcctgc cagagcgaga ttgggctgaa     720
   agtaccctga tgacacagaa gactggaatt gtgtcagggt ttggacggac acacgagaag     780
   ggccggcagt ctactagact gaaaatgctg gaagtgccct acgtcgacag gaacagttgt     840
   aagctgtcta gttcattcat catcacacag aacatgtttt gcgcaggcta tgacactaag     900
   caggaggatg cctgtcaggg ggacagcgga ggacctcacg tgacccgctt caaagatact     960
   tattttgtga ccggaatcgt cagctgggga gagggatgcg ctcgaaaggg caaatacggg    1020
   atctatacca aggtgacagc attcctgaaa tggattgaca ggagcatgaa gacccgcggc    1080
   ctgcctaagg ctaaatccca tgcaccagaa gtgatcacaa gctcccccct gaag           1134
<210> 82
   <211> 1152
   <212> DNA
   <213> Artificial Sequence
<220>
   <223> Séquence nucléique codant pour la protéine SEQ ID NO:66
<400> 82
   atgagatgga gttggatttt cctgctgctg ctgagtatca ccagtgccaa tgcaaacaag      60
   tataaagacg gagaccagtg cgagacatcc ccttgccaga accagggcaa gtgtaaagat     120
   gggctgggag agtacacctg cacatgtctg gaggggttcg aaggaaagaa ttgcgaactg     180
   tttactagaa aactgtgttc tctggataac ggggactgcg atcagttctg tcacgaggaa     240
   cagaattcag tggtctgcag ctgtgccagg gggtacaccc tggccgacaa cggaaaggct     300
   tgcatcccca ccggacccta tccttgtggc aaacagacac tggagagagg aggaggagga     360
   tccggaggag gagggtctcg gagaaagagg atcgtgggag gccaggagtg caaagatggg     420
   gaatgtcctt ggcaggccct gctgattaac gaggaaaatg agggattctg cgggggaact     480
   atcctgagcg aattttacat tctgaccgcc gctcactgtc tgtatcaggc caagaggttt     540
   aaagtgcgcg tcggcgaccg aaacacagag caggaggaag gcggggaagc tgtgcacgag     600
   gtcgaagtgg tcatcaagca taatcgcttc actaaagaga cctacgactt tgatatcgca     660
   gtgctgcgac tgaagacacc aattactttc cggatgaatg tcgcaccagc atgcctgcca     720
   gagcgagatt gggctgaaag taccctgatg acacagaaga ctggaattgt gtcaggcttt     780
   gggcggacac atgagaaggg ccggcagtct actagactga aaatgctgga agtgccttac     840
   gtcgacagga acagttgtaa gctgagctcc tctttcatca ttacacagaa tatgttttgc     900
   gcaggctatg acactaagca ggaggatgca tgtcaggggg acagcggagg accacacgtg     960
   acccgcttca aagatactta ttttgtgacc ggaatcgtca gctggggaga gggatgcgct    1020
   cgaaagggca aatacgggat ctataccaag gtgacagcat tcctgaaatg gattgacagg    1080
   agcatgaaga cccgcggcct gcctaaggct aaatcccatg caccagaagt gatcacaagt    1140
   tcacccctga ag                                                        1152
<210> 83
   <211> 1161
   <212> DNA
   <213> Artificial Sequence
<220>
   <223> Séquence nucléique codant pour la protéine SEQ ID NO:67
<400> 83
   atgcgatgga gctggatttt tctgctgctg ctgtcaatta catctgccaa cgccaacaag      60
   tacaaggacg gggatcagtg cgagacctcc ccttgccaga accagggcaa gtgtaaagat     120
   gggctgggag agtacacctg cacatgtctg gaggggttcg aaggaaagaa ttgcgaactg     180
   tttactcgca aactgtgttc tctggataac ggggactgcg atcagttctg tcacgaggaa     240
   cagaattcag tggtctgcag ctgtgccaga ggctacaccc tggccgacaa cggaaaggct     300
   tgcatcccca ccggacccta tccttgtggc aagcagacac tggagcgaag aaaacgagga     360
   ggaggaggat ccggaggagg agggtctagg cgcaagcgaa tcgtgggagg ccaggagtgc     420
   aaagatgggg aatgtccttg gcaggccctg ctgattaacg aggaaaatga gggattctgc     480
   gggggaacta tcctgagcga attttacatt ctgaccgccg ctcactgtct gtatcaggcc     540
   aagcggttca aggtgcgggt cggcgacaga aacacagagc aggaggaagg cggggaagct     600
   gtgcacgagg tcgaagtggt catcaagcat aatagattca ctaaagagac ctacgacttt     660
   gatatcgcag tgctgagact gaagacacca attactttca ggatgaatgt cgcaccagcc     720
   tgcctgcccg agagagattg ggctgaaagt accctgatga cacagaaaac tggaattgtg     780
   tcaggctttg ggaggacaca tgagaagggc aggcagtcta ctcgcctgaa aatgctggaa     840
   gtgccttacg tcgacaggaa cagttgtaag ctgagctcct ctttcatcat tacacagaat     900
   atgttttgcg caggctatga cactaagcag gaggatgcat gtcaggggga cagcggagga     960
   ccacacgtga cccgcttcaa agatacttat tttgtgaccg gaatcgtcag ctggggagag    1020
   ggatgcgctc gaaagggcaa atacgggatc tataccaagg tgacagcatt cctgaaatgg    1080
   attgaccgaa gcatgaagac ccggggcctg cctaaggcta aatcccatgc accagaagtg    1140
   atcacaagtt cacccctgaa g                                            1161
<210> 84
   <211> 1812
   <212> DNA
   <213> Artificial Sequence
<220>
   <223> Séquence nucléique codant pour la protéine SEQ ID NO:68
<400> 84
   atgagatgga gttggatttt tctgctgctg ctgagtatta cctctgctaa cgcaagcaat      60
   aaatacaaag atggcgacca gtgtgagacc agtccttgcc agaaccaggg caaatgtaaa     120
   gacggcctcg gggaatacac ctgcacctgt ttagaaggat tcgaaggcaa aaactgtgaa     180
   ttattcacac ggaagctctg cagcctggac aacggggact gtgaccagtt ctgccacgag     240
   gaacagaact ctgtggtgtg ctcctgcgcc cgcgggtaca ccctggctga caacggcaag     300
   gcctgcattc ccacagggcc ctacccctgt gggaaacaga ccctggaacg caggaagagg     360
   aggaagagga tcgtgggagg ccaggaatgc aaggacgggg agtgtccctg gcaggccctg     420
   ctcatcaatg aggaaaacga gggtttctgt ggtggaacca ttctgagcga gttctacatc     480
   ctaacggcag cccactgtct ctaccaagcc aagagattca aggtgagggt aggggaccgg     540
   aacacggagc aggaggaggg cggtgaggcg gtgcacgagg tggaggtggt catcaagcac     600
   aaccggttca caaaggagac ctatgacttc gacatcgccg tgctccggct caagaccccc     660
   atcaccttcc gcatgaacgt ggcgcctgcc tgcctccccg agcgtgactg ggccgagtcc     720
   acgctgatga cgcagaagac ggggattgtg agcggcttcg ggcgcaccca cgagaagggc     780
   cggcagtcca ccaggctcaa gatgctggag gtgccctacg tggaccgcaa cagctgcaag     840
   ctgtccagca gcttcatcat cacccagaac atgttctgtg ccggctacga caccaagcag     900
   gaggatgcct gccaggggga cagcgggggc ccgcacgtca cccgcttcaa ggacacctac     960
   ttcgtgacag gcatcgtcag ctggggagag ggctgtgccc gtaaggggaa gtacgggatc    1020
   tacaccaagg tcaccgcctt cctcaagtgg atcgacaggt ccatgaaaac caggggcttg    1080
   cccaaggcca agagccatgc cccggaggtc ataacgtcct ctccattaaa ggacaagaca    1140
   cacacatgcc ctccttgtcc agcccctgag ctgctgggcg gcccctccgt gttcctgttc    1200
   ccccccaagc ctaaggatac cctgatgatc agcagaaccc ccgaggtgac ctgcgtggtg    1260
   gtggacgtgt cccacgagga tcccgaggtg aagttcaact ggtacgtgga cggcgtggag    1320
   gtgcacaacg ctaagaccaa gcccagagag gagcagtaca acagcacata cagagtggtg    1380
   tctgtgctga ccgtgctgca ccaggactgg ctgaacggga aggagtacaa gtgcaaggtg    1440
   tccaacaagg ccctgcctgc ccctatcgag aagaccatct ctaaggctaa ggggcagccc    1500
   cgggagccac aggtgtacac cctgccaccc agccgcgacg agctgaccaa gaaccaggtg    1560
   tccctgacat gcctggtgaa gggattctac cccagcgaca tcgccgtgga gtgggagagc    1620
   aacggccagc ccgagaacaa ctacaagaca acccctcccg tgctggacag cgatggatcc    1680
   ttcttcctgt actccaagct gaccgtggac aagagcaggt ggcagcaggg aaacgtgttc    1740
   tcttgttccg tgatgcacga ggctctgcac aaccactaca cccagaagtc cctgagcctg    1800
   tctccaggca ag                                                        1812
<210> 85
   <211> 1827
   <212> DNA
   <213> Artificial Sequence
<220>
   <223> Séquence nucléique codant pour la protéine SEQ ID NO:69
<400> 85
   atgagatgga gttggatttt tctgctgctg ctgagtatta cctctgctaa cgcaagcaat 60
   aagtataaag acggggatca gtgcgagacc tccccatgtc agaaccaggg caagtgcaaa 120
   gacgggctgg gagagtacac atgcacttgt ctggaggggt tcgaaggaaa gaattgcgaa 180
   ctgtttacaa gaaagctgtg cagcctggat aacggcgact gcgatcagtt ctgtcacgag 240
   gaacagaata gtgtggtctg ctcatgtgcc agggggtaca ctctggctga caacggaaag 300
   gcatgcatcc ctactggacc ttatccatgt ggcaaacaga ccctggagcg gagaaagagg 360
   cgcaaacgca tcgtgggcgg gcaggagtgc aaggatggag aatgtccatg gcaggctctg 420
   ctgattaacg aggaaaatga gggcttctgc ggaggcacaa tcctgagcga gttctacatt 480
   ctgactgccg ctcattgtct gtatcaggct aagcggttca aggtgcgggt cggcgacaga 540
   aacaccgagc aggaggaagg gggagaagca gtgcacgagg tcgaagtggt catcaagcat 600
   aatcgcttca caaaagagac ttacgacttt gatatcgccg tgctgagact gaagaccccc 660
   attacattca ggatgaatgt ggcaccagca tgcctgcctg agcgagattg ggctgaatca 720
   actctgatga cccagaaaac aggaattgtg agcggctttg ggcgaactca cgagaagggc 780
   aggcagagca cccgcctgaa aatgctggaa gtgccctacg tcgaccggaa cagctgtaag 840
   ctgagctcct ctttcatcat tacccagaat atgttttgcg ccggctatga cacaaagcag 900
   gaggatgctt gtcaggggga ctccggagga cctcatgtga ccagattcaa agatacatat 960
   tttgtgactg gcatcgtctc ttggggagaa ggctgcgcca ggaagggcaa atacgggatc 1020
   tatactaagg tgaccgcctt cctgaaatgg attgatcgat ccatgaagac tcggggcctg 1080
   ccaaaggcaa aatctcacgc ccccgaagtg atcaccagtt cacctctgaa ggaacctaag 1140
   tcttgcgaca aaacccatac atgcccacct tgtccagcac ctgaactgct gggaggacca 1200
   tccgtgttcc tgtttccacc caagcccaaa gatacactga tgattagtcg gacccctgag 1260
   gtgacatgcg tggtcgtgga tgtctcacac gaggacccag aagtgaagtt taactggtac 1320
   gtggacggcg tggaagtcca taatgccaag accaaacctc gcgaggaaca gtacaacagt 1380
   acatatcgag tcgtgtcagt gctgactgtc ctgcaccagg attggctgaa cggaaaggag 1440
   tataagtgca aagtgagcaa taaggctctg ccagcaccca tcgagaaaac aatttccaag 1500
   gcaaaaggcc agccaaggga accccaggtg tacactctgc ctccaagccg cgatgagctg 1560
   acaaagaacc aggtgtccct gacttgtctg gtcaaagggt tctatccctc cgacatcgcc 1620
   gtggagtggg aatctaatgg acagcctgag aacaattaca agaccacacc ccctgtgctg 1680
   gactcagatg ggagcttctt tctgtattct aagctgactg tggacaaaag tagatggcag 1740
   cagggaaacg tgttttcttg cagtgtcatg cacgaggccc tgcacaatca ttacacccag 1800
   aagtcactga gcctgtcccc aggaaag 1827
<210> 86
   <211> 1824
   <212> DNA
   <213> Artificial Sequence
<220>
   <223> Séquence nucléique codant pour la protéine SEQ ID NO:70
<400> 86
   atgagatgga gttggatttt tctgctgctg ctgagtatta cctctgctaa cgcaaataag 60
   tataaagacg gggatcagtg cgagacctct ccttgtcaga accagggcaa gtgcaaagac 120
   gggctgggag agtacacatg cacttgtctg gaggggttcg aaggaaagaa ttgcgaactg 180
   tttacaagaa aactgtgtag cctggataac ggggactgcg atcagttctg tcacgaggaa 240
   cagaattccg tggtctgctc ttgtgcaagg gggtacaccc tggctgacaa cggaaaggca 300
   tgcatcccta ctggacctta tccatgtggc aaacagaccc tggagcggag aaagaggcgc 360
   aaacgcatcg tgggcgggca ggagtgcaag gatggagaat gtccatggca ggctctgctg 420
   attaacgagg aaaatgaggg cttctgcgga ggcacaatcc tgagcgaatt ttacattctg 480
   actgccgctc attgtctgta tcaggctaag cggttcaagg tgcgggtcgg cgacagaaac 540
   accgagcagg aggaaggggg agaagcagtg cacgaggtcg aagtggtcat caagcataat 600
   cgcttcacaa aagagactta cgactttgat atcgccgtgc tgagactgaa gacccccatt 660
   acattcagga tgaatgtggc accagcatgc ctgcctgagc gagattgggc tgaatctact 720
   ctgatgaccc agaaaacagg aattgtgagt ggctttgggc gaactcacga gaagggcagg 780
   cagtctaccc gcctgaaaat gctggaagtg ccctacgtcg accggaacag ctgtaagctg 840
   agctcctctt tcatcattac ccagaatatg ttttgcgccg gctatgacac aaagcaggag 900
   gatgcttgtc agggggacag cggaggacct catgtgacca gattcaaaga tacatatttt 960
   gtgactggca tcgtctcctg gggagaaggc tgcgcaagga agggcaaata cgggatctat 1020
   actaaggtga ccgccttcct gaaatggatt gatcgatcaa tgaagactcg gggcctgcca 1080
   aaggcaaaaa gccacgcccc cgaagtgatc accagttcac ctctgaagga accaaagagc 1140
   tgcgacaaaa cccatacatg cccaccttgt ccagcacctg aactgctggg aggaccatcc 1200
   gtgttcctgt ttccacccaa gcccaaagat acactgatga tttcccggac ccctgaggtg 1260
   acatgtgtgg tcgtggatgt ctctcacgag gacccagaag tgaagtttaa ctggtacgtg 1320
   gacggcgtgg aagtccataa tgccaagacc aaaccccgcg aggaacagta caactccaca 1380
   tatcgagtcg tgtctgtgct gactgtcctg caccaggatt ggctgaacgg aaaagagtac 1440
   aagtgcaaag tgagtaataa ggctctgcca gcacccatcg agaaaacaat ttccaaggca 1500
   aaaggccagc caagggaacc ccaggtgtac actctgcctc caagtcgcga tgagctgaca 1560
   aagaaccagg tgtcactgac ttgtctggtc aaagggttct atccctcaga catcgccgtg 1620
   gagtgggaaa gcaatggaca gcctgagaac aattacaaga ccacaccccc tgtgctggac 1680
   tctgatggga gtttctttct gtatagcaag ctgactgtgg acaaatccag atggcagcag 1740
   ggaaacgtgt tttcttgcag tgtcatgcac gaggccctgc acaatcatta cacccagaag 1800
   tcactgagcc tgtccccagg aaag 1824
<210> 87
   <211> 2547
   <212> DNA
   <213> Artificial Sequence
<220>
   <223> Séquence nucléique codant pour la protéine SEQ ID NO:71
<400> 87
   atgagatgga gttggatttt tctgctgctg ctgagtatta cctctgctaa cgcaagcaat      60
   aagtataaag acggggatca gtgcgagacc tccccatgtc agaaccaggg caagtgcaaa     120
   gacgggctgg gagagtacac atgcacttgt ctggaggggt tcgaaggaaa gaattgcgaa     180
   ctgtttacaa gaaagctgtg cagcctggat aacggcgact gcgatcagtt ctgtcacgag     240
   gaacagaata gtgtggtctg ctcatgtgcc agggggtaca ctctggctga caacggaaag     300
   gcatgcatcc ctactggacc ttatccatgt ggcaaacaga ccctggagcg gagaaagagg     360
   cgcaaacgca tcgtgggcgg gcaggagtgc aaggatggag aatgtccatg gcaggctctg     420
   ctgattaacg aggaaaatga gggcttctgc ggaggcacaa tcctgagcga gttctacatt     480
   ctgactgccg ctcattgtct gtatcaggct aagcggttca aggtgcgggt cggcgacaga     540
   aacaccgagc aggaggaagg gggagaagca gtgcacgagg tcgaagtggt catcaagcat     600
   aatcgcttca caaaagagac ttacgacttt gatatcgccg tgctgagact gaagaccccc     660
   attacattca ggatgaatgt ggcaccagca tgcctgcctg agcgagattg ggctgaatca     720
   actctgatga cccagaaaac aggaattgtg agcggctttg ggcgaactca cgagaagggc     780
   aggcagagca cccgcctgaa aatgctggaa gtgccctacg tcgaccggaa cagctgtaag     840
   ctgagctcct ctttcatcat tacccagaat atgttttgcg ccggctatga cacaaagcag     900
   gaggatgctt gtcaggggga ctccggagga cctcatgtga ccagattcaa agatacatat     960
   tttgtgactg gcatcgtctc ttggggagaa ggctgcgcca ggaagggcaa atacgggatc    1020
   tatactaagg tgaccgcctt cctgaaatgg attgatcgat ccatgaagac tcggggcctg    1080
   ccaaaggcaa aatctcacgc ccccgaagtg atcaccagtt cacctctgaa ggacaaaacc    1140
   catacatgcc caccttgtcc agcacctgaa ctgctgggag gaccatccgt gttcctgttt    1200
   ccacccaagc ccaaagatac actgatgatt agtcggaccc ctgaggtgac atgcgtggtc    1260
   gtggatgtct cacacgagga cccagaagtg aagtttaact ggtacgtgga cggcgtggaa    1320
   gtccataatg ccaagaccaa acctcgcgag gaacagtaca acagtacata tcgagtcgtg    1380
   tcagtgctga ctgtcctgca ccaggattgg ctgaacggaa aggagtataa gtgcaaagtg 1440
   agcaataagg ctctgccagc acccatcgag aaaacaattt ccaaggcaaa aggccagcca 1500
   agggaacccc aggtgtacac tctgcctcca agccgcgatg agctgacaaa gaaccaggtg 1560
   tccctgactt gtctggtcaa agggttctat ccctccgaca tcgccgtgga gtgggaatct 1620
   aatggacagc ctgagaacaa ttacaagacc acaccccctg tgctggactc agatgggagc 1680
   ttctttctgt attctaagct gactgtggac aaaagtagat ggcagcaggg aaacgtgttt 1740
   tcttgcagtg tcatgcacga ggccctgcac aatcattaca cccagaagtc actgagcctg 1800
   tccccaggag gaggaggagg aagcggagga ggaggctccg ggggaggcgg gtctgagccc 1860
   aagagctccg ataaaactca tacctgccca ccctgtcctg ctccagaact gctgggaggc 1920
   cctagcgtgt tcctgtttcc tccaaagcca aaagacacac tgatgatttc taggactccc 1980
   gaggtgacct gcgtggtggt cgatgtcagt cacgaggacc ctgaagtgaa gttcaactgg 2040
   tacgtggatg gagtcgaggt gcacaacgcc aagaccaaac cccgggagga acagtacaac 2100
   agcacctata gagtggtctc cgtgctgaca gtcctgcacc aggactggct gaacgggaag 2160
   gaatacaagt gcaaagtgtc caataaggcc ctgcccgctc ctatcgaaaa aaccatttct 2220
   aaggctaaag gccagccccg ggagccacag gtgtacacac tgcccccttc tcgggatgaa 2280
   ctgaccaaga accaggtgag tctgacatgt ctggtcaaag gcttctatcc aagtgacatc 2340
   gcagtggagt gggaatcaaa tgggcagccc gagaacaatt acaagactac cccacccgtg 2400
   ctggactccg atggctcttt ctttctgtat tcaaagctga ccgtggacaa aagcagatgg 2460
   cagcagggga acgtgttcag ctgcagtgtc atgcacgaag cactgcacaa tcattacact 2520
   cagaaatcac tgtcactgtc acctgga 2547
<210> 88
   <211> 2517
   <212> DNA
   <213> Artificial Sequence
<220>
   <223> Séquence nucléique codant pour la protéine SEQ ID NO:72
<400> 88
   atgagatgga gttggatttt tctgctgctg ctgagtatta cctctgctaa cgcaagcaat        60
   aagtataaag acggggatca gtgcgagacc tccccatgtc agaaccaggg caagtgcaaa       120
   gacgggctgg gagagtacac atgcacttgt ctggaggggt tcgaaggaaa gaattgcgaa       180
   ctgtttacaa gaaagctgtg cagcctggat aacggcgact gcgatcagtt ctgtcacgag       240
   gaacagaata gtgtggtctg ctcatgtgcc agggggtaca ctctggctga caacggaaag       300
   gcatgcatcc ctactggacc ttatccatgt ggcaaacaga ccctggagcg gagaaagagg       360
   cgcaaacgca tcgtgggcgg gcaggagtgc aaggatggag aatgtccatg gcaggctctg       420
   ctgattaacg aggaaaatga gggcttctgc ggaggcacaa tcctgagcga gttctacatt 480
   ctgactgccg ctcattgtct gtatcaggct aagcggttca aggtgcgggt cggcgacaga 540
   aacaccgagc aggaggaagg gggagaagca gtgcacgagg tcgaagtggt catcaagcat 600
   aatcgcttca caaaagagac ttacgacttt gatatcgccg tgctgagact gaagaccccc 660
   attacattca ggatgaatgt ggcaccagca tgcctgcctg agcgagattg ggctgaatca 720
   actctgatga cccagaaaac aggaattgtg agcggctttg ggcgaactca cgagaagggc 780
   aggcagagca cccgcctgaa aatgctggaa gtgccctacg tcgaccggaa cagctgtaag 840
   ctgagctcct ctttcatcat tacccagaat atgttttgcg ccggctatga cacaaagcag      900
   gaggatgctt gtcaggggga ctccggagga cctcatgtga ccagattcaa agatacatat      960
   tttgtgactg gcatcgtctc ttggggagaa ggctgcgcca ggaagggcaa atacgggatc 1020
   tatactaagg tgaccgcctt cctgaaatgg attgatcgat ccatgaagac tcggggcctg 1080
   ccaaaggcaa aatctcacgc ccccgaagtg atcaccagtt cacctctgaa ggacaaaacc 1140
   catacatgcc caccttgtcc agcacctgaa ctgctgggag gaccatccgt gttcctgttt 1200
   ccacccaagc ccaaagatac actgatgatt agtcggaccc ctgaggtgac atgcgtggtc 1260
   gtggatgtct cacacgagga cccagaagtg aagtttaact ggtacgtgga cggcgtggaa 1320
   gtccataatg ccaagaccaa acctcgcgag gaacagtaca acagtacata tcgagtcgtg 1380
   tcagtgctga ctgtcctgca ccaggattgg ctgaacggaa aggagtataa gtgcaaagtg 1440
   agcaataagg ctctgccagc acccatcgag aaaacaattt ccaaggcaaa aggccagcca 1500
   agggaacccc aggtgtacac tctgcctcca agccgcgatg agctgacaaa gaaccaggtg 1560
   tccctgactt gtctggtcaa agggttctat ccctccgaca tcgccgtgga gtgggaatct 1620
   aatggacagc ctgagaacaa ttacaagacc acaccccctg tgctggactc agatgggagc 1680
   ttctttctgt attctaagct gactgtggac aaaagtagat ggcagcaggg aaacgtgttt 1740
   tcttgcagtg tcatgcacga ggccctgcac aatcattaca cccagaagtc actgagcctg 1800
   tccccaggag gaggaggagg aagcgagccc aagagctccg ataaaactca tacctgccca 1860
   ccctgtcctg ctccagaact gctgggaggc cctagcgtgt tcctgtttcc tccaaagcca 1920
   aaagacacac tgatgatttc taggactccc gaggtgacct gcgtggtggt cgatgtcagt 1980
   cacgaggacc ctgaagtgaa gttcaactgg tacgtggatg gagtcgaggt gcacaacgcc 2040
   aagaccaaac cccgggagga acagtacaac agcacctata gagtggtctc cgtgctgaca 2100
   gtcctgcacc aggactggct gaacgggaag gaatacaagt gcaaagtgtc caataaggcc 2160
   ctgcccgctc ctatcgaaaa aaccatttct aaggctaaag gccagccccg ggagccacag     2220
   gtgtacacac tgcccccttc tcgggatgaa ctgaccaaga accaggtgag tctgacatgt     2280
   ctggtcaaag gcttctatcc aagtgacatc gcagtggagt gggaatcaaa tgggcagccc 2340
   gagaacaatt acaagactac cccacccgtg ctggactccg atggctcttt ctttctgtat 2400
   tcaaagctga ccgtggacaa aagcagatgg cagcagggga acgtgttcag ctgcagtgtc 2460
   atgcacgaag cactgcacaa tcattacact cagaaatcac tgtcactgtc acctgga 2517
<210> 89
   <211> 2559
   <212> DNA
   <213> Artificial Sequence
<220>
   <223> Séquence nucléique codant pour la protéine SEQ ID NO:73
<400> 89
   atgcgatggt cctggatttt tctgctgctg ctgagtatta cctctgccaa cgccgataag 60
   acacacactt gcccaccctg ccctgcccct gagctgctgg gcgggccaag cgtgttcctg 120
   tttcccccta agccaaaaga tacactgatg atcagtagaa ctcccgaagt gacctgcgtg 180
   gtcgtggacg tctcacacga ggaccccgaa gtgaagttca actggtacgt ggacggcgtg 240
   gaggtccata atgccaagac caaaccccgc gaggaacagt acaactccac ctatcgagtc 300
   gtgtctgtgc tgacagtcct gcaccaggat tggctgaacg gcaaggagta taagtgcaaa 360
   gtgtctaata aggctctgcc agcacccatc gagaaaacca ttagtaaggc aaaagggcag 420
   cctagggaac cacaggtgta cacactgcca cccagtcgcg atgagctgac taagaaccag 480
   gtgtcactga cctgtctggt caaaggattc tatccttcag acatcgccgt ggagtgggaa 540
   agcaatggcc agccagagaa caattacaag accacacctc cagtgctgga ctctgatgga 600
   agtttctttc tgtatagcaa gctgactgtg gacaaatcca gatggcagca gggcaacgtg 660
   ttttcttgca gtgtcatgca cgaggccctg cacaatcatt acacccagaa gtcactgagc 720
   ctgtcccctg gaggaggagg aggcagtgga ggaggagggt caggaggcgg gggaagcgag 780
   ccaaagagct ccgataaaac acatacttgc cccccttgtc ctgctccaga actgctggga 840
   ggaccttccg tgttcctgtt tccacccaag cctaaagaca cactgatgat ttccaggaca 900
   ccagaagtga cttgtgtcgt ggtcgacgtg tctcatgagg accccgaggt gaagtttaac 960
   tggtacgtgg atggagtcga agtgcacaac gccaagacca aaccccggga ggaacagtac 1020
   aatagtactt atagagtggt ctcagtgctg accgtcctgc accaggactg gctgaatggc 1080
   aaggaatata agtgcaaagt gagcaataag gccctgcccg ctcctatcga aaaaactatt 1140
   tccaaggcta aaggccagcc ccgagagcct caggtgtaca ccctgcctcc aagccgggat 1200
   gaactgacaa agaaccaggt gtccctgact tgtctggtca aagggttcta tccctccgac 1260
   atcgcagtgg agtgggaatc taatggacag cctgagaaca attacaagac taccccccct 1320
   gtgctggact cagatgggag cttctttctg tattctaagc tgaccgtgga taaaagtcgc 1380
   tggcagcagg gaaatgtgtt ttcttgtagt gtcatgcacg aagccctgca taaccactat     1440
   actcaaaagt cactgagcct gagcccagga ggaggaggag gctccaacaa gtataaagac     1500
   ggggatcagt gcgagacatc tccttgtcag aatcagggaa agtgtaaaga cggcctgggg     1560
   gagtacacct gcacatgtct ggagggcttc gaagggaaga actgcgaact gtttacaaga     1620
   aaactgtgta gcctggataa cggcgactgc gatcagttct gtcatgagga acagaattcc     1680
   gtggtctgct cttgtgccag gggctacacc ctggctgaca atgggaaggc atgcatccct     1740
   accgggccat atccctgtgg aaaacagaca ctggagcgga gaaagaggcg caaacggatc     1800
   gtggggggac aggagtgcaa ggatggcgaa tgtccatggc aggctctgct gattaacgag     1860
   gaaaatgagg ggttctgcgg cgggactatc ctgtccgaat tttacattct gaccgccgct     1920
   cactgtctgt atcaggctaa gcggttcaag gtgcgggtcg gcgacagaaa caccgagcag     1980
   gaggaaggag gcgaagcagt gcacgaggtc gaagtggtca tcaagcataa tcgcttcact     2040
   aaagagacct acgactttga tatcgccgtg ctgagactga agacacccat tactttcagg     2100
   atgaacgtgg caccagcatg cctgccagag cgagattggg ctgaatctac cctgatgaca     2160
   cagaaaactg gcattgtgag tggatttggc cgaacacatg agaaggggag gcagtctact     2220
   cgcctgaaaa tgctggaagt gccctacgtc gaccggaact cctgtaagct gtctagttca     2280
   ttcatcatca cacagaacat gttttgcgcc ggatatgaca ctaagcagga agatgcttgt     2340
   cagggcgaca gcggaggacc tcacgtgacc agattcaaag atacctattt tgtgacaggc     2400
   atcgtctcct ggggggaggg atgcgcaagg aagggaaaat acggcatcta taccaaggtg     2460
   acagccttcc tgaaatggat tgaccgatca atgaagacac ggggcctgcc caaggcaaaa     2520
   agccatgccc ctgaagtgat cactagctcc ccactgaaa                            2559
<210> 90
   <211> 2550
   <212> DNA
   <213> Artificial Sequence
<220>
   <223> Séquence nucléique codant pour la protéine SEQ ID NO:74
<400> 90
   atgcgatggt cctggatttt tctgctgctg ctgagtatta cctctgccaa cgccgataag      60
   acacacactt gcccaccctg ccctgcccct gagctgctgg gcgggccaag cgtgttcctg     120
   tttcccccta agccaaaaga tacactgatg atcagtagaa ctcccgaagt gacctgcgtg     180
   gtcgtggacg tctcacacga ggaccccgaa gtgaagttca actggtacgt ggacggcgtg     240
   gaggtccata atgccaagac caaaccccgc gaggaacagt acaactccac ctatcgagtc     300
   gtgtctgtgc tgacagtcct gcaccaggat tggctgaacg gcaaggagta taagtgcaaa     360
   gtgtctaata aggctctgcc agcacccatc gagaaaacca ttagtaaggc aaaagggcag 420
   cctagggaac cacaggtgta cacactgcca cccagtcgcg atgagctgac taagaaccag 480
   gtgtcactga cctgtctggt caaaggattc tatccttcag acatcgccgt ggagtgggaa 540
   agcaatggcc agccagagaa caattacaag accacacctc cagtgctgga ctctgatgga 600
   agtttctttc tgtatagcaa gctgactgtg gacaaatcca gatggcagca gggcaacgtg 660
   ttttcttgca gtgtcatgca cgaggccctg cacaatcatt acacccagaa gtcactgagc 720
   ctgtcccctg gaggaggagg aggcagtgga ggaggagggt caggaggcgg gggaagcgag 780
   ccaaagagct ccgataaaac acatacttgc cccccttgtc ctgctccaga actgctggga 840
   ggaccttccg tgttcctgtt tccacccaag cctaaagaca cactgatgat ttccaggaca 900
   ccagaagtga cttgtgtcgt ggtcgacgtg tctcatgagg accccgaggt gaagtttaac 960
   tggtacgtgg atggagtcga agtgcacaac gccaagacca aaccccggga ggaacagtac 1020
   aatagtactt atagagtggt ctcagtgctg accgtcctgc accaggactg gctgaatggc 1080
   aaggaatata agtgcaaagt gagcaataag gccctgcccg ctcctatcga aaaaactatt 1140
   tccaaggcta aaggccagcc ccgagagcct caggtgtaca ccctgcctcc aagccgggat 1200
   gaactgacaa agaaccaggt gtccctgact tgtctggtca aagggttcta tccctccgac 1260
   atcgcagtgg agtgggaatc taatggacag cctgagaaca attacaagac taccccccct 1320
   gtgctggact cagatgggag cttctttctg tattctaagc tgaccgtgga taaaagtcgc 1380
   tggcagcagg gaaatgtgtt ttcttgtagt gtcatgcacg aagccctgca taaccactat 1440
   actcaaaagt cactgagcct gagcccagga ggaggaggag gctccaacaa gtataaagac 1500
   ggggatcagt gcgagacatc tccttgtcag aatcagggaa agtgtaaaga cggcctgggg 1560
   gagtacacct gcacatgtct ggagggcttc gaagggaaga actgcgaact gtttacaaga 1620
   aaactgtgta gcctggataa cggcgactgc gatcagttct gtcatgagga acagaattcc 1680
   gtggtctgct cttgtgccag gggctacacc ctggctgaca atgggaaggc atgcatccct 1740
   accgggccat atccctgtgg aaaacagaca ctggagcgga gaaagaggat cgtgggggga 1800
   caggagtgca aggatggcga atgtccatgg caggctctgc tgattaacga ggaaaatgag 1860
   gggttctgcg gcgggactat cctgtccgaa ttttacattc tgaccgccgc tcactgtctg 1920
   tatcaggcta agcggttcaa ggtgcgggtc ggcgacagaa acaccgagca ggaggaagga 1980
   ggcgaagcag tgcacgaggt cgaagtggtc atcaagcata atcgcttcac taaagagacc 2040
   tacgactttg atatcgccgt gctgagactg aagacaccca ttactttcag gatgaacgtg 2100
   gcaccagcat gcctgccaga gcgagattgg gctgaatcta ccctgatgac acagaaaact 2160
   ggcattgtga gtggatttgg ccgaacacat gagaagggga ggcagtctac tcgcctgaaa 2220
   atgctggaag tgccctacgt cgaccggaac tcctgtaagc tgtctagttc attcatcatc 2280
   acacagaaca tgttttgcgc cggatatgac actaagcagg aagatgcttg tcagggcgac     2340
   agcggaggac ctcacgtgac cagattcaaa gatacctatt ttgtgacagg catcgtctcc     2400
   tggggggagg gatgcgcaag gaagggaaaa tacggcatct ataccaaggt gacagccttc     2460
   ctgaaatgga ttgaccgatc aatgaagaca cggggcctgc ccaaggcaaa aagccatgcc     2520
   cctgaagtga tcactagctc cccactgaaa                                      2550
<210> 91
   <211> 11
   <212> PRT
   <213> Artificial Sequence
<220>
   <223> Linker de l'exemple 20
<400> 91
   Gly Gly Gly Gly Ser Gly Gly Gly Gly Ser Arg
   1               5                   10
<210> 92
   <211> 8
   <212> PRT
   <213> Artificial Sequence
<220>
   <223> Séquence décrite à la figure 9
<400> 92
   Arg Lys Arg Ile Val Gly Gly Gln
   1               5
<210> 93
   <211> 12
   <212> PRT
   <213> Artificial Sequence
<220>
   <223> Séquence décrite à l'exemple 9
<400> 93
   Arg Arg Lys Arg Arg Lys Arg Ile Val Gly Gly Gln
   1               5                   10

## Description

La présente invention concerne des mutants du facteur X, et leur utilisation pour le traitement des troubles de la coagulation sanguine.

Le facteur X est une protéine présente dans le sang. Cette protéine joue un rôle important dans la cascade de la coagulation. La coagulation du sang est un processus complexe qui permet d'éviter l'écoulement du sang par les vaisseaux endommagés. Aussitôt qu'un vaisseau est brisé, les éléments responsables de la coagulation interagissent entre eux pour former un bouchon, le clou plaquettaire, à l'endroit où le vaisseau est brisé. Les facteurs de la coagulation sont requis pour tenir le clou plaquettaire en place et stabiliser le caillot.

La formation d'un caillot normal s'effectue en quatre étapes :
**Étape 1** Le vaisseau sanguin est endommagé.
**Étape 2** Le vaisseau sanguin se contracte de façon à restreindre l'apport de sang vers la zone lésée.
**Étape 3** Les plaquettes adhèrent à l'espace sous-endothélial exposé lors de la lésion du vaisseau ainsi qu'aux parois des vaisseaux sanguins stimulées. Les plaquettes s'étalent, c'est ce que l'on appelle « l'adhésion plaquettaire ». Ces plaquettes étalées libèrent des substances qui activent d'autres plaquettes avoisinantes de sorte qu'elles s'agglomèrent au siège de la lésion afin de former le clou plaquettaire. C'est ce que l'on appelle « l'agrégation plaquettaire ».
**Étape 4** La surface des plaquettes activées constitue ainsi une surface sur laquelle la coagulation du sang peut s'effectuer. Les protéines de la coagulation qui circulent dans le sang (dont le facteur X) sont activées à la surface des plaquettes et forment un caillot de fibrine.

Ces protéines de la coagulation (c'est-à-dire, les facteurs I, II, V, VIII, IX, X, XI, XII et XIII, ainsi que le facteur de Von Willebrand) fonctionnent en une réaction en chaîne, *i.e.* la cascade de la coagulation.

Le facteur X sous forme activée (Xa) intervient plus particulièrement dans l'activation de la prothrombine (facteur II) en thrombine (facteur IIa), notamment lorsqu'il est complexé avec le co-facteur V activé pour former le complexe prothrombinase. Ce facteur est un élément essentiel dans la cascade de la coagulation.

Lorsque ce facteur fait défaut, des saignements se manifestent, comme une épistaxis (saignements de nez), une hémarthrose (épanchement de sang dans une cavité articulaire) ou des saignements gastro-intestinaux. La déficience en facteur X est extrêmement rare. Sa transmission est autosomique récessive, et sa prévalence est de 1/1 000 000.

L'activation du FX (FXa) intervient :
- soit très précocement lors de l'étape d'initiation de la cascade de la coagulation par le complexe facteur VIIa/facteur tissulaire, dans une réaction peu efficace qui conduit à la formation de trace de thrombine ;
- soit lors de l'étape d'amplification de la cascade de la coagulation issue d'un rétrocontrôle positif effectué par les traces de thrombine conduisant à l'activation des facteurs VIII et IX.
Le FXa forme le complexe prothrombinase, qui catalyse la conversion de la prothrombine en thrombine. La thrombine catalyse quant à elle, la conversion du fibrinogène en fibrine, qui conduit à la formation des caillots dans le sang et à l'arrêt des saignements. L'activité du FXa peut être appelée « activité procoagulante ».

Les deux facteurs VIII et IX sont manquants chez les hémophiles A et B, causant ainsi un trouble hémorragique qui peut être fatal sans traitement. L'hémophilie A comme l'hémophilie B regroupe deux types d'hémophilie, l'hémophilie constitutionnelle et l'hémophile acquise.
L'hémophilie constitutionnelle de type A est une maladie hémorragique caractérisée par un déficit quantitatif ou qualitatif en FVIII résultant d'une anomalie du gène du FVIII. L'hémophilie constitutionnelle de type B est également une maladie hémorragique mais caractérisée par un déficit quantitatif ou qualitatif en FIX résultant d'une anomalie du gène du FIX.

L'hémophilie acquise de type A ou B se définit par l'apparition d'auto-anticorps dirigés contre ces FVIII ou FIX.
L'hémophilie se traduit par un déficit de la coagulation sanguine en réponse à une hémorragie. L'absence des facteurs VIII et IX ne permet pas de générer des quantités suffisantes de facteur X activé, pour juguler l'hémorragie.
Les patients atteints d'hémophilie A et B peuvent être traités par des concentrés comprenant respectivement du FVIII ou du FIX qui peuvent être des dérivés plasmatiques ou des produits issus du génie génétique. Ces concentrés peuvent être administrés à l'occasion de chaque hémorragie ; dans ce cas, il convient de commencer le traitement le plus rapidement possible, à l'apparition des premiers signes. Le traitement peut également être administré de façon prophylactique, régulièrement 2 à 3 fois par semaine de façon à prévenir les hémorragies. Cependant, le traitement peut donner lieu à l'apparition d'anticorps dirigés contre le FVIII ou le FIX appelés inhibiteurs. La présence de tels anticorps rend alors inefficace les administrations de facteurs VIII ou IX. Ces anticorps se développent tôt dès les premières administrations, souvent avant la dixième. Certains patients restent de faibles répondeurs (titre d'anticorps < 5 Unités Bethesda (UB)), d'autres appelés forts répondeurs atteignent des titres qui ne permettent plus de les traiter avec le facteur correspondant.

A ce jour, il n'existe pas de traitement permettant de prévenir et/ou traiter de façon satisfaisante l'existence d'un risque hémorragique chez les patients atteints d'hémophilie A ou B et présentant un inhibiteur. En effet, les produits disponibles peuvent être inefficaces (Astermark J, Donfield SM, DiMichele DM, Gringeri A, Gilbert SA, Waters J, Berntorp E, for the FSG. A randomized comparison of bypassing agents in hemophilia complicated by an inhibitor: the FEIBA NovoSeven Comparative (FENOC) Study. Blood. 2007; 109: 546-51) ou leur administration compliquée par des accidents thrombotiques (Aledort LM. Comparative thrombotic event incidence after infusion of recombinant factor VIIa versus factor VIII inhibitor bypass activity. J Thromb Haemost. 2004; 2: 1709).

FR2972114 décrit des facteurs X mutés sur la chaîne lourde, et comprenant le peptide d'activation. Il décrit notamment une chaîne lourde de facteur X mutée sur les positions 142 et 82.

Il existe donc un besoin avéré pour des alternatives thérapeutiques aux traitements existants. De telles alternatives doivent en outre présenter les avantages suivants :
- elles doivent permettre d'arrêter l'hémorragie,
- elles ne doivent pas provoquer de thrombose, et
- elles doivent permettre le traitement et/ou la prévention de l'accident hémorragique même en présence d'anticorps anti-FVIII ou FIX.

La présente invention répond à ce besoin. Elle a pour objet un facteur X modifié (appelé GPAD-FXa) consistant en la séquence SEQ ID NO :11 ou SEQ ID NO :25 ou SEQ ID NO :26, directement fusionnée, ou fusionnée via un linker, notamment -Arg-Lys-Arg-, à la séquence SEQ ID NO :6. Elle a également pour objet un facteur X (FX) modifié, ledit FX modifié (appelé GPAD-FXa) consistant en la séquence SEQ ID NO:11 directement fusionnée, ou fusionnée via un linker, notamment -Arg-Lys-Arg-, à la séquence SEQ ID NO :6.
Un tel facteur FXa modifié est notamment utile pour la prévention et/ou le traitement d'un accident hémorragique chez un patient atteint d'hémophilie A ou B.

La présente invention concerne donc une protéine consistant en la séquence SEQ ID NO :11 ou SEQ ID NO :25 ou SEQ ID NO :26, directement fusionnée, ou fusionnée via un linker, notamment -Arg-Lys-Arg-, à la séquence SEQ ID NO :6. Une telle protéine est également appelée GPAD-FXa dans la présente demande.

Des exemples de protéines FX selon l'invention sont notamment les séquences SEQ ID NO:7 et 9, 21 à 24 et 60 à 63.

Un autre objet de l'invention est un polynucléotide codant pour ladite protéine.
Un autre objet de l'invention est un vecteur d'expression comprenant ledit polynucléotide.
Un autre objet de l'invention est une cellule hôte comprenant ledit vecteur d'expression ou ledit polynucléotide.
Un autre objet de l'invention est l'utilisation de ladite protéine comme médicament. En particulier, ladite protéine peut être utilisée pour le traitement des troubles de la coagulation sanguine, notamment les troubles hémorragiques, tels que les hémophilies A, B et C (déficit en facteur XI), les déficits en facteur X, voire des besoins en coagulation d'urgence pour se substituer au Facteur VIIa. Lorsqu'une réponse procoagulante puissante et rapide est requise, ladite protéine peut être utilisée en combinaison avec d'autres molécules hémostatiques, telles que le facteur VIIa et/ou le fibrinogène, voire en association avec des composés procoagulants (transfusion de plaquettes, mélange procoagulant comme FEIBA, Kaskadil, Kanokad etc), qui pourront renforcer l'efficacité du traitement.

Tel qu'il est utilisé ici, les termes «protéine» et «polypeptide» sont utilisés ici de manière interchangeable et se réfèrent à une séquence d'acides aminés ayant plus de 100 acides aminés.

La présente invention se rapporte à un facteur X mutant consistant en la séquence SEQ ID NO :11 ou SEQ ID NO :25 ou SEQ ID NO :26, directement fusionnée, ou fusionnée via un linker, notamment -Arg-Lys-Arg-, à la séquence SEQ ID NO :6. De préférence, la présente invention se rapporte à un facteur X mutant consistant en la séquence SEQ ID NO:26 directement fusionnée, ou fusionnée via un linker -Arg-Lys-Arg-, à la séquence SEQ ID NO :6. De préférence, la présente invention se rapporte à un facteur X mutant consistant en la séquence SEQ ID NO :11 directement fusionnée, ou fusionnée via un linker -Arg-Lys-Arg-, à la séquence SEQ ID NO :6. De préférence, le GPAD-FXa selon l'invention est monocaténaire.

Le facteur X, appelé également facteur de Stuart-Prower, est codé par le gène F10 et se réfère à la sérine protéase EC3.4.21.6. Le facteur X est composé d'une chaîne lourde, de 306 acides aminés, et d'une chaîne légère, de 139 acides aminés.
Le facteur X est une protéine de 488 acides aminés, constitué d'un peptide signal, d'un propeptide, et des chaînes légère et lourde.

La séquence du facteur X humain peut être trouvée dans UniProtKB sous le numéro d'accession P00742.

La protéine est traduite sous forme de prépropeptide. Après clivage du peptide signal, le propeptide est finalement coupé, et le facteur X est clivé en une chaîne légère et une chaîne lourde (respectivement de 142 et 306 acides aminés) (zymogène), qui restent associées par des interactions non covalentes et/ou un pont disulfure. Sous cette forme bicaténaire, le facteur X est inactif. Suite au déclenchement de la coagulation, la chaîne lourde est finalement activée par clivage du peptide d'activation, pour ne contenir que 254 acides aminés (les 52 premiers acides aminés sont clivés lors du traitement): c'est la chaîne lourde du facteur Xa (SEQ ID NO :6).
Le prépropeptide de facteur X humain correspond à SEQ ID NO: 4. La chaîne lourde avec peptide d'activation correspond à SEQ ID NO: 1, et la chaîne légère correspond à SEQ ID NO: 5. Le peptide d'activation de la chaîne lourde correspond à SEQ ID NO :3, et comprend 52 acides aminés.
SEQ ID NO: 2 (peptide signal et chaîne légère) est identique aux acides aminés 1 à 182 de SEQ ID NO: 4.
SEQ ID NO: 1 (chaîne lourde avec peptide d'activation) est identique aux acides aminés 183 à 488 de SEQ ID NO: 4.
La chaîne lourde du facteur Xa (SEQ ID NO :6) correspond à la SEQ ID NO:1, dans laquelle le peptide SEQ ID NO :3 a été clivé.

Selon un mode de réalisation particulier, l'invention a pour objet un facteur X modifié (appelé GPAD-FXa) consistant en la séquence SEQ ID NO:11 ou SEQ ID NO :25 ou SEQ ID NO :26, directement fusionnée, ou fusionnée via un linker, notamment -Arg-Lys-Arg-, à la séquence SEQ ID NO :6, elle-même étant directement fusionnée, ou fusionnée via un linker à l'extrémité C-terminale de SEQ ID NO :6, à une séquence étiquette comprenant de 1 à 25 acides aminés, de préférence de 1 à 20 acides aminés. De préférence, la séquence étiquette est l'étiquette HPC4 de séquence SEQ ID NO :20, ou l'étiquette de séquence SEQ ID NO:20 délétée de l'acide aminé Cystéine en position 1. Préférentiellement, le linker placé en C-terminal de SEQ ID NO :6 est choisi parmi les séquences -GSSG-, -(GGGGS)n-, n étant compris entre 1 et 5 et -GSSGSSG-. Avantageusement, la séquence étiquette permet la visualisation de la protéine GPAD-FXa selon l'invention *in vitro, in vivo,* et/ou permet le dosage et/ou la purification de la protéine d'intérêt.

Dans ce mode de réalisation, les séquences nucléiques codant la protéine GPAD-FXa selon l'invention sont fusionnées à leur extrémité C-terminale à une séquence nucléique codant une séquence étiquette, directement ou via une séquence nucléique codant un linker précité.

Le facteur X modifié selon l'invention, GPAD-FXa, est dépourvu de son domaine γ-carboxyglutamic acid (Gla) de liaison aux phospholipides et de son peptide d'activation (SEQ ID NO :3).

Les 42 premiers acides aminés de la chaîne légère (résidus 1-42 de SEQ ID NO:5) représentent le domaine Gla, car il contient 11 résidus modifiés de façon post-traductionnelle (acide γ-carboxyglutamique). Une digestion par la chymotrypsine permet de supprimer les résidus 1-42 permettant de générer enzymatiquement un FX dépourvu de son domaine de fixation aux phospholipides. Alternativement cette molécule peut être aussi exprimée directement sans ce domaine par génie génétique. Le GPAD-FXa contient ainsi uniquement un fragment de la chaîne légère, i.e. la séquence SEQ ID NO :11 ou la séquence SEQ ID NO :25 ou la séquence SEQ ID NO :26. Les séquences SEQ ID NO :11, SEQ ID NO :25 et SEQ ID NO :26 ne diffèrent que par l'ajout éventuel d'un ou deux acides aminés en N-terminal. Le GPAD-FXa est également :
- soit fusionné directement à la séquence SEQ ID NO :6, notamment pour donner le mutant de séquence SEQ ID NO :9 ;
- soit fusionné via un linker, notamment -Arg-Lys-Arg-, à la séquence SEQ ID NO :6, notamment pour donner le mutant de séquence SEQ ID NO : 7.

De préférence, le facteur X modifié selon l'invention consiste en le fragment de la chaîne légère de séquence SEQ ID NO :11 ou SEQ ID NO :25 ou SEQ ID NO :26 fusionné directement à la séquence SEQ ID NO :6, qui est elle-même éventuellement fusionnée à une séquence étiquette. Le facteur X modifié consistant en :
- le fragment de la chaîne légère de séquence SEQ ID NO :11 fusionné directement à la séquence SEQ ID NO :6, , ou
- le fragment de la chaîne légère de séquence SEQ ID NO :25 fusionné directement à la séquence SEQ ID NO :6 (cette séquence totale correspond au mutant de séquence SEQ ID NO :21), ou
- le fragment de la chaîne légère de séquence SEQ ID NO :26 fusionné directement à la séquence SEQ ID NO :6,
correspond au mutant appelé GPAD1.

De préférence, le facteur X modifié selon l'invention consiste en le fragment de la chaîne légère de séquence SEQ ID NO :25 fusionné via un linker, notamment -Arg-Lys-Arg-, à la séquence SEQ ID NO:6, qui est elle-même éventuellement fusionnée à une séquence étiquette. Le facteur X modifié consistant en :
- le fragment de la chaîne légère de séquence SEQ ID NO :25 fusionné via un linker - Arg-Lys-Arg- à la séquence SEQ ID NO:6 (cette séquence correspond au mutant de séquence SEQ ID NO : 22), ou
- le fragment de la chaîne légère de séquence SEQ ID NO :11 fusionné via un linker - Arg-Lys-Arg- à la séquence SEQ ID NO :6, ou
- le fragment de la chaîne légère de séquence SEQ ID NO :26 fusionné via un linker - Arg-Lys-Arg- à la séquence SEQ ID NO :6,
correspond au mutant appelé GPAD2.

De préférence, le facteur X modifié selon l'invention est optimisé pour son expression par la cellule hôte. La cellule hôte peut être une cellule recombinante ou une cellule d'un animal transgénique. Dans ce cas, il est de préférence choisi parmi les séquences SEQ ID NO:23 (appelée GPAD1 optimisé) et SEQ ID NO:24 (appelée GPAD2 optimisé).

Un tel GPAD-FXa peut également comprendre d'autres modifications. Notamment, il peut comprendre au moins une mutation choisie parmi une substitution ponctuelle, une délétion et une insertion, de préférence une substitution ponctuelle ou une insertion. Ainsi, différentes mutations ont été introduites dans le gène codant pour le GPAD-FXa, permettant de garder une activité de génération de thrombine. Ces mutations peuvent être introduites par utilisation du kit QuickChange (Stratagene) et en suivant les recommandations du fabricant et selon la publication Wang & Malcolm (1999) - BioTechniques, 26 : 680-682. Ces mutations peuvent porter sur l'arginine 138 de la chaîne lourde du GPAD-FXa (i.e. SEQ ID NO : 6), qui peut être mutée pour donner tout autre acide aminé, préférentiellement la phénylalanine (par exemple SEQ ID NO :16), la glycine (par exemple SEQ ID NO :13), l'isoleucine (par exemple SEQ ID NO :14) ou la tyrosine (par exemple SEQ ID NO :15). Similairement la lysine 82 de la chaîne lourde (i.e. SEQ ID NO : 6) peut également être remplacée par un acide aminé tel que la tyrosine (par exemple SEQ ID NO :12). Le GPAD-FXa selon l'invention peut ainsi comprendre au moins l'une des mutations décrites ci-dessus. Il peut également comprendre la double mutation de l'arginine 138 de SEQ ID NO :6 en un acide aminé choisi parmi la phénylalanine, la glycine, l'isoleucine et la tyrosine, et de la lysine 82 de SEQ ID NO :6 en tyrosine. De préférence, il comprend la double mutation de l'arginine 138 de SEQ ID NO:6 en phénylalanine, et de la lysine 82 de SEQ ID NO:6 en tyrosine. De préférence, il comprend la double mutation de l'arginine 138 de SEQ ID NO :6 en glycine, et de la lysine 82 de SEQ ID NO :6 en tyrosine. De préférence, il comprend la double mutation de l'arginine 138 de SEQ ID NO :6 en isoleucine, et de la lysine 82 de SEQ ID NO :6 en tyrosine. De préférence, il comprend la double mutation de l'arginine 138 de SEQ ID NO :6 en tyrosine, et de la lysine 82 de SEQ ID NO :6 en tyrosine.

De préférence, le GPAD-FXa selon l'invention peut également être modifié par une insertion dans la séquence SEQ ID NO : 11, 25 ou 26.
De préférence, l'insertion dans la séquence SEQ ID NO : 11, 25 ou 26 correspond à l'insertion d'un linker. De préférence, le linker est choisi parmi les linkers : -GSSG-, -RGSSG-, -GSSGR-, -RKRGSSGR-, -(GGGGS)n-, -R(GGGGS)n-, - (GGGGS)nR-, -RKR(GGGGS)nR-, dans lequel n est un entier de 1 à 5, de préférence 1 à 3, et
X, R-X, X-R, et RKR-X-R, X étant un peptide de 4 à 52 acides aminés, G une glycine, S une sérine, R une arginine et K une lysine.
X est de préférence choisi parmi les séquences suivantes (provenant du site parts.igem.org):

| **Code d'accession** | **Description** | **Longueur (nombre d'acides aminés)** |
|---|---|---|
| | | |
| BBa_J18920 | 2aa GS linker | 6 |
| BBa_J18921 | 6aa [GS]x linker | 18 |
| BBa_J18922 | 10aa [GS]x linker | 30 |
| BBa_K105012 | 10 aa flexible protein domain linker | 30 |
| BBa_K133132 | 8 aa protein domain linker | 24 |
| BBa_K157009 | Split fluorophore linker; Freiburg standard | 51 |
| BBa_K157013 | 15 aa flexible glycine-serine protein domain linker; Freiburg standard | 45 |
| BBa_K243004 | Short Linker (Gly-Gly-Ser-Gly) | 12 |
| BBa_K243005 | Middle Linker (Gly-Gly-Ser-Gly)x2 | 24 |
| BBa_K243006 | Long Linker (Gly-Gly-Ser-Gly)x3 | 36 |
| BBa_K416001 | (Gly4Ser)3 Flexible Peptide Linker | 45 |
| BBa_K648005 | Short Fusion Protein Linker: GGSG with standard 25 prefix/suffix | 12 |
| BBa_K648006 | Long 10AA Fusion Protein Linker with Standard 25 Prefix/Suffix | 30 |
| BBa_K648007 | Médium 6AA Fusion Protein Linker: GGSGGS with Standard 25 Prefix/Suffix | 18 |

De préférence, la séquence SEQ ID NO :25 est modifiée pour comprendre, entre les acides aminés 99 et 100, une insertion du linker -GSSGR-, -RKRGSSGR-, - (GGGGS)nR- ou -RKR(GGGGS)nR-, dans lequel n est un entier de 1 à 5, de préférence de 1 à 3, X-R ou RKR-X-R où X est un peptide de 4 à 52 acides aminés, G une glycine, S une sérine, R une arginine et K une lysine.
Alternativement, de préférence, la séquence SEQ ID NO:25 est modifiée pour comprendre, entre les acides aminés 98 et 99, une insertion d'un linker. De préférence, le linker est un linker -GSSG-, -RGSSG-, -R(GGGGS)n- ou -(GGGGS)n-, dans lequel n est un entier de 1 à 5, de préférence de 1 à 3, X ou R-X où X est un peptide de 4 à 52 acides aminés et G une glycine, S une sérine, R une arginine.
De façon similaire, de préférence, la séquence SEQ ID NO :26 est modifiée pour comprendre, entre les acides aminés 98 et 99, une insertion du linker -GSSGR-, - RKRGSSGR-, -(GGGGS)nR- ou -RKR(GGGGS)nR-, dans lequel n est un entier de 1 à 5, de préférence de 1 à 3, ou X-R ou RKR-X-R où X est un peptide de 4 à 52 acides aminés, G une glycine, S une sérine, R une arginine et K une lysine.

Alternativement, de préférence, la séquence SEQ ID NO:26 est modifiée pour comprendre, entre les acides aminés 97 et 98, une insertion du linker -GSSG-, - RGSSG-, -R(GGGGS)n- ou -(GGGGS)n-, dans lequel n est un entier de 1 à 5, de préférence de 1 à 3, X ou R-X où X est un peptide de 4 à 52 acides aminés et G une glycine, S une sérine, R une arginine.

Préférentiellement, selon une première alternative, le GPAD-FXa selon l'invention consiste en le fragment de la chaîne légère de séquence SEQ ID NO :26 fusionné directement à la séquence SEQ ID NO :6, qui est elle-même éventuellement directement fusionnée à une séquence étiquette, dans laquelle la séquence SEQ ID NO :26 est modifiée pour comprendre, entre les acides aminés 98 et 99, une insertion du linker - GSSGR-, -RKRGSSGR-, -(GGGGS)nR- ou -RKR(GGGGS)nR-, dans lequel n est un entier de 1 à 5, de préférence de 1 à 3, ou X-R ou RKR-X-R où X est un peptide de 4 à 52 acides aminés, G une glycine, S une sérine, R une arginine et K une lysine.
Préférentiellement, selon une seconde alternative, le GPAD-FXa selon l'invention consiste en le fragment de la chaîne légère de séquence SEQ ID NO :26 fusionné à la séquence SEQ ID NO :6, qui est elle-même éventuellement fusionnée à une séquence étiquette, dans laquelle la séquence SEQ ID NO :26 est modifiée pour comprendre, entre les acides aminés 97 et 98, une insertion du linker -GSSG-, -RGSSG-, - R(GGGGS)n- ou -(GGGGS)n-, dans lequel n est un entier de 1 à 5, de préférence de 1 à 3, X ou R-X où X est un peptide de 4 à 52 acides aminés et G une glycine, S une sérine, R une arginine.

De telles insertions correspondent aux mutants nommés GPAD3.

De préférence, le GPAD-FXa selon l'invention est choisi parmi les séquences SEQ ID NO:28 (appelée GPAD3-LC), SEQ ID NO:29 (appelée GPAD3-LL) et SEQ ID NO:30 (appelée GPAD3-2F), SEQ ID NO :27, SEQ ID NO :52, SEQ ID NO:53, SEQ ID NO :64, SEQ ID NO :65, SEQ ID NO :66, SEQ ID NO :67, SEQ ID NO : 75, SEQ ID NO :76.

Le GPAD3 tel que défini ci-dessus peut notamment être muté sur l'arginine 138 de la chaîne lourde (i.e. de la séquence SEQ ID NO : 6), qui peut être substituée en phénylalanine, en glycine, en isoleucine ou en tyrosine. Similairement, le GPAD3 tel que défini ci-dessus peut être muté sur la lysine 82 de la chaîne lourde (i.e. de la séquence SEQ ID NO : 6), qui peut être remplacée par la tyrosine.
Selon un aspect particulier de l'invention, le GPAD3 peut être muté sur l'arginine 138 de la chaîne lourde (i.e. de la séquence SEQ ID NO : 6) et en outre muté sur la lysine 82 de la chaîne lourde (i.e. de la séquence SEQ ID NO : 6).

Le GPAD-FXa selon l'invention peut également être fusionné, en N-terminal ou en C-terminal, à au moins un fragment d'immunoglobuline de type sauvage. On entend, par fragment d'immunoglobuline de type sauvage, un fragment choisi parmi les fragments Fc de type sauvage et les fragments scFc de type sauvage.
Par "fragment Fc", on entend la région constante d'une immunoglobuline de longueur totale à l'exclusion du premier domaine de région constante d'immunoglobuline (i.e. CH1-CL). Ainsi le fragment Fc fait référence à un homodimère, chaque monomère comprenant les deux derniers domaines constants des IgA, IgD, IgG (i.e. CH2 et CH3), ou les trois derniers domaines constants des IgE et IgM (i.e. CH2, CH3 et CH4), et la région charnière flexible N-terminale de ces domaines. Le fragment Fc, lorsqu'il est issu d'IgA ou d'IgM, peut comprendre la chaîne J. De préférence, la région Fc d'une IgG1 se compose de la charnière flexible N-terminale et des domaines CH2-CH3, c'est-à-dire la portion à partir de l'acide aminé C226 jusqu'à l'extrémité C-terminale, la numérotation étant indiquée selon l'indice EU ou équivalent dans Kabat.
Par « fragment scFc » (« single chain Fc »), on entend un fragment Fc simple chaîne, obtenu par fusion génétique de deux monomères Fc reliés par un linker polypeptidique. Le scFc se replie naturellement en une région Fc dimérique fonctionnelle.
La fusion du GPAD-FXa à au moins un fragment d'immunoglobuline de type sauvage (notamment un fragment Fc ou scFc) en N-terminal ou en C-terminal, permet d'améliorer la stabilité et la rétention du GPAD-FXa dans l'organisme, et ainsi sa biodisponibilité ; elle permet également d'améliorer sa demi-vie dans l'organisme. En outre, elle peut permettre de simplifier la purification de la molécule obtenue par le ciblage du fragment Fc lors d'une des étapes de la purification. De préférence, le fragment Fc de type sauvage est choisi parmi la séquence SEQ ID NO:39 et la séquence SEQ ID NO : 54 éventuellement suivie d'une lysine en C-terminal (226 ou 227 acides aminés respectivement pour la SEQ ID NO :39 ; 231 ou 232 acides aminés respectivement pour la SEQ ID NO : 54). Le fragment Fc correspondant à la séquence SEQ ID NO:39 comprend les domaines constants CH2 et CH3 d'une IgG de type sauvage et la région charnière partielle en N-terminal (DKTHTCPPCP, SEQ ID NO :55). Le fragment correspondant à la séquence SEQ ID NO:54 comprend les domaines constants CH2 et CH3 d'une IgG de type sauvage et la région charnière entière en N-terminal (séquence EPKSCDKTHTCPPCP, SEQ ID NO :56).
Préférentiellement, le GPAD2 est utilisé pour être fusionné en C-terminal avec le Fc SEQ ID NO :39, suivi d'une lysine. De préférence, dans ce cas, le GPAD-FXa fusionné à un Fc selon l'invention correspond à la séquence SEQ ID NO :32 (GPAD2-FX-Fc). Alternativement, le GPAD2 est utilisé pour être fusionné en C-terminal avec le Fc SEQ ID NO : 54, suivi d'une lysine. De préférence, dans ce cas, le GPAD-FXa fusionné à un Fc selon l'invention correspond à la séquence SEQ ID NO:33 (GPAD2-Fcl). Préférentiellement, le GPAD2 peut également être directement fusionné en C-terminal avec le Fc SEQ ID NO :39 ou le Fc SEQ ID NO :54, ledit Fc étant lui-même fusionné en C-terminal à un autre Fc identique ou différent, via un linker -(GGGGS)n-, où n est un entier de 1 à 3, correspondant dans ce cas à un fragment scFc. De préférence, dans ce cas, le GPAD-FXa fusionné à un scFc selon l'invention est choisi parmi SEQ ID NO :35 (GPAD2-scFcL) et 36 (GPAD2-scFcS).
Le GPAD-FXa fusionné à un Fc selon l'invention peut également consister en la séquence SEQ ID NO :26 fusionnée via un linker -Arg-Lys-Arg- à la séquence SEQ ID NO :6, elle-même fusionnée au Fc SEQ ID NO :54 suivi d'une lysine. Il a dans ce cas pour séquence SEQ ID NO :34 (GPAD2-FcLss).
Alternativement, le GPAD2 ou le GPAD1 peut être utilisé pour être fusionné en N-terminal, directement ou via un linker -GGGGS-, avec le Fc SEQ ID NO :39. Préférentiellement, dans ce cas, le GPAD2 ou le GPAD1 est fusionné en N-terminal via un linker -GGGGS- avec le Fc SEQ ID NO :39 ou le Fc SEQ ID NO :54, ledit Fc étant lui-même fusionné en N-terminal à un autre Fc identique ou différent, via un linker - (GGGGS)n-, où n est un entier de 1 à 3. De préférence, dans ce cas, le GPAD-FXa fusionné à un Fc selon l'invention est choisi parmi SEQ ID NO :37 (scFcL-GPAD2) et 38 (scFcL-GPAD1).

Selon l'invention, dans sa forme immature, le GPAD-FXa correspond à une protéine monocaténaire dépourvue de peptide d'activation. Le clivage entre la chaîne lourde SEQ ID NO :6 et le fragment de la chaîne légère SEQ ID NO :11 ou SEQ ID NO :25 ou SEQ ID NO :26 peut soit se faire grâce à la présence de la séquence naturelle de clivage du facteur X par des enzymes cellulaires, les furines (RRKR), dans la séquence SEQ ID NO :11 ou 25 ou 26, soit se faire via un linker qui permet d'améliorer le clivage de la protéine (i) dans la cellule productrice ; (ii) à l'extérieur de la cellule lors du processus de production/purification de la molécule ; et/ou (iii) lors de l'activation de la molécule in vivo. Par « linker », on entend une courte séquence d'acides aminés, i.e. entre 2 et 5 acides aminés.
Parmi les linkers possibles, ceux clivés dans la cellule productrice peuvent sans limitations et à titre d'exemple être ceux de la furine RX(K ou R)R (Hosaka J Biol Chem 1991), ou bien de toute autre protéase impliquée dans la maturation ou l'activation des protéines intracellulaires. Le linker peut aussi être choisi parmi les sites de clivages des protéases impliquées dans la cascade de la coagulation telles que par exemple le FVIIa, le FIXa, le FXa, le FXIa, le FXIIa, la kallikréine ou la protéine C activée. Le linker peut aussi être composé d'une séquence peptidique connue par l'homme du métier pour séparer les protéines recombinantes telle par exemple celle reconnue par la TEV (Tobacco Etch Virus) ou l'entérokinase. De préférence, le linker est une séquence de 3 acides aminés. Plus préférentiellement, le linker est -Arg-Lys-Arg-.
De façon avantageuse, suite au clivage du GPAD-FXa entre la chaîne légère et la chaîne lourde, le GPAD-FXa est directement obtenu sous forme activée, et ne nécessite donc pas d'étape supplémentaire de clivage du peptide d'activation.

Ladite protéine selon l'invention est un facteur X muté qui est efficace dans le traitement de troubles de la coagulation.

La composition selon l'invention peut également être utilisée pour la prévention ou le traitement d'un accident hémorragique chez des patients hémophiles présentant des anticorps anti-facteur VIII (FVIII) ou facteur IX (FIX). Les anticorps sont apparus soit à la suite d'un traitement par des facteurs FVIII ou FIX, ou spontanément, comme dans l'hémophilie acquise.

Les séquences décrites dans la présente demande peuvent être résumées comme suit:

| **SEQ ID NO:** | **Protéine** |
|---|---|
| 1 | Chaîne lourde de facteur X humain (306 acides aminés), comprenant le peptide d'activation |
| 2 | Peptide signal et chaîne légère de facteur X humain (182 acides aminés) |
| 3 | Peptide d'activation de la chaîne lourde (52 acides aminés) |
| 4 | Prépropeptide de facteur X humain (488 acides aminés) |
| 5 | Chaîne légère de facteur X humain (142 acides aminés) |
| 6 | Chaîne lourde du Facteur X humain activé (FXa) (254 acides aminés) |
| 7 | Mutant de facteur X humain GPAD-FXa selon l'invention (357 acides aminés) |
| 8 | Séquence nucléique codant pour le mutant de SEQ ID NO :7 |
| 9 | Mutant de facteur X humain GPAD-FXa selon l'invention (354 acides aminés) |
| 10 | Séquence nucléique codant pour le mutant de SEQ ID NO:9 |
| 11 | Fragment de chaîne légère présent dans les séquences SEQ ID NO :7 et 9 (100 acides aminés) |
| 12 | Mutant de facteur X humain GPAD-FXa identique à SEQ ID NO :7 avec K82Y |
| 13 | Mutant de facteur X humain GPAD-FXa identique à SEQ ID NO:7 avec R138G |

| | |
|---|---|
| 14 | Mutant de facteur X humain GPAD-FXa identique à SEQ ID NO:7 avec R138I |
| 15 | Mutant de facteur X humain GPAD-FXa identique à SEQ ID NO:7 avec R138Y |
| 16 | Mutant de facteur X humain GPAD-FXa identique à SEQ ID NO:7 avec R138F |
| 17-18 | Amorces utilisées en exemple |
| 19 | Peptide signal MB7 |
| 20 | Etiquette HPC4 |
| 21 | GPAD1 |
| 22 | GPAD2 |
| 23 | GPAD1 optimisé |
| 24 | GPAD2 optimisé |
| 25 | SEQ ID NO :11 comprenant en plus les acides aminés Ser-Asn- en N terminal (soit 102 acides aminés au total) |
| 26 | SEQ ID NO :11 comprenant en plus l'acide aminé Asn-en N terminal (soit 101 acides aminés au total) |
| 27 | Exemple de GPAD-3 : SEQ ID NO :26 comprenant une insertion de -GSSG- entre les acides aminés 97 et 98, directement fusionnée à SEQ ID NO :6 |
| 28 | GPAD3-LC |
| 29 | GPAD3-LL |
| 30 | GPAD3-2F |
| 31 | Chaîne lourde du Facteur X humain activé (SEQ ID NO :6) comprenant en C-terminal un linker fusionné à une étiquette de séquence SEQ ID NO : 20 sans la cystéine en position 1 |
| 32 | GPAD2-FX-Fc |
| 33 | GPAD2-Fcl |
| 34 | GPAD2-FcLss |

| | |
|---|---|
| 35 | GPAD2-scFcL |
| 36 | GPAD2-scFcS |
| 37 | scFcL-GPAD2 |
| 38 | scFcL-GPAD 1 |
| 39 | Fragment Fc sauvage, éventuellement suivi d'une lysine |
| 40 à 51 | Séquences nucléiques codant respectivement les séquences SEQ ID NO :21 à 24 et 31 à 38 |
| 52 | Exemple de GPAD-3 : SEQ ID NO:26 comprenant une insertion de -RGGGGS- entre les acides aminés 97 et 98 fusionnée via un linker Arg-Lys-Arg à SEQ ID NO :6 |
| 53 | Exemple de GPAD-3 : SEQ ID NO:26 comprenant une insertion de -GSSGR- entre les acides aminés 98 et 99, directement fusionnée à SEQ ID NO :6 |
| 54 | SEQ ID NO:39 (Fragment Fc sauvage) comprenant la région charnière entière en N-terminal |
| 55 | Région charnière partielle N-terminal |
| 56 | Région charnière entière N-terminal |
| 57 à 59 | Séquences nucléiques codant respectivement les séquences SEQ ID NO :28 à 30 |
| 60 | GPAD1 avec peptide signal MB7 en N-terminal |
| 61 | GPAD2 avec peptide signal MB7 en N-terminal |
| 62 | GPAD1 optimisé avec peptide signal MB7 en N-terminal |
| 63 | GPAD2 optimisé avec peptide signal MB7 en N-terminal |
| 64 | Exemple de GPAD-3 avec peptide signal MB7 en N-terminal : SEQ ID NO :27 avec peptide signal MB7 en N-terminal |
| 65 | GPAD3-LC avec peptide signal MB7 en N-terminal |
| 66 | GPAD3-LL avec peptide signal MB7 en N-terminal |
| 67 | GPAD3-2F avec peptide signal MB7 en N-terminal |
| 68 | GPAD2-FX-Fc avec peptide signal MB7 en N-terminal |

| | |
|---|---|
| 69 | GPAD2-Fcl avec peptide signal MB7 en N-terminal |
| 70 | GPAD2-FcLss avec peptide signal MB7 en N-terminal |
| 71 | GPAD2-scFcL avec peptide signal MB7 en N-terminal |
| 72 | GPAD2-scFcS avec peptide signal MB7 en N-terminal |
| 73 | scFcL-GPAD2 avec peptide signal MB7 en N-terminal |
| 74 | scFcL-GPAD1 avec peptide signal MB7 en N-terminal |
| 75 | Exemple de GPAD-3 : SEQ ID NO:52 avec peptide signal MB7 en N-terminal |
| 76 | Exemple de GPAD-3 : SEQ ID NO:53 avec peptide signal MB7 en N-terminal |
| 77 à 90 | Séquences nucléiques codant respectivement les séquences SEQ ID NO :60 à 63 et 65 à 74 |
| 91 | Linker décrit à l'exemple 20 |
| 92 et 93 | Séquences décrites dans la figure 9 |

Un autre objet de l'invention est un acide nucléique (polynucléotide ou séquence nucléotidique) codant pour ladite protéine. La séquence nucléotidique codant pour le GPAD-FXa peut être synthétisée par voie chimique (Young L and Dong Q., 2004,-Nucleic Acids Res., Apr 1 5;32(7), Hoover, D.M. and Lubkowski, J. 2002,. Nucleic Acids Res., 30, Villalobos A, et al., 2006. BMC Bioinformatics, Jun 6;7:285). La séquence nucléotidique codant pour le GPAD-FXa peut être également amplifiée par PCR en utilisant des amorces adaptées.
Le GPAD-FXa peut également être produit par des techniques de génie génétique bien connues de l'homme du métier. La séquence nucléotidique codant pour le facteur X humain peut ainsi être clonée dans un vecteur d'expression ; la partie de la séquence nucléotidique codant pour le peptide signal, le propeptide et domaine Gla est délétée, un peptide signal est fusionné, comme celui du TIMP-1 (Crombez et al., 2005). L'ADN codant pour un tel FX modifié est inséré dans un plasmide d'expression et inséré dans une lignée cellulaire ad hoc pour sa production (par exemple la lignée HEK-293 FreeStyle), la protéine ainsi produite étant ensuite purifiée par chromatographie.

Ces techniques sont décrites en détails dans les manuels de référence : Molecular cloning : a laboratory manual, 3ieme édition-Sambrook and Russel eds. (2001) et Current Protocols in Molecular Biology - Ausubel et al. eds (2007).
Le polynucléotide selon l'invention est de préférence choisi parmi les séquences SEQ ID NO : 8, SEQ ID NO : 10, SEQ ID NO:40 à 51, SEQ ID NO:57 à 59 et SEQ ID NO :77 à 90.

Le polynucléotide selon l'invention codant pour le GPAD-FXa peut comprendre en N-terminal une séquence codant pour un peptide signal, notamment optimisé. Dans un mode de réalisation, le peptide signal utilisé est celui du TIMP-1. Dans un mode de réalisation particulièrement avantageux, le peptide signal utilisé est optimisé pour l'expression et la sécrétion d'une protéine GPAD-FXa selon l'invention. De tels peptides signaux sont décrits notamment dans la demande WO2011/114063. De préférence, un peptide signal utilisé est le MB7 (SEQ ID NO:19: MRWSWIFLLLLSITSANA).

Le polynucléotide selon l'invention codant pour le GPAD-FXa peut également comprendre des codons optimisés, notamment pour son expression dans certaines cellules. Par exemple, lesdites cellules comprennent les cellules COS, les cellules CHO, les cellules HEK, les cellules BHK, les cellules PER.C6, les cellules HeLa, les cellules NIH/3T3, 293 (ATCC # CRL1573), des cellules T2, les cellules dendritiques ou les monocytes. L'optimisation de codon a pour but de remplacer les codons naturels par des codons dont les ARN de transfert (ARNt) portant les acides aminés sont les plus fréquents dans le type cellulaire considéré. Le fait de mobiliser des ARNt fréquemment rencontrés a pour avantage majeur d'accroître la vitesse de traduction des ARN messagers (ARNm) et donc d'augmenter le titre final (Carton JM et al, Protein Expr Purif, 2007). L'optimisation de codons joue aussi sur la prédiction des structures secondaires d'ARNm qui pourraient ralentir la lecture par le complexe ribosomal. L'optimisation de codons a également un impact sur le pourcentage de G/C qui est directement lié à la demi-vie des ARNm et donc à leur potentiel de traduction (Chechetkin, J. of Theoretical Biology 242, 2006 922-934).

L'optimisation de codons peut être faite par substitution des codons naturels en utilisant des tables de fréquence des codons (codon Usage Table) pour mammifères et plus particulièrement pour *Homo sapiens.* Il existe des algorithmes présents sur internet et mis à disposition par les fournisseurs de gènes de synthèse (DNA2.0, GeneArt, MWG, Genscript) qui permettent de faire cette optimisation de séquence.
De préférence, le polynucléotide selon l'invention comprend des codons optimisés pour son expression dans les cellules HEK, telles que des cellules HEK293. Un tel polynucléotide est de préférence choisi parmi les séquences SEQ ID NO :42 et SEQ ID NO:43. Alternativement, le polynucléotide selon l'invention comprend des codons optimisés pour son expression dans les cellules d'animaux transgéniques, de préférence la chèvre, la lapine, la brebis ou la vache.
Le polynucléotide selon l'invention peut également avantageusement être choisi parmi les séquences SEQ ID NO :45 à 51.

Un autre objet de l'invention est une cassette d'expression comprenant ledit polynucléotide codant pour ladite protéine, ou un vecteur d'expression comprenant ledit polynucléotide ou ladite cassette d'expression. Selon l'invention, les vecteurs d'expression appropriés pour une utilisation selon l'invention peuvent comprendre au moins un élément de contrôle d'expression fonctionnellement lié à la séquence d'acide nucléique. Les éléments de contrôle d'expression sont insérés dans le vecteur et permettent de réguler l'expression de la séquence d'acide nucléique. Des exemples d'éléments de contrôle d'expression incluent notamment des systèmes lac, le promoteur du phage lambda, les promoteurs de levure ou les promoteurs viraux. D'autres éléments opérationnels peuvent être incorporés, comme une séquence de tête, des codons de terminaison, des signaux de polyadénylation et des séquences nécessaires pour la transcription et la traduction ultérieure de la séquence d'acide nucléique dans le système hôte. Il sera compris par l'homme de l'art que la combinaison correcte des éléments de contrôle d'expression dépend du système hôte choisi. Il sera également entendu que le vecteur d'expression doit contenir les éléments supplémentaires nécessaires pour le transfert et la réplication ultérieure du vecteur d'expression contenant la séquence d'acide nucléique dans le système hôte.

De tels vecteurs sont facilement construits en utilisant des méthodes conventionnelles ou disponibles dans le commerce. De préférence, de tels vecteurs sont ceux décrits dans les demandes WO2013/061010 et WO2013/117871.

Un autre objet de l'invention est une cellule recombinante comprenant un vecteur d'expression tel que décrit ci-dessus, ou un polynucléotide tel que décrit ci-dessus. Selon l'invention, des exemples de cellules hôtes qui peuvent être utilisées sont des cellules eucaryotes, comme les cellules animales, végétales, d'insectes et de levure ; et des cellules procaryotes, comme E. coli. Les moyens par lesquels le vecteur portant le gène peut être introduit dans les cellules comprennent notamment la microinjection, l'électroporation, la transduction ou la transfection à l'aide de DEAE-dextran, la lipofection, le phosphate de calcium ou d'autres procédures connues de l'homme de l'art. Dans un mode de réalisation préféré, les vecteurs d'expression eucaryotes qui fonctionnent dans les cellules eucaryotes sont utilisés. Des exemples de tels vecteurs comprennent les vecteurs viraux tels que les rétrovirus, adénovirus, virus de l'herpès, virus de la vaccine, virus de la variole, le poliovirus, lentivirus, les vecteurs d'expression bactériens ou des plasmides tels que pcDNA5. Les lignées cellulaires eucaryotes préférées comprennent les cellules COS, les cellules CHO, les cellules HEK, les cellules BHK, les cellules Per.C6, les cellules HeLa, les cellules NIH/3T3, 293 (ATCC # CRL1573), des cellules T2, les cellules dendritiques ou les monocytes.

La protéine selon l'invention peut être produite dans le lait d'animaux transgéniques. Dans ce cas, selon un premier aspect, l'expression d'une séquence d'ADN codant pour le GPAD-FXa selon l'invention est contrôlée par un promoteur de caséine de mammifère ou un promoteur de lactosérum de mammifère, ledit promoteur ne contrôlant pas naturellement la transcription dudit gène, et la séquence d'ADN contenant en outre une séquence de sécrétion de la protéine. La séquence de sécrétion comprend un signal de sécrétion interposé entre la séquence codante et le promoteur. Selon un aspect particulièrement avantageux, la séquence d'ADN codant pour le GPAD-FXa comprend des codons optimisés pour son expression dans les cellules d'animaux transgéniques.

L'animal transgénique utilisé est capable non seulement de produire la protéine désirée, mais également de transmettre cette capacité à sa descendance. La sécrétion de la protéine dans le lait facilite la purification et évite l'utilisation de produits sanguins. L'animal peut ainsi être choisi parmi la chèvre, la lapine, la brebis ou la vache.

Un tel procédé de production est notamment décrit dans le brevet EP0264166. Ce procédé peut comprendre les étapes suivantes :
(a) insérer dans un embryon de mammifère non humain une séquence d'ADN comprenant un polynucléotide selon l'invention, ledit polynucléotide étant sous le contrôle transcriptionnel d'un promoteur de la caséine de mammifère ou un promoteur de lactosérum de mammifère, ladite séquence d'ADN comprenant en outre une séquence signal permettant la sécrétion de la protéine GPAD-FXa,
(b) laisser ledit embryon se développer en un mammifère adulte,
(c) induire la lactation chez ledit mammifère ou dans un descendant femelle dudit mammifère dans lequel ledit polynucléotide, le promoteur et la séquence signal sont présents dans le génome du tissu mammaire,
(d) recueillir le lait dudit mammifère en lactation, et
(e) isoler ladite protéine à partir dudit lait recueilli.
De préférence, une séquence codant une enzyme furine ou une autre endopeptidase spécifique est insérée dans l'embryon de mammifère non humain à l'étape a).

De préférence, le procédé de production du GPAD-FXa selon l'invention comprend les étapes suivantes :
(a) insérer dans un embryon de mammifère non humain une séquence d'ADN comprenant un polynucléotide choisi parmi SEQ ID NO : 8, SEQ ID NO : 10, SEQ ID NO : 45 à 51, SEQ ID NO :57 à 59 et SEQ ID NO :77 à 90, ledit polynucléotide étant sous le contrôle transcriptionnel d'un promoteur de la caséine de mammifère ou un promoteur de lactosérum de mammifère, ladite séquence d'ADN comprenant en outre une séquence signal permettant la sécrétion de ladite protéine,
(b) laisser ledit embryon se développer en un mammifère adulte,
(c) induire la lactation chez ledit mammifère ou dans un descendant femelle dudit mammifère dans lequel ledit polynucléotide, le promoteur et la séquence signal sont présents dans le génome du tissu mammaire,
(d) recueillir le lait dudit mammifère en lactation, et
(e) isoler ladite protéine à partir dudit lait recueilli.
Un tel procédé utilise directement un polynucléotide qui ne contient pas de séquence codant un peptide d'activation.
De préférence, une séquence codant une enzyme furine ou une autre endopeptidase spécifique est insérée dans l'embryon de mammifère non humain à l'étape a).

La protéine selon l'invention peut également être produite selon le procédé suivant :
a) transfection de cellules eucaryotes, par exemple des cellules HEK293 ou CHO, avec des vecteurs d'expression comprenant au moins un polynucléotide selon l'invention. Le polynucléotide est de préférence choisi parmi SEQ ID NO : 8, SEQ ID NO : 10, SEQ ID NO : 45 à 51, SEQ ID NO :57 à 59 et SEQ ID NO:77 à 90. De préférence, les cellules sont également transfectées avec un vecteur exprimant la furine ;
b) culture des cellules obtenues en a), pour exprimer la protéine. La culture se fait selon les conditions classiques, bien connues de l'homme du métier. De préférence, lorsque les cellules co-expriment la protéine et la furine, la protéine produite est directement sous forme activée ; et
c) optionnellement, purification de la protéine obtenue. Là encore, un tel procédé utilise directement un polynucléotide qui ne contient pas de séquence codant un peptide d'activation

La protéine selon l'invention peut être utilisée en tant que médicament. Par conséquent, la protéine selon l'invention peut être introduite dans une composition pharmaceutique. En particulier, la protéine selon l'invention peut être utilisée pour le traitement des troubles de la coagulation, notamment des troubles hémorragiques.

De préférence, la protéine selon l'invention peut être utilisée dans la prévention ou le traitement chez un patient, notamment un être humain ou un animal, des évènements hémorragiques induits par la prise d'anticoagulants, qui sont des inhibiteurs spécifiques du facteur Xa. Dans une telle utilisation, la protéine selon l'invention sert d'antidote aux inhibiteurs spécifiques du facteur Xa. Par « antidote », on désigne les molécules, et notamment les protéines, capables de neutraliser ou de renverser tout ou partie de l'activité anticoagulante des anticoagulants. Cet effet doit pouvoir intervenir dans un délai plus ou moins court, en relation avec la localisation et l'ampleur de l'évènement hémorragique pour ralentir, atténuer ou interrompre totalement cet évènement hémorragique. L'activité anticoagulante des anticoagulants peut être mesurée par des tests coagulométriques globaux (temps de Quick (PT), temps de céphaline activé (aPTT)). En leur présence, il y a diminution de l'INR (International Normalized Ratio). La mesure de leurs effets peut aussi être envisagée par des tests de coagulation qui mesurent la génération de thrombine (TGT).

Par « inhibiteurs spécifiques du facteur Xa », on désigne les composés capables d'inhiber, directement ou indirectement, l'activité procoagulante du FXa qui consiste en la conversion de la prothrombine en thrombine *in vitro,* et/ou *ex vivo,* et/ou *in vivo.* Les inhibiteurs du FXa peuvent être classés en inhibiteurs de nature peptidique, extraits et purifiés ou obtenus par génie génétique et en inhibiteurs de nature non peptidique obtenus par synthèse chimique (Kher et al., 1998, La Lettre du pharmacologue, 12(6), 222-226). Les inhibiteurs qui bloquent le site actif du FXa sont appelés inhibiteurs directs alors que les inhibiteurs qui agissent en se liant et en catalysant l'effet de l'antithrombine vis-à-vis du FXa sont appelés inhibiteurs indirects. Des exemples d'inhibiteurs peptidiques directs du FXa incluent notamment le TAP (tick anticoagulant peptide) extrait de la salive de la tique, l'antistasine extraite des glandes salivaires de la sangsue *Haementeria officinalis,* l'ACAP (*ancylostoma caninum* anticoagulant peptide) isolé à partir de l'ankylostome ou ses formes recombinantes r Ac AP5, r Ac AP2 (également appelé NAP-5) ou encore FXa I (factor Xa inhibitor) extraite de la salive de la sangsue *Hirudo medicinalis* ou la protéine recombinante lui correspondant nommée Yagin. Parmi les inhibiteurs non peptidiques directs du FXa, on trouve le DX 9065a, le LY517717 et les xabans (eribaxaban, apixaban, betrixaban, edoxaban, otamixaban, rivaroxaban). Les HBPM, les oligosaccharides fondaparinux ou idraparinux et les héparinoïdes (danaparoïd, sulfodexide, sulfate de dermatan) constituent des exemples d'inhibiteurs indirects du FXa.

L'utilisation de la protéine selon l'invention comme antidote des inhibiteurs du FXa permet la titration de ces inhibiteurs. De préférence, l'activité inhibitrice de la protéine selon l'invention sur les inhibiteurs spécifiques de FXa est d'au moins environ 10%, 20%, 30%, 40%, 50%, 60%, 70%, 80%, 90% ou 100%.

La composition pharmaceutique de l'invention peut être combinée avec des excipients pharmaceutiquement acceptables, et éventuellement des matrices à libération prolongée, comme des polymères biodégradables, pour former une composition thérapeutique.
La composition pharmaceutique de la présente invention peut être administrée par voie orale, sublinguale, sous-cutanée, intramusculaire, intraveineuse, intra-artérielle, intrathécale, intra-oculaire, intra-cérébrale, transdermique, pulmonaire, locale ou rectale. Le principe actif, seul ou en association avec un autre principe actif, peut alors être administré sous forme unitaire d'administration, en mélange avec des supports pharmaceutiques classiques. Des formes unitaires d'administration comprennent les formes par voie orale telles que les comprimés, les gélules, les poudres, les granules et les solutions ou suspensions orales, les formes d'administration sublinguale et buccale, les aérosols, les implants sous-cutanés, transdermique, topique, intrapéritonéale, intramusculaire, intraveineuse, sous-cutanée, intrathécale, les formes d'administration par voie intranasale et les formes d'administration rectale.
De préférence, la composition pharmaceutique contient un véhicule pharmaceutiquement acceptable pour une formulation susceptible d'être injectée. Il peut s'agir en particulier de formules isotoniques, stériles, de solutions salines (avec phosphate monosodique ou disodique, chlorure de sodium, de potassium, de calcium ou de magnésium et analogues, ou des mélanges de tels sels), ou de compositions lyophilisées, qui, lors de l'addition d'eau stérilisée ou de sérum physiologique selon les cas, permettent la constitution de solutés injectables.
Les formes pharmaceutiques appropriées pour une utilisation injectable comprennent des solutions aqueuses stériles ou des dispersions, des formulations huileuses, y compris l'huile de sésame, l'huile d'arachide, et des poudres stériles pour la préparation extemporanée de solutions injectables stériles ou de dispersions. Dans tous les cas, la forme doit être stérile et doit être fluide dans la mesure où elle doit être injectée par seringue. Elle doit être stable dans les conditions de fabrication et de stockage et doit être préservée contre l'action contaminante de micro-organismes, comme les bactéries et les champignons.
Les dispersions selon l'invention peuvent être préparées dans du glycérol, des polyéthylèneglycols liquides ou leurs mélanges, ou dans des huiles. Dans des conditions normales de stockage et d'utilisation, ces préparations contiennent un conservateur pour empêcher la croissance des micro-organismes.
Le véhicule pharmaceutiquement acceptable peut être un solvant ou milieu de dispersion contenant, par exemple, l'eau, l'éthanol, un polyol (par exemple, la glycérine, le propylène glycol, le polyéthylène glycol, et analogues), des mélanges appropriés de ceux-ci, et/ou les huiles végétales. La fluidité convenable peut être maintenue, par exemple, par l'utilisation d'un tensioactif, tel que la lécithine. La prévention de l'action de micro-organismes peut être provoquée par divers agents antibactériens et antifongiques, par exemple, des parabènes, le chlorobutanol, le phénol, l'acide sorbique ou encore le thimérosal. Dans de nombreux cas, il sera préférable d'inclure des agents isotoniques, par exemple, des sucres ou du chlorure de sodium. L'absorption prolongée des compositions injectables peut être provoquée par l'utilisation dans les compositions d'agents retardant l'absorption, par exemple, le monostéarate d'aluminium ou la gélatine. Les solutions injectables stériles sont préparées en incorporant les substances actives en quantité requise dans le solvant approprié avec plusieurs des autres ingrédients énumérés ci-dessus, le cas échéant, suivie d'une stérilisation par filtration. En règle générale, les dispersions sont préparées en incorporant les divers ingrédients actifs stérilisés dans un véhicule stérile qui contient le milieu de dispersion basique et les autres ingrédients requis parmi ceux énumérés ci-dessus. Dans le cas de poudres stériles pour la préparation de solutions injectables stériles, les procédés de préparation préférés sont le séchage sous vide et la lyophilisation. Lors de la formulation, les solutions seront administrées d'une manière compatible avec la formulation posologique et en une quantité thérapeutiquement efficace. Les formulations sont facilement administrées dans une variété de formes galéniques, telles que les solutions injectables décrites ci-dessus, mais les capsules de libération de médicament et similaires peuvent également être utilisés. Pour l'administration parentérale dans une solution aqueuse par exemple, la solution doit être convenablement tamponnée et le diluant liquide rendu isotonique avec suffisamment de solution saline ou de glucose. Ces solutions aqueuses particulières conviennent particulièrement pour une administration intraveineuse, intramusculaire, sous-cutanée et intrapéritonéale. À cet égard, les milieux aqueux stériles qui peuvent être utilisés sont connus de l'homme de l'art. Par exemple, une dose peut être dissoute dans 1 ml de solution de NaCl isotonique puis ajoutée à 1000 ml de liquide approprié, ou injectée sur le site proposé de la perfusion. Certaines variations de posologie devront nécessairement se produire en fonction de l'état du sujet traité.

La composition pharmaceutique de l'invention peut être formulée dans un mélange thérapeutique comprenant environ 0.0001 à 1.0 milligrammes, soit environ 0.001 à 0.1 milligrammes, soit environ de 0.1 à 1.0 milligrammes, voire environ 10 milligrammes par dose ou plus. Des doses multiples peuvent également être administrées. Le niveau de dose thérapeutiquement efficace spécifique pour un patient particulier dépendra d'une variété de facteurs, y compris le trouble qui est traité et la gravité de la maladie, l'activité du composé spécifique employé, la composition spécifique utilisée, l'âge, le poids corporel, la santé générale, le sexe et le régime alimentaire du patient, le moment de l'administration, la voie d'administration, le taux d'excrétion du composé spécifique utilisé, la durée du traitement, ou encore les médicaments utilisés en parallèle.

Les exemples suivants sont donnés en vue d'illustrer divers modes de réalisation de l'invention.

Les légendes des figures sont les suivantes :

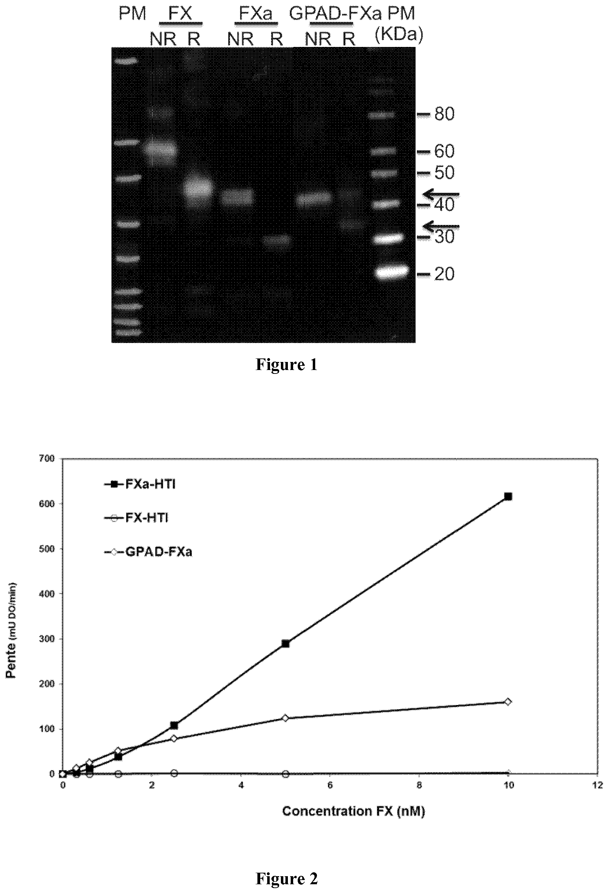

### Figure 1 : Analyse du GPAD-FXA produit en HEK293

Le FX plasmatique non activé (FX) ou activé (FXa) et le GPAD-FXa sont révélés en immunoblot par un anticorps polyclonal anti-FX après séparation en SDS-PAGE 4-12%. Les échantillons ont été non réduit (NR) ou réduit (R) par un traitement au β-mercaptoéthanol. Deux marqueurs de poids moléculaire distincts (PM) sont utilisés. Seules les valeurs de celui de droite sont indiquées. Les deux flèches indiquent le niveau de migration du GPAD-FXa non réduit (flèche supérieure) et réduit (flèche inférieure).

### Figure 2 : Activité amidolytique du GPAD-FXa

L'activité amidolytique vis-à-vis du substrat Pefachrome 8595 au cours du temps du GPAD-FXa (diamants blancs) est comparée à l'activité des facteurs X (ronds blancs) et X activé (carrés noirs).
- en abscisses : le temps (en minutes)
- en ordonnées : la vitesse d'apparition du produit de dégradation du Pefachrome 8595 en mUDO/min

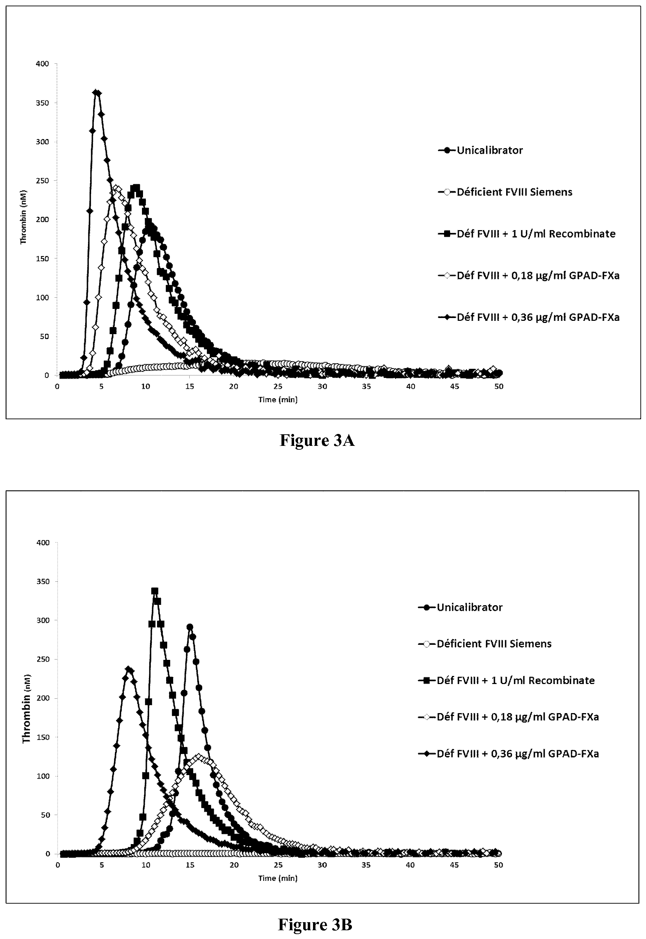

### Figure 3A : Thrombinogramme obtenu à partir de l'ajout de 0,18 µg/ml ou 0,36 µg/ml de GPAD-FXa dans un pool de plasma déficient en FVIII suite à l'activation par du facteur tissulaire:

- en abscisses : le temps (en minutes)
- en ordonnées : la concentration maximale de thrombine observée (en nM)

L'échantillon de pool de plasma normal est représenté par la courbe ● et l'échantillon correspondant à un plasma déficient en facteur VIII par la courbe ○. La courbe ■ représente un plasma déficient en FVIII substitué par 1 U/ml de FVIII recombinant (Recombinate).
Le GPAD-FXa a été ajouté à raison de 0,18 µg/ml (courbe ◊) ou 0,36 µg/ml (courbe de ◆) dans l'échantillon de plasma déficient en FVIII.

### Figure 3B : Thrombinogramme obtenu à partir de l'ajout de 0,18 µg/ml ou 0,36 µg/ml de GPAD-FXa dans un pool de plasma déficient en FVIII suite à l'activation par la céphaline:

- en abscisses : le temps (en minutes)
- en ordonnées : la concentration maximale de thrombine observée (en nM)

L'échantillon de pool de plasma normal est représenté par la courbe ● et l'échantillon correspondant à un plasma déficient en facteur VIII par la courbe ○. La courbe des ■ représente un plasma déficient en FVIII substitué par 1 U/ml de FVIII recombinant (Recombinate).

Le GPAD-FXa a été ajouté à raison de 0,18 µg/ml (courbe ◊) ou 0,36 µg/ml (courbe ◆) dans l'échantillon de plasma déficient en FVIII.

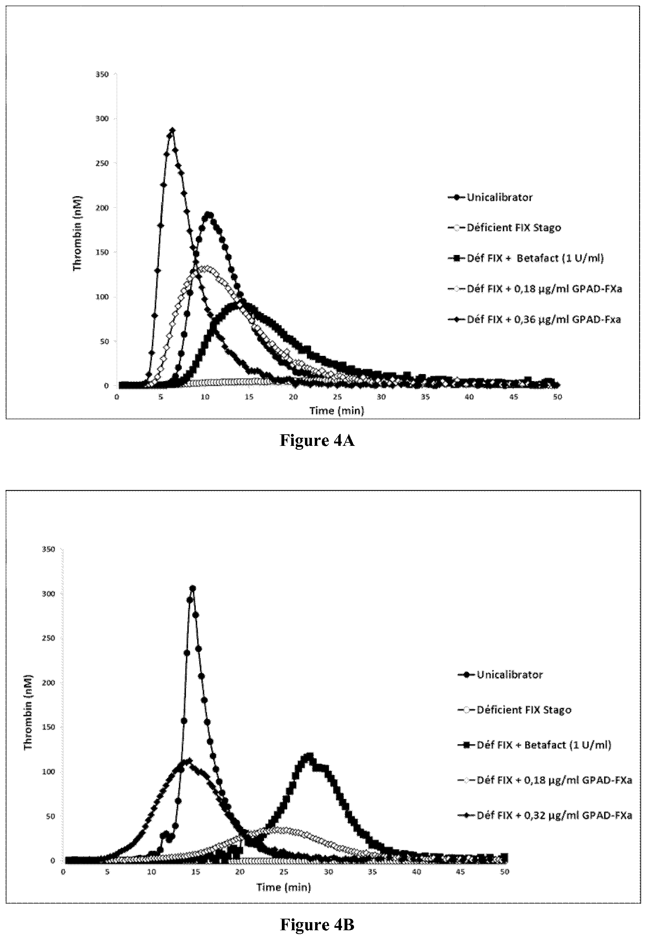

### Figure 4A : Thrombinogramme obtenu à partir de l'ajout de 0,18 µg/ml ou 0,36 µg/ml de GPAD-FXa dans un pool de plasma déficient en FIX suite à l'activation par du facteur tissulaire:

- en abscisses : le temps (en minutes)
- en ordonnées : la concentration maximale de thrombine observée (en nM)

L'échantillon de pool de plasma normal est représenté par la courbe ● et l'échantillon correspondant à un plasma déficient en facteur IX par la courbe ○. La courbe ■ représente un plasma déficient en FIX substitué par 1 U/ml de Betafact (FIX plasmatique).
Le GPAD-FXa a été ajouté à raison de 0,18 µg/ml (courbe ◊) ou 0,36 µg/ml (courbe ◆) dans l'échantillon de plasma déficient en FIX.

### Figure 4B : Thrombinogramme obtenu à partir de l'ajout de 0,18 µg/ml ou 0,36 µg/ml de GPAD-FXa dans un pool de plasma déficient en FIX suite à l'activation par la céphaline:

- en abscisses : le temps (en minutes)
- en ordonnées : la concentration maximale de thrombine observée (en nM)

L'échantillon de pool de plasma normal est représenté par la courbe ●. L'échantillon correspondant à un plasma déficient en facteur IX par la courbe ○. La courbe ■ représente un plasma déficient en FIX substitué par 1 U/ml de Betafact (FIX plasmatique).
Le GPAD-FXa a été ajouté à raison de 0,18 µg/ml (courbe ◊) ou 0,36 µg/ml (courbe ◆) dans l'échantillon de plasma déficient en FIX.

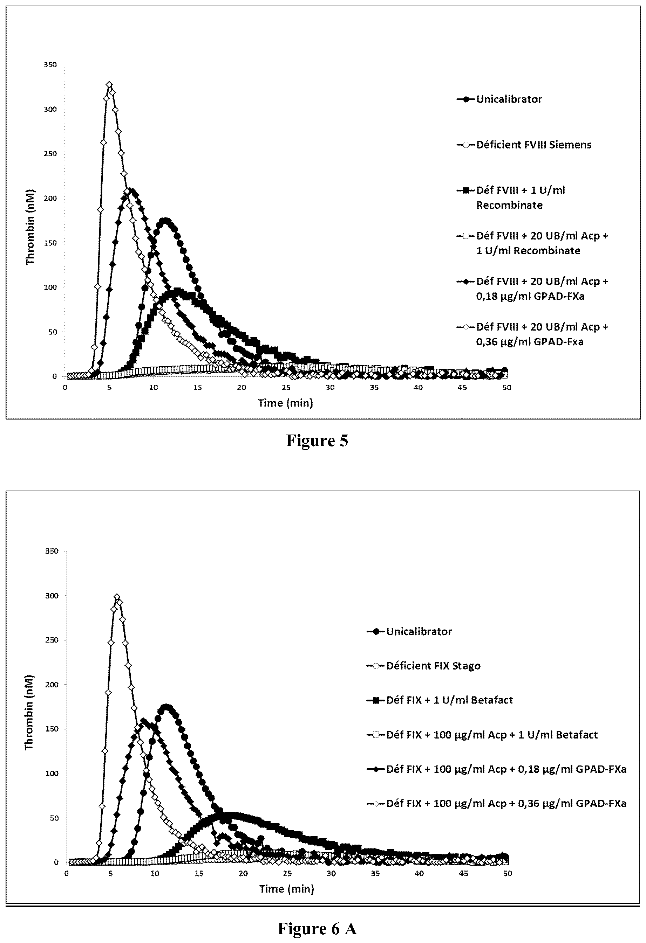

### Figure 5 : Thrombinogramme obtenu à partir de l'ajout de 0,18 µg/ml ou 0,36 µg/ml de GPAD-FXa dans un pool de plasma déficient en FVIII en présence d'anticorps inhibiteurs du FVIII suite à l'activation par du facteur tissulaire:

- en abscisses : le temps (en minutes)
- en ordonnées la concentration maximale de thrombine observée (en nM)

La génération de thrombine obtenue à partir d'un échantillon de pool de plasma normal est représentée par la courbe ●, à partir d'un plasma déficient en facteur VIII par la courbe ○. La courbe ■ représente un plasma déficient en FVIII substitué par 1 U/ml de FVIII recombinant (Recombinate) et, la courbe □ le même échantillon en présence d'anticorps polyclonaux anti-FVIII possédant une activité inhibitrice de 20 Unités Bethesda/ml. Les courbes ◆ et ◊ représentent le signal d'un plasma déficient en FVIII et contenant des anticorps polyclonaux anti-FVIII possédant une activité inhibitrice de 20 Unités Bethesda/ml en présence respectivement de 0,18 et 0,38 µg/ml GPAD-FXa.

### Figure 6A : Thrombinogramme obtenu à partir de l'ajout de 0,18 µg/ml ou 0,36 µg/ml de GPAD-FXa dans un pool de plasma déficient en FIX en présence d'anticorps inhibiteurs du FIX suite à l'activation par du facteur tissulaire:

- en abscisses : le temps (en minutes)
- en ordonnées la concentration maximale de thrombine observée (en nM)

La génération de thrombine obtenue à partir d'un échantillon de pool de plasma normal est représentée par la courbe ●, à partir d'un plasma déficient en facteur IX par la courbe ○. La courbe ■ représente un plasma déficient en FIX substitué par 1 U/ml de FIX plasmatique (Betafact) et, la courbe □ le même échantillon en présence d'anticorps polyclonaux anti-FIX (100 µg/ml). Les courbes ◆ et ◊ représentent le signal d'un plasma déficient en FIX et contenant des anticorps polyclonaux anti-FIX en présence respectivement de 0,18 et 0,38 µg/ml GPAD-FXa.

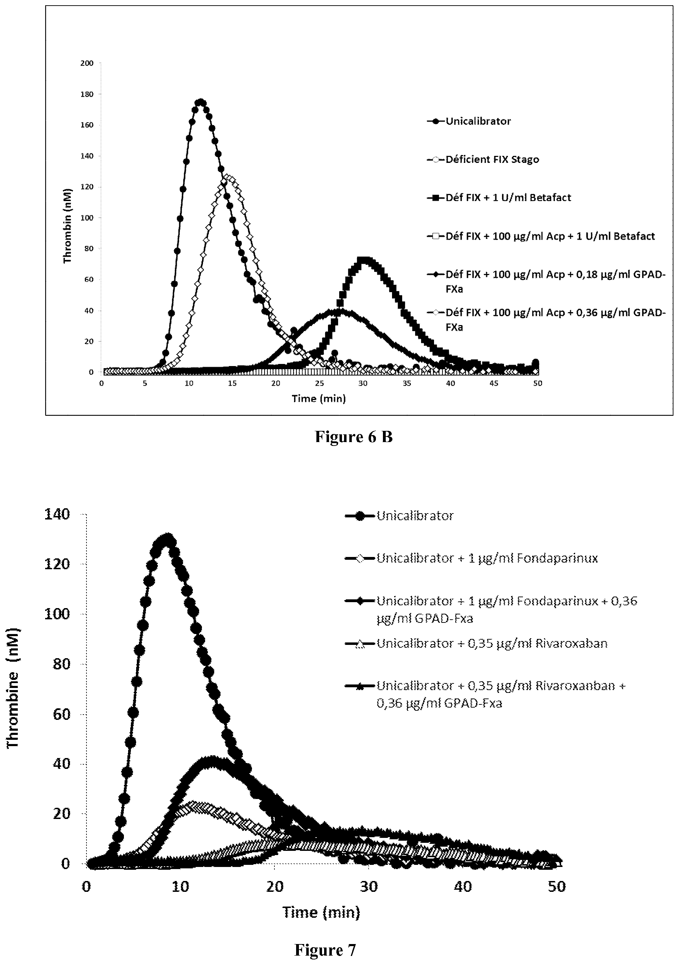

### Figure 6B : Thrombinogramme obtenu à partir de l'ajout de 0,18 µg/ml ou 0,36 µg/ml de GPAD-FXa dans un pool de plasma déficient en FIX en présence d'anticorps inhibiteurs du FIX suite à l'activation par la céphaline:

- en abscisses : le temps (en minutes)
- en ordonnées la concentration maximale de thrombine observée (en nM)

La génération de thrombine obtenue à partir d'un échantillon de pool de plasma normal est représentée par la courbe ●, à partir d'un plasma déficient en facteur IX par la courbe ○. La courbe ■ représente un plasma déficient en FIX substitué par 1 U/ml de FIX plasmatique (Betafact) et, la courbe □ le même échantillon en présence d'anticorps polyclonaux anti-FIX (100 µg/ml). Les courbes ◆ et ◊ représentent le signal d'un plasma déficient en FIX et contenant des anticorps polyclonaux anti-FIX en présence respectivement de 0,18 et 0,38 µg/ml GPAD-FXa.

### Figure 7 : Thrombinogrammes obtenus à partir de l'ajout de 0,36 µg/ml de GPAD-FXa en plasma déficient en FVIII contenant du Fondaparinux (1 µg/ml) ou du Rivaroxaban (0,35 µg/ml) suite à l'activation par la céphaline:

- en abscisses : le temps (en minutes)
- en ordonnées : la concentration maximale de thrombine observée (en nM)

L'échantillon de pool de plasma normal est représenté par la courbe ●. Les courbes (◆) et (◊) représentent respectivement l'effet de l'ajout de Fondaparinux (1µg/ml) dans le plasma avec ou sans GPAD-FXa (0,36 µg/ml). Les courbes (▲) et (Δ) représentent respectivement l'effet de l'ajout de Rivaroxaban (0,35 µg/ml) dans le plasma avec ou sans GPAD-FXa (0,36 µg/ml).

### Figure 8 : Effet de la présence de GPAD-FXa sur les temps de prothrombine et de céphaline activée en présence d'inhibiteurs de la coagulation

Les temps de prothrombine (Pt ; colonne noire) et de céphaline activée (TCA ; colonne blanche) ont été mesurés sur du plasma normal ou du plasma inhibé par le Fondaparinux (1 µg/ml) ou du Rivaroxaban (0, 35 µg/ml) en présence ou non de GPAD-FXa (0,36 µg/ml).

### Figure 9 : Constructions GPAD1, GPAD2, et leurs versions optimisées

Les constructions GPAD 1 et GPAD 2 et leur version optimisée sont schématiquement représentées ainsi que 2 constructions GPAD 3 contenant un linker de taille variable (L1 ou L2). Les séquences GPAD1-FXa et GPAD2-FXa diffèrent par l'adjonction d'un site furine supplémentaire dans le GPAD2-FXa en aval du site d'activation du FX (RKR en gras, flèche double). Le site de séparation de la chaine légère et de la chaine lourde est indiqué par la flèche jaune. Dans GPAD3, L1 est un linker de taille et de composition variable se terminant par une arginine à l'extrémité C-terminale ; L2 est un linker de taille et de composition variable débutant par la séquence RKR et se terminant à l'extrémité C- terminale par une arginine

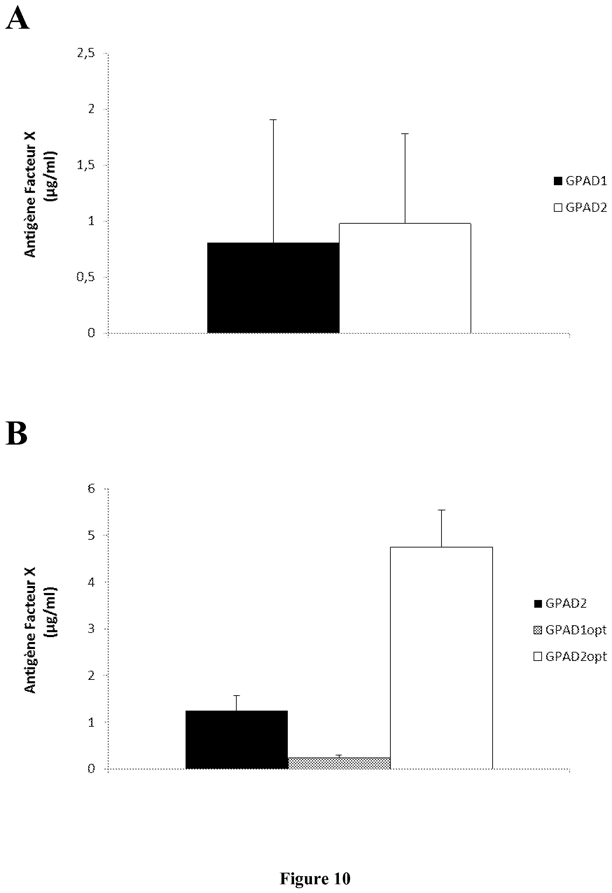

### Figure 10: Comparaison des productions des différentes molécules GPAD-FXa dans HEK293

Production (en µg/ml) des molécules GPAD1-FXa et GPAD2-FXa (panel A) et GPAD2-FXa, GPAD1opt-FXa et GPAD2opt-FXa (panel B) dans la lignée HEK293. Les productions sont une moyenne de valeurs obtenues suite à différentes transfections à partir de différents vecteurs d'expression (pCEP4, OptiHEK, pTT) et suite à différents temps de production (7 ou 11 jours). Panel A: GPAD1-FXa, n=6 ; GPAD2-FXa, n=20 ; Panel B : GPAD2-FXa, n= 4 ; GPAD1opt-FXa, n=7; GPAD2opt-FXa, n=4.

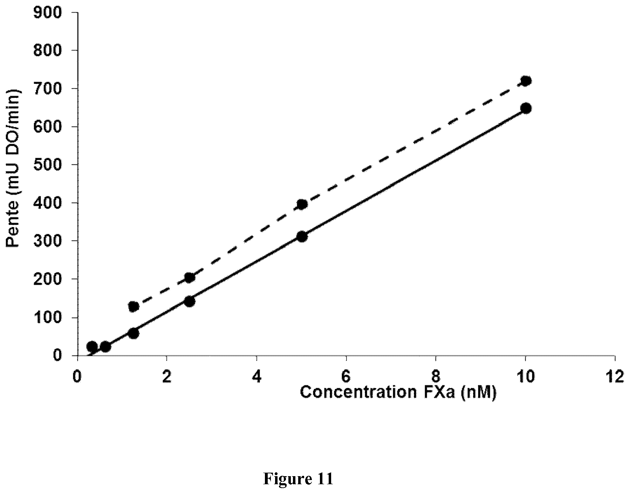

### Figure 11 : Activité chromogénique du GPAD2opt-FXa

Le GPAD2opt-FXa a été purifié suivant un protocole utilisant une colonne d'héparine suivie d'une colonne de phenyl-sepharose. L'activité amidolytique vis-à-vis du substrat Pefachrome 8595 au cours du temps du GPAD2opt-FXa (courbe pointillée) est comparée à l'activité du facteur X activé (courbe pleine).
- en abscisses : le temps (en minutes)
- en ordonnées : la vitesse d'apparition du produit de dégradation du Pefachrome 8595 en mUDO/min

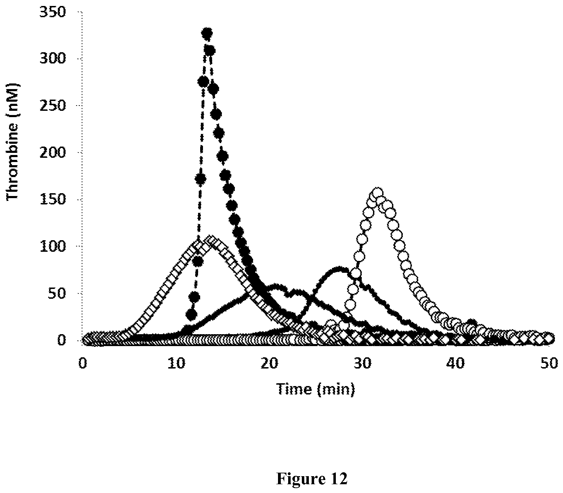

### Figure 12 : Thrombinogrammes obtenus à partir de l'ajout de GPAD2opt-FXa produit ou non en présence de furine dans un pool de plasma déficient en FVIII suite à l'activation par la céphaline:

- en abscisses : le temps (en minutes)
- en ordonnées : la concentration maximale de thrombine observée (en nM)

L'échantillon de pool de plasma normal est représenté par la courbe ● et l'échantillon correspondant à un plasma déficient en facteur VIII par la courbe ○ non reliée. La courbe pointillée ● représente un plasma déficient en FVIII substitué par 1 U/ml de FVIII recombinant (Recombinate) et celle reliée des ○ substitué par 0,1 U/ml.
Les GPAD2op-FXa (2 µg/ml) produits avec la furine (courbe ◊) ou sans furine (courbe ◆) ont été analysés dans du plasma déficient en FVIII.

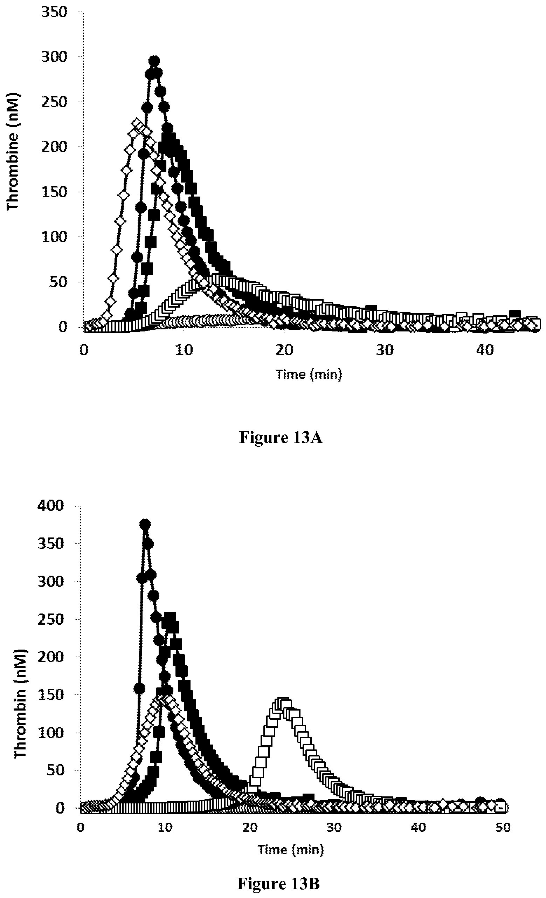

### Figure 13 : Thrombinogrammes obtenus à partir de l'ajout de GPAD2opt-FXa dans un pool de plasma déficient en FVIII:

- en abscisses : le temps (en minutes)
- en ordonnées : la concentration maximale de thrombine observée (en nM)

L'échantillon de pool de plasma normal est représenté par la courbe ● et l'échantillon correspondant à un plasma déficient en facteur FVIII par la courbe ○. Dans le panel A, la coagulation est stimulée par l'ajout d'un mélange de FT 0,5 nM/PL 4 µM. Dans le panel B, la stimulation est initiée par de la céphaline. La courbe ■ représente un plasma déficient en FVIII substitué par 1 U/ml de Recombinate (FVIII recombinant Baxter) et la courbe □ représente un plasma déficient en FVIII substitué par 0,1 U/ml. Le GPAD2op-FXa (2 µg/ml, courbe ◇) produit en présence de furine a été analysé dans du plasma déficient en FVIII.

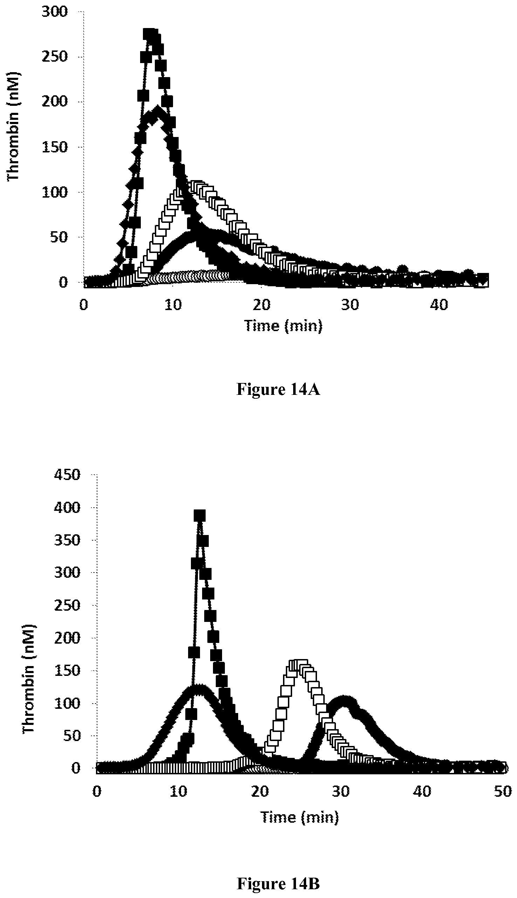

### Figure 14 : Thrombinogrammes obtenus à partir de l'ajout de GPAD2opt-FXa dans un pool de plasma déficient en FIX:

- en abscisses : le temps (en minutes)
- en ordonnées : la concentration maximale de thrombine observée (en nM)

L'échantillon de pool de plasma normal est représenté par la courbe ● et l'échantillon correspondant à un plasma déficient en facteur IX par la courbe ○. Dans le panel A, la coagulation est stimulée par l'ajout d'un mélange de FT 0,5 nM/PL 4 µM. Dans le panel B, la stimulation est initiée par de la céphaline. La courbe ■ représente un plasma déficient en FIX substitué par 1 U/ml de FIX (Betafact) et la courbe □ représente un plasma déficient en FIX substitué par 0,1 U/ml. Le GPAD2op-FXa (2 µg/ml, courbe ◆) produit en présence de furine a été analysé dans du plasma déficient en FIX.

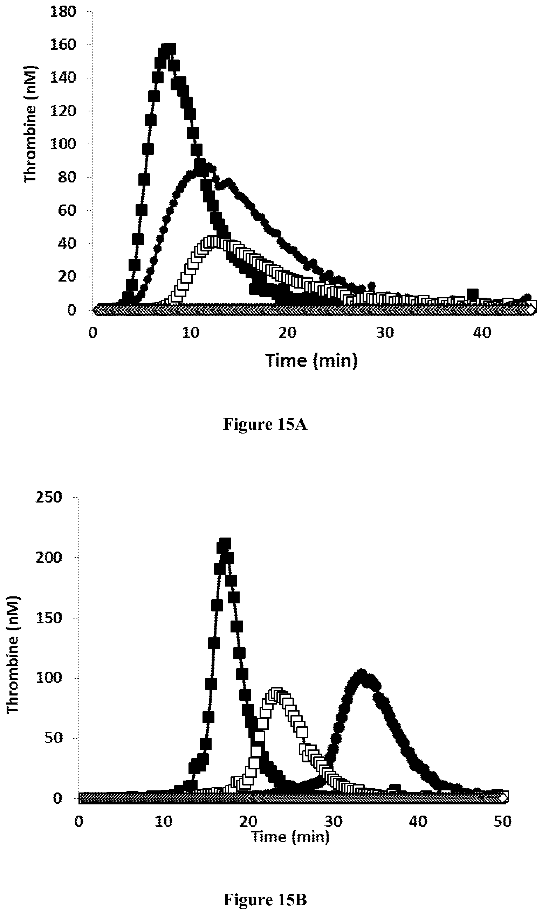

### Figure 15 : Thrombinogrammes obtenus à partir de l'ajout de GPAD2opt-FXa dans un pool de plasma déficient en FX:

- en abscisses : le temps (en minutes)
- en ordonnées : la concentration maximale de thrombine observée (en nM)

L'échantillon de pool de plasma normal est représenté par la courbe ● et l'échantillon correspondant à un plasma déficient en facteur X est représenté par la courbe ○. Cette dernière courbe n'est pas distinguable car elle se situe au niveau de l'axe des abscisses. Dans le panel A, la coagulation est stimulée par l'ajout d'un mélange de FT 0,5 nM/PL 4 µM. Dans le panel B, la stimulation est initiée par de la céphaline. La courbe ■ représente un plasma déficient en FX substitué par 10 µg/ml de FX plasmatique (Cryopep) et celle des □ substitué par 1 µg/ml. Le GPAD2op-FXa (2 µg/ml, courbe ◇) produit en présence de furine a été analysé dans du plasma déficient en FX.

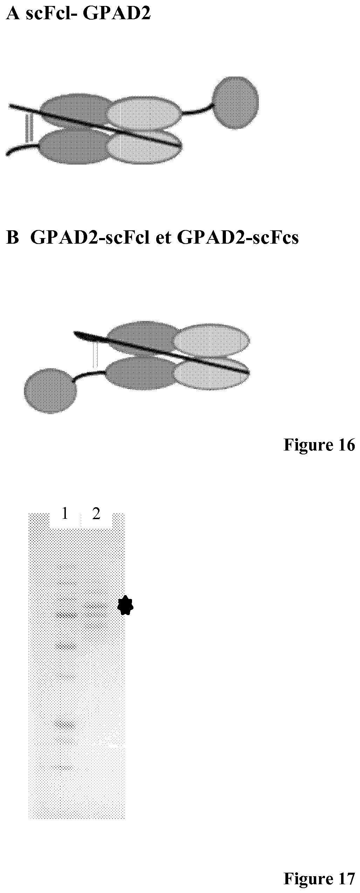

### Figure 16 : Schéma des constructions des GPAD2opt-FXa fusionnés aux scFc

Représentation schématique des molécules GPAD2opt-scFcs et GPAD2opt-scFcl (A) et scFcl-GPAD2opt-FXa (B). Les ellipses oranges et rouges représentent les domaines CH3 et CH2 respectivement des immunoglobulines gamma. Les linkers sont représentés par les lignes noires. Le domaine GPAD2opt-FXa est représenté par un cercle bleu. Dans les constructions GPAD2opt-scFcs et GPAD2opt-scFcl, le domaine GPAD2opt-FXa est cloné en position N-terminale par rapport au fragment scFc. Dans la construction scFcl-GPAD2opt-FXa, il est cloné en position C-Terminale. La différence entre GPAD2opt-scFcs et GPAD2opt-scFcl réside dans la taille du linker qui n'est pas montré sur cette figure.

### Figure 17 : Purification du scFcl-GPAD2opt-FXa

La molécule scFcl-GPAD2opt-FXa (900 µg) produite par expression transitoire dans HEK a été purifiée sur gel de protéine A puis analysée après séparation en SDS-PAGE et coloration au Nupage Blue. 1, Marqueurs moléculaires ; 2, scFcl-GPAD2opt purifié ; *, poids moléculaire attendu pour le scFcl-GPAD2opt-FXa

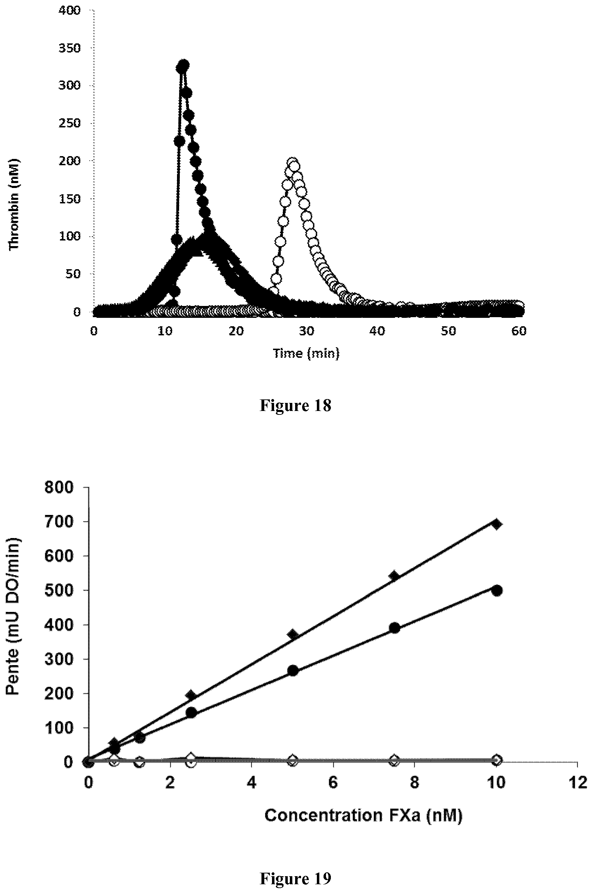

### Figure 18 : Thrombinogrammes obtenus à partir de l'ajout de GPAD2opt-FXa ou scFcl-GPAD2opt-FXa dans un pool de plasma déficient en FVIII suite à l'activation par la céphaline:

- en abscisses : le temps (en minutes)
- en ordonnées : la concentration maximale de thrombine observée (en nM)

Les échantillons de pool de plasma déficient en facteur VIII et substitué par 1 U/ml ou 0, 1 U/ml de FVIII sont représentés par les courbes ● et ○ respectivement. L'effet du GPAD2op-FXa (2 µg/ml, courbe ◆) et du scFcl GPAD2op-FXa (4 µg/ml, courbe ▲) a été analysé dans du plasma déficient en FVIII après activation par de la céphaline.

### Figure 19 : Inhibition de l'activité chromogénique du scFcl-GPAD2opt-FXa par le Rivaroxaban

Les activités chromogéniques sur le substrat Pefachrome 8595 du FXa d'origine plasmatique (courbe ●) ou du scFcl-GPAD2opt-FXa (courbe ◆) ont été mesurées comme décrit dans l'exemple 1. Ces activités ont aussi été mesurées en présence de Rivaroxaban (0,35 µg/ml). Les résultats du FXa d'origine plasmatique sont représentés par la courbe ○ et pour le scFcl-GPAD2opt-FXa par la courbe ◊.

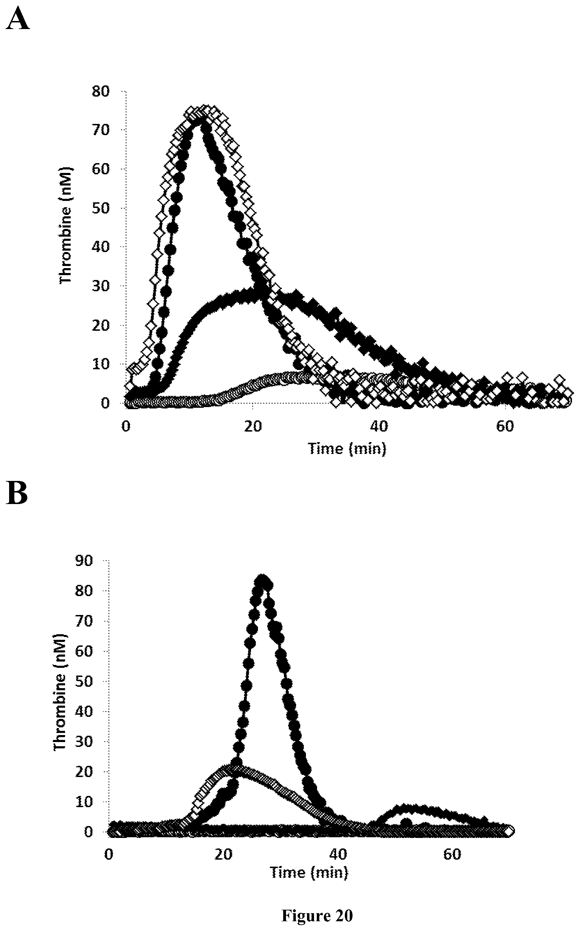

### Figure 20 : Thrombinogrammes obtenus à partir de l'ajout de scFcl-GPAD2opt-FXa dans un plasma normal supplémenté en Rivaroxaban

- en abscisses : le temps (en minutes)
- en ordonnées : la concentration maximale de thrombine observée (en nM)

Les échantillons de pool de plasma normal ou supplémenté en Rivaroxaban (0,25 µg/ml) sont représentés par les courbes ● et ○ respectivement. L'effet de l'adjonction de doses croissantes de scFcl GPAD2opt-FXa (25 ou 50 µg/ml) dans du plasma normal supplémenté en Rivaroxaban (0,25 µg/ml) est visualisé respectivement par les courbes: ◆ et ◇. Panel A, initiation du TGT par le mélange FT 0.5 pM/PL 4 µM. Panel B, initiation du TGT par de la céphaline.

### Exemples

### Exemple 1 : Préparation du GPAD-FXa

### Matériel :

Les facteurs X plasmatiques humain activé ou non (FXa/FX), les substrats chromogéniques pNAPEP 1025 et Pefachrome 8595, et le plasma d'hémophile A avec inhibiteurs et les anticorps polyclonaux anti-FX et anti-FIX proviennent de Haematologic Technologies (Cryopep Montpellier, France).
Le facteur VIII recombinant de contrôle (Recombinate) provient de chez Baxter. Le facteur IX plasmatique provient de LFB. Les plasmas déficients en FVIII proviennent de chez Siemens, ceux en FIX de chez Stago.
Les Anticorps polyclonaux anti-FVIII proviennent de chez Cedarlane.

### Méthodes :

### Production de l'ADNc de GPAD-FXa

L'ADNc codant pour un facteur X dénué du domaine gamma-carboxyglutamique et étiqueté par la séquence HPC4 (voir brevet FR 11 51637 déposée le 1er mars 2011) va servir de matrice pour la génération de GPAD-FXa. La délétion du peptide d'activation et son remplacement par un site furine (RKR) est apporté par l'utilisation de l'amorce sens (5'-ctggaacgcaggaagaggaggaagaggatcgtgggaggc, SEQ ID NO:17) et anti-sens (5'-: gcctcccacgatcctcttcctcctcttcctgcgttccag, SEQ ID NO :18). La modification de l'ADNc est réalisée avec la méthode Quickchange (Stratagène) et l'enzyme pFU Ultra.

### Production du GPAD-FXa recombinant

La veille de la transfection, les cellules HEK 293F sont repiquées à une concentration cellulaire de 7 X 10⁵ vc/ml. La densité cellulaire et la viabilité des cellules sont déterminées le jour de la transfection. Le volume de culture correspondant à 30 X 10⁶ cellules est centrifugé. Le surnageant est éliminé et le culot de cellules est repris dans 28 ml de milieu de culture F17 (Invitrogen), transféré dans un Erlenmeyer de 250 ml et incubé à 37 °C. Un complexe agent de transfection / ADN en rapport 2:1 est formé. L'agent de transfection et l'ADN correspondant au vecteur contenant une des séquences du GPAD-FXa sont préparés dans du milieu OptiMEM (Invitrogen) de la façon suivante:
- Ajout de 30 µg d'ADN dans 1 ml d'OptiMEM
- Ajout de 60 µl d'agent de transfection dans 1 ml d'OptiMEM.

Ces deux préparations sont incubées séparément pendant 5 minutes à température ambiante puis la solution contenant l'agent de transfection est ajoutée à celle contenant l'ADN. Le mélange est incubé pendant 25 minutes à température ambiante avant d'être ajouté au 28 ml de cellules HEK 293F. Les cellules sont alors incubées à 37°C en agitation à 125 rpm.
Un vecteur exprimant la GFP (Green Fluorescent Protein ; témoin positif de transfection) est également transfecté dans les mêmes conditions. L'efficacité de transfection est déterminée 24h post-transfection par microscopie à fluorescence en établissant le rapport du nombre de cellules exprimant la GFP sur le nombre total de cellules. Les cellules sont maintenues en culture durant 7 jours. La densité cellulaire et la viabilité sont déterminées tous les jours entre le 5^{ème} et le 7^{ème} jour à l'aide d'un automate d'analyse de culture cellulaire (Cedex, Innovatis - Roche Applied Science). La mesure de la viabilité est basée sur le comptage des cellules ayant incorporées du bleu trypan. Le 7^{ème} jour, les cellules sont centrifugées à 3000g pendant 15 minutes. Le culot cellulaire est éliminé et le surnageant cellulaire contenant le GPAD-FXa recombinant est filtré en 0,22 µm puis congelé à -20°C. Avant utilisation, les surnageants sont concentrés jusqu'à 10 fois sur des colonnes Vivaspin 10 kDa.

### Visualisation des protéines suivant une séparation en SDS-PAGE

Pour chaque gel au moins un témoin de masse moléculaire approprié est utilisé. Une quantité identique de protéine à détecter est ensuite chargée dans chaque puits en tampon de charge 2X concentré (Tris HCl 1M pH 6,8; 20% SDS; 20% glycérol ; 0,1% bleu bromophénol). Les échantillons réduits sont traités par du tampon de charge en présence de 0,5 M β-mercaptoéthanol. Le tampon de migration NuPAGE MOPS SDS 1x (Invitrogen) est utilisé pour la migration. Les échantillons sont laissés à migrer à 200V durant 50 minutes dans un gel NuPAGE Novex Bis-Tris 4-12 %.

Lorsque l'électrophorèse est terminée, les protéines séparées sur le gel sont immunodétectées après électrotransfert sur membrane de nitrocellulose ou PVDF en Tampon de Towbin (25 mM Tris, 192 mM glycine, 3,5 mM SDS, éthanol 10 %) en appliquant un courant de 0,8 mA/cm² pendant 1h30. La membrane est ensuite rincée en eau et incubée en tampon de saturation (PBS, 1% Sérum albumine bovine, 0,05% Tween-20). La membrane est ensuite incubée en tampon de saturation en présence de l'anticorps de mouton anti-FX humain (Cryopep 9-PAHFX-S) à 2 µg/ml pendant 1h puis en présence de l'anticorps secondaire (anti-mouton IgG H+L péroxidase - Jackson Laboratory) au 1/10 000^{ème} pendant 1h suite à 4 lavages par une solution d'eau physiologique (0,9% NaCl) contenant 0,05% de Tween-20. Un protocole de lavages identiques est appliqué avant révélation. L'immunoblot est révélé par chemiluminescence en utilisant le kit Super signal WEST PICO substrat chemiluminescent (PIERCE).

### Mesure de la concentration en facteur X

La mesure de concentration des FX, FXa et GPAD-FXa est effectuée grâce au kit commercial Zymutest Factor X (Hyphen Biomed). L'ELISA donne un signal linéaire pour des concentrations allant de 200 ng/ml à 1,5 ng/ml de factor X plasmatique. Les trois protéines sont reconnues de manière identique par le kit.

### Mesure de l'activité chromogénique du facteur X activé

La mesure de l'activité du FX, FXa et GPAD-FXa ont été étudiées à 37°C dans le tampon stop (50 mM Tris, pH 8,8, 0,475 M NaCl, 9 mM EDTA). L'activité chromogénique du FXa à différentes concentrations a été suivie dans le temps par la mesure de l'hydrolyse du substrat Pefachrome 8595 (250 µM) à 405 nm.

### Protocole de la mesure de génération de thrombine :

Le Fluoroskan Ascent, tous les réactifs après reconstitution et les échantillons sont préchauffées à 37°C. Les réactifs 0,5 pM FT/4 µM PL (concentration finale) et Céphaline (16,7% concentration finale) seront utilisés comme inducteurs de la réaction. Le réactif « Thrombin Calibrator » (Diagnostica Stago) est utilisé systématiquement comme contrôle des essais. Chaque échantillon à tester (80 µl) est étudié en dupliquat en présence de 20 µl du mélange FT/PL ou 20 µl de Céphaline. Le substrat fluorogénique (20 µL ; FluCa Kit (Diagnostica Stago) est alors ajouté et l'apparition de thrombine est suivie par une excitation à 390 nm et une émission à 460 nm durant 60 min. Les résultats sont ensuite analysés grâce au logiciel Thrombinoscope (Stago, Asnières, France).

### Exemple 2 : Analyse du GPAD-FXA produit en HEK293

Les surnageants de culture des cellules transfectées par un vecteur vide ou un vecteur codant pour GPAD-FXa ont été dosés avec le Kit Zymutest Factor X (Hyphen Biomed). Dans les milieux témoins, aucune quantité significative de FX n'a été détectée. En revanche dans les milieux des cellules exprimant GPAD-FXa des taux de 0,11 à 0,44 µg/ml ont été dosés. Ces concentrations varient en fonction des différents essais et des volumes de production. Ces données indiquent que GPAD-FXa est produit par les cellules HEK293 et qu'il est reconnu par le kit de détection du facteur X.

Afin de confirmer la présence et de visualiser la qualité du GPAD-FXa produit dans le surnageant de HEK293, un aliquot de milieu concentré a été analysé par SDS-PAGE sur gradient d'acrylamide (figure 1). Des quantités de protéines identiques ont été déposées sur gel et les signaux obtenus en immunoblot sont très proches suggérant que la quantification par ELISA de GPAD-FXa est correcte. Les échantillons ont été déposés réduits (R) par le β-mercaptoéthanol ou non (NR). Ils ont été comparés avec les FX et FXa plasmatiques traités de manière identique. Le GPAD-FXa non réduit migre sous une seule forme moléculaire à un poids moléculaire apparents de 42,1 kDa correspondant au poids attendu (flèche supérieure). Après réduction, le GPAD-FXa migre sous deux formes moléculaires l'une au même poids moléculaire que la forme non réduite et l'autre à un poids moléculaire de 35,6 kDa (flèche inférieure). Cette dernière forme migre de la même manière que la chaine lourde du FXa. Ces résultats suggèrent que le GPAD-FXa est produit sous deux formes moléculaires : une forme simple chaine non-clivée de 42,1 kDa et une forme dont les chaines lourdes et légères sont clivées et retenues par un pont disulfure.

### Exemple 3 : Activité amidolytique du GPAD-FXa

Les résultats en immunoblot suggèrent que la molécule est produite au moins partiellement sous sa forme active. Afin de confirmer cette hypothèse et de vérifier que le site catalytique du GPAD-FXa est resté fonctionnel, une mesure de l'activité amidolytique de la molécule est réalisée. Différentes concentrations de GPAD-FXa sont incubées 5 min à 37°C en présence du substrat chromogénique Pefachrome 8595, spécifique du FXa. La vitesse initiale d'apparition du substrat (en mUDO/min) est déterminée en fonction de la concentration initiale en FX(a) ou GPAD-FXa (figure 2). Comme attendu, le FX non activé (courbe ○) ne clive pas le substrat contrairement au FXa (courbe ■). De 0 à 10 nM de FXa la vitesse d'apparition du substrat augmente de manière linéaire. GPAD-FXa (courbe ◊) montre aussi une capacité à cliver le Pefachrome 8595. Cette capacité est toutefois plus faible que celle du FXa. Cependant la présence dans le surnageant de molécules de GPAD-FXa non activées par la furine, et donc dépourvues d'activité enzymatique peut expliquer cette différence quantitative.

### Exemple 4 : Le GPAD-FXa corrige un déficit en FVIII

La capacité du GPAD-FXa à restaurer la génération de thrombine en plasma déficient en FVIII a été évaluée suite à une induction par le facteur tissulaire (figure 3A) ou la céphaline (figure 3 B). Dans les conditions expérimentales, le plasma déficient en FVIII (Siemens) ne permet pas de générer de thrombine (courbe ○). La supplémentation de ce plasma par 1 unité/ml de FVIII (Recombinate, Baxter) permet une génération de thrombine importante (courbe ■). La quantité de thrombine générée totale est de 1579 nM de thrombine (Table I). L'adjonction de surnageant de culture contenant le GPAD-FXa à 0,18 µg/ml (1,8 % de la concentration plasmatique de FX soit 0,018 U/ml) permet de restaurer une production de thrombine (courbe ◊). La quantité de thrombine générée par GPAD-FXa (ETP) est de l'ordre de 1545 nM correspondant à 98 % de la génération de thrombine par du FVIII de substitution normalisateur. Par ailleurs, la présence de GPAD-FXa permet aussi de normaliser le temps d'apparition de la thrombine avec un temps pour atteindre le somment du pic à 6,83 min contre 8,67 min pour le plasma substitué. Une dose deux fois plus importante de GPAD-FXa (0,36 µg/ml) possède même un effet plus important que le FVIII normalisateur avec une quantité de thrombine générée de 1651 nM et un temps d'apparition du pic raccourci à 4,83 min. Le contrôle positif Unicalibrator (Stago) permet de vérifier la validité de l'expérience par la génération de thrombine dans un plasma sain (courbe ●). Une expérience similaire est réalisée en induisant la génération de thrombine par la céphaline. Dans ces conditions le plasma déficient en FVIII ne permet pas de générer de la thrombine (courbe ○). Au contraire du plasma déficient substitué en FVIII génère 1472 nM de thrombine (courbe ■). La présence de GPAD-FXa 0,18 µg/ml permet de générer une quantité de thrombine correspondant à 1160 nM (courbe ◊ ; 79 % du FVIII). Le signal dépend de la dose rajoutée puisque une dose double de GPAD-FXa (0,36 µg/ml) permet d'obtenir 1316 nM de thrombine (courbe ◆ ; 89% du FVIII). Le contrôle positif Unicalibrator (Stago) permet de vérifier la validité de l'expérience par la génération de thrombine dans un plasma sain (courbe ●).

**Table I : Paramètres cinétiques de la génération de thrombine en plasma déficient en FVIII activé par le facteur tissulaire**

| **Groupname** | **Unicalibrator** | **Déficient FVIII Siemens** | **Déf FVIII + Recombinate (1U/ml)** | **Déf FVIII + GPAD-Fxa (0,16 µg/ml)** | **Déf FVIII + GPAD-Fxa (0,32 µg/ml** |
|---|---|---|---|---|---|
| **Lagtime (min)** | **7,5** | **5,67** | **5,83** | **4** | **3** |
| **ETP (nM)** | **1293** | **400** | **1579** | **1545,5** | **1651** |
| **Peak (nM)** | **191,57** | **15,34** | **240,83** | **239,96** | **352,7** |
| **ttPeak (min)** | **10,83** | **22,5** | **8,67** | **6,83** | **4,83** |

**Table II : Paramètres cinétiques de la génération de thrombine en plasma déficient en FVIII activé par la céphaline**

| **Groupname** | **Unicalibrator** | **Déficient FVIII Siemens** | **Déf FVIII +Recombinate (1U/ml)** | **Déf FVIII + GPAD-Fxa (0,16 µg/ml)** | **Déf FVIII + GPAD-Fxa (0,32 µg/ml** |
|---|---|---|---|---|---|
| **Lagtime (min)** | **12,83** | **1,17** | **9,67** | **10,17** | **5,5** |
| **ETP (nM)** | **1139** | **0** | **1472,5** | **1160,5** | **1316** |
| **Peak (nM)** | **279,72** | **0,76** | **321,76** | **123,12** | **236,63** |
| **ttPeak (min)** | **15,5** | **32** | **11,33** | **16,5** | **8,33** |

### Exemple 5 : Le GPAD-FXa corrige un déficit en FIX

La capacité du GPAD-FXa à restaurer la génération de thrombine en plasma déficient en FIX a été évaluée suite à une induction par le facteur tissulaire (figure 4 A) ou la céphaline (figure 4 B). Dans les conditions expérimentales, le plasma déficient en FIX (Stago) ne permet pas de générer de thrombine (courbe ○). La supplémentation de ce plasma par 1 unité/ml de FIX (Betafact, LFB) permet une génération de thrombine importante (courbe ■). La quantité de thrombine générée totale est de 1123 nM de thrombine (Table III). L'adjonction de surnageant de culture contenant le GPAD-FXa à 0,18 µg/ml (1,8 % de la concentration plasmatique de FX soit 0,018 U/ml) permet de restaurer une production de thrombine (courbe ◊). La quantité de thrombine générée par GPAD-FXa (ETP) est de l'ordre de 1395 nM correspondant à 124 % de la génération de thrombine par du FIX de substitution normalisateur. Par ailleurs, la présence de GPAD-FXa permet aussi de normaliser le temps d'apparition de la thrombine avec un temps pour atteindre le somment du pic à 9,67 min contre 14,17 min pour le plasma substitué. Une dose deux fois plus importante de GPAD-FXa (courbe ◆ ; 0,36 µg/ml) possède un effet encore plus important que le FIX normalisateur avec une quantité de thrombine générée de 1437 nM et un temps d'apparition du pic raccourci à 6,17 min. Le contrôle positif Unicalibrator (Stago) permet de vérifier la validité de l'expérience par la génération de thrombine dans un plasma sain (courbe ●). Une expérience similaire est réalisée en induisant la génération de thrombine par la céphaline. Dans ces conditions le plasma déficient en FIX ne permet pas de générer de la thrombine (courbe ○). Au contraire du plasma déficient substitué en FIX génère 1030 nM de thrombine (courbe ■). La présence de GPAD-FXa 0,18 µg/ml permet de générer une quantité de thrombine correspondant à 493 nM (courbe ◊ ; 48 % du FIX). Le signal dépend de la dose rajoutée puisque une dose double de GPAD-FXa (0,36 µg/ml) permet d'obtenir 1032 nM de thrombine (courbe ◆ ; 100% du FIX). Cette dose de GPAD-FXa permet aussi de raccourcir le temps au pic de 28 à 14 min. Le contrôle positif Unicalibrator (Stago) permet de vérifier la validité de l'expérience par la génération de thrombine dans un plasma sain (courbe ●).

**Table III: Paramètres cinétiques de la génération de thrombine en plasma déficient en FIX activé par le facteur tissulaire**

| **Groupname** | **Unicalibrator** | **Déficient FIX Stago** | **Déf FIX + Betafact (1 U/ml)** | **Déf FIX + GPAD-FXa (0,18 µg/ml)** | **Déf FIX + GPAD-FXa (0,32 µg/ml)** |
|---|---|---|---|---|---|
| **Lagtime** | **7** | **5,17** | **8,33** | **5** | **3,83** |
| **ETP** | **1343,5** | **172** | **1123,5** | **1395,5** | **1437** |
| **Peak** | **190,37** | **5,85** | **90,61** | **132,02** | **279,63** |
| **ttPeak** | **10,67** | **23,83** | **14,17** | **9,67** | **6,17** |

**Table IV: Paramètres cinétiques de la génération de thrombine en plasma déficient en FIX activé par la céphaline**

| **Groupname** | **Unicalibrator** | **Déficient FIX Stago** | **Déf FIX + Betafact (1 U/ml)** | **Déf FIX + GPAD-FXa 0,18 µg/ml** | **Déf FIX + GPAD-FXa 0,36 µg/ml** |
|---|---|---|---|---|---|
| **Lagtime (min)** | **12,83** | **0** | **20** | **14,17** | **7,67** |
| **ETP (nM)** | **1161** | **0** | **1030** | **493,5** | **1032,5** |
| **Peak (nM)** | **293,25** | **0** | **112,68** | **34,47** | **109,16** |
| **ttPeak (min)** | **15** | **0** | **28,17** | **24,33** | **14** |

### Exemple 6 : Le GPAD-FXa corrige un déficit en FVIII en présence d'anticorps inhibiteurs du FVIII

Une expérience similaire à l'exemple 4 a été conduite mais en ajoutant dans le plasma des anticorps inhibiteurs du facteur VIII (figure 5). Les anticorps ont été dosés et une dose équivalente à 20 Unité Bethesda/ml a été rajoutée. Cette dose est capable d'inhiber toute génération de thrombine induite par 1 U/ml de FVIII (courbe □). En présence de ces anticorps, GPAD-FXa à 0,18 µg/ml (courbe ◆) ou 0,36 µg/ml (courbe ◊) est capable de restaurer une génération de thrombine supérieure au plasma déficient en FVIII substitué avec du FVIII (1 U/ml ; courbe ■) mais aussi à un plasma normal (●). Il est à noter que les temps de pic sont diminués en présence de GPAD-FXa par rapport aux différents contrôles.

### Exemple 7 : Le GPAD-FXa corrige un déficit en FIX en présence d'anticorps inhibiteurs du FIX

Une expérience similaire à l'exemple 6 a été conduite mais en ajoutant dans du plasma déficient en FIX des anticorps inhibiteurs du facteur IX (figure 6A). La génération de thrombine a été suivie soit après une induction par le facteur tissulaire (figure 7A) soit après une induction par la céphaline (figue 6B). L'efficacité des anticorps a été mesurée et une dose de 100 µg/ml d'anticorps a été rajoutée lors de la mesure de génération de thrombine. Cette dose est capable d'inhiber toute génération de thrombine induite par 1 U/ml de FIX (courbe □). En présence de ces anticorps, GPAD-FXa à 0,18 µg/ml (courbe ◆) ou 0,36 µg/ml (courbe ◊) est capable de restaurer une génération de thrombine supérieure au plasma déficient en FIX substitué avec du FIX (1U/ml ; courbe ■) mais aussi à un plasma normal (●). Comme pour la déficience en FVIII en présence d'inhibiteurs, les temps au pic sont aussi diminués par rapport aux contrôles.

### Exemple 8 : Le GPAD-FXa corrige la présence d'inhibiteurs de la coagulation en génération de thrombine

Un essai de TGT a été réalisé en additionnant dans du plasma normal des doses thérapeutiques soit de Fondaparinux (1µg/ml) soit de Rivaroxaban (0,35 µg/ml). La génération de thrombine est très diminuée aux concentrations d'inhibiteurs utilisées (figure 7, courbes ◊ et Δ) par rapport à la courbe témoin (●). La supplémentation de GPAD-FXa (0,36 µg/ml) permet de restaurer significativement une partie de la génération de thrombine (figure 7, courbes ◆ et ▲). Le GPAD-FXa semble plus efficace pour corriger la présence de Fondaparinux puisque plusieurs paramètres du TGT sont améliorés (temps de latence, hauteur du pic, aire sous la courbe et vélocité). La correction du Rivaroxaban intervient surtout sur la quantité de thrombine générée et non sur la cinétique de son apparition. La concentration molaire des inhibiteurs étant en grand excès par rapport à GPAD-FXa, la correction de la génération de thrombine demeure partielle par rapport au plasma témoin.

### Exemple 9 : Le GPAD-FXa corrige la présence d'inhibiteurs de la coagulation en tests chronométriques

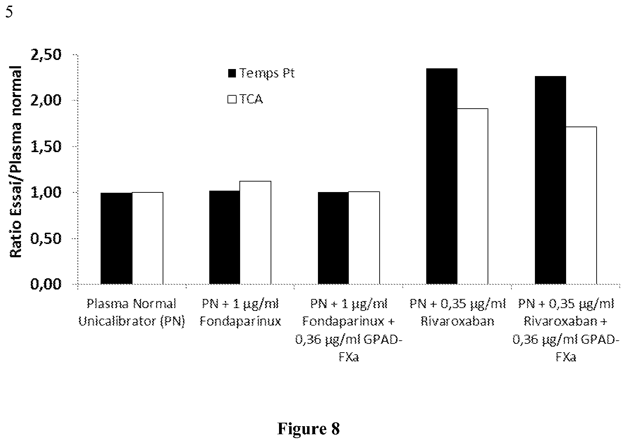

L'effet du GPAD-FXa en présence des inhibiteurs Fondaparinux (1µg/ml) et Rivaroxaban (0,35 µg/ml) a été mesuré par les tests chronométriques de mesure du temps de prothrombine (PT) et du temps de céphaline activée (TCA) (figure 8). Comme déjà démontré, la présence de Fondaparinux (1 µg/ml) n'affecte pas le temps de prothrombine (Smogorzewska A et al. Arch Pathol Lab Med. 2006 130(11): 1605-11). En conséquence, l'effet du GPAD-FXa ne peut être évalué. La présence de Rivaroxaban (0,35µg/ml) augmente le temps de prothrombine (de 13,75 à 32,3 s). La présence de GPAD-FXa ramène le temps de coagulation à 31,2 s soit une diminution de 4,5%.
La présence de Fondaparinux (1 µg/ml) augmente le TCA qui passe de 33,1 s à 37,3 s. La présence de GPAD-FXa permet de normaliser le TCA à 33,5 s en présence de cet inhibiteur. La présence de Rivaroxaban (0,35µg/ml) augmente très significativement le temps de prothrombine (de 33,1 à 63,25 s). La présence de GPAD-FXa permet de le diminuer à 56,65 s soit une diminution de 11 %.
Ces diminutions modestes de l'effet des inhibiteurs corrèlent avec les données obtenues dans l'exemple 8. Elles sont toutefois obtenues avec une dose de GPAD-FXa (0,36 µg/ml) beaucoup plus faible en molarité que celles des inhibiteurs (82 X et 115 X plus faible respectivement).

### Exemple 10 : Génération de constructions optimisées

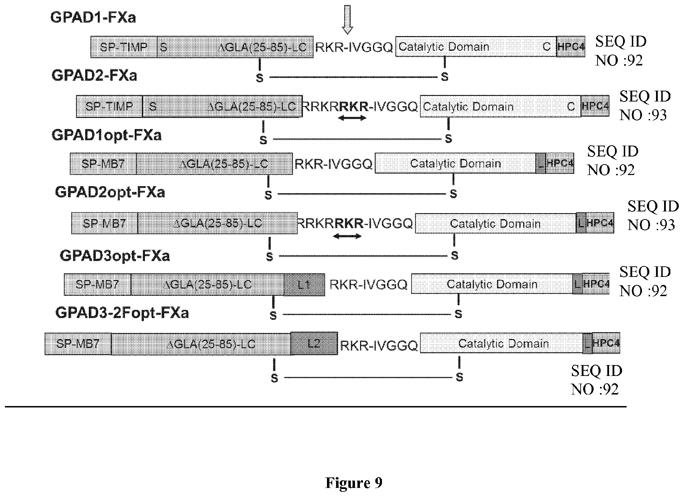

La construction GPAD-FXa correspondant à la fusion de la chaine légère tronquée du FX à la chaine lourde sans ajout de séquence supplémentaire est appelée GPAD1-FXa Cette fusion permet la formation naturelle d'une séquence protéique consensus correspondant à un site de clivage de la furine et qui sépare les deux chaines. La construction GPAD-FXa contenant un site de furine supplémentaire à la séquence protéique correspondant à une séquence fusionnée entre la chaine lourde et la chaine légère tronquée est appelée GPAD2-FXa (figure 9).. Pour tenter d'améliorer la production de ces molécules, des versions optimisées ont été réalisées en introduisant plusieurs modifications : un peptide signal du MB7 remplace le peptide signal TIMP, et la séquence codante a été optimisée pour une expression dans un système eucaryote. Les deux molécules résultantes sont nommées GPAD1opt-FXa et GPAD2opt-FXa (figure 9).
Par ailleurs, des structures de molécules GPAD3opt-FXa et GPAD3-2Fopt-FXa ont été réalisées soit en ajoutant un linker de taille variable se terminant par une arginine en amont du site RKR du peptide d'activation (L1 dans GPAD3opt-FXa), soit en ajoutant au même endroit des linkers débutant par la séquence RKR à l'extrémité N-terminale et se terminant par une arginine en C-terminale (L2 dans GPAD3-2Fopt-FXa, figure 9). Les molécules ont été exprimées de manière transitoire dans la lignée HEK293F et les taux de FX produits ont été mesurés par ELISA comme décrit dans l'exemple 1.
La comparaison de l'expression du GPAD1-FXa et du GPAD2-FXa montre un léger avantage non significatif pour l'expression de GPAD2-FXa (figure 10 A). La comparaison de l'expression de GPAD2-FXa, GPAD1opt-FXa et GPAD2opt-FXa montre elle, un avantage significatif à exprimer la molécule GPAD2opt-FXa (4,57 µg/ml) au lieu de 1,25 µg/ml de sa version non-optimisée (figure 10 B).

### Exemple 11 : Activité chromogénique de GPAD2opt-FXa

GPAD2opt-FXa produit dans HEK293 a été purifié suivant le protocole décrit dans l'article de Husi et al. J. Chromato 2001. Son activité chromogénique sur substrat Pefachrome 8595 a été mesurée comme décrit et comparée à celle d'un FXa plasmatique (figure 11). L'activité du GPAD2opt-FXa purifié est retrouvée identique à celle du FXa plasmatique. Ce résultat indique que l'activité enzymatique du GPAD2opt-FXa sur un petit substrat est identique à celle d'un FX plasmatique activé.

### Exemple 12 : Effet de la co-expression de la furine sur l'activité TGT du GPAD2opt-FXa

Le GPAD2opt-FXa a été exprimé dans HEK en sur-exprimant la furine ou non par co-transfection. Les activités des deux GPAD2opt-FXa ont été évaluées en génération de thrombine dans un plasma déficient en FVIII après induction de la coagulation par la céphaline (figure 12). La molécule GPAD2opt-FXa permet de corriger efficacement le déficit en FVIII après une induction par la céphaline (◆). La co-expression avec la furine permet d'obtenir une molécule de GPAD2opt-FXa active (◇) plus précocement et plus intensément qu'une molécule produite sans co-exprimer la furine (◆). Ces résultats montrent que la présence de furine lors de la production améliore l'activité spécifique de la molécule produite.

### Exemple 13 : Le GPAD2opt-FXa corrige la déficience en FVIII en génération de thrombine

Un essai de TGT a été réalisé en additionnant dans du plasma déficient en FVIII du GPAD2opt-FXa (figure 13A et B). Le plasma contrôle (courbe ●) possède une activité TGT attendue. La déficience en FVIII (courbe ○) abolit complètement la génération de thrombine induite par le mélange TF/PL ou par la céphaline (panel A et B, respectivement). La complémentation du plasma déficient par une dose thérapeutique de 1 U/ml de FVIII (courbe ■) permet d'obtenir un signal très proche du signal obtenu par un plasma normal. La supplémentation par 0,1 U/ml de facteur VIII recombinant (courbe □) permet d'obtenir un signal significativement supérieur au plasma de contrôle mais inférieur à la normale. La supplémentation avec GPAD2opt-FXa (2 µg/ml, courbe ◆) du plasma déficient en FVIII permet de restaurer significativement la génération de thrombine avec une célérité supérieure aux contrôles positifs en produisant toutefois une quantité de thrombine légèrement inférieure. Ce résultat indique que le GPAD2opt-FXa permet de restaurer une génération de thrombine significative en absence de FVIII.

### Exemple 14 : Le GPAD2opt-FXa corrige la déficience en FIX en génération de thrombine

Un essai de TGT a été réalisé en additionnant dans du plasma déficient en FIX du GPAD2opt-FXa (figure 14A et B). Le plasma contrôle (courbe ●) possède une activité TGT attendue. La déficience en FIX (courbe ○) abolit complètement la génération de thrombine induite par le mélange TF/PL ou par la céphaline (panel A et B, respectivement). La complémentation du plasma déficient par des doses thérapeutiques soit de 1 U/ml de FIX (courbe ■) soit de 0,1 U/ml de facteur IX plasmatique (courbe □) permet d'obtenir un signal supérieur au contrôle dans les deux cas. La supplémentation en GPAD2opt-FXa (2 µg/ml, courbe ◆) permet de restaurer significativement une partie de la génération de thrombine. Cette restauration correspond quasiment à une substitution en FIX suite à l'induction par le FT alors qu'elle demeure moins importante après induction par la céphaline. Ce résultat indique que le GPAD2opt-FXa permet de restaurer une génération de thrombine significative en absence de FIX.

### Exemple 15 : Le GPAD2opt-FXa ne corrige pas la déficience en FX en génération de thrombine

Un essai de TGT a été réalisé en additionnant dans du plasma déficient en FX du GPAD2opt-FXa (figure 15A et B). Le plasma contrôle (courbe ●) possède une activité TGT attendue. La déficience en FX (courbe ○) abolit complètement la génération de thrombine induite par le mélange TF/PL ou par la céphaline (panel A et B, respectivement). La complémentation du plasma déficient par des doses thérapeutiques soit de 10 µg/ml de FX plasmatique (courbe ■) soit de 1 µg/ml de facteur X plasmatique (courbe □) permet d'obtenir un signal supérieur au contrôle suite à l'induction par la céphaline. Suite à une induction par du facteur tissulaire, le FX plasmatique à 1 µg/ml est cependant moins efficace qu'un plasma normal à générer de la thrombine. Quel que soit l'inducteur la supplémentation en GPAD2opt-FXa (2 µg/ml, courbe ◇) ne permet pas de restaurer de génération de thrombine. La courbe résultante ne se différencie pas du niveau basal. Ce résultat indique que le GPAD2opt-FXa de par son absence de domaine gamma-carboxylé ne permet pas de restaurer de génération de thrombine en absence de FX. Cette absence de supplémentation en facteur X indique que le GPAD2opt-FXa ne conduira pas à un surdosage en facteur X qui pourrait s'avérer thrombogène. D'autre part ce résultat permet de confirmer que les résultats obtenus en TGT ne correspondent pas à une génération de thrombine qui serait non spécifique.

### Exemple 16: Construction de molécule de GPAD2opt-FXa fusionnée au scFc

La molécule de facteur X sous sa forme activée possède une demi-vie très courte dans la circulation (Invanciu et al. Nat. Biotech. 2011). Afin d'augmenter la demi-vie circulante du GPAD2opt-FXa, des molécules chimériques fusionnées au domaine Fc des anticorps sous la forme d'une simple chaine (scFc) ont été générées. Différentes combinaisons ont été testées avec la molécule GPAD2opt-FXa greffée soit en position N-Terminale soit en position C-Terminale (figure 16). De plus des linkers (1) entre le domaine scFc et la partie GPAD2opt-FXa ont été rajoutés (figure 16). Les molécules ont été produites dans la lignée HEK de manière similaire au GPAD2opt-FXa puis purifiées sur gel de protéine A dans un ratio de 100µl de gel pour 1 mg de protéine. Elles ont été éluées en tampon Citrate 25 mM pH 3,0 en 4 fractions de 200µl qui sont poolées et immédiatement neutralisées avec 32 µl de Tris 2M pH 9,0. Les molécules ont ensuite été dialysées contre du tampon Hepes 25 mM, NaCl 175 mM, pH 7,4.

Les molécules purifiées ont été dosées soit par reconnaissance de la partie FX comme décrit dans l'exemple 1 soit par dosage de la fraction Fc en utilisant le kit FastELISA, réf RDB-3257 (dosage des immunoglobulines G humaines, RD-Biotech, France) et en appliquant le protocole recommandé par le fournisseur. Les deux dosages permettent de trouver des valeurs similaires indiquant que la molécule chimérique est reconnue efficacement par ces deux méthodes. Par ailleurs le repliement de chacun des deux composants de la molécule doit s'effectuer de manière native pour que les deux révélations soient fonctionnelles. La qualité de la molécule scFcl-GPAD2opt-FXa est présentée figure 17 suite à une étape de purification sur gel de protéine A.

### Exemple 17: Mesure de l'activité de la molécule scFcl- GPAD2opt-FXa en plasma déficient en FVIII

La molécule scFcl-GPAD2opt-FXa sert de modèle pour l'étude de faisabilité d'une molécule long-lasting. Les propriétés de cette molécule purifiée sont comparées à celles du GPAD2opt-FXa.
La molécule scFcl- GPAD2opt-FXa a été comparée avec la molécule GPAD2opt-FXa dans un test de TGT en plasma déficient en FVIII par induction à la céphaline (Figure 18). La molécule chimérique a été utilisée de manière équimolaire (4 µg/ml) au GPAD2opt-FXa (2 µg/ml). Les deux molécules permettent d'obtenir une restauration de la génération de thrombine similaire indiquant que la partie scFc et le linker ne semblent pas gênants pour la fonction anti-hémophilique de la protéine.

### Exemple 18 : Mesure de l'activité de la molécule scFcl- GPAD2opt-FXa en activité chromogénique en présence ou non de Rivaroxaban

L'activité chromogénique sur le substrat Pefachrome 8595 de la molécule scFcl-GPAD2opt-FXa purifiée a été mesurée comme décrit dans l'exemple 1 (figure 18). L'activité résultante a été comparée à celle du facteur Xa plasmatique. Les deux molécules ont une activité chromogénique similaire indiquant que le site catalytique de la molécule scFcl- GPAD2opt-FXa a conservé ses propriétés enzymatiques. Un essai similaire a été réalisé en ajoutant 0,35 µg/ml de Rivaroxaban au milieu réactionnel. Le Rivaroxaban inhibe les deux molécules avec une efficacité maximale puisque leurs activités chromogéniques après traitement sont de l'ordre du bruit de fond. Ce résultat indique que la molécule scFcl- GPAD2opt-FXa a conservé sa capacité à être inhibée par le Rivaroxaban.

### Exemple 19 : Mesure de l'activité antidote de la molécule scFcl- GPAD2opt-FXa dans un test TGT

Un test de génération de thrombine sur un plasma normal contenant ou non du Rivaroxaban (0,25 µg/ml) a été pratiqué suite à une initiation soit par le mélange facteur tissulaire/phospholipides (figure 20A) ou de la céphaline (figure 20 B). A cette dose, le Rivaroxaban inhibe totalement la génération de thrombine issue de ce plasma (courbe ○). L'ajout de concentration croissante de scFcl- GPAD2opt-FXa (25 ou 50 µg/ml) permet, en fonction de la dose, de restaurer la formation d'une quantité significative de thrombine et d'améliorer la cinétique de formation. La dose de 50 µg/ml permet de normaliser complètement la génération de thrombine induite par le mélange FT/PL et de corriger très significativement l'action du Rivaroxaban suite à une induction par la céphaline. Ces résultats indiquent que la molécule scFcl- GPAD2opt-FXa est un antidote efficace des nouveaux anticoagulants oraux.

### Exemple 20: Effet de la longueur du peptide d'activation sur la structure du FXa

L'analyse en modélisation moléculaire de la structure du FXa fait apparaître que la longueur du peptide d'activation semble avoir un impact sur le repliement de la protéine. Ainsi lorsque nous supprimons le peptide d'activation (molécule GPAD1), nous observons une contrainte structurale forte sur le repliement de la molécule. Cette contrainte est représentée par la valeur élevée du RMSD (Root Mean Square Deviation) lors de la superposition du modèle structural GPAD1.M0013 sur la structure cristallographique du FXa, 2GD4 (chaîne principale) (Table V).

**Table V: Valeur des RMSD du FX-WT et des molécules tronquées**

| **Protein** | **RMSD (angstrom)** | **NRES (Number of Overlapping Residudes)** |
|---|---|---|
| **2GD4** | 0 | 288 |
| **GDFXa.M0015** | 0.297 | 288 |
| **GPAD1.M0013** | 1.095 | 288 |
| **GPAD2.M0025** | 0.526 | 288 |
| **GPAD3_GSSG.M0028** | 0.348 | 288 |
| **GPAD3_GGS.M0029** | 0.428 | 288 |
| **GPAD3_G4S2.M0020** | 0.349 | 288 |
| **GPAD3_G4S2.M0006** | 0.331 | 288 |

*Différentes molécules de FX tronquées ont été modélisées en utilisant la structure du FXa-WT 2GD4 comme matrice et les différences de RMSD ont été calculées par rapport à la valeur du FXa-WT (2GD4) cristallisé suite à la superposition du modèle sur la structure cristallographique.*

Cette valeur de 1.095 est très supérieure à celle du modèle 3D du FXa contenant toute la séquence du peptide d'activation, GDFXa.M0015 (0.297). Les mêmes analyses ont été réalisées sur des modèles 3D contenant, à la place de la séquence du peptide d'activation, le linker RKR(GPAD2.M0025), des linkers courts GSSGR et GGSR (GPAD3_GSSG.M0028 et GPAD3_GGS.M0029) ou un linker long GGGGSGGGGSR (SEQ ID NO:91) (GPAD3_G4S2.M0020 et GPAD3_G4S2.M0006). Nous avons observés que les linkers RKR et GGSR conservent une contrainte structurale significative sur le repliement bien que moins élevée que celle du GPAD1. En revanche les valeurs de RMSD des linkers GSSGR et GGGGSGGGGSR (SEQ ID NO :91) sont faibles et proche de celle du modèle contenant la séquence complète du peptide d'activation considéré comme non contraint, indiquant que l'ajout de ces 2 linkers permet de ne pas contraindre le repliement de la molécule GPAD. La présence de linker pourrait permettre une meilleure biosynthèse de la protéine, une meilleure productivité et un maintien de l'activité enzymatique.

## Revendications

1. Protéine consistant en la séquence SEQ ID NO : 11 ou SEQ ID NO :25 ou SEQ ID NO :26 directement fusionnée, ou fusionnée via un linker, à la séquence SEQ ID NO:6.

2. Protéine selon la revendication 1, **caractérisée en ce que** le linker consiste en la séquence -Arg-Lys-Arg-.

3. Protéine selon la revendication 1, **caractérisée en ce que** le linker est choisi parmi les sites de clivages du FVIIa, du FIXa, du FXa, du FXIa, du FXIIa, de la kallikréine et de la protéine C activée.

4. Protéine selon l'une des revendications 1 ou 2, **caractérisée en ce qu'**elle a pour séquence SEQ ID NO:7 ou SEQ ID NO:9, SEQ ID NO:21, SEQ ID NO:22, SEQ ID NO :23, SEQ ID NO :24, SEQ ID NO :60, SEQ ID NO :61, SEQ ID NO :62 ou SEQ ID NO :63.

5. Protéine selon l'une des revendications précédentes, **caractérisée en ce qu'**elle comprend au moins une mutation choisie parmi :
- une substitution de l'arginine 138 de SEQ ID NO :6 par la phénylalanine, la glycine, l'isoleucine ou la tyrosine ou de la lysine 82 de SEQ ID NO :6 par la tyrosine, et
- une insertion dans la séquence SEQ ID NO :11, 25 ou 26 étant une insertion d'un linker choisi parmi -GSSG-, -RGSSG-, -GSSGR-, -RKRGSSGR-, -R(GGGGS)n-, - RKR(GGGGS)nR-, -(GGGGS)n-, -(GGGGS)nR-, dans lequel n est un entier de 1 à 5, de préférence de 1 à 3, X, R-X, X-R, ou RKR-X-R dans lequel X est un peptide de 4 à 52 acides aminés.

6. Protéine selon la revendication 5, **caractérisée en ce que** l'insertion dans la séquence SEQ ID NO :25 consiste en :
- une insertion, entre les acides aminés 98 et 99, du linker -GSSG-, -RGSSG-, - R(GGGGS)n- ou -(GGGGS)n-, dans lequel n est un entier de 1 à 5, de préférence de 1 à 3, X, ou R-X, dans lequel X est un peptide de 4 à 52 acides aminés ; ou
- une insertion, entre les acides aminés 99 et 100, du linker -GSSGR-, -(GGGGS)nR-, - RKRGSSGR-, -RKR(GGGGS)nR- dans lequel n est un entier de 1 à 5, de préférence de 1 à 3, X-R ou RKR-X-R, dans lequel X est un peptide de 4 à 52 acides aminés.

7. Protéine selon la revendication 5, **caractérisée en ce que** l'insertion dans la séquence SEQ ID NO :26 consiste en :
- une insertion, entre les acides aminés 97 et 98, du linker -GSSG-, -RGSSG-, - R(GGGGS)n- ou -(GGGGS)n-, dans lequel n est un entier de 1 à 5, de préférence de 1 à 3, X ou R-X, dans lequel X est un peptide de 4 à 52 acides aminés ; ou
- une insertion, entre les acides aminés 98 et 99, du linker -GSSGR-, -(GGGGS)nR-, - RKRGSSGR-, -RKR(GGGGS)nR-, dans lequel n est un entier de 1 à 5, de préférence de 1 à 3, X-R ou RKR-X-R, dans lequel X est un peptide de 4 à 52 acides aminés.

8. Protéine selon l'une des revendications 5, 6 ou 7, **caractérisée en ce qu'**elle a pour séquence SEQ ID NO :28, SEQ ID NO :29, SEQ ID NO :30, SEQ ID NO :65, SEQ ID NO :66 ou SEQ ID NO :67.

9. Protéine selon l'une des revendications 1 à 8, **caractérisée en ce qu'**elle est monocaténaire.

10. Protéine selon l'une des revendications précédentes, **caractérisée en ce qu'**elle est fusionnée, en N-terminal ou en C-terminal, à un fragment Fc de type sauvage ou à un fragment scFc de type sauvage.

11. Protéine selon la revendication 10, **caractérisée en ce que** le fragment Fc a pour séquence SEQ ID NO :39 ou SEQ ID NO :54, éventuellement suivie d'une lysine en C-terminal.

12. Protéine selon l'une des revendications 10 ou 11, **caractérisée en ce qu'**elle est choisie parmi SEQ ID NO :32, SEQ ID NO :33, SEQ ID NO :34, SEQ ID NO :35, SEQ ID NO :36, SEQ ID NO :68, SEQ ID NO :69, SEQ ID NO :70, SEQ ID NO :71, SEQ ID NO :72, SEQ ID NO :37, SEQ ID NO :38, SEQ ID NO :73 et SEQ ID NO :74.

13. Acide nucléique **caractérisé en ce qu'**il code pour la protéine selon l'une des revendications précédentes.

14. Acide nucléique selon la revendication 13, **caractérisé en ce qu'**il est choisi parmi SEQ ID NO :8, SEQ ID NO :10, SEQ ID NO :45 à 51, SEQ ID NO :57 à 59 et SEQ ID NO :77 à 90.

15. Cassette d'expression comprenant l'acide nucléique selon la revendication 13 ou 14.

16. Vecteur d'expression, **caractérisé en ce qu'**il comprend la cassette d'expression selon la revendication 15 ou l'acide nucléique selon la revendication 13 ou 14.

17. Cellule recombinante comprenant l'acide nucléique selon la revendication 13 ou 14, ou le vecteur selon la revendication 16.

18. Protéine selon l'une des revendications 1 à 12 pour son utilisation en tant que médicament, de préférence pour traiter les troubles hémorragiques.

19. Protéine selon l'une des revendications 1 à 12 pour son utilisation dans la prévention ou le traitement chez un patient, notamment un être humain ou un animal, des évènements hémorragiques induits par la prise d'anticoagulants, qui sont des inhibiteurs spécifiques du facteur Xa.

20. Procédé de production d'une protéine selon l'une des revendications 1 à 12 comprenant les étapes suivantes :
a) transfection de cellules eucaryotes avec des vecteurs d'expression comprenant au moins un acide nucléique selon la revendication 13 ou 14, de préférence également avec un vecteur exprimant la furine ;
b) culture des cellules obtenues en a), pour exprimer la protéine ; et
c) optionnellement, purification de la protéine obtenue.

21. Procédé de production d'une protéine selon l'une des revendications 1 à 12 comprenant les étapes suivantes :
(a) insérer dans un embryon de mammifère non humain une séquence d'ADN comprenant un polynucléotide choisi parmi SEQ ID NO:8, SEQ ID NO:10, SEQ ID NO :45 à 51, SEQ ID NO :57 à 59 et SEQ ID NO :77 à 90, ledit polynucléotide étant sous le contrôle transcriptionnel d'un promoteur de la caséine de mammifère ou un promoteur de lactosérum de mammifère, ladite séquence d'ADN comprenant en outre une séquence signal permettant la sécrétion de ladite protéine,
(b) laisser ledit embryon se développer en un mammifère adulte,
(c) induire la lactation chez ledit mammifère ou dans un descendant femelle dudit mammifère dans lequel ledit polynucléotide, le promoteur et la séquence signal sont présents dans le génome du tissu mammaire,
(d) recueillir le lait dudit mammifère en lactation, et
(e) isoler ladite protéine à partir dudit lait recueilli.

## Patentansprüche

1. Protein, das in der Sequenz SEQ ID NR.: 11 oder SEQ ID NR. : 25 oder SEQ ID NR. : 26 besteht, welche mit der SEQ ID NR.: 6 direkt fusioniert oder über einen Linker fusioniert ist.

2. Protein nach Anspruch 1, **dadurch gekennzeichnet, dass** der Linker in der Sequenz -Arg-Lys-Arg- besteht.

3. Protein nach Anspruch 1, **dadurch gekennzeichnet, dass** der Linker aus den Spaltungsstellen des FVIIa, des FIXa, des FXa, des FXIa, des FXIIa, des Kallikreins und des aktivierten Proteins C ausgewählt ist.

4. Protein nach einem der Ansprüche 1 oder 2, **dadurch gekennzeichnet, dass** sie als Sequenz SEQ ID NR. : 7 oder SEQ ID NR.: 9, SEQ ID NR.: 21, SEQ ID NR.: 22, SEQ ID NR.: 23, SEQ ID NR.: 24, SEQ ID NR.: 60, SEQ ID NR.: 61, SEQ ID NR.: 62 oder SEQ ID NR.: 63 aufweist.

5. Protein nach einem der vorstehenden Ansprüche, **dadurch gekennzeichnet, dass** sie mindestens eine Mutation umfasst, ausgewählt aus:
- einer Substitution des Arginins 138 aus SEQ ID NR.: 6 durch Phenylalanin, Glycin, Isoleucin oder Tyrosin, oder des Lysins 82 aus SEQ ID NR.: 6 durch Tyrosin, und
- einer Einfügung in der Sequenz SEQ ID NR.: 11, 25 oder 26, welche eine Einfügung eines Linkers ist, ausgewählt aus -GSSG-, -RGSSG-, -GSSGR-, -RKRGSSGR-, -R(GGGGS)n-, - RKR(GGGGS)nR-, -(GGGGS)n-, -(GGGGS)nR-, wobei n eine ganze Zahl von 1 bis 5, vorzugsweise von 1 bis 3 ist, X, R-X, X-R, oder RKR-X-R, wobei X ein Peptid mit 4 bis 52 Aminosäuren ist.

6. Protein nach Anspruch 5, **dadurch gekennzeichnet, dass** die Einfügung in der Sequenz SEQ ID NR.: 25 besteht in:
- einer Einfügung, zwischen den Aminosäuren 98 und 99, des Linkers -GSSG-, -RGSSG-, -R(GGGGS)n- oder -(GGGGS)n-, wobei n eine ganze Zahl von 1 bis 5, vorzugsweise von 1 bis 3 ist, X oder R-X, wobei X ein Peptid mit 4 bis 52 Aminosäuren ist; oder
- einer Einfügung, zwischen den Aminosäuren 99 und 100, des Linkers -GSSGR-, -(GGGGS)nR-, -RKRGSSGR-, - RKR(GGGGS)nR-, wobei n eine ganze Zahl von 1 bis 5, vorzugsweise von 1 bis 3 ist, X-R oder RKR-X-R, wobei X ein Peptid mit 4 bis 52 Aminosäuren ist.

7. Protein nach Anspruch 5, **dadurch gekennzeichnet, dass** die Einfügung in der Sequenz SEQ ID NR.: 26 besteht in:
- einer Einfügung, zwischen den Aminosäuren 97 und 98, des Linkers -GSSG-, -RGSSG-, -R(GGGGS)n- oder -(GGGGS)n-, wobei n eine ganze Zahl von 1 bis 5, vorzugsweise von 1 bis 3 ist, X oder R-X, wobei X ein Peptid mit 4 bis 52 Aminosäuren ist; oder
- einer Einfügung, zwischen den Aminosäuren 98 und 99, des Linkers -GSSGR-, -(GGGGS)nR-, -RKRGSSGR-,-RKR(GGGGS)nR-, wobei n eine ganze Zahl von 1 bis 5, vorzugsweise von 1 bis 3 ist, X-R oder RKR-X-R, wobei X ein Peptid mit 4 bis 52 Aminosäuren ist.

8. Protein nach einem der Ansprüche 5, 6 oder 7, **dadurch gekennzeichnet, dass** es als Sequenz SEQ ID NR. : 28, SEQ ID NR.: 29, SEQ ID NR.: 30, SEQ ID NR.: 65, SEQ ID NR.: 66 oder SEQ ID NR.: 67 aufweist.

9. Protein nach einem der Ansprüche 1 bis 8, **dadurch gekennzeichnet, dass** es einzelsträngig ist.

10. Protein nach einem der vorstehenden Ansprüche, **dadurch gekennzeichnet, dass** es am N-Terminus oder am C-Terminus an ein Fc-Fragment vom Wildtyp oder an ein scFc-Fragment vom Wildtyp fusioniert ist.

11. Protein nach Anspruch 10, **dadurch gekennzeichnet, dass** das Fc-Fragment als Sequenz SEQ ID NR.: 39 oder SEQ ID NR.: 54 aufweist, gegebenenfalls gefolgt von einem Lysin am C-Terminus.

12. Protein nach einem der Ansprüche 10 oder 11, **dadurch gekennzeichnet, dass** es ausgewählt ist aus SEQ ID NR.: 32, SEQ ID NR.: 33, SEQ ID NR.: 34, SEQ ID NR.: 35, SEQ ID NR.: 36, SEQ ID NR.: 68, SEQ ID NR.: 69, SEQ ID NR.: 70, SEQ ID NR.: 71, SEQ ID NR. : 72, SEQ ID NR.: 37, SEQ ID NR.: 38, SEQ ID NR.: 73 und SEQ ID NR.: 74.

13. Nukleinsäure, **dadurch gekennzeichnet, dass** sie für das Protein nach einem der vorstehenden Ansprüche codiert.

14. Nukleinsäure nach Anspruch 13, **dadurch gekennzeichnet, dass** sie ausgewählt ist aus SEQ ID NR.: 8, SEQ ID NR.: 10, SEQ ID NR. : 45 bis 51, SEQ ID NR. : 57 bis 59 und SEQ ID NR.: 77 bis 90.

15. Expressionskassette, die die Nukleinsäure nach Anspruch 13 oder 14 umfasst.

16. Expressionsvektor, **dadurch gekennzeichnet, dass** er die Expressionskassette nach Anspruch 15 oder die Nukleinsäure nach Anspruch 13 oder 14 umfasst.

17. Rekombinante Zelle, die die Nukleinsäure nach Anspruch 13 oder 14 oder den Vektor nach Anspruch 16 umfasst.

18. Protein nach einem der Ansprüche 1 bis 12 zu seiner Verwendung als Arzneimittel, vorzugsweise um die Blutungsstörungen zu behandeln.

19. Protein nach einem der Ansprüche 1 bis 12 zu seiner Verwendung in der Vorbeugung oder der Behandlung bei einem Patienten, allem voran einem Menschen oder einem Tier, der Blutungsereignisse, die durch die Einnahme von Antikoagulanzien induziert werden, welche spezifische Inhibitoren des Faktors Xa sind.

20. Verfahren zur Herstellung eines Proteins nach einem der Ansprüche 1 bis 12, umfassend die folgenden Schritte:
a) Transfektion von eukaryotischen Zellen mit Expressionsvektoren, die mindestens eine Nukleinsäure nach Anspruch 13 oder 14 umfassen, vorzugsweise ebenfalls mit einem Vektor, der Furin exprimiert;
b) Kultur der in a) erhaltenen Zellen, um das Protein zu exprimieren; und
c) gegebenenfalls Aufreinigen des erhaltenen Proteins.

21. Verfahren zur Herstellung eines Proteins nach einem der Ansprüche 1 bis 12, umfassend die folgenden Schritte:
(a) Einfügen, in einen nicht menschlichen Säugetier-Embryo, einer DNA-Sequenz, die ein Polynukleotid umfasst, ausgewählt aus SEQ ID NR.: 8, SEQ ID NR.: 10, SEQ ID NR.: 45 bis 51, SEQ ID NR.: 57 bis 59 und SEQ ID NR.: 77 bis 90, wobei das Polynukleotid unter der Transkriptionskontrolle eines Protomors von Säugetier-Kasein oder eines Protomors von Säugetier-Laktoserum steht, wobei die DNA-Sequenz weiter eine Signalsequenz umfasst, welche die Sekretion des Proteins ermöglicht,
(b) Entwickelnlassen des Embryos in einem ausgewachsenen Säugetier,
(c) Induzieren der Laktation bei dem Säugetier oder in einem weiblichen Abkömmling des Säugetiers, bei dem das Polynukleotid, der Promotor und die Signalsequenz im Genom des Brustgewebes vorhanden sind,
(d) Gewinnen der Milch des laktierenden Säugetiers, und
(e) Isolieren des Proteins aus der gewonnenen Milch.

## Claims

1. A protein consisting of the sequence SEQ ID No.: 11 or SEQ ID No.: 25 or SEQ ID No.: 26, directly fused, or fused via a linker, to the sequence SEQ ID No.: 6.

2. The protein as claimed in claim 1, **characterized in that** the linker consists of the sequence -Arg-Lys-Arg-.

3. The protein as claimed in claim 1, **characterized in that** the linker is chosen from the cleavage sites of FVIIa, FIXa, FXa, FXIa, FXIIa, kallikrein and activated protein C.

4. The protein as claimed in either of claims 1 and 2, **characterized in that** it has the sequence SEQ ID No.: 7 or SEQ ID No.: 9, SEQ ID No.: 21, SEQ ID No.: 22, SEQ ID No.: 23, SEQ ID No.: 24, SEQ ID No.: 60, SEQ ID No.: 61, SEQ ID No.: 62 or SEQ ID No.: 63.

5. The protein as claimed in one of the preceding claims, **characterized in that** it comprises at least one mutation chosen from:
- a substitution of arginine 138 of SEQ ID No.: 6 by phenylalanine, glycine, isoleucine or tyrosine or of lysine 82 of SEQ ID No.: 6 by tyrosine, and
- an insertion into the sequence SEQ ID No.: 11, 25 or 26 being an insertion of a linker chosen from -GSSG-, -RGSSG-, -GSSGR-, -RKRGSSGR-, -R(GGGGS)n-, -RKR(GGGGS)nR-, -(GGGGS)n-, -(GGGGS)nR-, in which n is an integer from 1 to 5, preferably from 1 to 3, X, R-X, X-R or RKR-X-R in which X is a peptide of 4 to 52 amino acids.

6. The protein as claimed in claim 5, **characterized in that** the insertion into the sequence SEQ ID No.: 25 consists of:
- an insertion, between amino acids 98 and 99, of the linker -GSSG-, -RGSSG-, -R(GGGGS)n- or -(GGGGS)n-, in which n is an integer from 1 to 5, preferably from 1 to 3, X or R-X, in which X is a peptide of 4 to 52 amino acids; or
- an insertion, between amino acids 99 and 100, of the linker -GSSGR-, -(GGGGS)nR-, -RKRGSSGR-, -RKR(GGGGS)nR- in which n is an integer from 1 to 5, preferably from 1 to 3, X-R or RKR-X-R, in which X is a peptide of 4 to 52 amino acids.

7. The protein as claimed in claim 5, **characterized in that** the insertion into the sequence SEQ ID No.: 26 consists of:
- an insertion, between amino acids 97 and 98, of the linker -GSSG-, -RGSSG-, -R(GGGGS)n- or -(GGGGS)n-, in which n is an integer from 1 to 5, preferably from 1 to 3, X or R-X, in which X is a peptide of 4 to 52 amino acids; or
- an insertion, between amino acids 98 and 99, of the linker -GSSGR-, -(GGGGS)nR-, -RKRGSSGR-, -RKR(GGGGS)nR- in which n is an integer from 1 to 5, preferably from 1 to 3, X-R or RKR-X-R, in which X is a peptide of 4 to 52 amino acids.

8. The protein as claimed in one of claims 5, 6 or 7, **characterized in that** it has the sequence SEQ ID No.: 28, SEQ ID No.: 29, SEQ ID No.: 30, SEQ ID No.: 65, SEQ ID No.: 66 or SEQ ID No.: 67.

9. The protein as claimed in one of claims 1 to 8, **characterized in that** it is single-stranded.

10. The protein as claimed in one of the preceding claims, **characterized in that** it is fused, in N-terminal or in C-terminal, to a wild-type Fc fragment or to a wild-type scFc fragment.

11. The protein as claimed in claim 10, **characterized in that** the Fc fragment has the sequence SEQ ID No.: 39 or SEQ ID No.: 54, optionally followed by a lysine in the C-terminal position.

12. The protein as claimed in either of claims 10 and 11, **characterized in that** it is chosen from SEQ ID No.: 32, SEQ ID No.: 33, SEQ ID No.: 34, SEQ ID No.: 35, SEQ ID No.: 36, SEQ ID No.: 68, SEQ ID No.: 69, SEQ ID No.: 70, SEQ ID No.: 71 SEQ ID No.: 72, SEQ ID No.: 37, SEQ ID No.: 38, SEQ ID No.: 73 and SEQ ID No.: 74.

13. A nucleic acid **characterized in that** it encodes the protein as claimed in one of the preceding claims.

14. The nucleic acid as claimed in claim 13, **characterized in that** it is chosen from SEQ ID No.: 8, SEQ ID No.: 10, SEQ ID Nos.: 45 to 51, SEQ ID Nos.: 57 to 59 and SEQ ID Nos.: 77 to 90.

15. An expression cassette comprising the nucleic acid as claimed in claim 13 or 14.

16. An expression vector, **characterized in that** it comprises the expression cassette as claimed in claim 15 or the nucleic acid as claimed in claim 13 or 14.

17. A recombinant cell comprising the nucleic acid as claimed in claim 13 or 14, or the vector as claimed in claim 16.

18. The protein as claimed in one of claims 1 to 12, for use thereof as a medicament, preferably for treating hemorrhagic disorders.

19. The protein as claimed in one of claims 1 to 12, for use thereof in the prevention or treatment in a patient, in particular a human being or an animal, of hemorrhagic events induced by taking anticoagulants, which are factor Xa-specific inhibitors.

20. A process for producing a protein as claimed in one of claims 1 to 12, comprising the following steps:
a) transfecting eukaryotic cells with expression vectors comprising at least one nucleic acid as claimed in claim 13 or 14, preferably also with a vector expressing furin ;
b) culturing the cells obtained in a), so as to express the protein; and
c) optionally, purifying the protein obtained.

21. A process for producing a protein as claimed in one of claims 1 to 12, comprising the following steps:
(a) inserting into a non-human mammalian embryo a DNA sequence comprising a polynucleotide chosen from SEQ ID No.: 8, SEQ ID No.: 10, SEQ ID Nos.: 45 to 51, SEQ ID Nos.: 57 to 59 and SEQ ID Nos.: 77 to 90, said polynucleotide being under the transcriptional control of a mammalian casein promoter or a mammalian whey promoter, said DNA sequence also comprising a signal sequence allowing the secretion of said protein,
(b) leaving said embryo to develop in an adult mammal,
(c) inducing lactation in said mammal or in a female descendent of said mammal in which said polynucleotide, the promoter and the signal sequence are present in the genome of the mammalian tissue,
(d) collecting the milk of said lactating mammal, and
(e) isolating said protein from said collected milk.
